(19) Europäisches Patentamt
European Patent Office
Office européen des brevets

(11) EP 1 513 552 B1

(12) **EUROPEAN PATENT SPECIFICATION**

(45) Date of publication and mention of the grant of the patent:
**01.12.2010 Bulletin 2010/48**

(21) Application number: **03760672.0**

(22) Date of filing: **20.06.2003**

(51) Int Cl.:
***A61K 39/39*** *(2006.01)* ***A61P 37/04*** *(2006.01)*

(86) International application number:
**PCT/EP2003/006541**

(87) International publication number:
**WO 2004/000351 (31.12.2003 Gazette 2004/01)**

(54) **PACKAGED VIRUS-LIKE PARTICLES IN COMBINATION WITH CPG FOR USE AS ADJUVANTS WITH ALLERGENS : METHOD OF PREPARATION AND USE**

VERPACKTE VIRUSARTIGE PARTIKEL IN KOMBINATION MIT CPG ZUR VERWENDUNG ALS ADJUVANTIEN MIT ALLERGENEN. HERSTELLUNGSVERFAHREN UND VERWENDUNG

PARTICULES PSEUDO-VIRALES ENVELOPPEES COMBINEES AU CPG DESTINEES A ETRE UTILISEES EN TANT QU'ADJUVANTS AVEC DES ALLERGENES: PROCEDE DE PREPARATION ET UTILISATION DE CELLES-CI

(84) Designated Contracting States:
**AT BE BG CH CY CZ DE DK EE ES FI FR GB GR HU IE IT LI LU MC NL PT RO SE SI SK TR**

(30) Priority: **20.06.2002 US 389898 P**

(43) Date of publication of application:
**16.03.2005 Bulletin 2005/11**

(73) Proprietor: **Cytos Biotechnology AG**
**8952 Schlieren (CH)**

(72) Inventors:
- **BACHMANN, Martin, F.**
  **CH-8472 Seuzach (CH)**
- **RENNER, Wolfgang, A.**
  **CH-8802 Kilchberg (CH)**

(74) Representative: **Wichmann, Hendrik et al**
**Wuesthoff & Wuesthoff**
**Schweigerstrasse 2**
**81541 München (DE)**

(56) References cited:
**WO-A-00/32227** **WO-A-01/22972**
**WO-A-98/50071** **WO-A-03/024481**

- **GERBER S ET AL: "Human papillomavirus virus-like particles are efficient oral immunogens when coadministered with Escherichia coli heat-labile enterotoxin mutant R192G or CpG DNA" JOURNAL OF VIROLOGY, THE AMERICAN SOCIETY FOR MICROBIOLOGY, US, vol. 75, no. 10, May 2001 (2001-05), pages 4752-4760, XP002246338 ISSN: 0022-538X**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).



Printed by Jouve, 75001 PARIS (FR)

## Description

BACKGROUND OF THE INVENTION

Field of the Invention

The present invention is related to the fields of vaccinology, immunology and medicine. The invention provides compositions and methods for enhancing immunological responses against allergens mixed with virus-like particles (VLPs) packaged with oligonucleotides containing at least one non-methylated CpG sequence. The invention can be used to induce strong antibody and T cell responses particularly useful for the treatment of allergies.

Related Art

The essence of the immune system is built on two separate foundation pillars: one is specific or adaptive immunity which is characterized by relatively slow response-kinetics and the ability to remember; the other is non-specific or innate immunity exhibiting rapid response-kinetics but lacking memory. Lymphocytes are the key players of the adaptive immune system. Each lymphocyte expresses antigen-receptors of unique specificity. Upon recognizing an antigen via the receptor, lymphocytes proliferate and develop effector function. Few lymphocytes exhibit specificity for a given antigen or pathogen, and massive proliferation is usually required before an effector response can be measured - hence, the slow kinetics of the adaptive immune system. Since a significant proportion of the expanded lymphocytes survive and may maintain some effector function following elimination of the antigen, the adaptive immune system reacts faster when encountering the antigen a second time. This is the basis of its ability to remember.

In contrast to the situation with lymphocytes, where specificity for a pathogen is confined to few cells that must expand to gain function, the cells and molecules of the innate immune system are usually present in massive numbers and recognize a limited number of invariant features associated with pathogens (Medzhitov, R. and Janeway, C.A., Jr., Cell 91:295-298 (1997)). Examples of such patterns include lipopolysaccharides (LPS), non-methylated CG-rich DNA (CpG) or double stranded RNA, which are specific for bacterial and viral infections, respectively.

Most research in immunology has focused on the adaptive immune system and only recently has the innate immune system entered the focus of interest. Historically, the adaptive and innate immune system were treated and analyzed as two separate entities that had little in common. Such was the disparity that few researchers wondered why antigens were much more immunogenic for the specific immune system when applied with adjuvants that stimulated innate immunity (Sotomayor, E. M., et al., Nat. Med. 5:780 (1999); Diehl, L., et al., Nat. Med. 5:774 (1999); Weigle, W. O., Adv. Immunol. 30:159 (1980)). However, the answer posed by this question is critical to the understanding of the immune system and for comprehending the balance between protective immunity and autoimmunity.

Rationalized manipulation of the innate immune system and in particular activation of APCs involved in T cell priming to deliberately induce a self-specific T cell response provides a means for T cell-based tumor-therapy. Accordingly, the focus of most current therapies is on the use of activated dendritic cells (DCs) as antigen-carriers for the induction of sustained T cell responses (Nestle et al., Nat. Med. 4:328 (1998)). Similarly, in vivo activators of the innate immune system, such as CpGs or anti-CD40 antibodies, are applied together with tumor cells in order to enhance their immunogenicity (Sotomayor, E. M., et al., Nat. Med. 5:780 (1999); Diehl, L., et al., Nat. Med. 5:774 (1999)).

Generalized activation of APCs by factors that stimulate innate immunity may often be the cause for triggering self-specific lymphocytes and autoimmunity. This view is compatible with the observation that administration of LPS together with thyroid extracts is able to overcome tolerance and trigger autoimmune thyroiditis (Weigle, W. O., Adv. Immuno/. 30:159 (1980)). Moreover, in a transgenic mouse model, it was recently shown that administration of self-peptide alone failed to cause autoimmunity unless APCs were activated by a separate pathway (Garza, K. M., et al., J. Exp. Med. 191:2021 (2000)). The link between innate immunity and autoimmune disease is further underscored by the observation that LPS, viral infections or generalized activation of APCs delays or prevents the establishment of peripheral tolerance (Vella, A. T., et al., Immunity 2:261 (1995); Ehl, S., et al., J. Exp. Med. 187:763 (1998); Maxwell, J. R., et al., J. Immunol. 162:2024 (1999)). In this way, innate immunity not only enhances the activation of self-specific lymphocytes but also inhibits their subsequent elimination. These findings may extend to tumor biology and the control of chronic viral diseases.

Induction of cytotoxic T lymphocyte (CTL) responses after immunization with minor histocompatibility antigens, such as the HY-antigen, requires the presence of T helper cells (Th cells) (Husmann, L. A., and M. J. Bevan, Ann. NY. Acad Sci. 532:158 (1988); Guerder, S., and P. Matzinger, J. Exp. Med. 176:553 (1992)). CTL-responses induced by cross-priming, *i.e.* by priming with exogenous antigens that reached the class I pathway, have also been shown to require the presence of Th cells (Bennett, S. R. M., et al., J. Exp. Med. 186:65 (1997)). These observations have important consequences for tumor therapy where T help may be critical for the induction of protective CTL responses by tumor cells (Ossendorp, F., et al., J. Exp. Med. 187:693 (1998)).

An important effector molecule on activated Th cells is the CD40-ligand (CD40L) interacting with CD40 on B cells, macrophages and dendritic cells (DCs) (Foy, T.M., et al., Annu. Rev. Immunol. 14:591 (1996)). Triggering of CD40 on B cells is essential for isotype switching and the generation of B cell memory (Foy, T. M., et al., Ann. Rev. Immunol. 14:591 (1996)). More recently, it was shown that stimulation of CD40 on macrophages and DCs leads to their activation and maturation (Cella, M., et al., Curr. Opin. Immuno/. 9:10 (1997); Banchereau, J., and R. M. Steinman Nature 392: 245 (1998)). Specifically, DCs upregulate costimulatory molecules and produce cytokines such as IL-12 upon activation. Interestingly, this CD40L-mediated maturation of DCs seems to be responsible for the helper effect on CTL responses. In fact, it has recently been shown that CD40-triggering by Th cells renders DCs able to initiate a CTL-response (Ridge, J. P., et al., Nature 393:474 (1998); Bennett, S. R. M., et al., Nature 393:478 (1998); Schoenenberger, S. P., et al., Nature 393:480 (1998)). This is consistent with the earlier observation that Th cells have to recognize their ligands on the same APC as the CTLs, indicating that a cognate interaction is required (Bennett, S. R. M., et al., J. Exp. Med. 186: 65 (1997)). Thus CD40L-mediated stimulation by Th cells leads to the activation of DCs, which subsequently are able to prime CTL-responses.

In contrast to these Th-dependent CTL responses, viruses are often able to induce protective CTL-responses in the absence of T help (for review, see (Bachmann, M. F., et al., J. Immunol. 161:5791 (1998)). Specifically, lymphocytic choriomeningitis virus (LCMV) (Leist, T. P., et al., J. Immunol. 138:2278 (1987); Ahmed, R., et al., J. Virol. 62:2102 (1988); Battegay, M., et al., Cell Immunol. 167:115 (1996); Borrow, P., et al., J Exp. Med. 183:2129 (1996); Whitmire, J. K., et al., J. Virol. 70:8375 (1996)), vesicular stomatitis virus (VSV) (Kündig, T. M., et al., Immunity 5:41 (1996)), influenza virus (Tripp, R. A., et al., J. Immunol. 155:2955 (1995)), vaccinia virus (Leist, T. P., et al., Scand. J. Immunol. 30:679 (1989)) and ectromelia virus (Buller, R., et al., Nature 328:77 (1987)) were able to prime CTL-responses in mice deplete of $CD4^+$ T cells or deficient for the expression of class II or CD40. The mechanism for this Th cell independent CTL-priming by viruses is presently not understood. Moreover, most viruses do not stimulate completely Th cell independent CTL-responses, but virus-specific CTL-activity is reduced in Th-cell deficient mice. Thus, Th cells may enhance anti-viral CTL-responses but the mechanism of this help is not fully understood yet. DCs have recently been shown to present influenza derived antigens by cross-priming (Albert, M. L., et al., J. Exp. Med. 188:1359 (1998); Albert, M. L., et al., Nature 392:86 (1998)). It is therefore possible that, similarly as shown for minor histocompatibility antigens and tumor antigens (Ridge, J. P., et al., Nature 393:474 (1998); Bennett, S. R. M., et al., Nature 393:478 (1998); Schoenenberger, S. P., et al., Nature 393:480 (1998)), Th cells may assist induction of CTLs via CD40 triggering on DCs. Thus, stimulation of CD40 using CD40L or anti-CD40 antibodies may enhance CTL induction after stimulation with viruses or tumor cells.

However, although CD40L is an important activator of DCs, there seem to be additional molecules that can stimulate maturation and activation of DCs during immune responses. In fact, CD40 is not measurably involved in the induction of CTLs specific for LCMV or VSV (Ruedl, C., et al., J. Exp. Med. 189:1875 (1999)). Thus, although VSV-specific CTL responses are partly dependent upon the presence of $CD4^+$T cells (Kündig, T. M., et al., Immunity 5:41 (1996)), this helper effect is not mediated by CD40L. Candidates for effector molecules triggering maturation of DCs during immune responses include Trance and TNF (Bachmann, M. F., et al., J. Exp. Med. 189:1025 (1999); Sallusto, F., and A. Lanzavecchia, J Exp Med. 179:1109 (1994)), but it is likely that there are more proteins with similar properties such as, *e.g.,* CpGs.

It is well established that the administration of purified proteins alone is usually not sufficient to elicit a strong immune response; isolated antigen generally must be given together with helper substances called adjuvants. Within these adjuvants, the administered antigen is protected against rapid degradation, and the adjuvant provides an extended release of a low level of antigen.

Unlike isolated proteins, viruses induce prompt and efficient immune responses in the absence of any adjuvants both with and without T-cell help (Bachmann & Zinkernagel, Ann. Rev. Immunol. 15:235-270 (1997)). Although viruses often consist of few proteins, they are able to trigger much stronger immune responses than their isolated components. For B cell responses, it is known that one crucial factor for the immunogenicity of viruses is the repetitiveness and order of surface epitopes. Many viruses exhibit a quasi-crystalline surface that displays a regular array of epitopes which efficiently crosslinks epitope-specific immunoglobulins on B cells (Bachmann & Zinkernagel, Immunol. Today 17:553-558 (1996)). This crosslinking of surface immunoglobulins on B cells is a strong activation signal that directly induces cell-cycle progression and the production of IgM antibodies. Further, such triggered B cells are able to activate T helper cells, which in turn induce a switch from IgM to IgG antibody production in B cells and the generation of long-lived B cell memory - the goal of any vaccination (Bachmann & Zinkernagel, Ann. Rev. Immunol. 15:235-270 (1997)). Viral structure is even linked to the generation of anti-antibodies in autoimmune disease and as a part of the natural response to pathogens (*see* Fehr, T., et al., J. Exp. Med. 185:1785-1792 (1997)). Thus, antigens on viral particles that are organized in an ordered and repetitive array are highly immunogenic since they can directly activate B cells. However, soluble antigens not linked to a repetitive surface are poorly immunogenic in the absence of adjuvants. Since pathogens, allergen extracts and also tumors usually contain a multitude of antigens that may not all easily be expressed and conjugated to repetitive strucutures such as VLPs, it would be desirable to have adjuvants formulations that may simply be mixed with the antigen-preparations without the need for complex conjugation procedures.

In addition to strong B cell responses, viral particles are also able to induce the generation of a cytotoxic T cell response, another crucial arm of the immune system. These cytotoxic T cells are particularly important for the elimination of non-cytopathic viruses such as HIV or Hepatitis B virus and for the eradication of tumors. Cytotoxic T cells do not recognize native antigens but rather recognize their degradation products in association with MHC class I molecules (Townsend & Bodmer, Ann. Rev. Immunol. 7:601-624 (1989)). Macrophages and dendritic cells are able to take up and process exogenous viral particles (but not their soluble, isolated components) and present the generated degradation product to cytotoxic T cells, leading to their activation and proliferation (Kovacsovics-Bankowski et al., Proc. Natl. Acad Sci. USA 90:4942-4946 (1993); Bachmann et al., Eur. J. Immunol. 26:2595-2600 (1996)). In addition, activated DC's are also able to process and present soluble proteins.

Viral particles as antigens exhibit two advantages over their isolated components: (1) due to their highly repetitive surface structure, they are able to directly activate B cells, leading to high antibody titers and long-lasting B cell memory; and (2) viral particles but not soluble proteins are able to induce a cytotoxic T cell response, even if the viruses are non-infectious and adjuvants are absent.

Several new vaccine strategies exploit the inherent immunogenicity of viruses. Some of these approaches focus on the particulate nature of the virus particle; (*see* Harding, C. et al., J. Immunology 153:4925 (1994)), which discloses a vaccine consisting of latex beads and antigen; Kovacsovics-Bankowski, M., et al. (Proc. Natl. Acad Sci. USA 90:4942-4946 (1993)); which discloses a vaccine consisting of iron oxide beads and antigen; U.S. Patent No. 5,334,394 to Kossovsky, N., *et al.,* which discloses core particles coated with antigen; U.S. Patent No. 5,871,747, which discloses synthetic polymer particles carrying on the surface one or more proteins covalently bonded thereto; and a core particle with a non-covalently bound coating, which at least partially covers the surface of said core particle, and at least one biologically active agent in contact with said coated core particle (*see, e.g.,* WO 94/15585).

In a further development, virus-like particles (VLPs) are being exploited in the area of vaccine production because of both their structural properties and their non-infectious nature (*see, e.g.,* WO 98/50071). VLPs are supermolecular structures built in a symmetric manner from many protein molecules of one or more types. They lack the viral genome and, therefore, are noninfectious. VLPs can often be produced in large quantities by heterologous expression and can be easily be purified.

In addition, DNA rich in non-methylated CG motifs (CpG), as present in bacteria and most non-vertebrates, exhibits a potent stimulatory activity on B cells, dendritic cells and other APC's *in vitro* as well as *in vivo.* Although bacterial DNA, is immunostimulatory across many vertebrate species, the individual CpG motifs may differ. In fact, CpG motifs that stimulate mouse immune cells may not necessarily stimulate human immune cells and vice versa.

Although-DNA-oligonucleotides rich in CpG motifs can exhibit immunostimulatory capacity, their efficiency is often limited, since they are unstable *in vitro* and *in vivo.* Thus, they exhibit unfavorable pharmacokinetics. In order to render CpG-oligonucleotides more potent, it is therefore usually necessary to stabilize them by introducing phosphorothioate modifications of the phosphate backbone.

A second limitation for the use of CpGs to stimulate immune responses is their lack of specificity, since all APC's and B cells in contact with CpGs become stimulated. Thus, the efficiency and specificity of DNA oligonucleotides containing CpGs may be improved by stabilizing them or packaging them in a way that restricts cellular activation to those cells that also present the relevant antigen.

In addition, immunostimulatory CpG-oligodeoxynucleotides induce strong side effects by causing extramedullary hemopoiesis accomponied by splenomegaly and lymphadenopathy in mice (Sparwasser et al., J. Immunol. (1999), 162:2368-74). Recent evidence demonstrates that VLPs containing packaged CpGs are able to trigger very potent T cell responses against antigens conjugated to the VLPs (WO03/024481). In addition, packaging CpGs enhanced their stability and essentially removed their above mentioned side-effects such as causing extramedullary hemopoiesis accomponied by splenomegaly and lymphadenopathy in mice. In particular, packaged CpGs did not induce splenomegaly. However, as mentioned above, most pathogens, tumors and allergen extracts contain a multitude of antigens and it may be often difficult to express all these antigens recombinantly before conjugation to the VLPs. Hence, it would be desirable to have adjuvants formulations that may simply be mixed with the antigen-preparations without the need for complex conjugation procedures.

There have been remarkable advances made in vaccination strategies recently, yet there remains a need for improvement on existing strategies. In particular, there remains a need in the art for the development of new and improved vaccines that allow the induction of strong T and B cell responses without serious side-effects and without a need for conjugating the antigens to a carrier substance.

## SUMMARY OF THE INVENTION

It was a surprising finding that immunostimulatory substances such as DNA oligonucleotides can be packaged into VLPs which renders them more immunogenic. Unexpectedly, the nucleic acids and oligonucleotides, respectively, present in VLPs can be replaced specifically by the immunostimulatory substances and DNA-oligonucleotides containing CpG motifs, respectively. Surprisingly, these packaged immunostimulatory substances, in particular immunostimulatory nucleic acids such as unmethylated CpG-containing oligonucleotides retained their immunostimulatory capacity without widespread activation of the innate immune system. The compositions comprising VLP's and the immunostimulatory substances and in particular the CpG-VLPs are dramatically more immunogenic than their CpG-free counterparts and dramatically enhance B and T cell responses to antigens applied together, i.e. mixed with the packaged VLPs. Unexpectedly, coupling of the antigens to the VLPs was not required for enhancement of the immune response. Moreover, due to the packaging, the CpGs bound to the VLPs did not induce systemic side-effects, such as splenomegaly.

In a first embodiment, the invention provides a composition for enhancing an immune response in an animal comprising a virus-like particle and an immunostimulatory substance being an unmethylated CpG-containing oligonucleotide, where the oligonucleotide is packaged with the virus-like particle. The composition further comprises an allergen mixed with the virus-like particle.

The immunostimulatory nucleic acids, in particular the unmethylated CpG-containing oligonucleotides are stabilized by phosphorothioate modifications of the phosphate backbone. The immunostimulatory nucleic acids, in particular the unmethylated CpG-containing oligonucleotides are packaged into the VLPs by digestion of RNA within the VLPs and simultaneous addition of the DNA oligonucleotides containing CpGs of choice. In an equally preferred embodiment, the VLPs can be disassembled before they are reassembled in the presence of CpGs.

Immunostimulatory nucleic acids do not contain CpG motifs but nevertheless exhibit immunostimulatory activities. Such nucleic acids are described in WO 01/22972. All sequences described therein are hereby incorporated by way of reference.

In a preferred embodiment of the invention, the unmethylated CpG-containing oligonucleotide is not stabilized by phosphorothioate modifications of the phosphodiester backbone.

In a preferred embodiment, the unmethylated CpG containig oligonucleotide induces IFN-alpha in human cells. In another preferred embodiment, the IFN-alpha inducing oligonucleotides is flanked by guanosine-rich repeats and contains a palindromic sequence.

In a further preferred embodiment, the virus-like particle is a recombinant virus-like particle. Also preferred, the virus-like particle is free of a lipoprotein envelope. Preferably, the recombinant virus-like particle comprises, or alternatively consists of, recombinant proteins of Hepatitis B virus, measles virus, Sindbis virus, Rotavirus, Foot-and-Mouth-Disease virus, Retrovirus, Norwalk virus or human Papilloma virus, RNA-phages, Qβ-phage, GA-phage, fr-phage, AP205-phage and Ty. In a specific embodiment, the virus-like particle comprises, or alternatively consists of, one or more different Hepatitis B virus core (capsid) proteins (HBcAgs).

In a further preferred embodiment, the virus-like particle comprises recombinant proteins, or fragments thereof, of a RNA-phage. Preferred RNA-phages are Qβ-phage, AP 205-phage, GA-phage, fr-phage.

In another embodiment, the allergen is a recombinant antigen. In another embodiment, the allergen is extracted from a natural source, which includes but is not limited to: pollen, dust, fungi, insects, food, mammalian epidermals, hair, saliva, serum, bees, tumors, pathogens and feathers.

In yet another embodiment, the antigen can be a polypeptide suited to induce an immune response against allergens.

In a further embodiment, the antigen can be an organic molecule suited to induce an immune response against allergens.

The antigen may comprise, or alternatively consists of, a cytotoxic T cell or Th cell epitope. The antigen may also comprise, or alternatively consists of, a B cell epitope. The virus-like particle may also comprise the Hepatitis B virus core protein.

There is provided a method of enhancing an immune response in a human or other animal species comprising introducing into the animal a composition comprising a virus-like particle and immunostimulatory substance being an unmethylated CpG-containing oligonucleotide where the oligonucleotide is packaged with the virus-like particle and the virus-like particle is mixed with an allergen, several allergens or an allergen mixture. In yet another embodiment of the invention, the composition is introduced into an animal subcutaneously, intramuscularly, intranasally, intradermally, intravenously or directly into a lymph node.

In a preferred aspect of the invention, the immune response is a T cell response, and the T cell response against the antigen is enhanced. In a specific embodiment, the T cell response is a cytotoxic T cell response, and the cytotoxic T cell response against the antigen is enhanced. In another embodiment of the invention, the immune response is a B cell response, and the B cell response against the antigen is enhanced. The present invention also relates to a vaccine comprising an immunologically effective amount of the immune enhancing composition of the present invention together with a pharmaceutically acceptable diluent, carrier or excipient. The vaccine may further comprises at least one adjuvant, such as Alum or incomplete Freund's adjuvant. The invention also provides a method of immunizing and/or treating an animal comprising administering to the animal an immunologically effective amount of the disclosed vaccine.

The unmethylated CpG-containing oligonucleotide VLPs are used for vaccination of animals or humans against antigens mixed with the modified VLP. The vaccination can be for prophylactic or therapeutic purposes, or both. Also, the modified VLPs can be used to vaccinate against allergies, or diseases related to allergy such as asthma, in order to induce immune-deviation and/or antibody responses against the allergen. Such a vaccination and treatment, respectively, can then lead, for example, to a desensibilization of a former allergic animal and patient, respectively.

In the majority of cases, the desired immune response will be directed against allergens mixed with the unmethylated CpG-containing oligonucleotide VLPs. The allergens can be peptides, proteins or domains as well as mixtures thereof.

The route of injection is preferably subcutaneous or intramuscular, but it would also be possible to apply the CpG-containing VLPs intradermally, intranasally, intravenously or directly into the lymph node.

It is to be understood that both the foregoing general description and the following detailed description are exemplary and explanatory only and are intended to provide further explanation of the invention as claimed.

BRIEF DESCRIPTION OF THE DRAWINGS/FIGURES

Figure 1 shows VLPs in a native agarose gel electrophoresis (1% agarose) after control incubation or after digestion with RNase A upon staining with ethidium bromide (A) or Coomassie blue (B) in order to assess for the presence of RNA or protein. Recombinantly produced VLPs were diluted at a final concentration of 0.5 ug/ul protein in PBS buffer and incubated in the absence (lane 1) or presence (lane 2) of RNase A (100 ug/ml) (Sigma, Division of Fluka AG, Switzerland) for 2 h at 37˚C. The samples were subsequently complemented with 6-fold concentrated DNA-loading buffer (MBS Fermentas GmbH, Heidelberg, Germany) and run for 30 min at 100 volts in a 1% native agarose gel. The Gene Ruler marker (MBS Fermentas GmbH, Heidelberg, Germany) was used as reference for VLPs migration velocity (lane M). Rows are indicating the presence of RNA enclosed in VLPs (A) or VLPs itself (B). Identical results were obtained in 3 independent experiments.

Figure 2 shows VLPs in a native agarose gel electrophoresis (1% agarose) after control incubation or after digestion with RNase A in the presence of buffer only or CpG-containing DNA-oligonucleotides upon staining with ethidium bromide (A) or Comassie blue (B) in order to assess for the presence of RNA/DNA or protein. Recombinant VLPs were diluted at a final concentration of 0.5 ug/ul protein in PBS buffer and incubated in the absence (lane 1) or presence (lane 2 and 3) of RNase A (100 ug/ml) (Sigma, Division of Fluka AG, Switzerland) for 2 h at 37˚C. 5 nmol CpG-oligonucleotides (containing phosphorothioate modifications of the phosphate backbone) were added to sample 3 before RNase A digestion. The Gene Ruler marker (MBS Fermentas GmbH, Heidelberg, Germany) was used as reference for p33-VLPs migration velocity (lane M). Rows are indicating the presence of RNA/CpG-DNA enclosed in p33-VLPs (A) or p33-VLPs itself (B). Comparable results were obtained when CpG oligonucleotides with normal phosphor bonds were used for co-incubation of VLPs with RNase A.

Figure 3 shows p33-VLPs in a native agarose gel electrophoresis (1% agarose) before and after digestion with RNase A in the presence of CpG-containing DNA-oligonucleotides and subsequent dialysis (for the elimination of VLP-unbound CpG-oligonucleotides) upon staining with ethidium bromide (A) or Comassie blue (B) in order to assess for the presence of DNA or protein. Recombinant VLPs were diluted at a final concentration of 0.5 ug/ul protein in PBS buffer and incubated in absence (lane 1) or in presence (lanes 2 to 5) of RNase A (100 ug/ml) (Sigma, Division of Fluka AG, Switzerland) for 2 h at 37˚C. 50 nmol CpG-oligonucleotides (containing phosphorothioate bonds: lanes 2 and 3, containing normal phosphor modifications of the phosphate backbone: lanes 4 and 5) were added to VLPs before RNase A digestion. Treated samples were extensively dialysed for 24 hours against PBS (4500-fold dilution) with a 300 kDa MWCO dialysis membrane (Spectrum Medical Industries Inc., Houston, USA) to eliminate the in excess DNA (lanes 3 and 5). The Gene Ruler marker (MBS Fermentas GmbH, Heidelberg, Germany) was used as reference for p33-VLPs migration velocity (lane M). Rows are indicating the presence of RNA/CpG-DNA enclosed in VLPs (A) or VLPs itself (B).

Figure 4 shows VLPs in a native agarose gel electrophoresis (1% agarose) after control incubation or after digestion with RNase A where CpG-containing DNA-oligonucleotides were added only after completing the RNA digestion upon staining with ethidium bromide (A) or Comassie blue (B) in order to assess for the presence of RNA/DNA or protein. Recombinant VLPs were diluted at a final concentration of 0.5 ug/ul protein in PBS buffer and incubated in the absence (lane 1) or presence (lane 2 and 3) of RNase A (100 ug/ml) (Sigma, Division of Fluka AG, Switzerland) for 2 h at 37˚C. 5 nmol CpG-oligonucleotides (containing phosphorothioate modifications of the phosphate backbone) were added to sample 3 only after the RNase A digestion. The Gene Ruler marker (MBS Fermentas GmbH, Heidelberg, Germany) was used as reference for p33-VLPs migration velocity (lane M). Rows are indicating the presence of RNA/CpG-DNA enclosed in VLPs (A) or VLPs itself (B). Similar results were obtained when CpG oligonucleotides with normal phosphor bonds were used for reassembly of VLPs.

Figure 5 shows that RNase A treated VLPs derived from HBcAg carrying inside CpG-rich DNA (containing normal phosphodiester moieties), dialyzed from unbound CpG-oligonucleotides are effective at enhancing IgG responses against bee venom allergens (BV). Mice were subcutaneously primed with 5 μg of bee venom (ALK Abello) either alone or mixed with one of the following: 50 μg VLP alone, 50 μg VLP loaded and packaged, respectively, with CpG-oligonucleotides or 50 μg VLP mixed with 20 nmol CpG-oligonucleotides. Alternatively, mice were primed with 5 μg bee venom mixed with VLP alone or VLP loaded and packaged, respectively, with CpG-oligonucleotides in conjunction with aluminum hydroxide. 14 days later, mice were boosted with the same vaccine preparations and bled on day 21. Bee venom specific IgG responses in serum were assessed by ELISA. Results as shown as optical densities for indicated serum dilutions. Average of two mice each are shown.

Figure 6 shows that RNase A treated VLPs (HBc) carrying inside CpG-rich DNA (containing normal phosphor bonds), dialyzed from unbound CpG-oligonucleotides are effective at inducing IgG2a rather than IgG1 responses against the bee venom allergen PLA2 (Phospholipase A2). Mice were subcutaneously primed with 5 μg of bee venom (ALK Abello) either alone or mixed with one of the following: 50 μg VLP alone, 50 μg VLP loaded and packaged, respectively, with CpG-oligonucleotides or 50 μg VLP mixed with 24 nmol CpG-oligonucleotides. Alternatively, mice were primed with 5 μg bee venom mixed with VLP alone or VLP loaded and packaged, respectively, with CpG-oligonucleotides in conjunction with aluminum hydroxide. 14 days later, mice were boosted with the same vaccine preparations and bled on day 21. PLA2-specific IgG subclasses in serum from day 21 were assessed by ELISA. Note that presence of Alum favoured the induction of IgG1 even in the presence of CpG-packaged VLPs or free CpGs. Results are shown as optical densities for 20 fold diluted serum samples. Average of two mice each is shown.

Figure 7 shows that free CpGs but not CpGs packaged into VLPs (HBc) dramatically increase spleen size after vaccination. Mice were immunized with 100 μg VLP alone, CpGs alone (20 nmol), 100 μg VLPs mixed with 20 nmol CpGs, or containing packaged CpGs. Total lymphocyte numbers/spleen were measured 12 days later.

Figure 8 shows allergic body temperature drop in VLP(CpG) + Bee venom vaccinated mice. Two sets of mice have been tested. Group 1 (n = 7) received VLP(CpG) mixed together with Bee venom as vaccine. Group 2 (n = 6) received only VLP(CpG). After a challenge with a high dose of Bee venom (30ug), the allergic reaction was assessed in terms of changes in the body temperature of the mice. In group 1 receiving the Bee venom together with VLP (CpG) no significant changes of the body temperature was observed in any of the tested mice. In contrast, the group 2 receiving only VLP(CpG) as a desensitizing vaccine showed a pronounced body temperature drop in 4 out of 6 animals. Therefore, these mice have not been protected from allergic reactions. Note: The symbols in the figure represent the mean of 6 (for VLP(CpG)) or 7 (VLP(CpG) + Bee venom ) individual mice including standard deviation (SD).

Figure 9 shows detection of specific IgE and IgG serum antibodies in mice before and after desensitization. All mice have been sensitized with four injections of Bee venom in adjuvant (Alum). Then, the mice have been vaccinated with VLP(CpG) + Bee venom in order to induce a protective immune response or as a control with VLP(CpG) only. Blood samples of all mice were taken before and after desensitization and tested in ELISA for Bee venom specific IgE antibodies (panel A), IgG1 antibodies (panel B) and IgG2a antibodies (panel C), respectively. As shown in Figure 9A, an increased IgE titer is observed for VLP(CpG) + Bee venom vaccinated mice after desensitization. The results are presented as the optical density (OD450nm) at 1:250 serum dilution. The mean of 6 (VLP(CpG))or 7 (VLP(CpG) + Bee venom) individual mice including standard deviation (SD) is shown in the figure. Figure 9B reveals an increased anti-Bee venom IgG1 serum titer after desensitization only for mice vaccinated with VLP(CpG) + Bee venom. The same is true for Figure 9C were IgG2a serum titers have been determined. As expected for a successful desensitization, the increase in IgG2a antibody titers was most pronounced. The results are shown as means of 2 (VLP(CpG)) or 3 (VLP(CpG) + Bee venom) mice including SD for 1:12500 (IgG1)or 1:500 (IgG2a) serum dilutions, respectively.

Figure 10 shows the antibody responses of Balb/c mice immunized with grass pollen extract either mixed with Qb VLPs, Qb VLPs loaded and packaged, respectively, with CpG-2006 or with Alum. Polled sera of 5 mice per groups were used. An ELISA assay was performed with pollen extract coated to the plate. Wells were incubated with a dilution of 1:60 of the respective mouse sera from day 21 for detection of IgG1, IgG2a and Ig2b or with a dilution of 1:10 for the detection of IgE isotype antibodies and detection was performed with the corresponding isotype specific anti-mouse secondary antibodies coupled to horse raddish peroxidase. Optical densities at 450 nm are plotted after colour reaction.

Figure 11 shows the antibody responses of Balb/c mice which were sensitized with grass pollen extract mixed with Alum and subsequently desensitized with with grass pollen extract either mixed with Qb VLPs or with Qb VLPs loaded, and packaged, respectively, with CpG-2006 or with Alum. One group of mice was left untreated after sensitization. An ELISA assay was performed with pollen extract coated to the plate. Wells were incubated with serial dilutions of the respective mouse sera and detection was performed with the IgG1 and IgG2a isotype specific anti-mouse secondary antibodies coupled to horse raddish peroxidase. ELISA titers were calculated as the reciprocal of the dilution given 50% of the optical densities at saturation. Figure 11A shows the IgG1 titers, figure 11B the IgG2b titers.

Figure 12 depicts the analysis of g10gacga-PO packaging into HBc33 VLPs on a 1% agarose gel stained with ethidium bromide (A) and Coomassie Blue (B). Loaded on the gel are 15 μg of the following samples: 1. 1 kb MBI Fermentas DNA ladder; 2. HBc33 VLP untreated; 3. HBc33 VLP treated with RNase A; 4. HBc33 VLP treated with RNase A and packaged with g10gacga-PO; 5. HBc33 VLP treated with RNase A, packaged with g10gacga-PO, treated with Benzonase and dialysed.

Figure 13 shows electron micrographs of Qβ VLPs that were reassembled in the presence of different oligodeoxynucleotides. The VLPs had been reassembled in the presence of the indicated oligodeoxynucleotides or in the presence of tRNA but had not been purified to a homogenous suspension by size exclusion chromatography. As positive control served preparation of "intact" Qβ VLPs which had been purified from *E.coli.*

Figure 14 shows the analysis of nucleic acid content of the reassembled Qβ VLPs by nuclease treatment and agarose gelelectrophoresis: 5 μg of reassembled and purified Qβ VLPs and 5 μg of Qβ VLPs which had been purified from *E.coli,* respectively, were treated as indicated. After this treatment, samples were mixed with loading dye and loaded onto a 0.8% agarose gel. After the run the gel was stained first with ethidium bromide (A) and after documentation the same gel was stained with Coomassie blue (B).

Figure 15 A shows an electron micrograph of the disassembled AP205 VLP protein, while Figure 15 B shows the reassembled particles before purification. Figure 15C shows an electron micrograph of the purified reassembled AP205 VLPs. The magnification of Figure 15A-C is 200 000 X.

Figure 16 A and B show the reassembled AP205 VLPs analyzed by agarose gel electrophoresis. The samples loaded on the gel from both figures were, from left to right: untreated AP205 VLP, 3 samples with differing amount of AP205 VLP reassembled with CyCpG and purified, and untreated Qβ VLP. The gel on Figure 16A was stained with ethidium bromide, while the same gel was stained with Coomassie blue in Figure 16 B.

Figure 17 shows the SDS-PAGE analysis demonstrating multiple coupling bands consisting of one, two or three peptides coupled to the Qβ monomer (Arrows, Figure 17). For the sake of simplicity the coupling product of the peptide p33 and Qβ VLPs was termed, in particular, throughout the example section Qbx33.

Figure 18 depicts the analysis of B-CpGpt packaging into Qbx33 VLPs on a 1% agarose gel stained with ethidium bromide (A) and Coomassie Blue (B). (C) shows the analysis of the amount of packaged oligo extracted from the VLP on a 15% TBE/urea stained with SYBR Gold. Loaded on gel are the following samples: 1. BCpGpt oligo content of 2 μg Qbx33 VLP after proteinase K digestion and RNase A treatment; 2. 20 pmol B-CpGpt control; 3. 10 pmol B-CpGpt control; 4. 5 pmol B-CpGpt control. Figure 18 D and E show the analysis of g10gacga-PO packaging into Qbx33 VLPs on a 1% agarose gel stained with ethidium bromide (D) and Coomassie Blue (E). Loaded on the gel are 15 μg of the following samples: 1. MBI Fermentas 1 kb DNA ladder; 2. Qbx33 VLP untreated; 3. Qbx33 VLP treated with RNase A; 4. Qbx33 VLP treated with RNase A and packaged with g10gacga-PO; 5. Qbx33 VLP treated with RNase A, packaged with g10gacga-PO, treated with Benzonase and dialysed. Figure 18 E and F show the analysis of dsCyCpG-253 packaging into Qbx33 VLPs on a 1% agarose gel stained with ethidium bromide (E) and Coomassie Blue (F). Loaded on the gel are 15 μg of the following samples: 1. MBI Fermentas 1 kb DNA ladder; 2. Qbx33 VLP untreated; 3. Qbx33 VLP treated with RNase A; 4. Qbx33 VLP treated with RNase A, packaged with dsCyCpG-253 and treated with DNaseI; 5. Qbx33 VLP treated with RNase A, packaged with dsCyCpG-253, treated with DNaseI and dialysed.

## DETAILED DESCRIPTION OF THE INVENTION

Unless defined otherwise, all technical and scientific terms used herein have the same meanings as commonly understood by one of ordinary skill in the art to which this invention belongs. Although any methods and materials similar or equivalent to those described herein can be used in the practice or testing of the present invention, the preferred methods and materials are hereinafter described.

### 1. Definitions

Animal: As used herein, the term "animal" is meant to include, for example, humans, sheep, horses, cattle, pigs, dogs, cats, rats, mice, birds, reptiles, fish, insects and arachnids.

Antibody: As used herein, the term "antibody" refers to molecules which are capable of binding an epitope or antigenic determinant. The term is meant to include whole antibodies and antigen-binding fragments thereof, including single-chain antibodies. Most preferably the antibodies are human antigen binding antibody fragments and include, but are not limited to, Fab, Fab' and F(ab')2, Fd, single-chain Fvs (scFv), single-chain antibodies, disulfide-linked Fvs (sdFv) and fragments comprising either a $V_L$ or $V_H$ domain. The antibodies can be from any animal origin including birds and mammals. Preferably, the antibodies are human, murine, rabbit, goat, guinea pig, camel, horse or chicken. As used herein, "human" antibodies include antibodies having the amino acid sequence of a human immunoglobulin and include antibodies isolated from human immunoglobulin libraries or from animals transgenic for one or more human immunoglobulins and that do not express endogenous immunoglobulins, as described, for example, in U.S. Patent No. 5,939,598 by Kucherlapati et al.

In a preferred embodiment of the invention, compositions of the invention may be used in the design of vaccines for the treatment of allergies. Antibodies of the IgE isotype are important components in allergic reactions. Mast cells bind IgE antibodies on their surface and release histamines and other mediators of allergic response upon binding of specific antigen to the IgE molecules bound on the mast cell surface. Inhibiting production of IgE antibodies, therefore, is a promising target to protect against allergies. This should be possible by attaining a desired T helper cell response. T helper cell responses can be divided into type 1 ($T_H1$) and type 2 ($T_H2$) T helper cell responses (Romagnani, Immunol. Today 18:263-266 (1997)). $T_H1$ cells secrete interferon-gamma and other cytokines which trigger B cells to produce IgG antibodies. In contrast, a critical cytokine produced by $T_H2$ cells is IL-4, which drives B cells to produce IgE. In many experimental systems, the development of $T_H1$ and $T_H2$ responses is mutually exclusive since $T_H1$ cells suppress the induction of $T_H2$ cells and vice versa. Thus, antigens that trigger a strong $T_H1$ response simultaneously suppress the development of $T_H2$ responses and hence the production of IgE antibodies. The presence of high concentrations of IgG antibodies may prevent binding of allergens to mast cell bound IgE, thereby inhibiting the release of histamine. Thus, presence of IgG antibodies may protect from IgE mediated allergic reactions. Typical substances causing allergies include, but are not limited to: pollens (*e.g.* grass, ragweed, birch or mountain cedar); house dust and dust mites; mammalian epidermal allergens and animal danders; mold and fungus; insect bodies and insect venom; feathers; food; and drugs (*e.g.*, penicillin). *See* Shough, H. et al., REMINGTON'S PHARMACEUTICAL SCIENCES, 19th edition, (Chap. 82), Mack Publishing Company, Mack Publishing Group, Easton, Pennsylvania (1995), the entire contents of which is hereby incorporated by reference. Thus, immunization of individuals with allergens mixed with virus like particles containing packaged DNA rich in non-methylated CG motifs should be beneficial not only before but also after the onset of allergies.

Antigen: As used herein, the term "antigen" refers to a molecule capable of being bound by an antibody or a T cell receptor (TCR) if presented by MHC molecules. The term "antigen", as used herein, also encompasses T-cell epitopes. An antigen is additionally capable of being recognized by the immune system and/or being capable of inducing a humoral immune response and/or cellular immune response leading to the activation of B- and/or T-lymphocytes. This may, however, require that, at least in certain cases, the antigen contains or is linked to a Th cell epitope and is given in adjuvant. An antigen can have one or more epitopes (B- and T- epitopes). The specific reaction referred to above is meant to indicate that the antigen will preferably react, typically in a highly selective manner, with its corresponding antibody or TCR and not with the multitude of other antibodies or TCRs which may be evoked by other antigens. Antigens as used herein may also be mixtures of several individual antigens.

A "microbial antigen" as used herein is an antigen of a microorganism and includes, but is not limited to, infectious virus, infectious bacteria, parasites and infectious fungi. Such antigens include the intact microorganism as well as natural isolates and fragments or derivatives thereof and also synthetic or recombinant compounds which are identical to or similar to natural microorganism antigens and induce an immune response specific for that microorganism. A compound is similar to a natural microorganism antigen if it induces an immune response (humoral and/or cellular) to a natural microorganism antigen. Such antigens are used routinely in the art and are well known to the skilled artisan.

Examples of infectious viruses that have been found in humans include but are not limited to: Retroviridae (e.g. human immunodeficiency viruses, such as HTV-1 (also referred to as HTLV-III, LAV or HTLV-III/LAV, or HIV-III); and other isolates, such as HIV-LP); Picornaviridae (e.g. polio viruses, hepatitis A *virus*; enteroviruses, human Coxsackie viruses, rhinoviruses, echoviruses); Calciviridae (e.g. strains that cause gastroenteritis); Togaviridae (e.g. equine encephalitis viruses, rubella viruses); Flaviridae (e.g. dengue viruses, encephalitis viruses, yellow fever viruses); Coronoviridae (e.g. coronaviruses); Rhabdoviradae (e.g. vesicular stomatitis viruses, rabies viruses); Filoviridae (e.g. ebola viruses); Paramyxoviridae (e.g. parainfluenza viruses, mumps virus, measles virus, respiratory syncytial virus); Orthomyxoviridae (e.g. influenza viruses); Bungaviridae (e.g. Hantaan viruses, bunga viruses, phleboviruses and Nairo viruses); Arena viridae (hemorrhagic fever viruses); Reoviridae (e.g. reoviruses, orbiviurses and rotaviruses); Birnaviridae; Hepadnaviridae (Hepatitis B virus); Parvovirida (parvoviruses); Papovaviridae (papilloma viruses, polyoma viruses); Adenoviridae (most adenoviruses); Herpesviridae (herpes simplex virus (HSV) 1 and 2, varicella zoster virus, cytomegalovirus (CMV), herpes virus); Poxviridae (variola viruses, vaccinia viruses, pox viruses); and Iridoviridae (e.g. African swine fever virus); and unclassified viruses (e.g. the etiological agents of Spongiform encephalopathies, the agent of delta hepatitis (thought to be a defective satellite of hepatitis B virus), the agents of non-A, non-B hepatitis (class 1=internally transmitted; class 2=parenterally transmitted (i.e. Hepatitis C); Norwalk and related viruses, and astroviruses).

Both gram negative and gram positive bacteria serve as antigens in vertebrate animals. Such gram positive bacteria include, but are not limited to, Pasteurella species, Staphylococci species and Streptococcus species. Gram negative bacteria include, but are not limited to, Escherichia coli, Pseudomonas species, and Salmonella species. Specific examples of infectious bacteria include but are not limited to: *Helicobacter pyloris, Borelia burgdorferi, Legionella pneumophilia,* Mycobacteria sps. (e.g. *M. tuberculosis, M. avium, M intracellulare, M. kansaii, M. gordonae), Staphy-*

*lococcus aureus, Neisseria gonorrhoeae, Neisseria meningitidis, Listeria monocytogenes, Streptococcus pyogenes* (Group A Streptococcus), *Streptococcus agalactiae* (Group B Streptococcus), Streptococcus (viridans group), *Streptococcus faecalis, Streptococcus bovis,* Streptococcus (anaerobic sps.), *Streptococcus pneumoniae,* pathogenic Campylobacter sp., Enterococcus sp., *Haemophilus influenzae, Bacillus antracis, Corynebacterium diphtheriae,* Corynebacterium sp., *Erysipelothrix rhusiopathiae, Clostridium perfringers, Clostridium tetani, Enterobacter aerogenes, Klebsiella pneumoniae, Pasturella multocida,* Bacteroides sp., *Fusobacterium nucleatum, Streptobacillus moniliformis, Treponema pallidium, Treponema pertenue,* Leptospira, Rickettsia, *Actinomyces israelli* and *Chlamydia.*

Examples of infectious fungi include: *Cryptococcus neoformans, Histoplasma capsulatum, Coccidioides immitis, Blastomyces dermatitidis, Chlamydia trachomatis* and *Candida albicans.* Other infectious organisms (i.e., protists) include: Plasmodium such as *Plasmodium falciparum, Plasmodium malariae, Plasmodium ovale, Plasmodium vivax, Toxoplasma* gondii and *Shistosoma.*

Other medically relevant microorganisms have been descried extensively in the literature, *e.g., see* C. G. A. Thomas, "Medical Microbiology", Bailliere Tindall, Great Britain 1983, the entire contents of which is hereby incorporated by reference. The compositions and methods of the invention are also useful for treating cancer by stimulating an antigen-specific immune response against a cancer antigen. A "tumor antigen" as used herein is a compound, such as a peptide, associated with a tumor or cancer and which is capable of provoking an immune response. In particular, the compound is capable of provoking an immune response when presented in the context of an MHC molecule. Tumor antigens can be prepared from cancer cells either by preparing crude extracts of cancer cells, for example, as described in Cohen, et al., Cancer Research, 54:1055 (1994), by partially purifying the antigens, by recombinant technology or by de novo synthesis of known antigens. Tumor antigens include antigens that are antigenic portions of or are a whole tumor or cancer polypeptide. Such antigens can be isolated or prepared recombinantly or by any other means known in the art. Cancers or tumors include, but are not limited to, biliary tract cancer; brain cancer; breast cancer; cervical cancer; choriocarcinoma; colon cancer; endometrial cancer; esophageal cancer; gastric cancer; intraepithelial neoplasms; lymphomas; liver cancer; lung cancer (e.g. small cell and non-small cell); melanoma; neuroblastomas; oral cancer; ovarian cancer; pancreas cancer; prostate cancer; rectal cancer; sarcomas; skin cancer; testicular cancer; thyroid cancer; and renal cancer, as well as other carcinomas and sarcomas.

Allergens also serve as antigens in vertebrate animals. The term "allergen", as used herein, also encompasses "allergen extracts" and "allergenic epitopes." Examples of allergens include, but are not limited to: pollens (*e.g.* grass, ragweed, birch and mountain cedar); house dust and dust mites; mammalian epidermal allergens and animal danders; mold and fungus; insect bodies and insect venom; feathers; food; and drugs (*e.g.*, penicillin).

Antigenic determinant: As used herein, the term "antigenic determinant" is meant to refer to that portion of an antigen that is specifically recognized by either B- or T-lymphocytes. B-lyrnphocytes responding to antigenic determinants produce antibodies, whereas T-lymphocytes respond to antigenic determinants by proliferation and establishment of effector functions critical for the mediation of cellular and/or humoral immunity.

Antigen presenting cell: As used herein, the term "antigen presenting cell" is meant to refer to a heterogenous population of leucocytes or bone marrow derived cells which possess an immunostimulatory capacity. For example, these cells are capable of generating peptides bound to MHC molecules that can be recognized by T cells. The term is synonymous with the term "accessory cell" and includes, for example, Langerhans' cells, interdigitating cells, dendritic cells, B cells and macrophages. Under some conditions, epithelial cells, endothelial cells and other, non-bone marrow derived cells may also serve as antigen presenting cells.

Bound: As used herein, the term "bound" refers to binding that may be covalent, *e.g.,* by chemically coupling the unmethylated CpG-containing oligonucleotide to a virus-like particle, or non-covalent, *e.g.,* ionic interactions, hydrophobic interactions, hydrogen bonds, etc. Covalent bonds can be, for example, ester, ether, phosphoester, amide, peptide, imide, carbon-sulfur bonds, carbon-phosphorus bonds, and the like. The term also includes the enclosement, or partial enclosement, of a substance. The term "bound" is broader than and includes terms such as "coupled," "fused," "enclosed" and "attached." Moreover, with respect to the immunostimulatory substance being bound to the virus-like particle the term "bound" also includes the enclosement, or partial enclosement, of the immunostimulatory substance. Therefore, with respect to the immunostimulatory substance being bound to the virus-like particle the term "bound" is broader than and includes terms such as "coupled," "fused," "enclosed", "packaged" and "attached." For example, the immunostimulatory substance such as the unmethylated CpG-containing oligonucleotide can be enclosed by the VLP without the existence of an actual binding, neither covalently nor non-covalently, such that the oligonucleotide is held in place by mere "packaging." Coupled: As used herein, the term "coupled" refers to attachment by covalent bonds or by strong non-covalent interactions, typically and preferably to attachment by covalent bonds. Any method normally used by those skilled in the art for the coupling of biologically active materials can be used in the present invention.

Fusion: As used herein, the term "fusion" refers to the combination of amino acid sequences of different origin in one polypeptide chain by in-frame combination of their coding nucleotide sequences. The term "fusion" explicitly encompasses internal fusions, *i.e.,* insertion of sequences of different origin within a polypeptide chain, in addition to fusion to one of its termini.

CpG: As used herein, the term "CpG" refers to an oligonucleotide which contains at least one unmethylated cytosine, guanine dinucleotide sequence (*e.g*. "CpG-oligonucleotides" or DNA containing a cytosine followed by guanosine and linked by a phosphate bond) and stimulates/activates, *e.g*. has a mitogenic effect on, or induces or increases cytokine expression by, a vertebrate bone marrow derived cell. For example, CpGs can be useful in activating B cells, NK cells and antigen-presenting cells, such as dendritic cells, monocytes and macrophages. The CpGs can include nucleotide analogs such as analogs containing phosphorothioester bonds and can be double-stranded or single-stranded. Generally, double-stranded molecules are more stable *in vivo,* while single-stranded molecules have increased immune activity.

Coat protein(s): As used herein, the term "coat protein(s)" refers to the protein(s) of a bacteriophage or a RNA-phage capable of being incorporated within the capsid assembly of the bacteriophage or the RNA-phage. However, when referring to the specific gene product of the coat protein gene of RNA-phages the term "CP" is used. For example, the specific gene product of the coat protein gene of RNA-phage Qβ is referred to as "Qβ CP", whereas the "coat proteins" of bacteriophage Qb comprise the "Qβ CP" as well as the A1 protein. The capsid of Bacteriophage Qβ is composed mainly of the Qβ CP, with a minor content of the A1 protein. Likewise, the VLP Qβ coat protein contains mainly Qβ CP, with a minor content of A1 protein.

Epitope: As used herein, the term "epitope" refers to continuous or discontinuous portions of a polypeptide having antigenic or immunogenic activity in an animal, preferably a mammal, and most preferably in a human. An epitope is recognized by an antibody or a T cell through its T cell receptor in the context of an MHC molecule. An "immunogenic epitope," as used herein, is defined as a portion of a polypeptide that elicits an antibody response or induces a T-cell response in an animal, as determined by any method known in the art. (*See,* for example, Geysen et al., Proc. Natl. Acad Sci. USA 81:3998-4002 (1983)). The term "antigenic epitope," as used herein, is defined as a portion of a protein to which an antibody can immunospecifically bind its antigen as determined by any method well known in the art. Immunospecific binding excludes non-specific binding but does not necessarily exclude cross-reactivity with other antigens. Antigenic epitopes need not necessarily be immunogenic. Antigenic epitopes can also be T-cell epitopes, in which case they can be bound immunospecifically by a T-cell receptor within the context of an MHC molecule.

An epitope can comprise 3 amino acids in a spatial conformation which is unique to the epitope. Generally, an epitope consists of at least about 5 such amino acids, and more usually, consists of at least about 8-10 such amino acids. If the epitope is an organic molecule, it may be as small as Nitrophenyl.

Immune response: As used herein, the term "immune response" refers to a humoral immune response and/or cellular immune response leading to the activation or proliferation of B- and/or T-lymphocytes and/or antigen presenting cells. In some instances, however, the immune responses may be of low intensity and become detectable only when using at least one substance in accordance with the invention. "Immunogenic" refers to an agent used to stimulate the immune system of a living organism, so that one or more functions of the immune system are increased and directed towards the immunogenic agent. An "immunogenic polypeptide" is a polypeptide that elicits a cellular and/or humoral immune response, whether alone or linked to a carrier in the presence or absence of an adjuvant. Preferably, the antigen presenting cell may be activated.

Immunization: As used herein, the terms "immunize" or "immunization" or related terms refer to conferring the ability to mount a substantial immune response (comprising antibodies and/or cellular immunity such as effector CTL) against a target antigen or epitope. These terms do not require that complete immunity be created, but rather that an immune response be produced which is substantially greater than baseline.-For example, a mammal may be considered to be immunized against a target antigen if the cellular and/or humoral immune response to the target antigen occurs following the application of methods of the invention.

Immunostimulatory nucleic acid: As used herein, the term immunostimulatory nucleic acid refers to a nucleic acid capable of inducing and/or enhancing an immune response. Immunostimulatory nucleic acids, as used herein, comprise ribonucleic acids and in particular deoxyribonucleic acids. Preferably, immunostimulatory nucleic acids contain at least one CpG motif e.g. a CG dinucleotide in which the C is unmethylated. The CG dinucleotide can be part of a palindromic sequence or can be encompassed within a non-palindromic sequence. Immunostimulatory nucleic acids not containing CpG motifs as described above encompass, by way of example, nucleic acids lacking CpG dinucleotides, as well as nucleic acids containing CG motifs with a methylated CG dinucleotide. The term "immunostimulatory nucleic acid" as used herein should also refer to nucleic acids that contain modified bases such as 4-bromo-cytosine.

Immunostimulatory substance: As used herein, the term "immunostimulatory substance" refers to a substance capable of inducing and/or enhancing an immune response. Immunostimulatory substances, as used herein, include, but are not limited to, toll-like receptor activing substances and substances inducing cytokine secretion. Toll-like receptor activating substances include, but are not limited to, immunostimulatory nucleic acids, peptideoglycans, lipopolysaccharides, lipoteichonic acids, imidazoquinoline compounds, flagellins, lipoproteins, and immunostimulatory organic substances such as taxol.

Mixed: As used herein, the term "mixed" refers to the combination of two or more substances, ingredients, or elements that are added together, are not chemically combined with each other and are capable of being separated.

Oligonucleotide: As used herein, the terms "oligonucleotide" or "oligomer" refer to a nucleic acid sequence comprising 2 or more nucleotides, generally at least about 6 nucleotides to about 100,000 nucleotides, preferably about 6 to about 2000 nucleotides, and more preferably about 6 to about 300 nucleotides, even more preferably about 20 to about 300 nucleotides, and even more preferably about 20 to about 100 nucleotides. The terms "oligonucleotide" or "oligomer" also refer to a nucleic acid sequence comprising more than 100 to about 2000 nucleotides, preferably more than 100 to about 1000 nucleotides, and more preferably more than 100 to about 500 nucleotides. "Oligonucleotide" also generally refers to any polyribonucleotide or polydeoxribonucleotide, which may be unmodified RNA or DNA or modified RNA or DNA. The modification may comprise the backbone or nucleotide analogues. "Oligonucleotide" includes, without limitation, single- and double-stranded DNA, DNA that is a mixture of single- and double-stranded regions, single- and double-stranded RNA, and RNA that is mixture of single- and double-stranded regions, hybrid molecules comprising DNA and RNA that may be single-stranded or, more typically, double-stranded or a mixture of single- and double-stranded regions. In addition, "oligonucleotide" refers to triple-stranded regions comprising RNA or DNA or both RNA and DNA. Further, an oligonucleotide can be synthetic, genomic or recombinant, *e.g.*, $\lambda$-DNA, cosmid DNA, artificial bacterial chromosome, yeast artificial chromosome and filamentous phage such as M13.

The term "oligonucleotide" also includes DNAs or RNAs containing one or more modified bases and DNAs or RNAs with backbones modified for stability or for other reasons. For example, suitable nucleotide modifications/analogs include peptide nucleic acid, inosin, tritylated bases, phosphorothioates, alkylphosphorothioates, 5-nitroindole deoxyribofuranosyl, 5-methyldeoxycytosine and 5,6-dihydro-5,6-dihydroxydeoxythymidine. A variety of modifications have been made to DNA and RNA; thus, "oligonucleotide" embraces chemically, enzymatically or metabolically modified forms of polynucleotides as typically found in nature, as well as the chemical forms of DNA and RNA characteristic of viruses and cells. Other nucleotide analogs/modifications will be evident to those skilled in the art.

Packaged: The term "packaged" as used herein refers to the state of an immunostimulatory substance, in particular an immunostimulatory nucleic acid in relation to the VLP. The term "packaged" as used herein includes binding that may be covalent, *e.g.,* by chemically coupling, or non-covalent, *e.g.,* ionic interactions, hydrophobic interactions, hydrogen bonds, etc. Covalent bonds can be, for example, ester, ether, phosphoester, amide, peptide, imide, carbon-sulfur bonds, carbon-phosphorus bonds, and the like. The term "packaged" includes terms such as "coupled" and "attached", and in particular, and preferably, the term "packaged" also includes the enclosement, or partial enclosement, of a substance. For example, the immunostimulatory substance such as the unmethylated CpG-containing oligonucleotide can be enclosed by the VLP without the existence of an actual binding, neither covalently nor non-covalently. Therefore, in the preferred meaning, the term "packaged", and hereby in particular, if immunostimulatory nucleic acids are the immunostimulatory substances, the term "packaged" indicates that the nucleic acid in a packaged state is not accessible to DNAse or RNAse hydrolysis. In preferred embodiments, the immunostimulatory nucleic acid is packaged inside the VLP capsids, most preferably in a non-covalent manner.

PCR product: As used herein, the term "PCR product" refers to amplified copies of target DNA sequences that act as starting material for a PCR. Target sequences can include, for example, double-stranded DNA. The source of DNA for a PCR can be complementary DNA, also referred to as "cDNA", which can be the conversion product of mRNA using reverse transcriptase. The source of DNA for a PCR can be total genomic DNA extracted from cells. The source of cells from which DNA can be extracted for a PCR includes, but is not limited to, blood samples; human, animal, or plant tissues; fungi; and bacteria. DNA starting material for a PCR can be unpurified, partially purified, or highly purified. The source of DNA for a PCR can be from cloned inserts in vectors, which includes, but is not limited to, plasmid vectors and bacteriophage vectors. The term "PCR product" is interchangeable with the term "polymerase chain reaction product".

The compositions of the invention can be combined, optionally, with a pharmaceutically-acceptable carrier. The term "pharmaceutically-acceptable carrier" as used herein means one or more compatible solid or liquid fillers, diluents or encapsulating substances which are suitable for administration into a human or other animal. The term "carrier" denotes an organic or inorganic ingredient, natural or synthetic, with which the active ingredient is combined to facilitate the application.

Polypeptide: As used herein, the term "polypeptide" refers to a molecule composed of monomers (amino acids) linearly linked by amide bonds (also known as peptide bonds). It indicates a molecular chain of amino acids and does not refer to a specific length of the product. Thus, peptides, oligopeptides and proteins are included within the definition of polypeptide. This term is also intended to refer to post-expression modifications of the polypeptide, for example, glycosolations, acetylations, phosphorylations, and the like. A recombinant or derived polypeptide is not necessarily translated from a designated nucleic acid sequence. It may also be generated in any manner, including chemical synthesis.

A substance which "enhances" an immune response refers to a substance in which an immune response is observed that is greater or intensified or deviated in any way with the addition of the substance when compared to the same immune response measured without the addition of the substance. For example, the lytic activity of cytotoxic T cells can be measured, *e.g.* using a $^{51}$Cr release assay, with and without the substance. The amount of the substance at which the CTL lytic activity is enhanced as compared to the CTL lytic activity without the substance is said to be an amount sufficient to enhance the immune response of the animal to the antigen. In a preferred embodiment, the immune response in enhanced by a factor of at least about 2, more preferably by a factor of about 3 or more. The amount or type of cytokines secreted may also be altered. Alternatively, the amount of antibodies induced or their subclasses may be altered.

Effective Amount: As used herein, the term "effective amount" refers to an amount necessary or sufficient to realize a desired biologic effect. An effective amount of the composition would be the amount that achieves this selected result, and such an amount could be determined as a matter of routine by a person skilled in the art. For example, an effective amount for treating an immune system deficiency could be that amount necessary to cause activation of the immune system, resulting in the development of an antigen specific immune response upon exposure to antigen. The term is also synonymous with "sufficient amount."

The effective amount for any particular application can vary depending on such factors as the disease or condition being treated, the particular composition being administered, the size of the subject, and/or the severity of the disease or condition. One of ordinary skill in the art can empirically determine the effective amount of a particular composition of the present invention without necessitating undue experimentation.

Treatment: As used herein, the terms "treatment", "treat", "treated" or "treating" refer to prophylaxis and/or therapy. When used with respect to an infectious disease, for example, the term refers to a prophylactic treatment which increases the resistance of a subject to infection with a pathogen or, in other words, decreases the likelihood that the subject will become infected with the pathogen or will show signs of illness attributable to the infection, as well as a treatment after the subject has become infected in order to fight the infection, *e.g.,* reduce or eliminate the infection or prevent it from becoming worse.

Vaccine: As used herein, the term "vaccine" refers to a formulation which contains the composition of the present invention and which is in a form that is capable of being administered to an animal. Typically, the vaccine comprises a conventional saline or buffered aqueous solution medium in which the composition of the present invention is suspended or dissolved. In this form, the composition of the present invention can be used conveniently to prevent, ameliorate, or otherwise treat a condition. Upon introduction into a host, the vaccine is able to provoke an immune response including, but not limited to, the production of antibodies and/or cytokines and/or the activation of cytotoxic T cells, antigen presenting cells, helper T cells, dendritic cells and/or other cellular responses.

Optionally, the vaccine of the present invention additionally includes an adjuvant which can be present in either a minor or major proportion relative to the compound of the present invention. The term "adjuvant" as used herein refers to non-specific stimulators of the immune response or substances that allow generation of a depot in the host which when combined with the vaccine of the present invention provide for an even more enhanced immune response. A variety of adjuvants can be used. Examples include incomplete Freund's adjuvant, aluminum hydroxide and modified muramyldipeptide.

Virus-like particle: As used herein, the term "virus-like particle" (VLP) refers to a structure resembling a virus but which has not been demonstrated to be pathogenic. Typically, a virus-like particle in accordance with the invention does not carry genetic information encoding for the proteins of the virus-like particle. In general, virus-like particles lack the viral genome and, therefore, are noninfectious. Also, virus-like particles can often be produced in large quantities by heterologous expression and can be easily purified. Some virus-like particles may contain nucleic acid distinct from their genome. Typically, a virus-like particle in accordance with the invention is non replicative and noninfectious since it lacks all or part of the viral genome, in particular the replicative and infectious components of the viral genome. A virus-like particle in accordance with the invention may contain nucleic acid distinct from their genome. A typical and preferred embodiment of a virus-like particle in accordance with the present invention is a viral capsid such as the viral capsid of the corresponding virus, bacteriophage, or RNA-phage. The terms "viral capsid" or "capsid", as interchangeably used herein, refer to a macromolecular assembly composed of viral protein subunits. Typically and preferably, the viral protein subunits assemble into a viral capsid and capsid, respectively, having a structure with an inherent repetitive organization, wherein said structure is, typically, spherical or tubular. For example, the capsids of RNA-phages or HBcAg's have a spherical form of icosahedral symmetry. The term "capsid-like structure" as used herein, refers to a macromolecular assembly composed of viral protein subunits ressembling the capsid morphology in the above defined sense but deviating from the typical symmetrical assembly while maintaining a sufficient degree of order and repetitiveness.

VLP of RNA phage coat protein: The capsid structure formed from the self-assembly of 180 subunits of RNA phage coat protein and optionally containing host RNA is referred to as a "VLP of RNA phage coat protein". A specific example is the VLP of Qβ coat protein. In this particular case, the VLP of Qβ coat protein may either be assembled exclusively from Qβ CP subunits (SEQ ID: No 1) generated by expression of a Qβ CP gene containing, for example, a TAA stop codon precluding any expression of the longer A1 protein through suppression, see Kozlovska, T.M., et al., Intervirology 39: 9-15 (1996)), or additionally contain A1 protein subunits (SEQ ID: No 2) in the capsid assembly. The readthrough process has a low efficiency and is leading to an only very low amount A1 protein in the VLPs. An extensive number of examples have been performed with different combinations of ISS packaged and antigen coupled. No differences in the coupling efficiency and the packaging have been observed when VLPs of Qβ coat protein assembled exclusively from Qβ CP subunits or VLPs of Qβ coat protein containing additionally A1 protein subunits in the capsids were used. Furthermore, no difference of the immune response between these Qβ VLP preparations was observed. Therefore, for the sake of clarity the term "Qβ VLP" is used throughout the description of the examples either for VLPs of Qβ coat protein assembled exclusively from Qβ CP subunits or VLPs of Qβ coat protein containing additionally A1 protein subunits in the capsids.

The term "virus particle" as used herein refers to the morphological form of a virus. In some virus types it comprises a genome surrounded by a protein capsid; others have additional structures (*e.g.,* envelopes, tails, etc.).

Non-enveloped viral particles are made up of a proteinaceous capsid that surrounds and protects the viral genome. Enveloped viruses also have a capsid structure surrounding the genetic material of the virus but, in addition, have a lipid bilayer envelope that surrounds the capsid.

In a preferred embodiment of the invention, the VLP's are free of a lipoprotein envelope or a lipoprotein-containing envelope. In a further preferred embodiment, the VLP's are free of an envelope altogether.

One, a, or an: When the terms "one," "a," or "an" are used in this disclosure, they mean "at least one" or "one or more," unless otherwise indicated.

As will be clear to those skilled in the art, certain embodiments of the invention involve the use of recombinant nucleic acid technologies such as cloning, polymerase chain reaction, the purification of DNA and RNA, the expression of recombinant proteins in prokaryotic and eukaryotic cells, etc. Such methodologies are well known to those skilled in the art and can be conveniently found in published laboratory methods manuals (*e.g.,* Sambrook, J. et al., eds., MOLECULAR CLONING, A LABORATORY MANUAL, 2nd. edition, Cold Spring Harbor Laboratory Press, Cold Spring Harbor, N.Y. (1989); Ausubel, F. et al., eds., CURRENT PROTOCOLS IN MOLECULAR BIOLOGY, John H. Wiley & Sons, Inc. (1997)). Fundamental laboratory techniques for working with tissue culture cell lines (Celis, J., ed., CELL BIOLOGY, Academic Press, 2nd edition, (1998)) and antibody-based technologies (Harlow, E. and Lane, D., "Antibodies: A Laboratory Manual," Cold Spring Harbor Laboratory, Cold Spring Harbor, N.Y. (1988); Deutscher, M.P., "Guide to Protein Purification," Meth. Enzymol. 128, Academic Press San Diego (1990); Scopes, R.K., "Protein Purification Principles and Practice," 3rd ed., Springer-Verlag, New York (1994)) are also adequately described in the literature, all of which are incorporated herein by reference.

## 2. Compositions and Methods for Enhancing an Immune Response

The disclosed invention provides compositions and methods for enhancing an immune response against one or more allergens in an animal. Compositions of the invention comprise, or alternatively consist of, a virus-like particle and an immunostimulatory substance being an unmethylated CpG-containing oligonucleotide where the oligonucleotide is packaged within the virus-like particle and the resulting modified virus-like particle is mixed with an allergen, several allergens or an allergen mixture. Furthermore, the invention conveniently enables the practitioner to construct such a composition for various treatment and/or prevention purposes, which include the prevention and/or treatment of allergies or allergy-related diseases such as asthma, for example.

Virus-like particles in the context of the present application refer to structures resembling a virus particle but which are not pathogenic. In general, virus-like particles lack the viral genome and, therefore, are noninfectious. Also, virus-like particles can be produced in large quantities by heterologous expression and can be easily purified.

In a preferred embodiment, the virus-like particle is a recombinant virus-like particle. The skilled artisan can produce VLPs using recombinant DNA technology and virus coding sequences which are readily available to the public. For example, the coding sequence of a virus envelope or core protein can be engineered for expression in a baculovirus expression vector using a commercially available baculovirus vector, under the regulatory control of a virus promoter, with appropriate modifications of the sequence to allow functional linkage of the coding sequence to the regulatory sequence. The coding sequence of a virus envelope or core protein can also be engineered for expression in a bacterial expression vector, for example.

Examples of VLPs include, but are not limited, to, the capsid proteins of Hepatitis B virus (Ulrich, et al., Virus Res. 50:141-182 (1998)), measles virus (Warnes, et al., Gene 160:173-178 (1995)), Sindbis virus, rotavirus (U.S. Patent Nos. 5,071,651 and 5,374,426), foot-and-mouth-disease virus (Twomey, et al., Vaccine 13:1603-1610, (1995)), Norwalk virus (Jiang, X., et al., Science 250:1580-1583 (1990); Matsui, S.M., et al., J. Clin. Invest. 87:1456-1461 (1991)), the retroviral GAG protein (PCT Patent Appl. No. WO 96/30523), the retrotransposon Ty protein pl, the surface protein of Hepatitis B virus (WO 92/11291), human papilloma virus (WO 98/15631), RNA phages, Ty, fr-phage, GA-phage, AP 205-phage and, in particular, Qβ-phage.

As will be readily apparent to those skilled in the art, the VLP of the invention is not limited to any specific form. The particle can be synthesized chemically or through a biological process, which can be natural or non-natural. By way of example, this type of embodiment includes a virus-like particle or a recombinant form thereof.

In a more specific embodiment, the VLP can comprise, or alternatively essentially consist of, or alternatively consist of recombinant polypeptides, or fragments thereof, being selected from recombinant polypeptides of Rotavirus, recombinant polypeptides of Norwalk virus, recombinant polypeptides of Alphavirus, recombinant polypeptides of Foot and Mouth Disease virus, recombinant polypeptides of measles virus, recombinant polypeptides of Sindbis virus, recombinant polypeptides of Polyoma virus, recombinant polypeptides of Retrovirus, recombinant polypeptides of Hepatitis B virus (*e.g.*, a HBcAg), recombinant polypeptides of Tobacco mosaic virus, recombinant polypeptides of Flock House Virus, recombinant polypeptides of human Papillomavirus, recombinant polypeptides of bacteriophages, recombinant polypeptides of RNA phages, recombinant polypeptides of Ty, recombinant polypeptides of fr-phage, recombinant polypeptides of GA-phage, recombinant polypeptides of AP205-phage, and recombinant polypeptides of Qβ-phage. The virus-like particle can further comprise, or alternatively essentially consist of, or alternatively consist of, one or more fragments of such polypeptides, as well as variants of such polypeptides. Variants of polypeptides can share, for example, at least 80%, 85%, 90%, 95%, 97%, or 99% identity at the amino acid level with their wild-type counterparts.

In a preferred embodiment, the virus-like particle comprises, consists essentially of or alternatively consists of recombinant proteins, or fragments thereof, of a RNA-phage. Preferably, the RNA-phage is selected from the group consisting of a) bacteriophage Qβ; b) bacteriophage R17; c) bacteriophage fr; d) bacteriophage GA; e) bacteriophage SP; f) bacteriophage MS2; g) bacteriophage M11; h) bacteriophage MX1; i) bacteriophage NL95; k) bacteriophage f2; 1) bacteriophage PP7; and m) bacteriophage AP205.

In another preferred embodiment of the present invention, the virus-like particle comprises, consists essentially of or alternatively consists of recombinant proteins, or fragments thereof, of the RNA-bacteriophage Qβ, of the RNA-bacteriophage fr, or of the RNA-bacteriophage AP205.

In a further preferred embodiment of the present invention, the recombinant proteins comprise, consist essentially of or alternatively consist of coat proteins of RNA phages.

RNA-phage coat proteins forming capsids or VLP's, or fragments of the bacteriophage coat proteins compatible with self-assembly into a capsid or a VLP, are, therefore, further preferred embodiments of the present invention. Bacteriophage Qβ coat proteins, for example, can be expressed recombinantly in *E. coli.* Further, upon such expression these proteins spontaneously form capsids. Additionally, these capsids form a structure with an inherent repetitive organization.

Specific preferred examples of bacteriophage coat proteins which can be used to prepare compositions of the invention include the coat proteins of RNA bacteriophages such as bacteriophage Qβ (SEQ ID NO:1; PIR Database, Accession No. VCBPQβ referring to Qβ CP and SEQ ID NO: 2; Accession No. AAA16663 referring to Qβ A1 protein), bacteriophage R17 (SEQ ID NO:3; PIR Accession No. VCBPR7), bacteriophage fr (SEQ ID NO:4; PIR Accession No. VCBPFR), bacteriophage GA (SEQ ID NO:5; GenBank Accession No. NP-040754), bacteriophage SP (SEQ ID NO:6; GenBank Accession No. CAA30374 referring to SP CP and SEQ ID NO: 7; Accession No. NP 695026 referring to SP A1 protein), bacteriophage MS2 (SEQ ID NO:8; PIR Accession No. VCBPM2), bacteriophage M11 (SEQ ID NO:9; GenBank Accession No. AAC06250), bacteriophage MX1 (SEQ ID NO:10; GenBank Accession No. AAC14699), bacteriophage NL95 (SEQ ID NO:11; GenBank Accession No. AAC14704), bacteriophage f2 (SEQ ID NO: 12; GenBank Accession No. P03611), bacteriophage PP7 (SEQ ID NO: 13), bacteriophage AP205 (SEQ ID NO: 90). Furthermore, the A1 protein of bacteriophage Qβ (SEQ ID NO: 2) or C-terminal truncated forms missing as much as 100, 150 or 180 amino acids from its C-terminus may be incorporated in a capsid assembly of Qβ coat proteins. Generally, the percentage of A1 protein relative to Qβ CP in the capsid assembly will be limited, in order to ensure capsid formation.

Qβ coat protein has also been found to self-assemble into capsids when expressed in *E. coli* (Kozlovska TM. et al., GENE 137: 133-137 (1993)). The capsid contains 180 copies of the coat protein, which are linked in covalent pentamers and hexamers by disulfide bridges (Golmohammadi, R. et al., Structure 4: 543-5554 (1996)) leading to a remarkable stability of the capsid of Qβ coat protein. Capsids or VLP's made from -recombinant Qβ coat protein may contain, however, subunits not linked via disulfide links to other subunits within the capsid, or incompletely linked. Thus, upon loading recombinant Qβ capsid on non-reducing SDS-PAGE, bands corresponding to monomeric Qβ coat protein as well as bands corresponding to the hexamer or pentamer of Qβ coat protein are visible. Incompletely disulfide-linked subunits could appear as dimer, trimer or even tetramer band in non-reducing SDS-PAGE. Qβ capsid protein also shows unusual resistance to organic solvents and denaturing agents. Surprisingly, we have observed that DMSO and acetonitrile concentrations as high as 30%, and Guanidinium concentrations as high as 1 M do not affect the stability of the capsid. The high stability of the capsid of Qβ coat protein is an important feature pertaining to its use for immunization and vaccination of mammals and humans in particular.

Upon expression in *E. coli,* the N-terminal methionine of Qβ coat protein is usually removed, as we observed by N-terminal Edman sequencing as described in Stoll, E., et al., J Biol. Chem 252:990-993 (1977). VLP composed from Qβ coat proteins where the N-terminal methionine has not been removed, or VLPs comprising a mixture of Qβ coat proteins where the N-terminal methionine is either cleaved or present are also within the scope of the present invention.

Further preferred virus-like particles of RNA-phages, in particular of Qβ, in accordance of this invention are disclosed in WO 02/056905, the disclosure of which is herewith incorporated by reference in its entirety.

Further RNA phage coat proteins have also been shown to self-assemble upon expression in a bacterial host (Kastelein, RA. et al., Gene 23: 245-254 (1983), Kozlovskaya, TM. et al., Dokl. Akad. Nauh SSSR 287: 452-455 (1986), Adhin, MR. et al., Virology 170: 238-242 (1989), Ni, CZ., et al., Protein Sci. 5: 2485-2493 (1996), Priano, C. et al., J. Mol. Biol. 249: 283-297 (1995)). The Qβ phage capsid contains, in addition to the coat protein, the so called read-through protein A1 and the maturation protein A2. A1 is generated by suppression at the UGA stop codon and has a length of 329 aa. The capsid of phage Qβ recombinant coat protein used in the invention is devoid of the A2 lysis protein, and contains RNA from the host. The coat protein of RNA phages is an RNA binding protein, and interacts with the stem loop of the ribosomal binding site of the replicase gene acting as a translational repressor during the life cycle of the virus. The sequence and structural elements of the interaction are known (Witherell, GW. & Uhlenbeck, OC. Biochemistry 28: 71-76 (1989); Lim F. et al., J. Biol. Chem. 271: 31839-31845 (1996)). The stem loop and RNA in general are known to be involved in the virus assembly (Golmohammadi, R. et al., Structure 4: 543-5554 (1996)).

In a further preferred embodiment of the present invention, the virus-like particle comprises, or alternatively consists essentially of or alternatively consists of recombinant proteins, or fragments thereof of a RNA-phage, wherein the recombinant proteins comprise, consist essentially of or alternatively consist of mutant coat proteins of RNA phages. In another preferred embodiment, the mutant coat proteins have been modified by removal of at least one lysine residue by way of substitution, or by addition of at least one lysine residue by way of substitution. Alternatively, the mutant coat proteins have been modified by deletion of at least one lysine residue, or by addition of at least one lysine residue by way of insertion.

In another preferred embodiment, the virus-like particle comprises, consists essentially of, or alternatively consists of recombinant proteins, or fragments thereof, of the RNA-bacteriophage Qβ, wherein the recombinant proteins comprise, consist essentially of, or alternatively consist of coat proteins having an amino acid sequence of SEQ ID NO: 1, or a mixture of coat proteins having amino acid sequences of SEQ ID NO:1 and of SEQ ID NO: 2 or mutants of SEQ ID NO: 2 and wherein the N-terminal methionine is preferably cleaved.

In a further preferred embodiment of the present invention, the virus-like particle comprises, consists essentially of or alternatively consists of recombinant proteins of Qβ, or fragments thereof, wherein the recombinant proteins comprise, consist essentially of or alternatively consist of mutant Qβ coat proteins. In another preferred embodiment, these mutant coat proteins have been modified by removal of at least one lysine residue by way of substitution, or by addition of at least one lysine residue by way of substitution. Alternatively, these mutant coat proteins have been modified by deletion of at least one lysine residue, or by addition of at least one lysine residue by way of insertion.

Four lysine residues are exposed on the surface of the capsid of Qβ coat protein. Qβ mutants, for which exposed lysine residues are replaced by arginines can also be used for the present invention. The following Qβ coat protein mutants and mutant Qβ VLP's can, thus, be used in the practice of the invention: "Qβ-240" (Lys13-Arg; SEQ ID NO: 14), "Qβ-243" (Asn 10-Lys; SEQ ID NO:15), "Qβ-250" (Lys 2-Arg, Lys13-Arg; SEQ ID NO:16), "Qβ-251" (SEQ ID NO: 17) and "Qβ-259" (Lys 2-Arg, Lys16-Arg; SEQ ID NO:18). Thus, in further preferred embodiment of the present invention, the virus-like particle comprises, consists essentially of or alternatively consists of recombinant proteins of mutant Qβ coat proteins, which comprise proteins having an amino acid sequence selected from the group of a) the amino acid sequence of SEQ ID NO:14; b) the amino acid sequence of SEQ ID NO:15; c) the amino acid sequence of SEQ ID NO: 16; d) the amino acid sequence of SEQ ID NO:17; and e) the amino acid sequence of SEQ ID NO:18. The construction, expression and purification of the above indicated Qβ coat proteins, mutant Qβ coat protein VLP's and capsids, respectively, are described in WO 02/056905. In particular is hereby referred to Example 18 of above mentioned application.

In a further preferred embodiment of the present invention, the virus-like particle comprises, consists essentially of or alternatively consists of recombinant proteins of Qβ, or fragments thereof, wherein the recombinant proteins comprise, consist essentially of or alternatively consist of a mixture of either one of the foregoing Qβ mutants and the corresponding A1 protein.

In a further preferred embodiment, the virus-like particle comprises, or alternatively essentially consists of, or alternatively consists of recombinant proteins, or fragments thereof, of RNA phage AP205.

The AP205 genome consists of a maturation protein, a coat protein, a replicase and two open reading frames not present in related phages; a lysis gene and an open reading frame playing a role in the translation of the maturation gene (Klovins, J., et al., J. Gen. Virol. 83: 1523-33 (2002)). AP205 coat protein can be expressed from plasmid pAP283-58 (SEQ ID NO: 91), which is a derivative of pQb10 (Kozlovska, T. M.. et al., Gene 137:133-37 (1993)), and which contains an AP205 ribosomal binding site. Alternatively, AP205 coat protein may be cloned into pQb185, downstream of the ribosomal binding site present in the vector. Both approaches lead to expression of the protein and formation of capsids. Vectors pQb10 and pQb185 are vectors derived from pGEM vector, and expression of the cloned genes in these vectors is controlled by the *trp* promoter (Kozlovska, T. M. et al., Gene 137:133-37 (1993)). Plasmid pAP283-58 (SEQ ID NO: 91) comprises a putative AP205 ribosomal binding site in the following sequence, which is downstream of the XbaI site, and immediately upstream of the ATG start codon of the AP205 coat protein: *tctaga*ATTTTCTGCGCACCCATCCCG-GGTGGCGCCCAAAGT<u>GAGGAA</u>AATC AC*atg* (bases 77-133 of SEQ ID NO: 91). The vector pQb185 comprises a Shine Delagamo sequence downstream from the XbaI site and upstream of the start codon (*tctaga*TTAACCCAACGCGTAGGAG TCAGGCC*atg*, (SEQ ID NO: 92), Shine Delagarno sequence underlined).

In a further preferred embodiment of the present invention, the virus-like particle comprises, or alternatively essentially consists of, or alternatively consists of recombinant coat proteins, or fragments thereof, of the RNA-phage AP205.

This preferred embodiment of the present invention, thus, comprises AP205 coat proteins that form capsids. Such proteins are recombinantly expressed, or prepared from natural sources. AP205 coat proteins produced in bacteria spontaneously form capsids, as evidenced by Electron Microscopy (EM) and immunodiffusion. The structural properties of the capsid formed by the AP205 coat protein (SEQ ID NO: 90) and those formed by the coat protein of the AP205 RNA phage are nearly indistinguishable when seen in EM. AP205 VLPs are highly immunogenic, and can be linked with antigens and/or antigenic determinants to generate constructs displaying the antigens and/or antigenic determinants oriented in a repetitive manner. High titers are elicited against the so displayed antigens showing that bound antigens and/or antigenic determinants are accessible for interacting with antibody molecules and are immunogenic.

In a further preferred embodiment of the present invention, the virus-like particle comprises, or alternatively essentially consists of, or alternatively consists of recombinant mutant coat proteins, or fragments thereof, of the RNA-phage AP205. Assembly-competent mutant forms of AP205 VLPs, including AP205 coat protein with the subsitution of proline at amino acid 5 to threonine (SEQ ID NO: 93), may also be used in the practice of the invention and leads to a further preferred embodiment of the invention. These VLPs, AP205 VLPs derived from natural sources, or AP205 viral particles, may be bound to antigens to produce ordered repetitive arrays of the antigens in accordance with the present invention.

AP205 P5-T mutant coat protein can be expressed from plasmid pAP281-32 (SEQ ID No. 94), which is derived directly from pQb185, and which contains the mutant AP205 coat protein gene instead of the Qβ coat protein gene. Vectors for expression of the AP205 coat protein are transfected into *E. coli* for expression of the AP205 coat protein.

Methods for expression of the coat protein and the mutant coat protein, respectively, leading to self-assembly into VLPs are described in Examples 16 and 17. Suitable *E. coli* strains include, but are not limited to, *E. coli* K802, JM 109, RR1. Suitable vectors and strains and combinations thereof can be identified by testing expression of the coat protein and mutant coat protein, respectively, by SDS-PAGE and capsid formation and assembly by optionally first purifying the capsids by gel filtration and subsequently testing them in an immunodiffusion assay (Ouchterlony test) or Electron Microscopy (Kozlovska, T. M., et al., Gene 137:133-37 (1993)).

AP205 coat proteins expressed from the vectors pAP283-58 and pAP281-32 may be devoid of the initial Methionine amino-acid, due to processing in the cytoplasm of *E. coli.* Cleaved, uncleaved forms of AP205 VLP, or mixtures thereof are further preferred embodiments of the invention.

In a further preferred embodiment of the present invention, the virus-like particle comprises, or alternatively essentially consists of, or alternatively consists of a mixture of recombinant coat proteins, or fragments thereof, of the RNA-phage AP205 and of recombinant mutant coat proteins, or fragments thereof, of the RNA-phage AP205.

In a further preferred embodiment of the present invention, the virus-like particle comprises, or alternatively essentially consists of, or alternatively consists of fragments of recombinant coat proteins or recombinant mutant coat proteins of the RNA-phage AP205.

Recombinant AP205 coat protein fragments capable of assembling into a VLP and a capsid, respectively are also useful in the practice of the invention. These fragments may be generated by deletion, either internally or at the termini of the coat protein and mutant coat protein, respectively. Insertions in the coat protein and mutant coat protein sequence or fusions of antigen sequences to the coat protein and mutant coat protein sequence, and compatible with assembly into a VLP, are further embodiments of the invention and lead to chimeric AP205 coat proteins, and particles, respectively. The outcome of insertions, deletions and fusions to the coat protein sequence and whether it is compatible with assembly into a VLP can be determined by electron microscopy.

The particles formed by the AP205 coat protein, coat protein fragments and chimeric coat proteins described above, can be isolated in pure form by a combination of fractionation steps by precipitation and of purification steps by gel filtration using *e.g*. Sepharose CL-4B, Sepharose CL-2B, Sepharose CL-6B columns and combinations thereof. Other methods of isolating virus-like particles are known in the art, and may be used to isolate the virus-like particles (VLPs) of bacteriophage AP205. For example, the use of ultracentrifugation to isolate VLPs of the yeast retrotransposon Ty is described in U.S. Patent No. 4,918,166, which is incorporated by reference herein in its entirety.

The crystal structure of several RNA bacteriophages has been determined (Golmohammadi, R. et al., Structure 4:543-554 (1996)). Using such information, one skilled in the art could readily identify surface exposed residues and modify bacteriophage coat proteins such that one or more reactive amino acid residues can be inserted. Thus, one skilled in the art could readily generate and identify modified forms of bacteriophage coat proteins which can be used for the present invention. Thus, variants of proteins which form capsids or capsid-like structures (*e.g*., coat proteins of bacteriophage Qβ, bacteriophage R17, bacteriophage fr, bacteriophage GA, bacteriophage SP, bacteriophage MS2, and bacteriophage AP205) can also be used to prepare compositions of the present invention.

Although the sequence of the variants proteins discussed above will differ from their wild-type counterparts, these variant proteins will generally retain the ability to form capsids or capsid-like structures. Thus, the invention further includes compositions and vaccine compositions, respectively, which further include variants of proteins which form capsids or capsid-like structures, as well as methods for preparing such compositions and vaccine compositions, respectively, individual protein subunits used to prepare such compositions, and nucleic acid molecules which encode these protein subunits. Thus, included within the scope of the invention are variant forms of wild-type proteins which form capsids or capsid-like structures and retain the ability to associate and form capsids or capsid-like structures.

As a result, the invention further includes compositions and vaccine compositions, respectively, comprising proteins, which comprise, or alternatively consist essentially of, or alternatively consist of amino acid sequences which are at least 80%, 85%, 90%, 95%, 9.7%, or 99% identical to wild-type proteins which form ordered arrays and having an inherent repetitive structure, respectively. In many instances, these proteins will be processed to remove signal peptides (*e.g.*, heterologous signal peptides).

In particular embodiments, the invention further includes compositions comprising proteins, which comprise, or alternatively consist essentially of, or alternatively consist of amino acid sequences which are at least 80%, 85%, 90%, 95%, 97%, or 99% identical to any of the amino acid sequences shown in SEQ ID NOs:1-11.

Proteins suitable for use in the present invention also include C-terminal truncation mutants of proteins which form capsids or capsid-like structures, as well as other ordered arrays. Specific examples of such truncation mutants include proteins having an amino acid sequence shown in any of SEQ ID NOs:1-11 where 1, 2, 5, 7, 9, 10, 12, 14, 15, or 17 amino acids have been removed from the C-terminus. Typically, theses C-terminal truncation mutants will retain the ability to form capsids or capsid-like structures.

Further proteins suitable for use in the present invention also include N-terminal truncation mutants of proteins which form capsids or capsid-like structures. Specific examples of such truncation mutants include proteins having an amino acid sequence shown in any of SEQ ID NOs:1-11 where 1, 2, 5, 7, 9, 10, 12, 14, 15, or 17 amino acids have been removed from the N-terminus. Typically, these N-terminal truncation mutants will retain the ability to form capsids or capsid-like structures.

Additional proteins suitable for use in the present invention include N- and C-terminal truncation mutants which form capsids or capsid-like structures. Suitable truncation mutants include proteins having an amino acid sequence shown in any of SEQ ID NOs:1-11 where 1, 2, 5, 7, 9, 10, 12, 14, 15, or 17 amino acids have been removed from the N-terminus and 1,2, 5, 7, 9,10,12,14, 15, or 17 amino acids have been removed from the C-terminus. Typically, these N-terminal and C-terminal truncation mutants will retain the ability to form capsids or capsid-like structures. The invention further includes compositions comprising proteins which comprise, or alternatively consist essentially of, or alternatively consist of, amino acid sequences which are at least 80%, 85%, 90%, 95%, 97%, or 99% identical to the above described truncation mutants.

The invention thus includes compositions and vaccine compositions prepared from proteins which form ordered arrays, methods for preparing these compositions from individual protein subunits and VLP's or capsids, methods for preparing these individual protein subunits, nucleic acid molecules which encode these subunits, and methods for vaccinating and/or eliciting immunological responses in individuals using these compositions of the present invention.

Fragments of VLPs which retain the ability to induce an immune response can comprise, or alternatively consist of, polypeptides which are about 15, 20, 25, 30, 35, 40, 45, 50, 55, 60, 65, 70, 75, 80, 85, 90, 95, 100, 110, 120, 130, 140, 150, 160, 170, 180, 190, 200, 250, 300, 350, 400, 450 or 500 amino acids in length, but will obviously depend on the length of the sequence of the subunit composing the VLP. Examples of such fragments include fragments of proteins discussed herein which are suitable for the preparation of the immune response enhancing composition.

In another preferred embodiment of the invention, the VLP's are free of a lipoprotein envelope or a lipoprotein-containing envelope. In a further preferred embodiment, the VLP's are free of an envelope altogether.

The lack of a lipoprotein envelope or lipoprotein-containing envelope and, in particular, the complete lack of an envelope leads to a more defined virus-like particle in its structure and composition. Such more defined virus-like particles, therefore, may minimize side-effects. Moreover, the lack of a lipoprotein-containing envelope or, in particular, the complete lack of an envelope avoids or minimizes incorporation of potentially toxic molecules and pyrogens within the virus-like particle.

As previously stated, the invention includes virus-like particles or recombinant forms thereof. Skilled artisans have the knowledge to produce such particles and mix antigens thereto. By way of providing other examples, the invention provides herein for the production of Hepatitis B virus-like particles as virus-like particles (Example 1).

In one embodiment, the particles used in compositions of the invention are composed of a Hepatitis B capsid (core) protein (HBcAg) or a fragment of a HBcAg. In a further embodiment, the particles used in compositions of the invention are composed of a Hepatitis B capsid (core) protein (HBcAg) or a fragment of a HBcAg protein, which has been modified to either eliminate or reduce the number of free cysteine residues. Zhou et al. (J. Virol. 66:5393-5398 (1992)) demonstrated that HBcAgs which have been modified to remove the naturally resident cysteine residues retain the ability to associate and form multimeric structures. Thus, core particles suitable for use in compositions of the invention include those comprising modified HBcAgs, or fragments thereof, in which one or more of the naturally resident cysteine residues have been either deleted or substituted with another amino acid residue (*e.g.*, a serine residue).

The HBcAg is a protein generated by the processing of a Hepatitis B core antigen precursor protein. A number of isotypes of the HBcAg have been identified and their amino acids sequences are readily available to those skilled in the art. For example, the HBcAg protein having the amino acid sequence shown in SEQ ID NO: 71 is 183 amino acids in length and is generated by the processing of a 212 amino acid Hepatitis B core antigen precursor protein. This processing results in the removal of 29 amino acids from the N-terminus of the Hepatitis B core antigen precursor protein. Similarly, the HBcAg protein that is 185 amino acids in length is generated by the processing of a 214 amino acid Hepatitis B core antigen precursor protein.

In most instances, compositions and vaccine compositions, respectively, of the invention will be prepared using the processed form of a HBcAg (*i.e.*, a HBcAg from which the N-terminal leader sequence of the Hepatitis B core antigen precursor protein have been removed).

Further, when HBcAgs are produced under conditions where processing will not occur, the HBcAgs will generally be expressed in "processed" form. For example, when an *E. coli* expression system directing expression of the protein to the cytoplasm is used to produce HBcAgs of the invention, these proteins will generally be expressed such that the N-terminal leader sequence of the Hepatitis B core antigen precursor protein is not present.

The preparation of Hepatitis B virus-like particles, which can be used for the present invention, is disclosed, for example, in WO 00/32227, and hereby in particular in Examples 17 to 19 and 21 to 24, as well as in WO 01/85208, and hereby in particular in Examples 17 to 19, 21 to 24, 31 and 41, and in WO 02/056905. For the latter application, it is in particular referred to Example 23, 24, 31 and 51. All three documents are explicitly incorporated herein by reference.

The present invention also includes HBcAg variants which have been modified to delete or substitute one or more additional cysteine residues. Thus, the vaccine compositions of the invention include compositions comprising HBcAgs in which cysteine residues not present in the amino acid sequence shown in SEQ ID NO: 71 have been deleted.

It is well known in the art that free cysteine residues can be involved in a number of chemical side reactions. These side reactions include disulfide exchanges, reaction with chemical substances or metabolites that are, for example; injected or formed in a combination therapy with other substances, or direct oxidation and reaction with nucleotides upon exposure to UV light. Toxic adducts could thus be generated, especially considering the fact that HBcAgs have a strong tendency to bind nucleic acids. The toxic adducts would thus be distributed between a multiplicity of species, which individually may each be present at low concentration, but reach toxic levels when together.

In view of the above, one advantage to the use of HBcAgs in vaccine compositions which have been modified to remove naturally resident cysteine residues is that sites to which toxic species can bind when antigens or antigenic determinants are attached would be reduced in number or eliminated altogether.

A number of naturally occurring HBcAg variants suitable for use in the practice of the present invention have been identified. Yuan *et al.,* (*J. Virol. 73*:10122-10128 (1999)), for example, describe variants in which the isoleucine residue at position corresponding to position 97 in SEQ ID NO:19 is replaced with either a leucine residue or a phenylalanine residue. The amino acid sequences of a number of HBcAg variants, as well as several Hepatitis B core antigen precursor variants, are disclosed in GenBank reports AAF121240 (SEQ ID NO:20), AF121239 (SEQ ID NO:21), X85297 (SEQ ID NO:22), X02496 (SEQ ID NO:23), X85305 (SEQ ID NO:24), X85303 (SEQ ID NO:25), AF151735 (SEQ ID NO:26), X85259 (SEQ ID NO:27), X85286 (SEQ ID NO:28), X85260 (SEQ ID NO:29), X85317 (SEQ ID NO:30), X85298 (SEQ ID NO:31), AF043593 (SEQ ID NO:32), M20706 (SEQ ID NO:33), X85295 (SEQ ID NO:34), X80925 (SEQ ID NO:35), X85284 (SEQ ID NO:36), X85275 (SEQ ID NO:37), X72702 (SEQ ID NO:38), X85291 (SEQ ID NO:39), X65258 (SEQ ID NO:40), X85302 (SEQ ID NO:41), M32138 (SEQ ID NO:42), X85293 (SEQ ID NO:43), X85315 (SEQ ID NO:44), U95551 (SEQ ID NO:45), X85256 (SEQ ID NO:46), X85316 (SEQ ID NO:47), X85296 (SEQ ID NO:48), AB033559 (SEQ ID NO:49), X59795 (SEQ ID NO:50), X85299 (SEQ ID NO:51), X85307 (SEQ ID NO:52), X65257 (SEQ ID NO:53), X85311 (SEQ ID NO:54), X85301 (SEQ ID NO:55), X85314 (SEQ ID NO:56), X85287 (SEQ ID NO:57), X85272 (SEQ ID NO:58), X85319 (SEQ ID NO:59), AB010289 (SEQ ID NO:60), X85285 (SEQ ID NO:61), AB010289 (SEQ ID NO:62), AF121242 (SEQ ID NO:63), M90520 (SEQ ID NO:64), P03153 (SEQ ID NO:65), AF110999 (SEQ ID NO:66), and M95589 (SEQ ID NO:67), the disclosures of each of which are incorporated herein by reference. These HBcAg variants differ in amino acid sequence at a number of positions, including amino acid residues which corresponds to the amino acid residues located at positions 12, 13, 21, 22, 24, 29, 32, 33, 35, 38, 40, 42, 44, 45, 49, 51, 57, 58, 59, 64, 66, 67, 69, 74, 77, 80, 81, 87, 92, 93, 97, 98, 100, 103, 105, 106, 109, 113, 116, 121, 126, 130, 133, 135, 141, 147, 149, 157, 176, 178, 182 and 183 in SEQ ID NO:68. Further HBcAg variants suitable for use in the compositions of the invention, and which may be further modified according to the disclosure of this specification are described in WO 01/98333, WO 00/177158 and WO 00/214478. HbcAgs suitable for use in the present invention can be derived from any organism so long as they are able to enclose or to be coupled or otherwise attached to an unmethylated CpG-containing oligonucleotide and induce an immune response.

As noted above, generally processed HBcAgs (*i.e.*, those which lack leader sequences) will be used in the compositions and vaccine compositions, respectively, of the invention. The present invention includes vaccine compositions, as well as methods for using these compositions, which employ the above described variant HBcAgs.

Further included within the scope of the invention are additional HBcAg variants which are capable of associating to form dimeric or multimeric structures. Thus, the invention further includes compositions and vaccine compositions, respectively, comprising HBcAg polypeptides comprising, or alternatively consisting of, amino acid sequences which are at least 80%, 85%, 90%, 95%, 97%, or 99% identical to any of the wild-type amino acid sequences, and forms of these proteins which have been processed, where appropriate, to remove the N-terminal leader sequence.

Whether the amino acid sequence of a polypeptide has an amino acid sequence that is at least 80%, 85%, 90%, 95%, 97% or 99% identical to one of the above wild-type amino acid sequences, or a subportion thereof, can be determined conventionally using known computer programs such the Bestfit program. When using Bestfit or any other sequence alignment program to determine whether a particular sequence is, for instance, 95% identical to a reference amino acid sequence, the parameters are set such that the percentage of identity is calculated over the full length of the reference amino acid sequence and that gaps in homology of up to 5% of the total number of amino acid residues in the reference sequence are allowed.

The HBcAg variants and precursors having the amino acid sequences set out in SEQ ID NOs: 20-63 and 64-67 are relatively similar to each other. Thus, reference to an amino acid residue of a HBcAg variant located at a position which corresponds to a particular position in SEQ ID NO:68, refers to the amino acid residue which is present at that position in the amino acid sequence shown in SEQ ID NO:68. The homology between these HBcAg variants is for the most part high enough among Hepatitis B viruses that infect mammals so that one skilled in the art would have little difficulty reviewing both the amino acid sequence shown in SEQ ID NO:68 and that of a particular HBcAg variant and identifying "corresponding" amino acid residues. For example, the HBcAg amino acid sequence shown in SEQ ID NO: 64, which shows the amino acid sequence of a HBcAg derived from a virus which infect woodchucks, has enough homology to the HBcAg having the amino acid sequence shown in SEQ ID NO:68 that it is readily apparent that a three amino acid residue insert is present in SEQ ID NO:64 between amino acid residues 155 and 156 of SEQ ID NO:68.

The invention also includes vaccine compositions which comprise HBcAg variants of Hepatitis B viruses which infect birds, as wells as vaccine compositions which comprise fragments of these HBcAg variants. For these HBcAg variants one, two, three or more of the cysteine residues naturally present in these polypeptides could be either substituted with another amino acid-residue or deleted-prior to their inclusion in vaccine compositions of the invention.

As discussed above, the elimination of free cysteine residues reduces the number of sites where toxic components can bind to the HBcAg, and also eliminates sites where cross-linking of lysine and cysteine residues of the same or of neighboring HBcAg molecules can occur. Therefore, in another embodiment of the present invention, one or more cysteine residues of the Hepatitis B virus capsid protein have been either deleted or substituted with another amino acid residue.

In other embodiments, compositions and vaccine compositions, respectively, of the invention will contain HBcAgs from which the C-terminal region (*e.g.*, amino acid residues 145-185 or 150-185 of SEQ ID NO:68) has been removed. Thus, additional modified HBcAgs suitable for use in the practice of the present invention include C-terminal truncation mutants. Suitable truncation mutants include HBcAgs where 1, 5, 10, 15, 20, 25, 30, 34, 35, amino acids have been removed from the C-terminus. HBcAgs suitable for use in the practice of the present invention also include N-terminal truncation mutants. Suitable truncation mutants include modified HBcAgs where 1, 2, 5, 7, 9, 10, 12, 14, 15, or 17 amino acids have been removed from the N-terminus.

Further HBcAgs suitable for use in the practice of the present invention include N- and C-terminal truncation mutants. Suitable truncation mutants include HBcAgs where 1, 2, 5, 7, 9, 10, 12, 14, 15, and 17 amino acids have been removed from the N-terminus and 1, 5, 10, 15, 20, 25, 30, 34, 35 amino acids have been removed from the C-terminus as long as truncation of the C terminus is compatible with binding of CpG-containing oligonucleotides.

The invention further includes vaccine compositions comprising HBcAg polypeptides comprising, or alternatively consisting of, amino acid sequences which are at least 80%, 85%, 90%, 95%, 97%, or 99% identical to the above described truncation mutants.

In certain embodiments of the invention, a lysine residue is introduced into a HBcAg polypeptide, to mediate the binding of the antigen or antigenic determinant to the VLP of HBcAg. In preferred embodiments, compositions of the invention are prepared using a HBcAg comprising, or alternatively consisting of, amino acids 1-144, or 1-149, or 1-185 of SEQ ID NO:68, which is modified so that the amino acids corresponding to positions 79 and 80 are replaced with a peptide having the amino acid sequence of Gly-Gly-Lys-Gly-Gly (SEQ ID NO:95), resulting in the HBcAg variant having the amino acid sequence of SEQ ID NO: 96. In further preferred embodiments, the cysteine residues at positions 48 and 107 of SEQ ID NO:68 are mutated to serine (SEQ ID NO: 97). The invention further includes compositions comprising the corresponding polypeptides having amino acid sequences shown in any of SEQ ID NOs:20-67, which also have above noted amino acid alterations. Further included within the scope of the invention are additional HBcAg variants which are capable of associating to form a capsid or VLP and have the above noted amino acid alterations. Thus, the invention further includes compositions comprising HBcAg polypeptides which comprise, or alternatively consist of, amino acid sequences which are at least 80%, 85%, 90%, 95%, 97% or 99% identical to any of the wild-type amino acid sequences, and forms of these proteins which have been processed, where appropriate, to remove the N-terminal leader sequence and modified with above noted alterations.

Compositions of the invention may comprise mixtures of different HBcAgs. Thus, these compositions may be composed of HBcAgs which differ in amino acid sequence. For example, compositions could be prepared comprising a "wild-type" HBcAg and a modified HBcAg in which one or more amino acid residues have been altered (*e.g.*, deleted, inserted or substituted). Further, preferred vaccine compositions of the invention are those which present highly ordered and repetitive antigen arrays. In one aspect of the invention a virus-like particle, to which an unmethylated CpG-containing oligonucleotide is bound, is mixed with antigen/immunogen against which an enhanced immune response is desired. In some instances, a single antigen will be mixed with the so modified virus-like particle. In other instances, the so modified VLPs will be mixed with several antigens or even complex antigen mixtures. The antigens can be produced recombinantly or be extracted from natural sources, which include but are not limited to pollen, dust, fungi, insects, food, mammalian epidermals, feathers, bees, tumors, pathogens and feathers.

As previously disclosed, the invention is based on the surprising finding that modified VLP's, i.e. VLP's to which immunostimulatory substances, preferably immunostimulatory nucleic acids and even more preferably DNA oligonucleotides or alternatively poly (I:C) are bound, and preferably to which immunostimulatory substances, preferably immunostimulatory nucleic acids and even more preferably DNA oligonucleotides or alternatively poly (I:C) are bound to leading to packaged VLPs, can enhance B and T cell responses against antigens solely through mixing the so modified VLPs with antigens. Surprisingly, no covalent linkage or coupling of the antigen to the VLP is required. In addition, the T cell responses against both the VLPs and antigens are especially directed to the Th1 type. Furthermore, the packaged nucleic acids and CpGs, respectively, are protected from degradation, *i.e.*, they are more stable. Moreover, non-specific activation of cells from the immune system is dramatically reduced.

The innate immune system has the capacity to recognize invariant molecular pattern shared by microbial pathogens. Recent studies have revealed that this recognition is a crucial step in inducing effective immune responses. The main mechanism by which microbial products augment immune responses is to stimulate APC, expecially dendritic cells to produce proinflammatory cytokines and to express high levels costimulatory molecules for T cells. These activated dendritic cells subsequently initiate primary T cell responses and dictate the type of T cell-mediated effector function.

Two classes of nucleic acids, namely 1) bacterial DNA that contains immunostimulatory sequences, in particular unmethylated CpG dinucleotides within specific flanking bases (referred to as CpG motifs) and 2) double-stranded RNA synthesized by various types of viruses represent important members of the microbial components that enhance immune responses. Synthetic double stranded (ds) RNA such as polyinosinic-polycytidylic acid (poly I:C) are capable of inducing dendritic cells to produce proinflammatory cytokines and to express high levels of costimulatory molecules.

A series of studies by Tokunaga and Yamamoto et al. has shown that bacterial DNA or synthetic oligodeoxynucleotides induce human PBMC and mouse spleen cells to produce type I interferon (IFN) (reviewed in Yamamoto et al., Springer Semin Immunopathol. 22:11-19). Poly (I:C) was originally synthesized as a potent inducer of type I IFN but also induces other cytokines such as IL-12.

Ribonucleic acid encompass polyinosinic-polycytidylic acid double-stranded RNA (poly I:C). Ribonucleic acids and modifications thereof as well as methods for their production have been described by Levy, H.B (Methods Enzymol. 1981, 78:242-251), DeClercq, E (Methods Enzymol. 1981,78:227-236) and Torrence, P.F. (Methods Enzymol 1981;78: 326-331) and references therein. Ribonucleic acids comprise polynucleotides of inosinic acid and cytidiylic acid such poly (IC) of which two strands forms double stranded RNA. Ribonucleic acids can be isolated from organisms. Ribonucleic acids also encompass further synthetic ribonucleic acids, in particular synthetic poly (I:C) oligonucleotides that have been rendered nuclease resistant by modification of the phosphodiester backbone, in particular by phosphorothioate modifications. In a further embodiment the ribose backbone of poly (I:C) is replaced by a deoxyribose. Those skilled in the art know procedures how to synthesize synthetic oligonucleotides.

Molecules that active toll-like receptors (TLR) are disclosed. Ten human toll-like receptors are known uptodate. They are activated by a variety of ligands. TLR2 is activated by peptidoglycans, lipoproteins, lipopolysacchrides, lipoteichonic acid and Zymosan, and macrophage-activating lipopeptide MALP-2; TLR3 is activated by double-stranded RNA such as poly (I:C); TLR4 is activated by lipopolysaccharide, lipoteichoic acids and taxol and heat-shock proteins such as heat shock protein HSP-60 and Gp96; TLR5 is activated by bacterial flagella, especially the flagellin protein; TLR6 is activated by peptidoglycans, TLR7 is activated by imiquimoid and imidazoquinoline compounds, such as R-848, loxoribine and bropirimine and TLR9 is activated by bacterial DNA, in particular CpG-oligonucleotides. Ligands for TLR1, TLR8 and TLR10 are not known so far. However, recent reports indicate that same receptors can react with different ligands and that further receptors are present. The above list of ligands is not exhaustive and further ligands are within the knowledge of the person skilled in the art.

In general, the unmethylated CpG-containing oligonucleotide comprises the sequence:

$$5'\ X_1X_2CGX_3X_4\ 3'$$

wherein $X_1$, $X_2$, $X_3$ and $X_4$ are any nucleotide. In addition, the oligonucleotide can comprise about 6 to about 100,000 nucleotides, preferably about 6 to about 2000 nucleotides, more preferably about 20 to about 2000 nucleotides, and even more preferably comprises about 20 to about 300 nucleotides. In addition, the oligonucleotide can comprise more than 100 to about 2000 nucleotides, preferably more than 100 to about 1000 nucleotides, and more preferably more than 100 to about 500 nucleotides.

In a preferred embodiment, the CpG-containing oligonucleotide contains one or more phosphothioester modifications of the phosphate backbone. For example, a CpG-containing oligonucleotide having one or more phosphate backbone modifications or having all of the phosphate backbone modified and a CpG-containing oligonucleotide wherein one, some or all of the nucleotide phosphate backbone modifications are phosphorothioate modifications are included within the scope of the present invention.

The CpG-containing oligonucleotide can also be recombinant, genomic, synthetic, cDNA, plasmid-derived and single or double stranded. For use in the instant invention, the nucleic acids can be synthesized de novo using any of a number of procedures well known in the art. For example, the b-cyanoethyl phosphoramidite method (Beaucage, S. L., and Caruthers, M. H., Tet. Let. 22:1859 (1981); nucleoside H-phosphonate method (Garegg et al., Tet. Let. 27: 4051-4054 (1986); Froehler et al., Nucl. Acid. Res. 14:5399-5407 (1986); Garegg et al., Tet. Let. 27:4055-4058 (1986), Gaffney et al., Tet. Let. 29:2619-2622 (1988)). These chemistries can be performed by a variety of automated oligonucleotide synthesizers available in the market. Alternatively, CpGs can be produced on a large scale in plasmids, (see Sambrook, T., et al., "Molecular Cloning: A Laboratory Manual," Cold Spring Harbor laboratory Press, New York, 1989) which after being administered to a subject are degraded into oligonucleotides. Oligonucleotides can be prepared from existing nucleic acid sequences (e.g., genomic or cDNA) using known techniques, such as those employing restriction enzymes, exonucleases or endonucleases.

The immunostimulatory substances, the immunostimulatory nucleic acids as well as the unmethylated CpG-containing oligonucleotide can be bound to the VLP by any way known is the art provided the composition enhances an immune response in an animal. For example, the oligonucleotide can be bound either covalently or non-covalently. In addition, the VLP can enclose, fully or partially, the immunostimulatory substances, the immunostimulatory nucleic acids as well as the unmethylated CpG-containing oligonucleotide. The immunostimulatory nucleic acid as well as the unmethylated CpG-containing oligonucleotide can be bound to a VLP site such as an oligonucleotide binding site (either naturally or non-naturally occurring), a DNA binding site or a RNA binding site. The VLP can site comprise an arginine-rich repeat or a lysine-rich repeat.

One specific use for the compositions is to activate dendritic cells for the purpose of enhancing a specific immune response against antigens. The dendritic cells can be enhanced using ex vivo or in vivo techniques. The ex vivo procedure can be used on autologous or heterologous cells, but is preferably used on autologous cells. The dendritic cells are isolated from peripheral blood or bone marrow, but can be isolated from any source of dendritic cells. Ex vivo manipulation of dendritic cells for the purposes of cancer immunotherapy have been described in several references in the art, including Engleman, E. G., Cytotechnology 25:1 (1997); Van Schooten, W., et al., Molecular Medicine Today, June, 255 (1997); Steinman, R. M., Experimental Hematology 24:849 (1996); and Gluckman, J. C., Cytokines, Cellular and Molecular Therapy 3:187 (1997).

The dendritic cells can also be contacted with the compositions using in vivo methods. In order to accomplish this, the CpGs are administered in combination with the VLP mixed with antigens directly to a subject in need of immunotherapy. It is preferred that the VLPs/CpGs be administered in the local region of the tumor, which can be accomplished in any way known in the art, *e.g.*, direct injection into the tumor.

In a further very preferred embodiment of the present invention, the unmethylated CpG-containing oligonucleotide comprises, or alternatively consists essentially of, or alternatively consists of the sequence GGGGGGGGGGGACGATCGTCGGGGGGGGGG (SEQ ID NO: 122). The latter was previously found to be able to stimulate blood cells in vitro (Kuramoto E. et al., Japanese Journal Cancer Research 83, 1128-1131 (1992).

In another preferred embodiment of the present invention, the immunostimulatory substance is an unmethylated CpG-containing oligonucleotide, wherein the CpG motif of said unmethylated CpG-containing oligonucleotide is part of a palindromic sequence. Preferably said palindromic sequence is GACGATCGTC (SEQ ID NO: 105). In another preferred embodiment, the palindromic sequence is flanked at its 3'-terminus and at its 5'-terminus by less than 10 guanosine entities, wherein preferably said palindromic sequence is GACGATCGTC (SEQ ID NO: 105). In a further preferred embodiment the palindromic sequence is flanked at its N-terminus by at least 3 and at most 9 guanosine entities and wherein said palindromic sequence is flanked at its C-terminus by at least 6 and at most 9 guanosine entities. These inventive immunostimulatory substances have unexpectedly found to be very -efficiently packaged into VLPs. The packaging ability was hereby enhanced as compared to the corresponding immunostimulatory substance having the sequence GACGATCGTC (SEQ ID NO: 105) flanked by 10 guanosine entitites at the 5' and 3' terminus.

In a preferred embodiment of the present invention, the palindromic sequence comprises, or alternatively consist essentially of, or alternatively consists of or is GACGATCGTC (SEQ ID NO: 105), wherein said palindromic sequence is flanked at its 5'-terminus by at least 3 and at most 9 guanosine entities and wherein said palindromic sequence is flanked at its 3'-terminus by at least 6 and at most 9 guanosine entities.

In a further very preferred embodiment of the present invention, the immunostimulatory substance is an unmethylated CpG-containing oligonucleotide, wherein the CpG motif of said unmethylated CpG-containing oligonucleotide is part of a palindromic sequence, wherein said unmethylated CpG-containing oligonucleotide has a nucleic acid sequence selected from (a) GGGGACGATCGTCGGGGGG ((SEQ ID NO: 106); and typically abbreviated herein as G3-6), (b) GGGGGACGATCGTCGGGGGG ((SEQ ID NO: 107); and typically abbreviated herein as G4-6), (c) GGGGGGACGATCGTCGGGGGG ((SEQ ID NO: 108); and typically abbreviated herein as G5-6), (d) GGGGGGGACGATCGTCGGGGGG ((SEQ ID NO: 109); and typically abbreviated herein as G6-6), (e) GGGGGGGGACGATCGTCGGGGGGG ((SEQ ID NO: 110); and typically abbreviated herein as G7-7), (f) GGGGGGGGGACGATCGTCGGGGGGGG ((SEQ ID NO: 111); and typically abbreviated herein as G8-8), (g) GGGGGGGGGGACGATCGTCGGGGGGGGG ((SEQ ID NO: 112); and typically abbreviated herein as G9-9), and (h) GGGGGGCGACGACGATCGTCGTCGGGGGGG ((SEQ ID NO: 113); and typically abbreviated herein as G6).

In a further preferred embodiment of the present invention the immunostimulatory substance is an unmethylated CpG-containing oligonucleotide, wherein the CpG motif of said unmethylated CpG-containing oligonucleotide is part of a palindromic sequence, wherein said palindromic sequence is GACGATCGTC (SEQ ID NO: 105), and wherein said palindromic sequence is flanked at its 5'-terminus of at least 4 and at most 9 guanosine entities and wherein said palindromic sequence is flanked at its 3'-terminus of at least 6 and at most 9 guanosine entities.

In another preferred embodiment of the present invention the immunostimulatory substance is an unmethylated CpG-containing oligonucleotide, wherein the CpG motif of said unmethylated CpG-containing oligonucleotide is part of a palindromic sequence, wherein said unmethylated CpG-containing oligonucleotide has a nucleic acid sequence selected from (a) GGGGGACGATCGTCGGGGGG ((SEQ ID NO: 107), and typically abbreviated herein as G4-6); (b) GGGGGGACGATCGTCGGGGGG ((SEQ ID NO: 108), and typically abbreviated herein as G5-6); (c) GGGGGGGACGATCGTCGGGGGG ((SEQ ID NO: 109),; and typically abbreviated herein as G6-6); (d) GGGGGGGGACGATCGTCGGGGGGG ((SEQ ID NO: 110), and typically abbreviated herein as G7-7); (e) GGGGGGGGGACGATCGTCGGGGGGGG ((SEQ ID NO: 111), and typically abbreviated herein as G8-8); (f) GGGGGGGGGGACGATCGTCGGGGGGGGG ((SEQ ID NO: 112), and typically abbreviated herein as G9-9).

In a further preferred embodiment of the present invention the immunostimulatory substance is an unmethylated CpG-containing oligonucleotide, wherein the CpG motif of said unmethylated CpG-containing oligonucleotide is part of a palindromic sequence, wherein said palindromic sequence is GACGATCGTC (SEQ ID NO: 105), and wherein said palindromic sequence is flanked at its 5'-terminus of at least 5 and at most 8 guanosine entities and wherein said palindromic sequence is flanked at its 3'-terminus of at least 6 and at most 8 guanosine entities.

The experimental data show that the ease of packaging of the preferred inventive immunostimulatory substances, i.e. the guanosine flanked, palindromic and unmethylated CpG-containing oligonucleotides, wherein the palindromic sequence is GACGATCGTC (SEQ ID NO: 105), and wherein the palindromic sequence is flanked at its 3'-terminus and at its 5'-terminus by less than 10 guanosine entities, into VLP's increases if the palindromic sequences are flanked by fewer guanosine entities. However, decreasing the number of guanosine entities flanking the palindromic sequences leads to a decrease of stimulating blood cells in vitro. Thus, packagability is paid by decreased biological activity of the indicated inventive immunostimulatory substances. The preferred embodiments represent, thus, a compromise between packagability and biological activity.

In another preferred embodiment of the present invention the immunostimulatory substance is an unmethylated CpG-containing oligonucleotide, wherein the CpG motif of said unmethylated CpG-containing oligonucleotide is part of a palindromic sequence, wherein said unmethylated CpG-containing oligonucleotide has a nucleic acid sequence selected from (a) GGGGGGACGATCGTCGGGGGG ((SEQ ID NO: 108), and typically abbreviated herein as G5-6); (b) GGGGGGGACGATCGTCGGGGGG ((SEQ ID NO: 109), and typically abbreviated herein as G6-6); (c) GGGGGGGGACGATCGTCGGGGGGG ((SEQ ID NO: 110), and typically abbreviated herein as G7-7); (d) GGGGGGGGGACGATCGTCGGGGGGGG ((SEQ ID NO: 111), and typically abbreviated herein as G8-8).

In a very preferred embodiment of the present invention the immunostimulatory substance is an unmethylated CpG-containing oligonucleotide, wherein the CpG motif of said unmethylated CpG-containing oligonucleotide is part of a palindromic sequence, wherein said unmethylated has the nucleic acid sequence of SEQ ID NO: 111, i.e.the immunostimulatory substance is G8-8.

As mentioned above, the optimal sequence used to package into VLPs is a compromise between packagability and biological activity. Taking this into consideration, the G8-8 immunostimulatoy substance is a further very preferred embodiment of the present invention since it is biologically highly active while it still reasonably well packaged.

The inventive composition further comprises an allergen or allergenic determinant mixed with the modified virus-like particle. The invention provides for compositions that vary according to the allergen or allergenic determinant selected in consideration of the desired therapeutic effect. Antigens or antigenic determinants suitable for use in the present invention are disclosed in WO 00/32227, in WO 01/85208 and in WO 02/056905.

The antigen can be any antigen of known or yet unknown provenance. It can be isolated from bacteria; viruses or other pathogens; tumors; or trees, grass, weeds, plants, fungi, mold, dust mites, food, or animals known to trigger allergic responses in sensitized patients. Alternatively, the antigen can be a recombinant antigen obtained from expression of suitable nucleic acid coding therefor. In a preferred embodiment, the antigen is a recombinant antigen. The selection of the antigen is, of course, dependent upon the immunological response desired and the host

The present invention is applicable to a wide variety of allergens. The allergen may be a protein, polypeptide or peptide.

Antigens can be selected from the group consisting of the following: (a) polypeptides suited to induce an immune response against cancer cells; (b) polypeptides suited to induce an immune response against infectious diseases; (c) polypeptides suited to induce an immune response against allergens; (d) polypeptides suited to induce an immune response in farm animals or pets; (e) carbohydrates naturally present on the polypeptides and (f) fragments (*e.g.*, a domain) of any of the polypeptides set out in (a)-(e).

Antigens include those from a pathogen (*e.g*. virus, bacterium, parasite, fungus) tumors (especially tumor-associated antigens or "tumor markers") and allergens. Other antigens are autoantigens and self antigens, respectively.. In specific embodiments described in the Examples, the antigen is bee venom. Up to 3% of the population are allergic to bee venom and it is possible to sensitize mice to bee venom in order to make them allergic. Hence, bee venom is an ideal allergen mixture that allows the study of immune responses induced by such mixtures in the presence or absence of various adjuvants, such as CpG-packaged VLPs. (*See* inter alia Example 4 and Example 9.)

In some Examples, VLPs containing peptide p33 were used. It should be noted that the VLPs containing peptide p33 were used only for reasons of convenience, and that wild-type VLPs can likewise be used in the present invention. The peptide p33 derived from lymphocytic choriomeningitis virus (LCMV). The p33 peptide represents one of the best studied CTL epitopes (Pircher et al., "Tolerance induction in double specific T-cell receptor transgenic mice varies with antigen," Nature 342:559 (1989); Tissot et al., "Characterizing the functionality of recombinant T-cell receptors in vitro: a pMHC tetramer based approach," J Immunol Methods 236:147 (2000); (Bachmann et al., "Four types of Ca2+-signals after stimulation of naive T cells with T cell agonists, partial agonists and antagonists," Eur. J. Immunol. 27:3414 (1997); Bachmann et al., "Functional maturation of an anti-viral cytotoxic T cell response," J. Virol. 71:5764 (1997); Bachmann et al., "Peptide induced TCR-down regulation on naive T cell predicts agonist/partial agonist properties and strictly correlates with T cell activation," Eur. J. Immunol. 27:2195 (1997); Bachmann et al., "Distinct roles for LFA-1 and CD28 during activation of naive T cells: adhesion versus costimulation," Immunity 7:549 (1997)). p33-specific T cells have been shown to induce lethal diabetic disease in transgenic mice (Ohashi et al., "Ablation of 'tolerance' and induction of diabetes by virus infection in viral antigen transgenic mice," Cell 65:305 (1991)) as well as to be able to prevent growth of tumor cells expressing p33 (Kündig et al., "Fibroblasts act as efficient antigen-presenting cells in lymphoid organs," Science 268:1343 (1995); Speiser et al., "CTL tumor therapy specific for an endogenous antigen does not cause autoimmune disease," J. Exp. Med. 186:645 (1997)). This specific epitope, therefore, is particularly well suited to study autoimmunity, tumor immunology as well as viral diseases.

In specific embodiments, compositions of the invention are an immunotherapeutic that can be used for the treatment and/or prevention of allergies.

The selection of allergens or allergenic determinants for the preparation of compositions and for use in methods of treatment for allergies would be known to those skilled in the medical arts treating such disorders. Representative examples of such allergens or allergenic determinants include the following: bee venom phospholipase $A_2$; Amb a 1 (ragweed pollen allergen), Bet v I (birch pollen allergen); 5 Dol m V (white-faced hornet venom allergen); Der p 1, Der f 2 and Der 2 (house dust mite allergens); Lep d 2 (dust mite allergen); Alt a 1, Asp f 1, and Asp f 16 (fungus allergens); Ara h 1, Ara h 2, and Ara h3 (peanut allergens) as well as fragments of each which can be used to elicit immunological responses. Moreover, the invention is particularly useful for the use of allergen mixtures that have been isolated from organisms or parts of organisms, such as pollen extracts or bee venom. In a preferred embodiment, pollen extracts comprise, or alternatively consist of trees, grasses, weeds, and garden plants. Examples of tree pollen extracts include, but are not limited to, the following: acacia, alder (grey), almond, apple, apricot, arbor vitae, ash, aspen, bayberry, beech, birch (spring), birch (white), bottle brush, box elder, carob tree, cedar, including but not limited to the japanese cedar, cherry, chestnut, cottonwood, cypress, elderberry, elm (American), eucalyptus, fir, hackberry, hazelnut, hemlock, hickory, hop-hornbeam, ironwood, juniper, locust, maple, melaleuca, mesquite, mock orange, mulberry, oak (white), olive, orange, osage orange, palo verde, peach, pear, pecan, pepper tree, pine, plum, poplar, privet, redwood, Russian olive, spruce, sweet gum, sycamore, tamarack, tree of heaven, walnut and willow. Examples of grass pollen extracts include, but are not limited to, the following: bahia, barley, beach, bent, Bermuda grass, bluegrass (Kentucky), brome, bunch, canarygrass, chess, corn, fescue (meadow), grama, johnson, june grass, koeler's, oats, orchard grass, quack, redtop, rye grass (perennial), salt, sorghum, sudan, sweet vernal grass, timothy grass, velvetgrass, wheat and wheatgrass. Examples of weed and garden plant extracts include, but are not limited to, the following: alfalfa, amaranth, aster, balsam root, bassia, beach bur, broomwood, burrow bush, careless weed, castor bean, chamise, clover, cocklebur, coreopsis, cosmos, daffodil, dahlia, daisy, dandelion, dock, dog fennel, fireweed, gladiolus, goldenrod, greasewood, hemp, honeysuckle, hops, iodone bush, Jerusalem oak, kochia, lamb's quarters, lily; marigold, marshelder, Mexican tea, mugwort, mustard, nettle, pickleweed, pigweed, plaintain (English), poppy, povertyweed, quailbush, ragweed (giant), ragweed (short), ragweed (western), rose, Russian thistle, sagebrush, saltbrush, scale, scotch broom, sea blight, sheep sorrel, snapdragon, sugar beet, sunflower, western waterhemp, winter fat, wormseed, wormwood.

In a preferred embodiment, pollen extracts comprise, or alternatively consist of rye. The seasonal appearance of ragweed pollen (September-October) induces asthma in many individuals (Marshall, J. et al., J. Allergy Clin. Immunol. 108:191-197 (2001)). Asthma is characterized by pulmonary inflammation, reversible airflow obstruction, and airway hyperresponsivess. A complex cascade of immunological responses to aeroallergens leads to leukocyte recruitment in the airways. Specifically, lymphocytes, macrophages, eosinophils, neutrophils, plasma cells, and mast cells infiltrate the bronchial mucosa (Redman, T. et al., Exp. Lung Res. 27:433-451 (2001)). Eosinophil recruitment is associated with increased production of the TH2 cytokines IL-4 and IL-5, key factors in asthma pathogenesis that support the chronic inflammatory process (Justice, J. et al., Am. J. Physiol. Lung Cell Mol. Physiol. 282:L302-L309 (2002), the entire contents of which is hereby incorporated by reference). The immunodominant ragweed allergen in short ragweed (*Ambrosia artemisiifolia*) is Amb a 1 (Santeliz, J. et al., J. Allergy Clin. Immunol. 109:455-462 (2002)). In a specific embodiment of the invention, the composition comprises the Amb a 1 mixed with the virus-like particle. (*See* Example 6.)

In yet another preferred embodiment, dust extracts comprise, or alternatively consist of house dusts and dust mites. Examples of house dusts include, but are not limited to: house dust, mattress dust, and upholstrey dust. Examples of dust mites include, but are not limited to, *D. farniae*, *D. ptreronysiinus,* mite mix, and *L. destructor.* Dust extracts also include, but are not limited to, cedar and red cedar dust, cotton gin dust, oak dust, grain (elevator) dust, paduk dust and wood dust.

Dust mites are an important source of perennial indoor allergens in homes in humid climates of developed countries (Arlian, L., Current Allergy and Asthma Reports I:581-586 (2001)). About 60-85% of all patients with allergic bronchial asthma are sensitized to the house dust mite *Dermatophogoldes pteronyssinus* (Arlian, L., Current Allergy and Asthma Reports I:581-586 (2001)). Immunodominant *D. pteronyssinus* dust mite allergens include Der p 1, Der f 2, and Der 2 (Kircher, M. et al., J. Allergy Clin. Immunol.109:517-523 (2002) and Clarke, A. et al., Int. Arch. Allergy Immunol. 120:126-134 (1999), the entire contents of which are hereby incorporated by reference). In a specific embodiment of the invention, the composition comprises the Der p 1, Der f 2, Der 2, or fragments thereof, or an antigenic mixture thereof mixed with the virus-like particle. An important cause of allergic reactions to dust, especially in farming communities, is *Lepidoglyphus destructor* (Ericksson, T. et al., Clinical and Exp. Allergy 31:1181-1890 (2001)). An immunodominant *L. destructor* dust mite allergen is Lep d 2 (Ericksson, T. et al., Clinical and Exp. Allergy 31:1181-1890 (2001)). In a specific embodiment of the invention, the composition comprises the Lep d 2 mixed with the virus-like particle. (*See* Example 8.)

In a preferred embodiment, fungal extracts comprise, or alternatively consist of alternaria, aspergillus, botrytis, candida, cephalosporium, cephalothecium, chaetomium, cladosporium, crytococcus, curvularia, epicoccum, epidermophyton, fusarium, gelasinospora, geotrichum, gliocladium, helminthosporium, hormodendrum, microsporium, mucor, mycogone, nigraspora, paecilomyces, penicillium, phoma, pullularia, rhizopus, rhodotorula, rusts, saccharomyces, smuts, spondylocladium, stemphylium, trichoderma, trichophyton and verticillium.

*Alternaria alternata* is considered to be one of the most important fungi causing allergic disease in the United States. *Alternaria* is the major asthma-associated allergen in desert regions of the United States and Australia and has been reported to cause serious respiratory arrest and death in the US Midwest (Vailes, L. et al., J. Allergy Clin. Immunol. 107:641 (2001) and Shampain, M. et al., Am. Rev. Respir. Dis. 126:493-498 (1982), the entire contents of which are hereby incorporated by reference). The immunodominant *Alternaria alternata* antigen is Alt a 1 (Vailes, L. et al., J. Allergy Clin. Immunol. 107:641 (2001)). Greater than 80% of *Alternaria* sensitized individuals have Ig E antibody against Alt a 1 (Vailes, L. et al., Clinical and Exp. Allergy 31:1891-1895 (2001)). In a specific embodiment of the invention, the composition comprises the Alt a 1 mixed with the virus-like particle. (*See* Example 7.)

Another opportunistic fungi is *Aspergillus fumigatus,* which is involved in a broad spectrum of pulmonary diseases, including allergic asthma. Immunodominant *Aspergillus fumigatus* antigens include Asp f 1 and Asp f 16 (Vailes, L. et al., J. Allergy Clin Immunol. 107:641 (2001)). In a specific embodiment of the invention, the composition comprises the Asp f 1 or Asp f 16 or an antigenic mixture thereof mixed with the virus-like particle. (*See* Example 7.)

In yet another preferred embodiment, insect extracts comprise, or alternatively consist of, stinging insects whose whole body induces allergic reactions, stinging insects whose venom protein induces allergic reactions, and insects that induce inhaled allergic reactions. Examples of stinging insects whose whole body induces allergic reactions include, but are not limited to: ant (black), ant (red), ant (carpenter), ant mix (black/red), ant (fire). Examples of stinging insects whose venom protein induces allergic reactions include, but are not limited to: honey bee, yellow hornet, wasp, yellow jacket, white-faced hornet and mixed vespid. Examples of insects that induce inhaled allergic reactions include, but are not limited to: aphid, black fly, butterfly, caddis fly, cicada/locust, cricket, cockroach, daphnia, deerfly, fruit fly, honey bee (whole body), horse fly, house fly, leafhopper, may fly, Mexican bean weevil, mites (dust), mosquito, moth, mushroom fly, screwworm fly, sow bugs, spider and water flea. (*See* Example 4.)

In yet another preferred embodiment, food extracts comprise, or alternatively consist of, animal products and plant products. Examples of animal products include, but are not limited to: beef, chicken, deer, duck, egg (chicken), fish, goat, goose, lamb, milk (cow), milk (goat), pork, rabbit, shellfish and turkey. Examples of plant products include, but are not limited to: apple, apricot, arrowroot, artichoke, asparagus, avodaco, banana, bean, beet, berries, cabbage family, carrot, celery, cherry, chocolate, citrus fruits, coconut, coffee, cucumber, date, eggplant, grain, grape, greens, gums, hops, lettuce, malt, mango, melon, mushroom, nuts, okra, olive, onion, papaya, parsnip, pea, peanut, pear, pimento, pineapple, plum, potato, prune, pumpkin, radish, rhubarb, spice/condiment, spinach, squash, tapioca, tea, tomato, watermelon and yeast.

Allergies to peanuts and tree nuts account for the majority of fatal and near-fatal anaphylactic reactions (Sampson, H., N. Engl. J. Med. 346(17):1294-1299 (2002)). About 1.1 percent of Americans, or 3 million people, are allergic to peanuts, tree nuts, or both (Sampson, H., N. Engl. J. Med. 346(17):1294-1299 (2002)). About 6 percent of Americans have serologic evidence of sensitivity to peanuts (i.e. the presence of IgE antibodies specific for peanut proteins), although the majority of these people will not have an allergic reaction when they eat peanuts (Sampson, H., N. Engl. J. Med. 346(17):1294-1299 (2002) and Helm, R et al., J. Allergy Clin. Immunol. 109:136-142 (2002)). Peanut allergy usually develops at an early age, often following exposure to peanut protein in utero, during breast-feeding, or early in childhood and is often a lifelong disorder (Sampson, H., N. Engl. J. Med. 346(17):1294-1299 (2002); Li, X. et al., J. Allergy Clin. Immunol. 108:639-646 (2001); and Helm, R et al., J. Allergy Clin. Immunol. 109:136-142 (2002)). Infants who have peanut allergy tend to have more severe allergic reacts as they get older (Sampson, H., N. Engl. J. Med., 346 (17):1294-1299 (2002)). It has been suggested that the promotion of peanut products as a nutritional source for pregnant and lactating women has contributed the rising prevalence of peanut allergy in westernized countries (Sampson, H., N. Engl. J. Med. 346(17):1294-1299 (2002)).

Peanut allergy symptoms may develop within minutes to a few hours after ingestion of food, and in life-threatening cases, symptoms include severe bronchospasm. Currently, treatment of peanut allergy consists of teaching patients and their families how to avoid the accidental ingestion of peanuts, how to recognize early symptoms of allergic reaction, and how to manage the early stages of anaphylactic reaction (Sampson, H., N. Engl. J. Med. 346(17):1294-1299 (2002)). Inadvertent exposures result in an allergic reaction every three to five years in the average patient with peanut allergy (Sampson, H., N. Engl. J. Med. 346(17):1294-1299 (2002)). These inadvertant exposures may occur as a result of peanut contamination of equipment used in the manufacture of various products, inadequate food labeling, cross-contamination of food during cooking in restaurants, and unanticipated exposures (*e.g.* the inhalation of peanut dust in airplanes) (Sampson, H., N. Engl. J. Med. 346(17):1294-1299 (2002)). Current therapy of an acute reaction to peanuts includes aggressive treatment with intramuscular epinephrine; oral, intramuscular, or intravenous histamine $H_1$- and $H_2$-receptor antagonists; oxygen; inhaled albuterol; and systemic coorticosteroids (Sampson, H., N. Engl. J. Med. 346 (17):1294-1299 (2002)). In addition, a three-day course of oral prednisone and antihistamine is often recommended following an acute reaction to peanuts. Given the severity, prevalence, and frequently lifelong persistence of peanut allergy there is a need for a preventive or curative therapy for peanut allergy (Sampson, H., N. Engl. J. Med. 346(17): 1294-1299 (2002)).

Two major allergenic peanut proteins, which are recognized by more than 95% of patients with peanut allergy, are Ara h 1 and Ara h 2 (Bannon, G., et al., Int. Arch. Allergy Immunol. 124:70-72 (2001) and Li, X. et al., J. Allergy Clin. Immunol. 106:150-158 (2000), the entire contents of which are hereby incorporated by reference). Ara h 3 is recognized by about 45% of patients with peanut allergy (Li, X., et al., J Allergy Clin. Immunol. 106:150-158 (2000)). In a specific embodiment of the invention, the composition comprises the antigen Ara h 1, Ara h 2, or Ara h 3 or an antigenic mixture thereof mixed with the virus-like particle. (*See* Example 5.)

In another preferred embodiment, mammalian epidermal allergens include, but are not limited to: camel, cat hair, cat pelt, chinchilla, cow, deer, dog, gerbil, goat, guinea pig, hamster, hog, horse, mohair, monkey, mouse, rabbit, wool (sheep). In yet another preferred embodiment, feathers include, but are not limited to: canary, chicken, duck, goose, parakeet, pigeon, turkey. In another preferred embodiment, other inhalants include, but are not limited to: acacia, algae, castor bean, cotton linters, cottonseed, derris root, fern spores, grain dusts, hemp fiber, henna, flaxseed, guar gum, jute, karaya gum, kapok, leather, lycopodium, orris root, pyrethrum, silk (raw), sisal, tobacco leaf, tragacanth and wood dusts.

In another preferred embodiment, typically defined mammalian allergens, either purified from natural sources or recombinantly expressed are included. These include, but are not limited, to Fel d 1, Fel d 3 (cystatin) from cats and albumins from cat, camel, chinchilla, cow, deer, dog, gerbil, goat, guinea pig, hamster, hog, horse, mohair, monkey, mouse, rabbit, wool (sheep).

The antigen or antigenic determinant may be selected from the group consisting of: (a) a recombinant polypeptide of HIV; (b) a recombinant polypeptide of Influenza virus (*e.g.*, an Influenza virus M2 polypeptide or a fragment thereof); (c) a recombinant polypeptide of Hepatitis C virus; (d) a recombinant polypeptide of Hepatitis B virus; (e) a recombinant polypeptide of *Toxoplasma*; (f) a recombinant polypeptide of *Plasmodium falciparum*; (g) a recombinant polypeptide of *Plasmodium vivax*; (h) a recombinant polypeptide of *Plasmodium ovale*; (i) a recombinant polypeptide of *Plasmodium malariae*; (j) a recombinant polypeptide of breast cancer cells; (k) a recombinant polypeptide of kidney cancer cells; (1) a recombinant polypeptide of prostate cancer cells; (m) a recombinant polypeptide of skin cancer cells;

(n) a recombinant polypeptide of brain cancer cells; (o) a recombinant polypeptide of leukemia cells; (p) a recombinant profiling; (q) a recombinant polypeptide of bee sting allergy; (r) a recombinant polypeptide of nut allergy; (s) a recombinant polypeptide of pollen; (t) a recombinant polypeptide of house-dust; (u) a recombinant polypeptide of cat or cat hair allergy; (v) a recombinant protein of food allergies; (w) a recombinant protein of asthma; (x) a recombinant protein of *Chlamydia*; (y) antigens extracted from any of the protein sources mentioned in (a-x); and (z) a fragment of any of the proteins set out in (a)-(x). The antigen mixed with the virus-like particle packaged with the immunostimulatory substance, the immunostimulatory nucleic acid or the unmethylated CpG-containing oligonucleotide of the invention, is a T cell epitope, either a cytotoxic or a Th cell epitope. The antigen mixed with the virus-like particle packaged with the immunostimulatory substance, the immunostimulatory nucleic acid or the unmethylated CpG-containing oligonucleotide of the invention is a B cell epitope. The antigen can be a combination of at least two, preferably different, epitopes, wherein the at least two epitopes are linked directly or by way of a linking sequence. These epitopes are preferably selected from the group consisting of cytotoxic and Th cell epitopes.

The indicated epitope or epitopes, can be synthesized or recombinantly expressed and coupled to the virus-like particle, or fused to the virus-like particle using recombinant DNA techniques. Exemplary procedures describing the attachment of antigens to virus-like particles are disclosed in WO 00/32227, in WO 01/85208 and in WO 02/056905.

Provided is a method of producing a composition for enhancing an immune response in an animal comprising a VLP and an unmethylated CpG-containing oligonucleotide bound to the VLP which comprises incubating the VLP with the oligonucleotide, adding RNase and purifying said composition. The method comprises incubating the VLP with RNase, adding the oligonucleotide and purifying the composition. In one embodiment, the VLP is produced in a bacterial expression system. In another embodiment, the RNase is RNase A.

Further provided is a method of producing a composition for enhancing an immune response in an animal comprising a VLP bound to an unmethylated CpG-containing oligonucleotide which comprises disassembling the VLP, adding the oligonucleotide and reassembling the VLP. The method can further comprise removing nucleic acids of the at least partially disassembled VLP and/or purifying the composition after reassembly.

Also provided are vaccine compositions which can be used for preventing and/or attenuating diseases or conditions. Vaccine compositions comprise, or alternatively consist of, an immunologically effective amount of the inventive immune enhancing composition together with a pharmaceutically acceptable diluent, carrier or excipient. The vaccine can also optionally comprise an adjuvant.

Further provided are vaccination methods for preventing and/or attenuating diseases or conditions in animals. Provided are vaccines for the prevention of infectious diseases in a wide range of animal species, particularly mammalian species such as human, monkey, cow, dog, cat, horse, pig, etc. Vaccines can be designed to treat infections of viral etiology such as HIV, influenza, *Herpes,* viral hepatitis, Epstein Bar, polio, viral encephalitis, measles, chicken pox, etc.; or infections of bacterial etiology such as pneumonia, tuberculosis, syphilis, etc.; or infections of parasitic etiology such as malaria, trypanosomiasis, leishmaniasis, trichomoniasis, amoebiasis, etc.

Provided are vaccines for the prevention of cancer in a wide range of species, particularly mammalian species such as human, monkey, cow, dog, cat, horse, pig, etc. Vaccines can be designed to treat all types of cancer including, but not limited to, lymphomas, carcinomas, sarcomas and melanomas.

As would be understood by one of ordinary skill in the art, when compositions of the invention are administered to an animal, they can be in a composition which contains salts, buffers, adjuvants or other substances which are desirable for improving the efficacy of the composition. Examples of materials suitable for use in preparing pharmaceutical compositions are provided in numerous sources including REMINGTON'S PHARMACEUTICAL SCIENCES (Osol, A, ed., Mack Publishing Co., (1990)).

Various adjuvants can be used to increase the immunological response, depending on the host species, and include but are not limited to, Freund's (complete and incomplete), mineral gels such as aluminum hydroxide, surface active substances such as lysolecithin, pluronic polyols, polyanions, peptides, oil emulsions, keyhole limpet hemocyanins, dinitrophenol, and potentially useful human adjuvants such as BCG (bacille Calmette-Guerin) and *Corynebacterium parvum.* Such adjuvants are also well known in the art. Further adjuvants that can be administered with the compositions of the invention include, but are not limited to, Monophosphoryl lipid immunomodulator, AdjuVax 100a, QS-21, QS-18, CRL1005, Aluminum salts (Alum), MF-59, OM-174, OM-197, OM-294, and Virosomal adjuvant technology. The adjuvants can also comprise a mixture of these substances.

Immunologically active saponin fractions having adjuvant activity derived from the bark of the South American tree Quillaja Saponaria Molina are known in the art. For example QS21, also known as QA21, is an Hplc purified fraction from the Quillaja Saponaria Molina tree and it's method of its production is disclosed (as QA21) in U.S. Pat. No. 5,057,540. Quillaja saponin has also been disclosed as an adjuvant by Scott et al, Int. Archs. Allergy Appl. Immun., 1985, 77, 409. Monosphoryl lipid A and derivatives thereof are known in the art. A preferred derivative is 3 de-o-acylated monophosphoryl lipid A, and is known from British Patent No. 2220211. Further preferred adjuvants are described in WO00/00462.

Compositions of the invention are said to be "pharmacologically acceptable" if their administration can be tolerated by a recipient individual. Further, the compositions of the invention will be administered in a "therapeutically effective amount" (*i.e.*, an amount that produces a desired physiological effect).

The compositions of the present invention can be administered by various methods known in the art. The particular mode selected will depend of course, upon the particular composition selected, the severity of the condition being treated and the dosage required for therapeutic efficacy. The methods of the invention, generally speaking, can be practiced using any mode of administration that is medically acceptable, meaning any mode that produces effective levels of the active compounds without causing clinically unacceptable adverse effects. Such modes of administration include oral, rectal, parenteral, intracistemal, intravaginal, intraperitoneal, topical (as by powders, ointments, drops or transdermal patch), bucal, or as an oral or nasal spray. The term "parenteral" as used herein refers to modes of administration which include intravenous, intramuscular, intraperitoneal, intrasternal, subcutaneous and intraarticular injection and infusion. The composition of the invention can also be injected directly in a lymph node.

Components of compositions for administration include sterile aqueous (*e.g.*, physiological saline) or non-aqueous solutions and suspensions. Examples of non-aqueous solvents are propylene glycol, polyethylene glycol, vegetable oils such as olive oil, and injectable organic esters such as ethyl oleate. Carriers or occlusive dressings can be used to increase skin permeability and enhance antigen absorption. Combinations can be administered either concomitantly, *e.g.*, as an admixture, separately but simultaneously or concurrently; or sequentially. This includes presentations in which the combined agents are administered together as a therapeutic mixture, and also procedures in which the combined agents are administered separately but simultaneously, *e.g.*, as through separate intravenous lines into the same individual. Administration "in combination" further includes the separate administration of one of the compounds or agents given first, followed by the second. Dosage levels depend on the mode of administration, the nature of the subject, and the quality of the carrier/adjuvant formulation. Typical amounts are in the range of about 0.001 $\mu$g to about 20 mg per subject. Preferred amounts are at least about 1 $\mu$g to about 100 $\mu$g per subject. Multiple administration to immunize the subject is preferred, and protocols are those standard in the art adapted to the subject in question. Typical amounts of the antigen are in a range comparable, similar or identical to the range typically used for administration without the addition of the VLP's.

The compositions can conveniently be presented in unit dosage form and can be prepared by any of the methods well-known in the art of pharmacy. Methods include the step of bringing the compositions of the invention into association with a carrier which constitutes one or more accessory ingredients. In general, the compositions are prepared by uniformly and intimately bringing the compositions of the invention into association with a liquid carrier, a finely divided solid carrier, or both, and then, if necessary, shaping the product.

Compositions suitable for oral administration can be presented as discrete units, such as capsules, tablets or lozenges, each containing a predetermined amount of the compositions of the invention. Other compositions include suspensions in aqueous liquids or non-aqueous liquids such as a syrup, an elixir or an emulsion.

Other delivery systems can include time-release, delayed release or sustained release delivery systems. Such systems can avoid repeated administrations of the compositions of the invention described above, increasing convenience to the subject and the physician. Many types of release delivery systems are available and known to those of ordinary skill in the art.

Other embodiments of the invention include processes for the production of the compositions of the invention and methods of medical treatment for cancer and allergies using said compositions.

Thus, the present invention, inter alia, relates to the finding that virus like particles (VLPs) can be loaded and packaged, respectively, with DNA oligonucleotides rich in non-methylated C and G (CpGs). If such CpG-VLPs are mixed with antigens, the immunogenicity of these antigens was dramatically enhanced. In addition, the T cell responses against the antigens are especially directed to the Th1 type. Surprisingly, no covalent linkage of the antigen to the VLP was required but it was sufficient to simply mix the VLPs with the adjuvants for co-administration. In addition, VLPs did not enhance immune responses unless they were loaded and packaged, respectively, with CpGs. Antigens mixed with CpG-packaged VLPs may therefore be ideal vaccines for prophylactic or therapeutic vaccination against allergies, tumors and other self-molecules and chronic viral diseases.

Provided is a method of producing a composition for enhancing an immune response in an animal comprising a virus-like particle and an immunostimulatory substance packaged within said virus-like particle, said method comprises (a) incubating -said virus-like particle with said immunostimulatory substance; (b) adding RNase; and (c) purifying said composition.

Provided is a method of producing a composition for enhancing an immune response in an animal comprising a virus-like particle and an immunostimulatory substance packaged within said virus-like particle, said method comprises (a) incubating said virus-like particle with RNase; (b) adding said immunostimulatory substance; and (c) purifying said composition.

Provided is a method of producing a composition for enhancing an immune response in an animal comprising a virus-like particle and an immunostimulatory substance packaged within said virus-like particle, said method comprises: (a) disassembling said virus-like particle; (b) adding said immunostimulatory substance; and (c) reassembling said virus-like particle. In an alternative embodiment, the method of producing a composition for enhancing an immune response in an animal according to the invention further comprises removing nucleic acids of the disassembled virus-like particle. In yet an alternative embodiment, the method of producing a composition for enhancing an immune response in an animal according to the invention further comprises purifying the composition after reassembly (c).

Provided is a method of producing a composition for enhancing an immune response in an animal comprising a virus-like particle and an immunostimulatory substance packaged within said virus-like particle, said method comprises (a) incubating said virus-like particle with solutions comprising metal ions capable of hydrolizing the nucleic acids of said virus-like particle; (b) adding said immunostimulatory substance; and (c) purifying said composition. Preferably, the metal ions capable of hydrolyzing the nucleic acids of the virus-like particle are selected from the group of (a) zinc (Zn) ions; (b) copper (Cu) ions; (c) iron (Fe) ions; (d) any mixtures of at least one ion of (a), (b) and/or (c). In preferred embodiments of the methods of producing a composition for enhancing an immune respons in an animal according to the invention, indicated above, the immunostimulatory immunostimulatory substance is an immunostimulatory nucleic acid selected from the group consisting of, or alternatively consisting essentially of: (a) ribonucleic acids, preferably poly-(I:C) or a derivative thereof; (b) deoxyribonucleic acids, preferably oligonucleotides free of unmethylated CpG motifs, and even more preferably unmethylated CpG-containing oligonucleotides; (c) chimeric nucleic acids; and (d) any mixtures of at least one nucleic acid of (a), (b) and/or (c).

In another preferred embodiments of the methods of producing a composition for enhancing an immune respons in an animal according to the invention, indicated above, the virus-like particle is produced in a bacterial or in a mammalian expression system, in a further preferred embodiment, the RNase is RNaseA.

The following examples are illustrative only and are not intended to limit the scope of the invention as defined by the appended claims.

EXAMPLE 1

Generation of VLPs.

The DNA sequence of HBcAg containing peptide p33 from LCMV is given in SEQ ID NO: 70. The p33-HBcAg VLPs (p33-VLPs) were generated as follows: Hepatitis B clone pEco63 containing the complete viral genome of Hepatitis B virus was purchased from ATCC. The generation of the expression plasmid has been described previously (*see* WO 03/024481).

A clone of E. coli K802 selected for good expression was transfected with the plasmid, and cells were grown and resuspended in 5 ml lysis buffer (10 mM $Na_2HPO_4$, 30 mM NaCl, 10 mM EDTA, 0.25 % Tween-20, pH 7.0). 200 μl of lysozyme solution (20 mg/ml) was added. After sonication, 4 μl Benzonase and 10 mM $MgCl_2$ was added and the suspension was incubation for 30 minutes at RT, centrifuged for 15 minutes at 15,000 rpm at 4˚C and the supernatant was retained. Next, 20 % (w/v) (0.2 g/ml lysate) ammonium sulfate was added to the supernatant. After incubation for 30 minutes on ice and centrifugation for 15 minutes at 20,000 rpm at 4˚C the supernatant was discarded and the pellet resuspended in 2-3 ml PBS. 20 ml of the PBS-solution was loaded onto a Sephacryl S-400 gel filtration column (Amersham Pharmacia Biotechnology AG), fractions were loaded onto a SDS-Page gel and fractions with purified p33-HBcAg VLP capsids were pooled. Pooled fractions were loaded onto a Hydroxyappatite column. Flow through (which contains purified p33-HBcAg VLP capsids) was collected. Electron microscopy was performed according to standard protocols. A representative example is shown in Figure 1 ofStomi T., et al.,(2002) J Immunol.; 168(6):2880-6.

It should be noted that the VLPs containing peptide p33 were used only for reasons of convenience, and that wild-type VLPs can likewise be used in the present invention. Throughout the description the terms p33-HBcAg VLP, HBcAg-p33 VLP, p33-VLPs and HBc33 are used interchangeably. In particular, the VLPs used in Examples 1-4, 9, and 10, 18 are p33-HBcAg VLPs.

EXAMPLE 2

CpG-containing oligonucleotides can be packaged into HBcAg VLPs.

Recombinant VLPs generated as described in Example 1 were run on a native agarose (1%) gel electrophoresis and stained with ethidium bromide or Coomassie blue for the detection of RNA/DNA or protein (Figure 1). Bacterial produced VLPs contain high levels of single stranded RNA, which is presumably binding to the arginine repeats appearing near the C-terminus of the HBcAg protein and being geographically located inside the VLPs as shown by X-ray crystallography. The contaminating RNA can be easily digested and so eliminated by incubating the VLPs with RNase A. The highly active RNase A enzyme has a molecular weight of about 14 kDa and is presumably small enough to enter the VLPs to eliminate the undesired ribonucleic acids.

The recombinant VLPs were supplemented with CpG-rich oligonucleotides (see SEQ ID NO: 69) before digestion with RNase A. As shown in Figure 2 the presence of CpG- oligonucleotides preserved the capsids structure as shown by similar migration compared to untreated p33-VLPs. The CpG- oligonucleotides containing VLPs were purified from unbound oligonucleotides via dialysis (4500-fold dilution in PBS for 24 hours using a 300 kDa MWCO dialysis membrane) (see Figure 3).

EXAMPLE 3

CpG-containing oligonucleotides can be packaged into VLPs by removal of the RNA with RNAse and subsequent packaging of oligonucleotides into VLPs.

The VLPs (containing bacterial single-stranded RNA and generated as described in Example 1) were first incubated with RNaseA to remove the RNA and in a second step the immunostimulating CpG-oligonucleotides (with normal phosphodiester moieties but also with phosphorothioate modifications of the phosphate backbone) was supplemented to the samples (Figure 4). This experiment clearly shows that the CpG-oligonucleotides are is not absolutely required simultaneously during the RNA degradation reaction but can be added at a later time.

EXAMPLE 4

VLPs containing CpG-oligonucleotides induce strong IgG responses against co-administered bee venom.

The VLP generated as described in Example 1 was used for this experiment. Mice were subcutaneously primed with 5 μg of bee venom (ALK Abello) either alone or mixed with one of the following: 50 μg VLP alone, 50 μg VLP loaded and packaged, respectively, with CpG-oligonucleotides or 50 μg VLP mixed with 20 nmol CpG-oligonucleotides. Alternatively, mice were primed with 5 μg bee venom mixed with VLP alone or VLP loaded and packaged, respectively, with CpG-oligonucleotides in conjunction with aluminum hydroxide. 14 days later, mice were boosted with the same vaccine preparations and bled on day 21. Bee venom specific IgG responses in sera from day 21 were assessed by ELISA. RNase A treated VLPs derived from HBcAg carrying inside CpG-oligonucleotides (containing normal phosphodiester moieties), dialyzed from unbound CpG-oligonucleotides were effective at enhancing IgG responses against bee venom allergens (BV). As shown in Figure 5, the presence of either free CpGs or VLPs loaded and packaged, respectively, with CpGs dramatically enhanced the IgG response against the bee venom. The VLP without CpGs did not enhance the immune response. The presence of Alum as an adjuvant further increased the IgG response. If IgG subclasses were measured (Figure 6), it was evident that CpG-packaged VLPs shifted the response from an IgG1 dominance to a IgG2a dominance, indicating that a Th1 response was used. Interestingly, the presence of Alum enhanced the Th2-associated IgG1 isotype. Hence, addition of CpG-packaged VLPs to the bee venom in Alum resulted in high IgG titers but the response was still dominated by IgG1. Importantly, although CpGs packaged into VLPs were similarly effective as free CpGs at enhancing IgG responses against bee venom both in the presence or absence of Alum, they did not show signs of systemic immune activation (Figure 7). Specifically, while vaccination of mice in the presence of free CpGs induced splenomegaly with spleens up to 4 fold increased total lymphocyte numbers, CpGs packaged into VLPs did not result in increased total lymphocyte numbers.

EXAMPLE 5

VLPs used against peanut allergy.

In the following examples 5 to 8, the VLP used is Qb core particle (SEQ ID NO: 1) packaged with G10-PO (SEQ ID NO: 122). Female C3H/HeJ mice 5 weeks of age are sensitized to peanuts by intragastric gavage with 5 mg of freshly ground, roasted whole peanut together with 10 μg of cholera toxin on day 0. Mice are boosted 1 and 3 weeks later. One week after the final sensitization dose, mice receive either VLP mixed with 10 mg of crude peanut extract, VLP mixed with 5 μg of Ara h 1, VLP mixed with 5 μg of Ara h 2, VLP mixed with 5 μg of Ara h 3, or VLP mixed with 5 μg each of Ara h 1, Ara h 2 and Ara h 3. Naïve mice, mice receiving VLP alone, mice receiving 10 mg of crude peanut extract alone, or mice receiving VLP mixed with 5 μg of an irrelevant antigen serve as controls.

Levels of peanut-specific IgE are measured by using ELISA. IgE antibodies specific for Ara h 1, Ara h 2, and Ara h 3 are monitored in pooled sera from peanut-sensitized mice. Plates are coated with Ara h 1, Ara h 2, and Ara h 3 (2 μg/ml). Levels of IgG subclasses, specifically IgG1 and IgG2a, are also measured by ELISA in order to determine if a TH1 or a TH2 response is used.

Anaphylactic symptoms are evaluated for 30 to 40 minutes after the second challenge dose by-using the following scoring system: 0, no symptoms; 1, scratching and rubbing around the nose and head; 2, puffiness around the eyes and mouth, diarrhea, pilar erecti, reduced activity, and/or decreased activity with increased respiratory rate; 3, wheezing, labored respiration, and cyanosis around the mouth and the tail; 4, no activity after prodding or tremor and convulsion; 5, death.

Blood is collected 30 minutes after the second intragastric gavage challenge. Plasma histamine levels are determined using an enzyme immunoassay kit (ImmunoTECH Inc, Marseille, France) as described by the manufacturer.

Spleens are removed from peanut-sensitized and naive mice after rechallenge at week 5. As a measure of their activation state, the ability of splenocytes to proliferate following *in vitro* stimulation with peanut antigens is determined. Specifically, spleen cells are isolated and suspended in complete culture medium (RPMI-1640 plus 10% FBS, 1% penicillin-streptomycin, and 1% glutamine). Spleen cells (1 x $10^6$/well in 0.2 mL) are incubated in triplicate cultures in microwell plates in the presence or absence of crude peanut extract, Ara h 1, Ara h 2, or Ara h 3 (10 or 50 $\mu$g/ml). Cells stimulated with Con A (2 $\mu$g/ml) are used as positive controls. Six days later, the cultures are pulsed for 18 hours with 1 $\mu$Ci per well of $^3$H-thymidine. The cells are harvested, and the incorporated radioactivity is counted in a $\beta$-scintillation counter.

Spleen cells are also cultured in 24-well plates (4 x $10^6$/well/ml) in the presence or absence of crude peanut extract (50 $\mu$g/ml) or Con A (2 $\mu$g/ml). Supernatants are collected 72 hours later. IL-4, IL-5, IL-13, and IFN-$\gamma$ are determined by ELISA, according to the manufacturer's instructions, in order to determine if a TH1 or a TH2 response is used.

EXAMPLE 6

VLPs used against ragweed allergy.

Male C3H/HeJ mice 6-10 weeks of age are sensitized to ragweed (RW) by intraperitoneal injection of 80 $\mu$g RW on days 0 and 4 (endotoxin content >2.3 ng/mg RW; Greer Laboratories, Lenoir, NC). Sensitization solution consists of 1 mg of RW in 1 ml of 0.9% NaCl (Baxter, Deerfield, IL) plus 333 ml of Imject alum (Pierce, Rockford, IL). One week after the final sensitization dose, mice receive either VLP mixed with 160 ug of RW or VLP mixed with 80 ug of Amb a 1. Naive mice, mice receiving VLP alone, mice receiving 160 ug of RW alone, or mice receiving VLP mixed with 80 ug of an irrelevant antigen serve as controls.

On day 25, 0.5 ml of peripheral blood from the tail vein is collected, mice are anesthetized with ketamine (90 $\mu$g/kg body wt) and xylazine (10 mg/kg body wt) and then are challenged by intratracheal administration of RW (10 $\mu$g of RW in 0.1 ml of 0/9% NACl). 12 h following RW challenge, 0.5 ml of peripheral blood from the tail vein is collected and lungs are lavaged with a single 1 ml aliquot of PBS. Samples are centrifuged at 2,000 rpm for 5 min and bronchoalveolar lavage fluid is collected. Interleukin IL-4 and IL-5 levels are determined using two-site immunoenzymetric assay kits (Endogen, Cambridge, MA) according to the manufacturer's instructions. The lower limits of detection are 1 pg/ml for both IL-4 and IL-5. After lungs are lavaged, they are removed. The lungs are infused with 4% paraformaldehyde (in PBS) for 30 min, rinsed with PBS and immersed in 0.5 M sucrose (in PBS) overnight at 4° C. Lungs are inflated and embedded in parafin. Tissues sections are stained with hematoxylin and eosin and the degree of inflammation eosinophil infiltration is quantified by image analysis.

White blood cells are isolated from peripheral blood by centrifugation on a discontinuous Percoll gradient with subsequent hypotonic lysis of remaining red blood cells. Eosinophils are enriched from white blood cells by the negative-selection process using anti-CD90 and anti-CD45R antibodies to deplete the B- and T-cell populations using the MACS magnetic bead separation method per the manufacturer's suggested protocol (Miltenyi Biotechnical, Auburn, CA). Eosinophil fractions are routinely enriched to <98%.

Purified peripheral blood eosinophils are resuspended in RPMI-1640 (GIBCO-BRL) and 5% fetal calf serum (GIBCO-BRL) at a cell density of 1 x $10^6$ cells/ml. The cells are stimulated with $10^{-7}$ M phorbol 12-myristate 13-acetate (PMA) and $10^{-7}$ M A-23187 (Sigma) in 96-well plates at 37° C for 30 min, 1 h, and 16 h or Amb a 1 (20 $\mu$g/ml) for 6 days. Following stimulation, the ability of VLPs to reverse the TH2-dominant cytokine secretion profile induced by Amb a 1 is analyzed. Specfically, the ability of eosinophils to produce the IFN-$\gamma$, IL-4 and IL-5 is analyzed by sandwich ELISA.

Levels of ragweed-specific IgE are measured by using ELISA. IgE antibodies specific for Amb a 1 are monitored in pooled sera from ragweed-sensitized mice. Plates are coated with Amb a 1 (2 $\mu$g/ml). Levels of IgG subclasses, specifically IgG1 and IgG2a, are also measured by ELISA in order to determine if a TH1 or a TH2 response is used.

EXAMPLE 7

VLPs used against fungal allergies.

Naïve New Zealand white rabbits at 7 days of age are immunized with VLP mixed with 10 $\mu$g of Alt a 1, a heat-stable dimer of 28 kd, which is extracted and purified from *Alternaria alternata* extract or with VLP mixed with 10 $\mu$g of Asp f 1 and or 10 $\mu$g of Asp f 16, proteins which are extracted and purified from *Aspergillus fumigatus.* Naïve rabbits, rabbits receiving VLP alone and rabbits receiving 210 ng protein/ml of lyophilized *Alternaria alternata* or *Aspergillus fumigatus* extract, reconstituted in normal saline, serve as controls. Rabbit anti-*Alternaria* and anti-*Aspergillus* IgE is measured by homologous passive cutaneous anaphylaxis (PCA). Naïve 3-month old New Zealand white rabbits are injected intracutaneously along the back with 0.2 ml serum dilutions from 3-month-old immunized rabbits. Serums from nonimmunized rabbits and rabbits immunized with bovine serum albumin are tested as controls. After a latent period of 3 days the recipient rabbits are injected intravenously with 2.1 ng protein of *Alternaria* or *Aspergillus* extract diluted in 5 ml of 2.5 % Evans blue dye (Fisher Scientific Company, Fair Lawn, NJ). To gauge skin test responsiveness, histamine phosphate (0.2 ml of 0.275 mg/ml) and normal saline are injected intracutaneously 10 min before the extract-dye mixture is given. Blueing of the individual injection sites is measured 1 h after dye administration. A positive response for any dilution is a blue spot 5 mm or greater in diameter.

Three month old immunized rabbits as well as nonimmunized control rabbits are anesthetized with 1 to 3 ml of sodium methohexital (Brevitol, Eli Lilly Co., Indianapolis, IN), 10 mg/ml in normal saline, given intravenously. The rabbits are intubated with a 3.5 mm endotracheal tube (Portex Inc., Woburn MA). A latex balloon (Young Rubber Co., Trenton, NJ), 3 cm in length, attached to a P-240 catheter (Clay Adams, Parsippany, NJ) is placed in the esophagus. A 4-cm segment of a 9-mm diameter endotracheal tube is placed to the back of the oropharynx covering the esophageal catheter and small endotracheal tube to prevent damage to them by the rabbits' posterior teeth. The mouth is taped shut and the animal is allowed to awaken over 2 h. After introduction of a small volume of air into the balloon, the position of the balloon is adjusted to the point where the end-expiratory pressure is most negative and cardiac artifact least. The esophageal balloon catheter is connected to a Hewlett-Packard Model 270 differential pressure transducer (Minneapolis, MN) and the difference between balloon and endotracheal tube pressure is recorded as transpulmonary pressure. Baseline measurements are made after the animals are fully awakened. These measurements included respiratory frequency, inspiratory and expiratory flow rates, tidal volume and transpulmonary pressure.

After baseline measurements are made, The animals are challenged with aerosols of either normal saline, *Alternaria alternata* extract, or *Aspergillus fumigatus* extract diluted 1:20 weight/volume in normal saline. One ml of either normal saline, *Alternaria* extract, or *Aspergillus* extract is nebulized over 5 min directly into the endotracheal tube using an air flow of 4L/min (with compressed air). At the end of the 5-min challenge, and pulmonary function measurements are made every 30 min through 6 h.

Levels of Alt a 1, Asp f 1 or Asp f 16-specific IgE are measured by using ELISA. IgE antibodies specific for Alt a 1, Asp f 1 or Asp f 16 are monitored in pooled sera from *Alternaria* or *Aspergillus*-sensitized mice. Plates are coated with Alt a 1, Asp f 1 or Asp f 16 (2 μg/ml). Levels of IgG subclasses, specifically IgG1 and IgG2a, are also measured by ELISA in order to determine if a TH1 or a TH2 response is used.

EXAMPLE 8

VLPs used against dust mite allergies.

Male C57BL/6 mice 6 weeks of age are sensitized to *Dermatophogoldes pteronyssinus* or *Lepidoglyphus destructor* by subcutaneous injection of 10 μg *D. pteronyssinus* or *L. destructor* whole extract on day 0.

On Day 14, mice that are sensitized to *D. pteronyssinus* are immunized with either VLP mixed with 10 μg of *D. pteronyssinus,* VLP mixed with 5 μg Der p 1, Der f 2, and/or Der 2, which is extracted and purified from whole *D. pteronyssinus* extract. Naïve mice, mice receiving VLP alone, mice receiving 10 μg of *D. pteronyssinus* alone, or mice receiving VLP mixed with 5 μg of an irrelevant antigen serve as controls.

On Day 14, mice that are sensitized to L. destructor are immunized with either VLP mixed with 10 μg of L. destructor, VLP mixed with 5 μg Lep d 2, which is extracted and purified from whole L. destructor extract. Naïve mice, mice receiving VLP alone, mice receiving 10 μg of L. destructor alone, or mice receiving VLP mixed with 5 μg of an irrelevant antigen serve as controls.

On day 28, 0.5 ml of peripheral blood from the tail vein is collected, mice are anesthetized with ketamine (90 μg/kg body wt) and xylazine (10 mg/kg body wt) and then are challenged intranasally with 10 μg of *D. pteronyssinus* or *L. destructor.* 72 h following *D. pteronyssinus* or *L. destructor* challenge, 0.5 ml of peripheral blood from the tail vein is collected and lungs are removed. The lungs are infused with 4% paraformaldehyde (in PBS) for 30 min, rinsed with PBS and immersed in 0.5 M sucrose (in PBS) overnight at 4° C. Lungs are inflated and embedded in parafin. Tissues sections are stained with hematoxylin and eosin and the degree of inflammation eosinophil infiltration is quantified by image analysis.

White blood cells are isolated from peripheral blood by centrifugation on a discontinuous Percoll gradient with subsequent hypotonic lysis of remaining red blood cells. White blood cells are isolated from peripheral blood on a discontinuous Percoll gradient. Eosinophils are enriched from both populations by the negative-selection process using anti-CD90 and anti-CD45R antibodies to deplete the B- and T-cell populations using the MACS magnetic bead separation method per the manufacturer's suggested protocol (Miltenyi Biotechnical, Auburn, CA). Eosinophil fractions are routinely enriched to <98%.

Purified peripheral blood eosinophils are resuspended in RPMI-1640 (GIBCO-BRL) and 5% fetal calf serum (GIBCO-BRL) at a cell density of 1 x $10^6$ cells/ml. The cells are stimulated with $10^{-7}$ M phorbol 12-myristate 13-acetate (PMA) and $10^{-7}$ M A-23187 (Sigma) in 96-well plates at 37° C for 30 min, 1 h, and 16 h 5 μg Der p 1, Der f 2, Der 2, or Lep d 2 (20 μg/ml) for 6 days. Following stimulation, the ability of VLPs to reverse the TH2-dominant cytokine secretion profile induced Der p 1, Der f 2, Der 2, or Lep d 2 is analyzed. Specfically, the ability of eosinophils to produce the IFN-γ, IL-4 and IL-5 is analyzed by sandwich ELISA.

Levels of *D. pteronyssinus* or *L. destructor*-specific IgE are measured by using ELISA. IgE antibodies specific for induced Der p 1, Der f 2, Der 2 and Lep d 2 are monitored in pooled sera from D. pteronyssinus or L. destructor-sensitized mice. Plates are coated with Der p 1, Der f 2, Der 2 and Lep d 2 (2 μg/ml). Levels of IgG subclasses, specifically IgG1 and IgG2a, are also measured by ELISA in order to determine if a TH1 or a TH2 response is used.

## EXAMPLE 9

Desensitization of mice against Bee venom challenge Packaging of VLPs with CpG and immunization of mice with VLP (CpG) mixed with Bee venom

VLPs having the sequence as shown in SEQ ID NO: 70 were produced in *E. coli.* and contain amounts of RNA which can be digested and so eliminated by incubating the VLPs with RNase A. The highly active RNase A enzyme used has a molecular weight of about 14 kDa. Recombinantly produced HBc VLPs concentrated at 0.8 mg/ml in PBS buffer pH7.2 were incubated in the absence or presence of RNase A (300 μg/ml, Qiagen AG, Switzerland) for 3h at 37°C. After RNase A digestion VLPs were supplemented with 130nmol/ml CpG oligonucleotides (of the sequence as shown in SEQ ID NO: 69) with phosphorothioate backbone and incubated for 3h at 37°C. VLP preparations for mouse immunization were extensively dialysed (10,000-fold diluted) for 24 h against PBS pH7.2 with a 300 kDa MWCO dialysis membrane (Spectrum Medical Industries Inc., Houston, TX, USA) to eliminate RNase A and the excess of CpG-oligonucleotides.

A group of 13 CBA/J mice have been sensitized by repeated injections of 0.2ug Bee venom (Pharmalgen) and 1mg Alum (Pierce), mixed with PBS, on day 0, 9, 23 and 38. The mice received a total volume of 66ul s.c. (33ul per each side) per injection day. After four times of sensitization the mice were desensitized with VLP(CpG) + Bee venom or with VLP(CpG) alone at day 65, 73, and 80. The first group of seven mice received three injections each of 50ug VLP (CpG) + 5ug Bee venom in PBS. A total volume of 200ul was given s.c. in two doses à 100ul per each side. The second group of six mice received the same amount of VLP(CpG) but no Bee venom following the same immunization schedule as for the first group (d65, d73 and d80). Finally, at day 87 all mice were challenged with 30ug Bee venom s.c. in a total volume of 300ul PBS.

Throughout the description and figures the terms VLP(CpG) and VLP-CpG are used interchangeably and mean VLP packaged with CpG.

## EXAMPLE 10

Assessment of temperature changes and serum analysis of vaccinated mice challenged with Bee venom

In order to assess the protective outcome of the desensitization with the VLP(CpG) conjugates, the body temperature of the mice was measured in 10min. intervals for 1h after the Bee venom challenge (Figure 8). Figure 8 shows allergic body temperature drop in VLP(CpG) + Bee venom vaccinated mice. Two sets of mice have been tested. Group 1 (n = 7) received VLP(CpG) mixed together with Bee venom as vaccine. Group 2 (n = 6) received only VLP (CpG). After the challenge with a high dose of Bee venom (30ug), the allergic reaction was assessed in terms of changes in the body temperature of the mice. In group 1 receiving the Bee venom together with VLP(CpG) no significant changes of the body temperature was observed in any of the tested mice. In contrast, the group 2 receiving only VLP(CpG) as a desensitizing vaccine showed a pronounced body temperature drop in 4 out of 6 animals. Therefore, these mice have not been protected from allergic reactions. Note: The symbols in the figure represent the mean of 6 (for VLP(CpG)) or 7 (VLP(CpG) + Bee venom ) individual mice including standard deviation (SD)

For serological analysis the mice were bled retroorbitally at day 0 (pre-immune), day 58 (after sensitization) and day 86 (after desensitization). The ELISA tests were performed as follows. ELISA plates were coated overnight at 4°C with 5ug Bee venom per 1ml coating buffer (0.1M NaHCO, pH 9.6). The plates were blocked with blocking buffer (2% bovine serum albumin (BSA) in PBS (pH 7.4)/0.05% Tween20) for 2 hours at 37°C, washed with PBS (pH7.4)/ 0.05% Tween20 and then incubated for 2 hours at room temperature with serially diluted mouse sera in blocking buffer. For IgE-detection the immune sera were pre-absorbed on a protein G column. The plates were washed with PBS (pH 7.4)/0.05% Tween20 and then incubated with horse radish peroxidase-labeled goat anti-mouse IgE, IgG1 or IgG2a antibodies at 1ug/ml (Jackson ImmunoResearach) for 1h at room temperature. The plates were washed with PBS (pH 7.4)/0.05% Tween20 and the substrate solution was added (0.066M $Na_2HPO_4$, 0.03 5M citric acid (pH5.0) + 0.4mg OPD (1.2-Phenylenediamine dihydrochloride) + 0.01% $H_2O_2$). After 10min. the color reaction was stopped with 5% $H_2SO_4$ and absorbance was read at 450nm. As a control, pre-immune sera of the same mice were also tested. ELISA titers were presented as optical density ($OD_{450nm}$) of 1:250 (IgE), 1:12500 (IgG1) or 1:500 (IgG2a) diluted sera (Figure 9). Figure 9 shows detection of specific IgE and IgG serum antibodies in mice before and after desensitization. Blood samples of all mice were taken before and after desensitization and tested in ELISA for Bee venom specific IgE antibodies (panel A), IgG1 antibodies (panel B) and IgG2a antibodies (panel C), respectively. As shown in Figure 9A, an increased IgE titer is observed for VLP(CpG) + Bee venom vaccinated mice after desensitization. The results are presented as the optical density (OD450nm) at 1:250 serum dilution. The mean of 6 (VLP(CpG))or 7 (VLP(CpG) + Bee venom) individual mice including standard deviation (SD) is shown in the figure. Figure 9B reveals an increased anti-Bee venom IgG1 serum titer after desensitization only for mice vaccinated with VLP(CpG) + Bee venom. The same is true for Figure 9C were IgG2a serum titers have been determined. As expected for a successful desensitization, the increase in IgG2a antibody titers was most pronounced. The results are shown as means of 2 (VLP(CpG)) or 3 (VLP(CpG) + Bee venom) mice including SD for 1:12500 (IgG1)or 1:500 (IgG2a) serum dilutions, respectively.

EXAMPLE 11

VLPs containing CpG-oligonucleotides induce IgG responses against co-administered grass pollen extract.

VLPs formed by the coat protein of the RNA bacteriophage Qb was used for this experiment. They were used either untreated or after packaging with CpG-2006 oligonucleotides (SEQ-ID NO: 114) having phosphorothioate modifications of the phosphorus backbone. Packaging of CpG-2006 was achieved by incubating 8 ml of a Qb VLP solution (2.2 mg/ml) at 60˚C overnight in the presence of 0.2 ml of a 100 mM $ZnSO_4$ solution. This treatment leads to hydrolysis of the RNA contained in the Qb VLPs. After dialysis against 20 mM Hepes, pH 7.5 using a dialysis tube (cut-off MWCO 300000), CpG-2006 was added at 130 nmol / 1 ml VLP solution and incubated for 3 h at 37˚C under shaking at 650 rpm. Removal of unpackaged CpG-2006 was achieved by subsequent treatment with 50 U/ml Benzonase (Merck) for 3 h at 37˚C in the presence of 1 mM $MgCl_2$ followed by a dialysis against 20 mM Hepes, pH 7.5 as discribed above. Packaging of CpG-2006 was verified by agarose gel electrophoresis stained with ethidium bromide for visualization of nucleic acids and subsequently with Coomassie Blue for visualization of protein. In addition packaged VLPs were analysed on TBE-urea gels and amounts of packaged CpG-oligonucleotides estimated. About 6.7 nmol of CpG-2006 were packaged in 100 ug Qb VLPs.

Female Balb/c mice were subcutaneously immunized with 1.9 B.U. of the grass pollen extract (5-gras-mix Pangramin, Abello, prepared from perennial rye, orchard, timothy, kentucky bluegrass and meadow fescue pollen) mixed with one of the following: 50 μg Qb VLP alone, 50 μg Qb VLP loaded and packaged, respectively, with CpG-2006 or 3 mg aluminium hydroxide (Imject, Pierce). 14 days later, mice were boosted with the same vaccine preparations and bled on day 21. IgG responses in sera from day 21 were assessed by ELISA. As shown in Figure 10, the presence of VLPs loaded and packaged, respectively, with CpG-2006 enhanced the IgG2b response against the pollen extract. No IgE against pollen extract was induced in the presence of Qb VLPs loaded and packaged, respectively, with CpG-2006 while in the presence of Alum a strong IgE response was observed. In contrast to Alum did the Qb-VLP loaded and packaged, respectively, with CpG-2006 not induce IgG1 antibodies. This indicates the absence of a Th2 biased response.

EXAMPLE 12

VLPs containing CpG-oligonucleotides induce IgG responses against co-administered grass pollen extract in allergic mice

VLPs formed by the coat protein of the RNA bacteriophage Qb was used for this experiment. They were used after packaging with CpG-2006 oligonucleotides (SEQ-ID NO: 114) as described in EXAMPLE 11.

Female Balb/c mice were subcutaneously sensitized with 1.9 B.U. of the grass pollen extract (see EXAMPLE 11) mixed with 3 mg aluminium hydroxide (Imject, Pierce). 14 days later, mice were boosted with the same vaccine preparation. One group of mice was left untreated. Further groups underwent desensitization treatment at day 21, day 28 and day 35 by injection of 1.9 B.U. of the grass pollen extract alone or mixed with one of the following: 50 μg Qb VLP alone, 50 μg Qb VLP loaded and packaged, respectively, with CpG-2006 or 3 mg Alum (Imject, Pierce). A further group of mice was desensitized with 50 μg Qb VLP loaded and packaged, respectively, with CpG-2006. IgG responses in sera from days 14, 21, 28, 35 and 42 were assessed by ELISA. As shown in Figure 11, in the presence of pollen and VLPs loaded and packaged, respectively, with CpG-2006 a strong IgG2b response was induced against the pollen extract which was absent in untreated mice or mice treated with pollen extract. The IgG1 response was higher for mice desensitized with Qb VLPs loaded and packaged, respectively, with CpG-2006 than for mice treated with pollen extract alone. Untreated mice and mice treated with Qb VLPs loaded, and packaged, respectively, with CpG-2006 in the absence of pollen did not induce IgG1 antibodies.

EXAMPLE 13

VLPs containing CpG-oligonucleotides induce IgG responses against co-administered tree pollen extract in allergic mice

VLPs formed by the coat protein of the RNA bacteriophage Qb are used for this experiment. They are used after packaging with CpG-2006 oligonucleotides (SEQ-ID NO: 114) as described in EXAMPLE 11.

Female Balb/c mice were subcutaneously sensitized with tree pollen extract. One group of mice receives 2 B.U. of the tree pollen extract mix (3 trees mix, Abello) containing pollen extracts of *Alnus glutinosa, Betula verrucosa* and *Corylus avellana.* A second group receives *Alnus glutinosa* extract only, group three receives *Betula verrucosa* pollen extract only and group four *Corylus avellana* pollen extract only, group five receives japanes cedar (*Cryptomeria japonica*) pollen extract only. 14 days later, mice are boosted with the same vaccine preparation. One group of mice is left untreated. Further groups undergo desensitization treatment at day 21, day 28 and day 35 by injection of 2 B.U. of the same tree pollen extract that was used for sensitization. This corresponding extract is either used alone or mixed with one of the following: 50 μg Qb VLP alone, 50 μg Qb VLP loaded and packaged, respectively, with CpG-2006 or 3 mg aluminium hydroxide (Imject, Pierce). IgG responses in sera from days 14, 21, 28, 35 and 42 are assessed by ELISA.

EXAMPLE 14

VLPs containing CpG-oligonucleotides induce IgG responses against co-administered cat allergen extract in allergic mice

VLPs formed by the coat protein of the RNA bacteriophage Qb are used for this experiment. They are used after packaging with CpG-2006 oligonucleotides (SEQ-ID NO: 114) as described in EXAMPLE 11.

Two groups of female Balb/c mice were subcutaneously sensitized with cat allergen extract corresponding to 0.5 μg and 5 μg Feld1 protein. 14 days later, mice are boosted with the same vaccine preparation. One group of mice is left untreated. Further groups undergo desensitization treatment at day 21, day 28 and day 35 by injection of the same cat allergen extract that was used for sensitization. This corresponding extract is either used alone or mixed with one of the following: 50 μg Qb VLP alone, 50 μg Qb VLP loaded and packaged, respectively, with CpG-2006 or 3 mg aluminium hydroxide (Imject, Pierce). IgG responses in sera from days 14, 21, 28, 35 and 42 are assessed by ELISA.

EXAMPLE 15

VLPs containing G10-PO induce IgG responses against co-administered allergen extract.

VLPs formed by the coat protein of the RNA bacteriophage Qb was used for this experiment. They were used either untreated or after packaging with G10-PO (SEQ-ID NO: 122). Packaging of G10 was achieved by the following method:

*Disassembly:* 45 mg Qβ VLP (as determined by Bradford analysis) in PBS (20 mM Phosphate, 150 mM NaCl, pH 7.8), was reduced with 5 mM DTT for 15 min at RT under stirring conditions. A second incubation of 30 min at RT under stirring conditions followed after addition of magnesium chloride to a final concentration of 700 mM, leading to precipitation of the RNA. The solution was centrifuged 10 min at 10000 g at 4°C in order to isolate the precipitated RNA in the pellet. The disassembled Qβ coat protein dimer, in the supernatant, was used directly for the chromatography purification steps.

*Two-step purification method of disassembled* Qβ *coat protein by cation ion exchange chromatography*: The supernatant of the disassembly reaction, containing disassembled coat protein and remaining RNA, was applied onto a SP-Sepharose FF. During the run, which was carried out at RT with a flow rate of 5ml/min, the absorbance at 260nm and 280nm was monitored. The column was equilibrated with 20mM sodium phosphate buffer pH 7, 150 mM NaCl; the sample was diluted 1:10 to reach a conductivity below 10mS/cm. The elution step (in 5ml fractions) followed with a gradient to 20mM sodium phosphate and 500mM sodium chloride in order to isolate pure Qβ coat protein dimer from contaminants.

Optionally, in a subsequent step, the isolated Qβ coat protein dimer (the eluted fraction from the cation exchange column) was applied onto a Sepharose CL4B (Amersham pharmacia biotech) equilibrated with buffer (20mM sodium phosphate, 250mM sodium chloride; pH 7.2). Absorbance was monitored at 260nm and 280nm and fractions corresponding to the Qb dimer were pooled.

*Reassembly*: Purified Qβ coat protein dimer at a concentration of 1 mg/ml was used for the reassembly of Qβ VLP in the presence of the oligodeoxynucleotide G10-PO. The oligodeoxynucleotide concentration in the reassembly

reaction was of 35μM. The concentration of coat protein dimer in the reassembly solution was 70μM. Urea was added to the solution to give final concentrations of 1M urea. Alternatively, 2.5mM DTT was added in addition to the urea. Sodium chloride was added to a total concentratio of 250mM. The oligodeoxynucleotide to be packaged during the reassembly reaction was added last giving a final volume of the reassembly reaction of 25ml. This solution was first diafiltrated for 100 min against buffer containing 20 mM sodium phosphate, 250 mM NaCl, pH 7.2 using a Pellikon XL Biomax 5 membrane with a MWCO of 5 kDa at room temperature. This was followed by a second diafiltration without or alternatively after incubation with 7 mM hydrogen peroxide for 1 h. In the second diafiltration 20 mM sodium phosphate, 150 mM NaCl, pH 7.2 using a Pellikon XL Biomax 100 membrane with a MWCO of 100 kDa or a membrane with a MWCO of 300 kDa were used.

Analysis of Qβ VLPs which had been reassembled in the presence of oligodeoxynucleotides:

*A) Hydrodynamic size of reassembled capsids:* Qβ capsids, which had been reassembled in the presence of oligodeoxynucleotide G10-PO, were analyzed by dynamic light scattering (DLS) and compared to intact Qβ VLPs, which had been purified from *E.coli.* Reassembled capsids showed a similar hydrodynamic size (which depends both on mass and conformation) as the intact Qβ VLPs.
*B) Disulfide-bond formation in reassembled capsids:* Reassembled Qβ VLPs were analyzed by non-reducing SDS-PAGE and compared to intact Qβ VLPs, which had been purified from *E.coli.* Reassembled capsids displayed a similar disulfide-bond pattern, with the presence of pentamers and hexamers, as the intact Qβ VLPs.
*C) Analysis of nucleic acid content of the Qβ VLPs which had been reassembled in the presence of oligodeoxynucleotides by agarose gelelectrophoresis and by denaturing polyacrylamide TBE-Urea gelelectrophoresis*: Reassembled Qβ VLPs were loaded on a 1% agarose gel and was stained with ethidium bromide and Coomassie Brilliant Blue. Reassembled Qβ VLPs were treated with proteinase K as described in Example 18. The reactions were then mixed with a TBE-Urea sample buffer and loaded on a 15% polyacrylamide TBE-Urea gel. As a qualitative as well as quantitative standard, 10 pmol, 20 pmol and 40 pmol of the oligodeoxynucleotide which was used for the reassembling reaction, was loaded on the same gel. This gel was stained with SYBR®-Gold (Molecular Probes Cat. No. S-11494). The SYBR®-Gold stain showed that the reassembled Qβ capsids contained nucleic acid comigrating with the oligodeoxynucleotides which were used in the reassembly reaction. The agarose gel showed same migration of oligonucleotide stain and protein stain. Taken together, comigration of the nucleic acid content of the Qβ VLPs with protein and isolation of the oligodeoxynucleotide from purified particles by proteinase K digestion, demonstrate packaging of the oligodeoxynucleotide.

Female Balb/c mice were subcutaneously sensitized with grass pollen extract or with cat hair extract as described in EXAMPLES 11 and 14.
One group of each sensitized mouse groups is left untreated. Further groups undergo desensitization treatment at day 21, day 28 and day 35 by injection of same allergen extract that was used for sensitization. The corresponding extract is either used alone or mixed with one of the following: 50 μg Qb VLP alone, 50 μg Qb VLP loaded and packaged, respectively, with G10-PO or 3 mg aluminium hydroxide (Imject, Pierce). IgG responses in sera from days 14,21,28,35 and 42 are assessed by ELISA.

EXAMPLE 16:

Cloning of the AP205 Coat Protein gene

The cDNA of AP205 coat protein (CP) (SEQ ID NO: 90) was assembled from two cDNA fragments generated from phage AP205 RNA by using a reverse transcription-PCR technique and cloning in the commercial plasmid pCR 4-TOPO for sequencing. Reverse transcription techniques are well known to those of ordinary skill in the relevant art. The first fragment, contained in plasmid p205-246, contained 269 nucleotides upstream of the CP sequence and 74 nucleotides coding for the first 24 N-terminal amino acids of the CP. The second fragment, contained in plasmid p205-262, contained 364 nucleotides coding for amino acids 12-131 of CP and an additional 162 nucleotides downstream of the CP sequence. Both p205-246 and p205-262 were a generous gift from J. Klovins.
The plasmid 283.-58 was designed by two-step PCR, in order to fuse both CP fragments from plasmids p205-246 and p205-262 in one full-length CP sequence.
An upstream primer p1.44 containing the *NcoI* site for cloning into plasmid pQb185, or p1.45 containing the *XbaI* site for cloning into plasmid pQb10, and a downstream primer p1.46 containing the *HindIII* restriction site were used (recognition sequence of the restriction enzyme underlined):

p1.44 5'-NNCC ATG GCA AAT AAG CCA ATG CAA CCG-3' (SEQ ID NO: 100)

p1.45 5'-NNTCTAGAATTTTCTGCGCACCCATCCCGG-3' (SEQ ID NO: 101)
p1.46 5'-NNAAGC TTA AGC AGT AGT ATC AGA CGA TAC G-3' (SEQ ID NO: 102)

Two additional primers, p1.47, annealing at the 5' end of the fragment contained in p205-262, and p1.48, annealing at the 3' end of the fragment contained in plasmid p205-246 were used to amplify the fragments in the first PCR. Primers p1.47 and p1.48 are complementary to each other.
p1.47: 5'-GAGTGATCCAACTCGTTTATCAACTACATTT-TCAGCAAGTCTG-3' (SEQ ID NO: 103)
p1.48: 5'-CAGACTTGCTGAAAATGTAGTTGATAAACGA-GTTGGATCACTC-3' (SEQ ID NO: 104)
In the first two PCR reactions, two fragments were generated. The first fragment was generated with primers p1.45 and p1.48 and template p205-246. The second fragment was generated with primers p1.47 and p1.46, and template p205-262. Both fragments were used as templates for the second PCR reaction, a splice-overlap extension, with the primer combination p1.45 and p1.46 or p1.44 and p1.46. The product of the two second-step PCR reactions were digested with *XbaI* or *NcoI* respectively, and *HindIII,* and cloned with the same restriction sites into pQb10 or pQb185 respectively, two pGEM-derived expression vectors under the control of *E.coli* tryptophan operon promoter.
Two plasmids were obtained, pAP283-58 (SEQ ID NO: 91), containing the gene coding for wt AP205 CP (SEQ ID NO: 90) in pQb10, and pAP281-32 (SEQ ID NO: 94) with mutation Pro5→Thr (SEQ ID NO: 93), in pQb185 . The coat protein sequences were verified by DNA sequencing. PAP283-58 contains 49 nucleotides upstream of the ATG codon of the CP, downstream of the XbaI site, and contains the putative original ribosomal binding site of the coat protein mRNA.

EXAMPLE 17:

Expression and Purification of Recombinant AP205 VLP

A. Expression of recombinant AP205 VLP

*E.coli* JM109 was transformed with plasmid pAP283-58. 5 ml of LB liquid medium with 20 μg/ml ampicillin were inoculated with a single colony, and incubated at 37 ˚C for 16-24 h without shaking.
The prepared inoculum was diluted 1:100 in 100-300 ml of LB medium, containing 20 μg/ml ampicillin and incubated at 37 ˚C overnight without shaking. The resulting second inoculum was diluted 1:50 in 2TY medium, containing 0.2 % glucose and phosphate for buffering, and incubated at 37 ˚C overnight on a shaker. Cells were harvested by centrifugation and frozen at -80˚C.

B. Purification of recombinant AP205 VLP

Solutions and buffers:

Lysis buffer
50mM Tris-HCl pH 8.0 with 5mM EDTA , 0.1% tritonX100 and PMSF at 5 micrograms per ml.
SAS
Saturated ammonium sulphate in water
Buffer NET.
20 mM Tris-HCl, pH 7.8 with 5mM EDTA and 150 mM NaCl.
PEG
40% (w/v) polyethylenglycol 6000 in NET

Lysis:

Frozen cells were resuspended in lysis buffer at 2 ml/g cells. The mixture was sonicated with 22 kH five times for15 seconds, with intervals of 1min to cool the solution on ice. The lysate was then centrifuged for 20 minutes at 12 000 rpm, using a F34-6-38 rotor (Eppendorf). The centrifugation steps described below were all performed using the same rotor, except otherwise stated. The supernatant was stored at 4˚ C, while cell debris were washed twice with lysis buffer. After centrifugation, the supernatants of the lysate and wash fractions were pooled.
Ammonium-sulphate precipitation can be further used to purify AP205 VLP. In a first step, a concentration of ammonium-sulphate at which AP205 VLP does not precipitate is chosen. The resulting pellet is discarded. In the next step, an ammonium sulphate concentration at which AP205 VLP quantitatively precipitates is selected, and AP205 VLP is isolated from the pellet of this precipitation step by centrifugation (14 000 rpm, for 20 min). The obtained pellet is solubilised in NET buffer.

Chromatography:

The capsid protein from the pooled supernatants was loaded on a Sepharose 4B column (2.8 X 70 cm), and eluted with NET buffer, at 4 ml/hour/fraction. Fractions 28-40 were collected, and precipitated with ammonium sulphate at 60% saturation.

The fractions were analyzed by SDS-PAGE and Western Blot with an antiserum specific for AP205 prior to precipitation. The pellet isolated by centrifugation was resolubilized in NET buffer, and loaded on a Sepharose 2B column (2.3 X 65 cm), eluted at 3 ml/h/fraction. Fractions were analysed by SDS-PAGE, and fractions 44-50 were collected, pooled and precipitated with ammonium sulphate at 60% saturation.

The pellet isolated by centrifugation was resolubilized in NET buffer, and purified on a Sepharose 6B column (2.5 X 47 cm), eluted at 3 ml/hour/fraction. The fractions were analysed by SDS-PAGE. Fractions 23-27 were collected, the salt concentration adjusted to 0.5 M, and precipitated with PEG 6000, added from a 40% stock in water and to a final concentration of 13.3%. The pellet isolated by centrifugation was resolubilized in NET buffer, and loaded on the same Sepharose 2B column as above, eluted in the same manner. Fractions 43-53 were collected, and precipitated with ammonium sulphate at a saturation of 60%. The pellet isolated by centrifugation was resolubilized in water, and the obtained protein solution was extensively dialyzed against water. About 10 mg of purified protein per gram of cells could be isolated.

Examination of the virus-like particles in Electron microscopy showed that they were identical to the phage particles.

EXAMPLE 18

Immunostimulatory nucleic acids can be packaged into HBcAg VLPs.

HBcAg VLPs, when produced in *E. coli* by expressing the Hepatitis B core antigen fusion protein p33-HBcAg (HBc33) (see Example 1) contain RNA which can be digested and so eliminated by incubating the VLPs with RNase A. It should be noted that the VLPs containing peptide p33 were used only for reasons of convenience, and that wild-type VLPs can likewise be used in the present invention.

*Enzymatic RNA hydrolysis:* Recombinantly produced HBcAg-p33 (HBc33) VLPs at a concentration of 1.0 mg/ml in 1 x PBS buffer (KCl 0.2g/L, KH2PO4 0.2g/L, NaCl 8 g/L, Na2HPO4 1.15 g/L) pH 7.4, were incubated in the presence of 300 μg/ml RNase A (Qiagen AG, Switzerland) for 3 h at 37°C in a thermomixer at 650 rpm.

*Packaging of immunostimulatory nucleic acids*: After RNA digestion with RNAse A HBcAg-p33 VLPs were supplemented with 130 nmol/ml CpG-oligonucleotides B-CpG, NKCpG, G10-PO (Table 1). Similarly, the 150mer single-stranded Cy150-1 and 253mer double stranded dsCyCpG-253, both containing multiple copies of CpG motifs, were added at 130 nmol/ml or 1.2 nmol/ml, respectively, and incubated in a thermomixer for 3 h at 37°C. Double stranded CyCpG-253 DNA was produced by cloning a double stranded multimer of CyCpG into the EcoRV site of pBluescript KS-. The resulting plasmid, produced in *E. coli* XL1-blue and isolated using the Qiagen Endofree plasmid Giga Kit, was digested with restriction endonucleases XhoI and XbaI and resulting restriction products were separated by agarose electrophoresis. The 253 bp insert was isolated by electro-elution and ethanol precipitation. Sequence was verified by sequencing of both strands.

Table 1: Terminology and sequences of immunostimulatory nucleic acids used in the Examples.

| Small letters indicate deoxynucleotides connected via phosphorothioate bonds while large letters indicate deoxynucleotides connected via phosphodiester bonds | | |
|---|---|---|
| Terminology | Sequence | SEQ ID NO |
| CyCpGpt | tccatgacgttcctgaataat | 69 |
| CpG-2006 | tcgtcgttttgtcgttttgtcgt | 114 |
| CyCpG | TCCATGACGTTCCTGAATAAT | 116 |
| B-CpGpt | tccatgacgttcctgacgtt | 117 |
| B-CpG | TCCATGACGTTCCTGACGTT | 118 |
| NKCpGpt | ggggtcaacgttgaggggg | 119 |
| NKCpG | GGGGTCAACGTTGAGGGGG | 120 |
| CyCpG-rev-pt | attattcaggaacgtcatgga | 121 |
| g10gacga-PO (G10-PO) | GGGGGGGGGGGACGATCGTCGGGGGGGGGG | 122 |
| g10gacga-PS (G10-PS) | ggggggggggacgatcgtcgggggggggg | 123 |
| (CpG)200pA | CGCGCGCGCGCGCGCGCGCGCGCGCGCGCGC<br>GCGCGCGAAATGCA<br>TGTCAAAGACAGCAT | 124 |
| Cy(CpG) 20 | TCCATGACGTTCCTGAATAATCGCGCGCGCG<br>CGCGCGCGCGCGCG<br>CGCGCGCGCGCGCG | 125 |
| Cy(CpG)20-OpA | TCCATGACGTTCCTGAATAATCGCGCGCGCG<br>CGCGCGCGCGCGCG<br>CGCGCGCGCGCGCGAAATGCATGTCAAAGAC<br>CAT | 126 |
| CyOpA | TCCATGACGTTCCTGAATAATAAATGCATGT<br>AAGACAGCAT | 127 |

(continued)

| Small letters indicate deoxynucleotides connected via phosphorothioate bonds while large letters indicate deoxynucleotides connected via phosphodiester bonds | | |
|---|---|---|
| Terminology | Sequence | SEQ ID NO |
| CyCyCy | TCCATGACGTTCCTGAATAATTCCATGACGT<br>CTGAATAATTCCAT<br>GACGTTCCTGAATAAT | 128 |
| Cy150-1 | TCCATGACGTTCCTGAATAATTCCATGACGT<br>CTGAATAATTCCAT<br>GACGTTCCTGAATAATTGGATGACGTTGGTG<br>TAATTCCATGACGT<br>TCCTGAATAATTCCATGACGTTCCTGAATAA<br>CCATGACGTTCCTG<br>AATAATTCC | 129 |
| dsCyCpG-253 (complementary strand not shown) | CTAGAACTAGTGGATCCCCCGGGCTGCAGGA<br>TCGATTCATGACTT<br>CCTGAATAATTCCATGACGTTGGTGAATAAT<br>CATGACGTTCCTGA<br>ATAATTCCATGACGTTCCTGAATAATTCCAT<br>CGTTCCTGAATAAT<br>TCCATGACGTTCCTGAATAATTCCATGACGT<br>CTGAATAATTCCAT<br>GACGTTCCTGAATAATTCCATGACGTTCCTG<br>AATTCCAATCAAGC<br>TTATCGATACCGTCGACC | 130 |

*DNAse I treatment:* Packaged HBcAg-p33 VLPs were subsequently subjected to DNaseI digestion (5 U/ml) for 3 h at 37˚C (DNaseI, RNase free Fluka AG, Switzerland) and were extensively dialysed (2 x against 200-fold volume) for 24 h against PBS pH 7.4 with a 300 kDa MWCO dialysis membrane (Spectrum Medical industries Inc., Houston, USA) to eliminate RNAse A and the excess of CpG-oligonucleotides.

*Benzonase treatment:* Since some single stranded oligodeoxynucleotides were partially resistant to DNaseI treatment, Benzonase treatment was used to eliminate free oligonucleotides from the preparation. 100-120 U/ml Benzonase (Merck KGaA, Darmstadt, Germany) and 5 mM $MgCl_2$ were added and incubated for 3 h at 37˚C before dialysis.

*Dialysis:* VLP preparations packaged with immunostimulatroy nucleic acids used in mouse immunization experiments were extensively dialysed (2x against 200fold volume) for 24 h against PBS pH 7.4 with a 300 kDa MWCO dialysis membrane (Spectrum Medical Industries, Houston, US) to eliminate added enzymes and free nucleic acids.

*Analytics of packaging: release of packaged immunostimulatory nucleic acids:* To 50 μl capsid solution 1 μl of proteinase K (600 U/ml, Roche, Mannheim, Germany), 3 μl 10% SDS-solution and 6 μl 10fold proteinase buffer (0.5 M NaCl, 50 mM EDTA, 0.1 M Tris pH 7.4) were added and subsequently incubated overnight at 37˚C. VLPs are completed hydrolysed under these conditions. Proteinase K was inactivated by heating for 20 min at 65˚C. 1 μl RNAse A (Qiagen, 100 μg/ml, diluted 250 fold) was added to 25 μl of capsid. 2-30 μg of capsid were mixed with 1 volume of 2x loading buffer (1xTBE, 42% w/v urea, 12% w/v Ficoll, 0.01 % Bromphenolblue), heated for 3 min at 95˚C and loaded on a 10% (for oligonucleotides of about 20 nt length) or 15% (for > than 40 mer nucleic acids) TBE/urea polyacrylamid gel (Invitrogen). Alternatively samples were loaded on a 1% agarose gel with 6x loading dye (10 mM Tris pH 7.5, 50 mM EDTA, 10% v/v glycerol, 0.4 % orange G). TBE/urea gels were stained with SYBRGold and agarose gels with stained with ethidium bromide.

Fig. 12 shows the packaging of G10-PO oligonucleotides into HBc33. RNA content in the VLPs was strongly reduced after RNaseA treatment (Fig. 12A) while most of the capsid migrated as a a slow migrating smear presumably due to the removal of the negatively charged RNA (Fig. 12B). After incubation with an excess of oligonucleotid the capsids contained a higher amount of nucleic acid than the RNAseA treated capsids and therefore migrated at similar velocity as the untreated capsids. Additional treatment with DNAse I or Benzonase degraded the free oligonucleotides while oligonucleotides packaged in the capsids did not degrade, clearly showing packaging of oligonucleotides. The finding that oligonucleotides restore the migration of the capsids clearly demonstrated packaging of oligonucleotides.

Analogous results and figures have been obtained for the other oligonucleotides used and indicated within this example.

EXAMPLE 19

Qβ Disassembly Reassembly and Packaging.

*Disassembly and Reassembly of Qβ VLP*

*Disassembly*: 10 mg Qβ VLP (also termed interchangeably Qβ capsids) (as determined by Bradford analysis) in 20 mM HEPES, pH 7.4, 150 mM NaCl was precipitated with solid ammonium sulfate at a final saturation of 60%. Precipitation was performed over night at 4˚C and precipitated VLPs were sedimented by centrifugation for 60 minutes at 4˚C (SS-34 rotor). Pellets were resuspended in 1 ml of 6 M Guanidine hydrochloride (GuHCl) containing 100 mM DTT (final concentration) and incubated for 8 h at 4˚C.

*Purification of Qβ coat protein by size exclusion chromatography*: The solution was clarified for 10 minutes at 14000 rpm (Eppendorf 5417 R, in fixed angle rotor F45-30-11, used in all the following steps) and dialysed against a buffer containing 7 M urea, 100 mM TrisHCl, pH 8.0, 10 mM DTT (2000 ml) over night. Dialysis buffer was exchanged once and dialysis continued for another 2 h. The resulting suspension was centrifuged at 14 000 rpm for 10 minutes at 4˚C. A negligible sediment was discarded, and the supernatant was kept as "load fraction" containing dissasembled coat protein and RNA. Protein concentration was determined by Bradford analysis and 5mg total protein was applied onto a HiLoad™ Superdex™ 75 prep grade column (26/60, Amersham Biosciences) equilibrated with 7 M urea, 100 mM TrisHCl and 10 mM DTT. Size exclusion chromatography was performed with the equilibration buffer (7 M urea, 100 mM TrisHCl pH 8.0, 10 mM DTT) at 12˚C with a flow-rate of 0.5 ml/min. During the elution absorbance at 254 nm and 280 nm was monitored. Two peaks were isolated. A high molecular weight peak preceded a peak of lower apparent molecular weight. Peaks were collected in fractions of 1.5 ml and aliquots were analysed by SDS-PAGE followed by Coomassie staining as well as SYBR®Gold staining. This showed that the RNA could be separated from the coat protein which eluted in the second peak.

*Purification of Qβ coat protein by ion exchange chromatography*: Alternatively, the clearified supernatant was dialysed against a buffer containing 7 M urea, 20 mM MES, 10 mM DTT, pH 6.0 (2000 ml) over night Dialysis buffer was exchanged once and dialysis continued for another 2 h. The resulting suspension was centrifuged at 14 000 rpm for 10 minutes at 4˚C. A negligible sediment was discarded, and the supernatant was kept as "load fraction" containing disassembled coat protein and RNA. Protein concentration was determined by Bradford analysis and 10 mg total protein was diluted to a final volume of 10 ml with buffer A (see below) and applied with a flowrate of 1 ml/min to a 1 ml HiTrap™ SP HP column (Amersham Biosciences, Cat. No. 17-1151-01) equilibrated with buffer A: 7 M urea, 20 mM MES, 10 mM DTT, pH 6.0. The flowthrough which contained the RNA was collected as one fraction. After the column was extensively washed with buffer A (30 CV) the bound Qβ coat protein was eluted in a linear gradient from 0% - 100% buffer B (gradient length was 5 CV; buffer A: see above, buffer B: 7 M urea, 20 mM MES, 10 mM DTT, 2 M NaCl, pH 6.0). During the loading, wash and elution the absorbance at 254 nm and 280 nm was monitored. Peak fractions of 1 ml were collected and analysed by SDS-PAGE followed by Coomassie staining as well as SYBR®Gold staining. Fractions containing the Qβ coat protein but not the RNA were identified and the pH was adjusted by addition of 100 μl 1 M TrisHCl, pH 8.0.

Samples containing the Qβ coat protein but no RNA were pooled and dialysed against 0.87 M urea, 100 mM TrisHCl, 10 mM DTT (2000 ml) over night and buffer was exchanged once and dialysis continued for another 2 h. The resulting suspension was centrifuged at 14 000 rpm for 10 minutes at 4°C. A negligible sediment was discarded, and the supernatant was kept as "disassembled coat protein". Protein concentration was determined by Bradford analysis.

*Reassembly*: Purified Qβ coat protein with a concentration of 0.5 mg/ml was used for the reassembly of VLPs in the presence of an oligodeoxynucleotide. For the reassembly reaction the oligodeoxynucleotide was used in a tenfold excess over the calculated theoretical amount of Qβ-VLP capsids (monomer concentration divided by 180). After the Qβ coat protein was mixed with the oligodeoxynucleotide to be packaged during the reassembly reaction, this solution (volume up to 5 ml) was first dialysed for 2 h against 500 ml NET buffer containing 10% β-mercaptoethanol at 4°C, then dialyzed in a continuous mode, with a flow of NET buffer of 8 ml/h over 72 h at 4°C, and finally for another 72 h with the same continous mode with a buffer composed of 20 mM TrisHCl pH 8.0,150 mM NaCl. The resulting suspension was centrifuged at 14 000 rpm for 10 minutes at 4°C. A negligible sediment was discarded, and the supernatant contained the reassembled and packaged VLPs. Protein concentration was determined by Bradford analysis and if needed reassembled and packaged VLPs were concentrated with centrifugal filter devices (Millipore, UFV4BCC25, 5K NMWL) to a final proteinconcentration of 3 mg/ml.

*Purification of reassembled and packaged VLPs*: Up to 10 mg total protein was loaded onto a Sepharose™ CL-4B column (16/70, Amersham Biosciences) equilibrated with 20 mM HEPES pH 7.4, 150 mM NaCl. Size exclusion chromatography was performed with the equilibration buffer (20 mM HEPES pH 7.4, 150 mM NaCl) at room temperature with a flow-rate of 0.4 ml/min. During the elution absorbance at 254 nm and 280 nm was monitored. Two peaks were isolated. A high molecular weight peak preceded a peak of lower apparent molecular weight. Fractions of 0.5 ml were collected and identified by SDS-PAGE followed by Coomassie blue staining. Calibration of the column with intact and highly purified Qβ capsids from *E.coli* revealed that the apparent molecular weight of the major first peak was consistent with Qβ capsids.

Analysis of Qβ VLPs which had been reassembled in the presence of oligodeoxynucleotides:

*A) Overall structure of the capsids:* Qβ VLPs that were reassembled either in the presence of one of the following oligodeoxynucleotides (CyOpA (SEQ ID NO: 127), Cy(CpG)200pA (SEQ ID NO: 126), Cy(CpG)20 (SEQ ID NO: 125), CyCyCy (SEQ ID NO: 128), (CpG)200pA) (SEQ ID NO: 124), or in the presence of tRNA from *E.coli* (Roche Molecular Biochemicals, Cat. No. 109541) were analyzed by electron microscopy (negative staining with uranylacetate pH 4.5) and compared to intact Qβ VLPs purified from *E.coli.* As a negative control served a reassembly reaction where nucleic acid was omitted. Reassembled capsids display the same structural features and properties as the intact Qβ VLPs (Figure 13).

*B) Hydrodynamic size of reassembled capsids:* Qβ capsids which had been reassembled in the presence of oligodeoxynucleotides were analyzed by dynamic light scattering (DLS) and compared to intact Qβ VLPs which had been purified from *E.coli*.Reassembled capsids showed the same hydrodynamic size (which depends both on mass and conformation) as the intact Qβ VLPs.

*C) Disulfide-bond formation in reassembled capsids:* Reassembled Qβ VLPs were analyzed by native polyacrylamid gelelectrophoresis and compared to intact Qβ VLPs which had been purified from *E.coli.* Reassembled capsids displayed the same disulfide-bond pattern as the intact Qβ VLPs.

*D) Analysis of nucleic acid content of the Qβ VLPs which had been reassembled in the presence of oligodeoxynucleotides by agarose gelelectrophoresis*: 5 μg reassembled Qβ VLPs were incubated in total reaction volume of 25 μl either with 0.35 units RNase A (Qiagen, Cat. No. 19101), 15 units DNAse I (Fluka, Cat. No. 31136), or without any further addition of enzymes for 3 h at 37°C. Intact Qβ VLPs which had been purified from *E.coli* served as control and were incubated under the same conditions as described for the capsids which had been reassembled in the presence of oligodeoxynucleotides. The reactions were then loaded on a 0.8% agarose gel that was first stained with ethidumbromide (Figure 14A) and subsequently with Coomassie blue (Figure 14B). The ethidium bromide stain shows, that none of the added enzymes could digest the nucleic acid content in the reassembled Qβ capsids showing that the nucleic acid content (i.e. the oligodeoxynucleotides) is protected. This result indicates that the added oligodeoxynucleotides were packaged into the newly formed capsids during the reassembly reaction. In contrast, the nucleic acid content in the intact Qβ VLPs which had been purified from *E.coli* was degraded upon addition of RNase A, indicating that the nucleic acid content in this VLPs consists of RNA. In addition, both the ethidium bromide stain and the Coomasie blue stain of the agarose gel shows that the nucleic acid containing Qβ VLPs (reassembled and purified from *E.coli,* respectively) are migrating at about the same size, which indicates that the reassembly reaction led to Qβ VLPs of comparable size to intact Qβ VLPs which had been purified from *E. coli.*

The gel thus shows that DNAse I protected oligodeoxynucleotides were present in the reassembled Qβ VLP. Furthermore, -after the packaged oligodeoxynuleotides had been extracted by phenol/chloroform they were digestable by DNAse I, but not by RNAse A. Oligodeoxynucleotides could thus be successfully packaged into Qβ VLPs after initial disassembly of the VLP, purification of the disassembled coat protein from nucleic acids and subsequent reassembly of the VLPs in the presence of oligodeoxynucleotides.

*E) Analysis of nucleic acid content of the Qβ VLPs which had been reassembled in the presence of oligodeoxynucleotides by denaturing polyacrylamide TBE-Urea gelelectrophoresis:* 40 μg reassembled Qβ VLPs (0.8 mg/ml) were incubated in a total reaction volume of 60 μl with 0.5 mg/ml proteinase K (PCR-grade, Roche Molecular Biochemicals, Cat. No. 1964364) and a reaction buffer according to the manufacturers instructions for 3 h at 37˚C. Intact Qβ VLPs which had been purified from *E.coli* served as control and were incubated with proteinase K under the same conditions as described for the capsids which had been reassembled in the presence of oligodeoxynucleotides. The reactions were then mixed with a TBE-Urea sample buffer and loaded on a 15% polyacrylamide TBE-Urea gel (Novex®, Invitrogen Cat. No. EC6885). As a qualitative as well as quantitative standard, 1 pmol, 5 pmol and 10 pmol of the oligodeoxynucleotide which was used for the reassembling reaction, were loaded onto the same gel. This gel was fixed with 10% acetic acid, 20% methanol, equilibrated to neutral pH and stained with SYBR®-Gold (Molecular Probes Cat. No. S-11494). The SYBR®-Gold stain showed, that the reassembled Qβ capsids contained nucleic acid comigrating with the oligodeoxynucleotides which were used in the reassembly reaction. Note that intact Qβ VLPs (which had been purified from *E.coli*) did not contain a nucleic acid of similar size. Taken together, analysis of the nucleic acid content of the Qβ VLPs which had been reassembled in the presence of oligodeoxynucleotides showed that oligodeoxynucleotides were protected from DNase I digestion, meaning that they were packaged) and that the added oligodeoxynucleotides could be reisolated by proper means (e.g. proteinase K digestion of the Qβ VLP).

Figure 13 shows electron micrographs of Qβ VLPs that were reassembled in the presence of different oligodeoxynucleotides. The VLPs had been reassembled in the presence of the indicated oligodeoxynucleotides or in the presence of tRNA but had not been purified to a homogenous suspension by size exclusion chromatography. As positive control served preparation of "intact" Qβ VLPs which had been purified from *E.coli.* Importantly, by adding any of the indicated nucleic acids during the reassembly reaction, VLPs of the correct size and conformation could be formed, when compared to the "positive" control. This implicates that the reassembly process in general is independent of the nucleotide sequence and the length of the used oligodeoxynucleotides. Note that adding of nucleic acids during the reassembly reaction is required for the formation of Qβ VLPs, since no particles had been formed if nucleic acids were omitted from the reassembly reaction.

Figure 14 shows the analysis of nucleic acid content of the reassembled Qβ VLPs by nuclease treatment and agarose gelelectrophoresis: 5 μg of reassembled and purified Qβ VLPs and 5 μg of Qβ VLPs which had been purified from *E. coli,* respectively, were treated as indicated. After this treatment, samples were mixed with loading dye and loaded onto a 0.8% agarose gel. After the run the gel was stained first with ethidium bromide (A) and after documentation the same gel was stained with Coomassie blue (B). Note that the nucleic acid content of the reassembled and purified Qβ VLPs were resistant towards RNase A digestion while the nucleic acid content of Qβ VLPs purified from *E.coli* was digested upon incubation with RNase A. This indicates that the nucleic acid content of the reassembled Qβ capsids consists out of deoxynucleotides which of course are protected from RNase A digestion. Hence, oligodeoxynucleotides were packaged into Qβ VLPs during the reassembly reaction.

EXAMPLE 20

AP205 Disassembly-Purification-Reassembly and Packaging of immunostimulatory nucleic acids.

*A. Disassembly and Reassembly of AP205 VLP from material able to reassemble without addition of oligonucleotide*

*Disassembly:* 40 mg of lyophilized purified AP205 VLP (SEQ-ID: 90 or 93) were resolubilized in 4 ml 6 M GuHCl, and incubated overnight at 4˚C. The disassembly mixture was centrifuged at 8000 rpm (Eppendorf 5810 R, in fixed angle rotor F34-6-38, used in all the following steps). The pellet was resolubilized in 7 M urea, while the supernatant was dialyzed 3 days against NET buffer (20 mM Tris-HCl, pH 7.8 with 5mM EDTA and 150 mM NaCl) with 3 changes of buffer. Alternatively, dialysis was conducted in continuous mode over 4 days. The dialyzed solution was centrifuged at 8000 rpm for 20 minutes, and the pellet was resolubilized in 7 M urea, while the supernatant was pelletted with ammonium sulphate (60% saturation), and resolubilized in a 7 M urea buffer containing 10 mM DTT. The previous pellets all resolubilized in 7 M urea were joined, and precipitated with ammonium sulphate (60% saturation), and resolubilized in a 7 M urea buffer containing 10 mM DTT. The materials resolubilized in the 7 M urea buffer containing 10 mM DTT were joined and loaded on a Sephadex G75 column equilibrated and eluted with the 7 M urea buffer containing 10 mM DTT at 2ml/h. One peak eluted from the column. Fractions of 3 ml were collected. The peak fractions containing AP205 coat protein were pooled and precipitated with ammonium sulphate (60% saturation). The pellet was isolated by centrifugation at 8000 rpm, for 20 minutes. It was resolubilized in 7 M urea, 10 mM DTT, and loaded on a short Sepharose 4B column (1.5 X 27 cm Sepharose 4B, 2 ml/h, 7 M urea, 10 mM DTT as elution buffer). Mainly one peak, with a small shoulder eluted from the column. The fractions containing the AP205 coat protein were identified by SDS-PAGE, and pooled, excluding the shoulder. This yielded a sample of 10.3 ml. The protein concentration was estimated spectrophotometrically by measuring an aliquot of protein diluted 25-fold for the measurement, using the following formula: (1.55 x OD280 - 0.76 x OD260) x volume. The average concentration was of 1 nmol/ml of VLP (2.6 mg/ml). The ratio of absorbance at 280 nm vs. 260 nm was of 0.12/0.105.

*Reassembly:* 1.1 ml beta-mercaptoethanol was added to the sample, and the following reassembly reactions were set up:

1 ml of AP205 coat protein, no nucleic acids
1 ml of AP205 coat protein, rRNA (approx. 200 OD260 units, 10 nmol)
9 ml of AP205 coat protein, CyCpG (370 ul of 225 pmol/μl solution, i.e. 83 nmol).

These mixtures were dialyzed 1 hour against 30 ml of NET buffer containing 10% beta-mercaptoethanol. The mixture containing no nucleic acids was dialyzed separately. The dialysis was then pursued in a continuous mode, and 1 l of NET buffer was exchanged over 3 days. The reaction mixtures were subsequently extensively dialyzed against water (5 changes of buffer), and lyophilized. They were resolubilized in water, and analyzed by EM. All mixtures contained capsids, showing that AP205 VLP reassembly is independent of the presence of detectable nucleic acids, as measured by agarose gel electrophoresis using ethidium bromide staining and evidenced by EM analysis. The EM procedure was as follows: A suspension of the proteins was absorbed on carbon-formvar coated grids and stained with 2% phosphotungstic acid (pH 6,8). The grids were examined with a JEM 100C (JEOL,Japan) electron microscope at an accelerating voltage of 80 kV. Photographic records (negatives) were performed on Kodak electron image film and electron micrographs were obtained by printing of negatives on Kodak Polymax paper.The VLP reassembled in the presence of the CyCpG was purified over a Sepharose 4B column (1 X 50 cm), eluted with NET buffer (1 ml/h). The fractions were analyzed by Ouchterlony assay, and the fractions containing VLP were pooled. This resulted in a sample of 8 ml, which was desalted against water by dialysis, and dried. The yield of capsid was of 10 mg. Analysis of resolubilized material in a 0.6% agarose gel stained with ethidium-bromide showed that the capsids were empty of nucleic acids. Samples of the reassembly reaction containing CyCpG taken after the reassembly step and before extensive dialysis were analysed on a 0.6% agarose gel. A band migrating at the same height than intact AP205 VLP and staining both for ethidium-bromide and Coomassie blue staining could be obtained, showing that AP205 VLP containing oligodeoxynucleotide had been reassembled. The extensive dialysis steps following the reassembly procedure are likely to have led to diffusion of the oligodeoxynucleotide outside of the VLPs. Significantly, the AP205 VLPs could also be reassembled in the absence of detectable oligodeoxynucleotide, as measured by agarose gel electrophoresis using ethidium bromide staining. Oligodeoxynucleotides could thus be successfully bound to AP205 VLP after initial disassembly of the VLP, purification of the disassembled coat protein from nucleic acids and subsequent reassembly of the VLP in the presence of oligodeoxynucleotide.

*B. Reassembly of AP205 VLP using disassembled material which does not reassemble in the absence of added oligonucleotide*

*Disassembly:* 100 mg of purified and dried recombinant AP205 VLP were used for disassembly as described above. All steps were performed essentially as described under disassembly in part A, but for the use of 8 M urea to solublize the pellets of the ammonium sulphate precipitation steps and the omission of the gel filtration step using a CL-4B column prior to reassembly. The pooled fractions of the Sephadex G-75 column contained 21 mg of protein as determined by spectroscopy using the formula described in part A. The ratio of absorbance at 280 nm to the absorbance at 260 nm of the sample was of 0.16 to 0.125. The sample was diluted 50 times for the measurement.

*Reassembly:* The protein preparation resulting from the Sephadex G-75 gel filtration purification step was precipitated with ammonium sulphate at 60% saturation, and the resulting pellet solubilized in 2 ml 7 M urea, 10 mM DTT. The sample was diluted with 8 ml of 10% 2-mercaptoethanol in NET buffer, and dialyzed for 1 hour against 40 ml of 10% 2-mercaptoethanol in NET buffer. Reassembly was initiated by adding 0.4 ml of a CyCpG solution (109 nmol/ml) to the protein sample in the dialysis bag. Dialysis in continous mode was set up, and NET buffer used as eluting buffer. Dialysis was pursued for two days and a sample was taken for EM analysis after completion of this dialysis step (Figure 44 B). The dialyzed reassembly solution was subsequently dialyzed against 50% v/v Glycerol in NET buffer, to achieve concentration. One change of buffer was effected after one day of dialysis. The dialysis was pursued over a total of three days.

The dialyzed and concentrated reassembly solution was purified by gel filtration over a Sepharose 4-B column (1X60 cm) at a flow rate of 1 ml/hour, in NET buffer. Fractions were tested in an Ouchterlony assay, and fractions containing capsids were dried, resuspended in water, and rechromatographed on the 4-B column equilibrated in 20 mM Hepes pH 7.6. Using each of the following three formula:

$$1.\ (183 * OD^{230\,nm} - 75.8 * OD^{260\,nm}) * \text{volume (ml)} - 2.\ ((OD^{235\,nm} - OD^{280\,nm})/2.51) \times \text{volume} - 3.\ ((OD^{228.5\,nm} - OD^{234.5\,nm}) * 0.37) \times \text{volume}$$

protein amounts of 6-26 mg of reassembled VLP were determined.

The reassembled AP205 VLPs were analyzed by EM as described above, agarose gel electrophoresis and SDS-PAGE under non-reducing conditions.

The EM analysis of disassembled material shows that the treatment of AP205 VLP with guanidinium-chloride essentially disrupts the capsid assembly of the VLP. Reassembly of this disassembled material with an oligonucleotide yielded capsids (Figure 15B), which were purified and further enriched by gel filtration (Figure 15 C). Two sizes of particles were obtained; particles of about 25 nm diameter and smaller particles are visible in the electron micrograph of Figure 44C. No reassembly was obtained in the absence of oligonucleotides. Loading of the reassembled particles on agarose electrophoresis showed that the reassembled particles contained nucleic acids. Extraction of the nucleic acid content by phenol extraction and subsequent loading on an agarose gel stained with ethidium bromide revealed that the particles contained the oligonucleotide used for reassembly (Figure 45A). Identity of the packaged oligonucleotide was controlled by loading a sample of this oligonucleotide side to side to the nucleic acid material extracted from the particles. The agarose gel where the reassembled AP205 VLP had been loaded and previously stained with ethidium bromide was subsequently stained with Coomassie blue, revealing comigration of the oligonucleotide content with the protein content of the particles (Figure 16B), showing that the oligonucleotide had been packaged in the particles. Loading of the reassembled AP205 VLP on an SDS-PAGE gel, run in the absence of reducing agent demonstrated that the reassembled particles have formed disulfide bridges, as is the case for the untreated AP205 VLP. Moreover, the disulfide bridge pattern is identical to the untreated particles.

Depicted on Figure 15 A is an electron micrograph of the disassembled AP205 VLP protein, while Figure 15 B shows the reassembled particles before purification. Figure 15C shows an electron micrograph of the purified reassembled AP205 VLPs. The magnification of Figure 15A-C is 200 000 X.

Figure 16 A and B show the reassembled AP205 VLPs analyzed by agarose gel electrophoresis. The samples loaded on the gel from both figures were, from left to right: untreated AP205 VLP, 3 samples with differing amount of AP205 VLP reassembled with CyCpG and purified, and untreated Qβ VLP. The gel on Figure 16A was stained with ethidium bromide, while the same gel was stained with Coomassie blue in Figure 16 B.

EXAMPLE 21

Immunostimulatory nucleic acids can be packaged into Qβ VLPs.

*Coupling of p33 peptides to Qβ VLPs:*

Recombinantly produced virus-like particles of the RNA-bacteriophage Qb (Qβ VLPs) were used untreated or after coupling to p33 peptides containing an N-terminal CGG or and C-terminal GGC extension (CGG-KAVYNFATM (SEQ ID NO: 115) and KAVYNFATM-GGC (SEQ ID NO: 131)). Recombinantly produced Qβ VLPs were derivatized with a 10 molar excess of SMPH (Pierce) for 0.5 h at 25˚C, followed by dialysis against 20 mM HEPES, 150 mM NaCl, pH 7.2 at 4˚C to remove unreacted SMPH. Peptides were added in a 5 fold molar excess and allowed to react for 2 h in a thermomixer at 25˚C in the presence of 30% acetonitrile. Figure 17 shows the SDS-PAGE analysis demonstrating multiple coupling bands consisting of one, two or three peptides coupled to the Qβ monomer (Arrows, Figure 17). For the sake of simplicity the coupling product of the peptide p33 and Qβ VLPs was termed, in particular, throughout the example section Qbx33. It should be noted that the VLPs containing peptide p33 were used only for reasons of convenience, and that wild-type VLPs can likewise be used in the present invention.

Qβ VLPs, when produced in *E. coli* by expressing the bacteriophage Qβ capsid protein, contain RNA which can be digested and so eliminated by incubating the VLPs with RNase A.

*Low ionic strength and low Qβ concentration allow RNA hydrolysis of Qβ VLPs by RNAse A:*

Qβ VLPs at a concentration of 1.0 mg/ml in 20mM Hepes/150mM NaCl buffer (HBS) pH 7.4 were either digested directly by addition of RNase A (300 μg/ml, Qiagen AG, Switzerland) or were diluted with 4 volumes $H_2O$ to a final 0.2 x HBS concentration and then incubated with RNase A (60 μg/ml, Qiagen AG, Switzerland). Incubation was allowed for 3 h at 37˚C in a thermomixer at 650 rpm. Agarose gel electrophoresis and ethidium bromide staining demonstrate that in 1xHBS only a very weak reduction of RNA content was observed, while in 0.2x HBS most of the RNA was hydrolysed. In agreement, capsid migration was unchanged after addition of RNAse A in 1x HBS, while migration was slower after addition of RNAse in 0.2xHBS.

*Low ionic strength increases nucleic acid packaging in Qβ VLPs:*

After RNase A digestion in 0.2 x HBS the Qβ VLPs were concentrated to 1 mg/ml using Millipore Microcon or Centriplus concentrators and aliquots were dialysed against 1x HBS or 0.2 x HBS. Qβ VLPs were supplemented with 130 nmol/ml CpG-oligonucleotide B-CpG and incubated in a thermomixer for 3 h at 37˚C. Subsequently Qβ VLPs were subjected to Benzonase digestion (100 U/ml) for 3 h at 37˚C. Samples were analysed on 1% agarose gels after staining with ethidium bromide or Coomassie Blue. It was shown that in 1x HBS only a very low amount of oligonucleotides could be packaged, while in 0.2 x HBS a strong ethidium bromide stained band was detectable, which colocalized with the Coomassie blue stain of the capsids.

*Different immunostimulatory nucleic acids can be packaged in Qβ and Qbx33 VLPs:*

After RNase A digestion in 0.2 x HBS the Qβ VLPs or Qbx33 VLPs were concentrated to 1 mg/ml using Millipore Microcon or Centriplus concentrators and supplemented with 130 nmol/ml CpG-oligonucleotides B-CpGpt, g10gacga and the 253 mer dsCyCpG-253 (Table 1) and incubated in a thermomixer for 3 h at 37˚C. Subsequently Qβ VLPs or Qbx33 VLPs were subjected to DNAse I digestion (5 U/ml) or Benzonase digestion (100 U/ml) for 3 h at 37˚C. Samples were analysed on 1% agarose gels after staining with ethidium bromide or Coomassie Blue. Figure 18 shows that the different nucleic acids B-CpGpt, g10gacga and the 253mer dsDNA could be packaged into Qbx33. Packaged nucleic acids were resistant to DNAse I digestion and remained packaged during dialysis (Figure 18). Packaging of B-CpGpt was confirmed by release of the nucleic acid by proteinase K digestion followed by agarose electrophoresis and ethidium bromide staining (Figure 18C).

Figure 18 depicts the analysis of B-CpGpt packaging into Qbx33 VLPs on a 1% agarose gel stained with ethidium bromide (A) and Coomassie Blue (B). Loaded on the gel are 50 μg of the following samples: 1. Qbx33 VLP untreated; 2. Qbx33 VLP treated with RNase A; 3. Qbx33 VLP treated with RNase A and packaged with B-CpGpt; 4. Qb33 VLP treated with RNase A, packaged with B-CpGpt, treated with DNaseI and dialysed; 5. 1 kb MBI Fermentas DNA ladder. (C) depicts the analysis of the amount of packaged oligo extracted from the VLP on a 15% TBE/urea stained with SYBR Gold. Loaded on gel are the following samples: 1. BCpGpt oligo content of 2 μg Qbx33 VLP after proteinase K digestion and RNase A treatment; 2. 20 pmol B-CpGpt control; 3. 10 pmol B-CpGpt control; 4. 5 pmol B-CpGpt control

Figure 18 D and E depict the analysis of g10gacga-PO packaging into Qbx33 VLPs on a 1% agarose gel stained with ethidium bromide (D) and Coomassie Blue (E). Loaded on the gel are 15 μg of the following samples: 1. MBI Fermentas 1 kb DNA ladder; 2. Qbx33 VLP untreated; 3. Qbx33 VLP treated with RNase A; 4. Qbx33 VLP treated with RNase A and packaged with g10gacga-PO; 5. Qbx33 VLP treated with RNase A, packaged with g10gacga-PO, treated with Benzonase and dialysed.

Figure 18 E and F depict the analysis of dsCyCpG-253 packaging into Qbx33 VLPs on a 1% agarose gel stained with ethidium bromide (E) and Coomassie Blue (F). Loaded on the gel are 15 μg of the following samples: 1. MBI Fermentas 1 kb DNA ladder; 2. Qbx33 VLP untreated; 3. Qbx33 VLP treated with RNase A; 4. Qbx33 VLP treated with RNase A, packaged with dsCyCpG-253 and treated with DNaseI; 5. Qbx33 VLP treated with RNase A, packaged with dsCyCpG-253, treated with DNaseI and dialysed.

EXAMPLE 22

Packaging of immunostimulatory nucleic acids into VLPs.

*RNaseA and $ZnSO_4$ mediated degradation of the nucleic acid content of a VLP.*

Qβ VLPs were treated with RNaseA as described in Example 21 under low ionic strength conditions (20 mM Hepes pH 7.4 or 4 mM Hepes, 30 mM NaCl, pH 7.4). Alternatively, Qβ VLPs and AP205 VLPs were treated with $ZnSO_4$ under low ionic strength conditions (20 mM Hepes pH 7.4 or 4 mM Hepes, 30 mM NaCl pH 7.4) similar as described in Example 11. AP205 VLP (1 mg/ml) in either 20 mM Hepes pH 7.4 or 20 mM Hepes, 1 mM Tris, pH 7.4 was treated for 48 h with 2.5 mM $ZnSO_4$ at 50˚C in an Eppendorf Thermomixer comfort at 550 rpm. Qβ and AP205 VLP samples were centrifuged at 14000 rpm and supernatants were dialysed in 10.000 MWCO Spectra/Por® dialysis tubing (Spectrum, Cat. nr. 128 118) against first 2120 mM Hepes, pH 7.4 for 2 h at 4˚C and, after buffer exchange, overnight. Samples were clarified after dialysis similar as described in Example 11 and protein concentration in the supernatants was determined by Bradford analysis.

*Packaging of ISS into RnaseA and $ZnSO_4$ treated VLPs.*

After RNA hydrolysis and dialysis, Qβ and AP205 VLPs (1-1.5 mg/ml) were mixed with 130 μl of CpG oligonucleotides (NKCpG - cf. Table 1; G3-6, G8-8 - cf. Table 2; 1 mM oligonucleotide stock in 10 mM Tris pH 8) per ml of VLPs. Samples were incubated for 3 h at 37˚C in a thermoshaker at 650 rpm. Subsequently, samples were treated with 125 U Benzonase/ml VLPs (Merck KGaA, Darmstadt, Germany) in the presence of 2 mM $MgCl_2$ and incubated for 3 h at 37˚C before dialysis. Samples were dialysed in 300.000 MWCO Spectra/Por® dialysis tubing (Spectrum, Cat. nr. 131 447) against 20 mM Hepes, pH 7.4 for 2 h at 4˚C, and after buffer exchange overnight against the same buffer. After dialysis samples were centrifuged at 14000 rpm and protein concentration in the supernatants were determined by Bradford analysis.

Agarose gel electrophoresis and subsequent staining with ethidium bromide and Coomassie Blue showed that oligonucleotides were packaged in the VLPs.

EXAMPLE 23

Packaging of immunostimulatory guanosine flanked oligonucleotides into VLPs.

Qbx33 VLPs (Qβ VLPs coupled to peptide p33, see Example 21) were treated with RNaseA under low ionic conditions (20 mM Hepes pH 7.4) as described in Example 21 to hydrolyse RNA content of the Qbx33 VLP. After dialysis against 20 mM Hepes pH 7.4, Qbx33 VLPs were mixed with guanosine flanked oligonucleotides (Table 2: G3-6, G7-7, G8-8, G9-9 or G6, from a 1 mM oligonucleotide stock in 10 mM Tris pH 8) and incubated as described in Example 22. Subsequently, Qbx33 VLPs were treated with Benzonase and dialysed in 300.000 MWCO tubing. Samples with oligos G7-7, G8-8 and G9-9 were extensively dialysed over 3 days with 4 buffer exchanges to remove free oligo. Packaging was confirmed on 1% agarose gels and, after proteinase K digestion, on TBE/urea gels.

Table 2. Sequences of immunostimulatory nucleic acids used in the Examples.

| Small letters indicate deoxynucleotides connected via phosphorothioate bonds while larger letters indicate deoxynucleotides connected via phosphodiester bonds | | |
|---|---|---|
| ISS name | 5'-3' sequence | SEQ ID NO |
| | GACGATCGTC | 105 |
| G3-6 | GGGGACGATCGTCGGGGGG | 106 |
| G4-6 | GGGGGACGATCGTCGGGGGG | 107 |
| G5-6 | GGGGGGACGATCGTCGGGGGG | 108 |
| G6-6 | GGGGGGGACGATCGTCGGGGGG | 109 |
| G7-7 | GGGGGGGGACGATCGTCGGGGGGG | 110 |
| G8-8 | GGGGGGGGGACGATCGTCGGGGGGGG | 111 |
| G9-9 | GGGGGGGGGGACGATCGTCGGGGGGGGG | 112 |

| | | |
|---|---|---|
| G6 | GGGGGGCGACGACGATCGTCGTCGGGGGG | 113 |

EXAMPLE 24

Packaging ribonucleic acid into VLPs.

*$ZnSO_4$ dependent degradation of the nucleic acid content of a VLP.*

Qβ VLPs were treated with $ZnSO_4$ under low ionic strength conditions (20 mM Hepes pH 7.4 or 4 mM Hepes, 30 mM NaCl, pH 7.4) similar as described in Example 11. AP205 VLPs (1 mg/ml) in either 20 mM Hepes pH 7.4 or 20 mM Hepes, 1 mM Tris, pH 7.4 were treated for 48 h with 2.5 mM ZnSO4 at 50˚C in an Eppendorf Thermomixer comfort at 550 rpm. Qβ and AP205 VLP samples were centrifuged at 14000 rpm and dialysed against 20 mM Hepes, pH 7.4 as in Example 22.

*Packaging of poly (I:C) into $ZnSO_4$-treated VLPs:*

The immunostimulatory ribonucleic acid poly (I:C), (Cat. nr. 27-4732-01, poly(I)-poly(C), Pharmacia Biotech) was dissolved in PBS (Invitrogen cat. nr. 14040) or water to a concentration of 4 mg/ml (9μM). Poly (I:C) was incubated for 10 minutes at 60˚C and then cooled to 37˚C. Incubated poly (I:C) was added in a 10-fold molar excess to either $ZnSO_4$-treated Qβ or AP205 VLPs (1-1.5 mg/ml) and the mixtures were incubated for 3 h at 37˚C in a thermomixer at 650 rpm. Subsequently, excess of free poly (I:C) was enzymatically hydrolysed by incubation with 125 U Benzonase per ml VLP mixture in the presence of 2 mM $MgCl_2$ for 3 h at 37˚C in a thermomixer at 300 rpm. Upon Benzonase hydrolysis samples were centrifuged at 14000 rpm and supernatants were dialysed in 300.000 MWCO Spectra/Por® dialysis tubing (Spectrum, Cat nr. 131 447) against 2120 mM Hepes, pH 7.4 for 2 h at 4˚C, and after buffer exchange overnight against the same buffer.. After dialysis, samples were centrifuged at 14000 rpm and protein concentration in the supernatants were determined by Bradford analysis.
Packaging is confirmed on 1% agarose gels and, after proteinase K digestion, on TBE/urea gels.

EXAMPLE 25

Packaging of immunostimulatory guanosine flanked oligonucleotides into HBcAg VLPs.

HBcAg VLPs are treated with RNaseA under low ionic strength conditions (20 mM Hepes pH 7.4) as described in Example 21 to hydrolyse RNA content of the VLP. After dialysis against 20 mM Hepes, pH 7.4, VLPs are mixed with guanosine flanked oligonucleotides (Table 2; G3-6, CG7-7, G8-8, G9-9, G10-PO or G6, 1 mM stock in 10 mM Tris pH 8) and incubated as described in Example 22. Subsequently, VLPs are treated with Benzonase and dialysed in 300.000 MWCO tubing. Packaging is analysed on 1% agarose gels and on TBE/urea gels after proteinase K digestion.

EXAMPLE 26

Packaging ribonucleic acid into HBcAg VLPs.

HBcAg VLPs are treated with $ZnSO_4$ under low ionic strength conditions (20 mM Hepes pH 7.4 or 4 mM Hepes, 30 mM NaCl, pH 7.4 ) similar as described in Example 11 and are dialysed against 20 mM Hepes pH 7.4 as in Example 22. Poly (I:C) is added in a 10-fold molar excess to HBcAg VLPs (1-1.5 mg/ml) and incubated for 3 h at 37˚C in a thermomixer at 650 rpm as described in Example 24. Subsequently, excess of free poly (I:C) is enzymatically hydrolysed by incubation with 125 U Benzonase per ml VLP mixture in the presence of 2 mM $MgCl_2$ for 3 h at 37˚C in a thermomixer at 300 rpm. Samples are clarified after Benzonase hydrolysis similar as described in Example 11 and dialysed as in Example 24. After dialysis, samples are centrifuged at 14000 rpm and protein concentration in the supernatants are determined by Bradford analysis.

EXAMPLE 27

Qβ Disassembly Reassembly and Packaging.

*Disassembly and Reassembly of Qβ VLP*

*Disassembly:* 45 mg Qβ VLP (as determined by Bradford analysis) in PBS (20 mM Phosphate, 150 mM NaCl, pH 7.5), was reduced with 10 mM DTT for 15 min at RT under stirring conditions. A second incubation of 15 min at RT under stirring conditions followed after addition of magnesium chloride to a final concentration of 700 mM, leading to precipitation of the RNA. The solution was centrifuged 10 min at 4000 rpm at 4˚C (Eppendorf 5810 R, in fixed angle rotor A-4-62 used in all following steps) in order to isolate the precipitated RNA in the pellet. The disassembled Qβ coat protein dimer, in the supernatant, was used directly for the chromatography purification steps.

*Two-step purification method of disassembled Qβ coat protein by cation ion exchange chromatography and size exclusion chromatography*: The supernatant of the disassembly reaction, containing disassembled coat protein and remaining RNA, was applied onto a SP-Sepharose FF (16/20; 6ml; Amersham pharmacia biotech). During the run, which was carried out at RT with a flow rate of 5ml/min, the absorbance at 260nm and 280nm was monitored. The column was equilibrated with 20mM sodium phosphate buffer pH 7; the sample was diluted 1:10 to reach a conductivity below 9mS/cm (dilution to this conductivity was necessary, and was done using 0.5x equilibration buffer). The elution step (in 5ml fractions) followed with a gradient to 20mM sodium phosphate and 500mM sodium chloride in order to isolate pure Qβ coat protein dimer from contaminants. The column was regenerated with 0.5M NaOH.

In the second step, the isolated Qβ coat protein dimer (the eluted fraction from the cation exchange column) was applied (in two runs) onto a Sephacryl S-100 HR column (26/60; 320ml; Amersham pharmacia biotech) equilibrated with buffer (20mM sodium phosphate, 150mM sodium chloride; pH 6.5). Chromatography was performed at RT with a flow rate of 2.5ml/min. Absorbance was monitored at 260nm and 280nm. Fractions of 5 ml were collected. The column was regenerated with 0.5 M NaOH.

*Reassembly*: Purified Qβ coat protein dimer at a concentration of 2 mg/ml was used for the reassembly of Qβ VLP in the presence of the oligodeoxynucleotide G8-8. The oligodeoxynucleotide concentration in the reassembly reaction was of 10μM. The concentration of coat protein dimer in the reassembly solution was 40μM. Urea and DTT were added to the solution to give final concentrations of 1M urea and 5mM DTT respectively. The oligodeoxynucleotide to be packaged during the reassembly reaction was added last, together with $H_2O$, giving a final volume of the reassembly reaction of 3ml. This solution was first dialysed for 72 h against 1500 ml buffer containing 20 mM TrisHCl, 150 mM NaCl, pH 8.0 at 4˚C. The dialysed reassembly mixture was centrifuged at 14 000 rpm for 10 minutes at 4˚C. A negligible sediment was discarded while the supernatant contained the reassembled and packaged VLPs. Protein concentration was determined by Bradford analysis. Reassembled and packaged VLPs were concentrated with centrifugal filter devices (Millipore, UFV4BCC25, 5KNMWL) to a final protein concentration of 3 mg/ml.

*Purification of reassembled and packaged VLPs*: Up to 10 mg total protein was loaded onto a Sepharose™ CL-4B column (16/70, Amersham Biosciences) equilibrated with 20 mM HEPES, 150 mM NaCl, pH 7.4. Size exclusion chromatography was performed with the equilibration buffer (20 mM HEPES, 150 mM NaCl, pH 7.4) at room temperature at a flow-rate of 0.4 ml/min. Absorbance was monitored at 254 nm and 280 nm. Two peaks were isolated. A high molecular weight peak preceded a peak of lower apparent molecular weight. Fractions of 0.5 ml were collected and Qb VLPs containing fractions identified by SDS-PAGE followed by Coomassie blue staining. Calibration of the column with intact and highly purified Qβ capsids from *E.coli* revealed that the apparent molecular weight of the major first peak was consistent with Qβ capsids.

Analysis of Qβ VLPs which had been reassemble in the presence of oligodeoxynucleotides:

*A) Hydrodynamic size of reassembled capsids:* Qβ capsids, which had been reassembled in the presence of oligodeoxynucleotide G8-8, were analyzed by dynamic light scattering (DLS) and compared to intact Qβ VLPs, which had been purified from *E.coli.* Reassembled capsids showed the same hydrodynamic size (which depends both on mass and conformation) as the intact Qβ VLPs.

*B) Disulfide-bond formation in reassembled capsids:* Reassembled Qβ VLPs were analyzed by non-reducing SDS-PAGE and compared to intact Qβ VLPs, which had been purified from *E.coli.* Reassembled capsids displayed the same disulfide-bond pattern, with the presence of pentamers and hexamers, as the intact Qβ VLPs.

*C) Analysis of nucleic acid content of the Qβ VLPs which had been reassembled in the presence of oligodeoxynucleotides by denaturing polyacrylamide TBE-Urea gelelectrophoresis*: Reassembled Qβ VLPs (0.4 mg/ml) containing G8-8 oligonucleotides were incubated for 2 h at 37˚C with 125 U benzonase per ml Qβ VLPs in the presence of 2 mM $MgCl_2$. Subsequently the benzonase treated Qβ VLPs were treated with proteinase K (PCR-grade, Roche Molecular Biochemicals, Cat. No. 1964364) as described in Example 11. The reactions were then mixed with a TBE-Urea sample buffer and loaded on a 15% polyacrylamide TBE-Urea gel (Novex®, Invitrogen Cat. No. EC6885).

As a qualitative as well as quantitative standard, 1 pmol, 5 pmol and 10 pmol of the oligodeoxynucleotide which was used for the reassembling reaction, was loaded on the same gel. This gel was stained with SYBR®-Gold (Molecular Probes Cat. No. S-11494). The SYBR®-Gold stain showed that the reassembled Qβ capsids contained nucleic acid comigrating with the oligodeoxynucleotides which were used in the reassembly reaction. Taken together, resistance to benzonase digestion of the nucleic acid content of the Qβ VLPs which had been reassembled in the presence of oligodeoxynucleotides and isolation of the oligodeoxynucleotide from purified particles by proteinase K digestion, demonstrate packaging of the oligodeoxynucleotide.

EXAMPLE 28

VLPs containing G10-PO induce Th1 type responses against co-administered grass pollen extract in the presence of Alum.

VLPs formed by the coat protein of the RNA bacteriophage Qb was used for this experiment. They were used either untreated or after packaging with G10-PO (SEQ-ID: 122 ) as described in Example 15. Female Balb/c mice were subcutaneously immunized with 1.9 B.U. of the grass pollen extract (5-gras-mix Pangramin, Abello, prepared from perennial rye, orchard, timothy, kentucky bluegrass and meadow fescue pollen) mixed with Alum (Imject, Pierce) in the presence of 50 μg Qb VLP alone or 50 μg Qb VLP loaded and packaged, respectively with G10-PO. A control group of mice received pollen extract mixed with Alum only. 50 days later, mice were boosted with the same vaccine preparations and bled on day 57. IgG responses in sera from day 57 were assessed by ELISA. The control group showed anti-pollen antibodies of the IgG1 isotype, but none of the IgG2a isotype. The presence of VLPs loaded with G10-PO induced a IgG2a response against the pollen extract. No IgE against pollen extract was induced in the presence of Qb VLPs loaded, and packaged, respectively, with G10-PO while in the presence of Alum only an IgE response was observed. This indicates that G10-PO loaded into VLPs is able to induce a Th1 response and suppress the Alum induced IgE production.

## Claims

1. A composition for enhancing an immune response in an animal comprising:

   (a) a virus-like particle;
   (b) an immunostimulatory substance, wherein said immunostimulatory substance is an unmethylated CpG-containing oligonucleotide, and wherein said immunostimulatory substance is packaged within said virus-like particle (a); and
   (c) an antigen, wherein said antigen is an allergen, and wherein said antigen is mixed with said virus-like particle (a).

2. The composition of claim 1, wherein the CpG motif of said unmethylated CpG-containing oligonucleotide is part of a palindromic sequence, and wherein preferably said palindromic sequence is GACGATCGTC (SEQ ID NO:105), and wherein further preferably said palindromic sequence is flanked at its 5'-terminus by at least 4 and at most 9 guanosine entities, and wherein said palindromic sequence is flanked at its 3'-terminus by at least 6 and at most 9 guanosine entities.

3. The composition of claim 1, wherein said unmethylated CpG-containing oligonucleotide has a nucleic acid sequence selected from:

   (a) GGGGACGATCGTCCGGGGG (SEQ ID NO:106);
   (b) GGGGGACGATCGTCGGGGGG (SEQ ID NO:107);
   (c) GGGGGGACGATCGTCGGGGGG (SEQ ID NO:108);
   (d) GGGGGGGACGATCGTCGGGGGG (SEQ ID NO:109);
   (e) GGGCCGGGACGATCGTCGGGGGGG (SEQ ID NO:110);
   (f) GGGGGGCGGACGATCGTCGGOGGGCG (SEQ ID NO:111);
   (g) GGGGGGGGGGACGATCGTCGGGGGGGGG (SEQ ID NO:112); and
   (h) GGGGGGCGACGACGATCGTCGTCGGGGGGG (SEQ ID NO:113).

4. The composition of claim 1, wherein said unmethylated CpG-containing oligonucleotide comprises, or alternatively consists essentially of, or alternatively consists of the sequence GACGATCGTC GGGGGGGGG (SEQ ID NO:122).

5. The composition of claim 1, wherein said unmethylated CpG-containing oligonucleotide consists of the sequence GGGGGGGGGG GACGATCGTC GGGGGGGGGG (SEQ ID NO:122).

6. The composition of any one of the preceding claims, wherein said unmethylated CpG-containing oligonucleotide is not stabilized by phosphorothioate modifications of the phosphodiester backbone.

7. The composition of any one of the preceding claims, wherein said immunostimulatory substance is not accessible to DNAse hydrolysis.

8. The composition of any one of the preceding claims, wherein said virus-like particle comprises recombinant proteins, or fragments thereof, of an RNA-phage, wherein preferably said RNA-phage is selected from the group consisting of:

   (a) bacteriophage Qβ;
   (b) bacteriophage R17;
   (c) bacteriophage fr;
   (d) bacteriophage GA;
   (e) bacteriophage SP;
   (f) bacteriophage MS2;
   (g) bacteriophage M11;
   (h) bacteriophage MX1;

(i) bacteriophage NL95;
(j) bacteriophage f2;
(k) bacteriophage PP7; and
(l) bacteriophage AP205.

**9.** The composition of any one of the preceding claims, wherein said virus-like particle comprises recombinant proteins, or fragments thereof, of a RNA-phage, wherein said RNA-phage is Qβ.

**10.** The composition of claim 9, wherein said recombinant proteins comprise, consist essentially of, or alternatively consist of coat proteins having an amino acid sequence of SEQ m NO:1.

**11.** The composition of any one of claims 1 to 7, wherein said virus-like particle comprises recombinant proteins of an RNA-phage, wherein said recombinant proteins consist of coat proteins having an amino acid sequence of SEQ ID NO: 1.

**12.** The composition of any one of the preceding claims, wherein said antigen (c) is isolated from a natural source, wherein preferably said natural source is selected from the group consisting of:

(a) pollen extract;
(b) dust extract;
(c) dust mite extract;
(d) fungal extract;
(f) mammalian epidermal extract;
(g) feather extract;
(h) insect extract;
(i) food extract,
(j) hair extract;
(k) saliva extract, and
(l) serum extract.

**13.** The composition of any one of claims 1 to 11, wherein said allergen is derived from pollen extract.

**14.** The composition of any one of claims 1 to 11, wherein said allergen is derived from dust extract or dust mite extract.

**15.** The composition of any one of claims 1 to 11, wherein said allergen is selected from the group consisting of:

(a) trees;
(b) grasses;
(c) house dust;
(d) house dust mite;
(c) aspergillus;
(f) animal hair;
(g) animal feather;
(h) bee venom;
(i) animal products; and
(j) plant products.

**16.** A vaccine comprising an immunologically effective amount of the composition of any one of claims 1 to 15, together with a pharmaceutically acceptable diluent, carrier or excipient, wherein preferably said vaccine further comprises an adjuvant, and wherein further preferably said adjuvant is Alum or incomplete Freund's adjuvant.

**17.** Use of a composition according to any one of claims 1 to 15 or use of a vaccine according to claim 16 in the manufacture of a pharmaceutical for the treatment of allergies.

**18.** A composition according to any one of claims 1 to 15 or vaccine according to claim 16 for use in the prevention and/or treatment of allergies.

**19.** A composition comprising:

(a) a virus-like particle; and
(b) an immunostimulatory substance, wherein said immunostimulatory substance is an unmethylated CpG-containing oligonucleotide, and wherein said immunostimulatory substance is packaged within said virus-like particle; and
(c) an antigen, wherein said antigen is an allergen, and wherein said antigen is mixed with said virus-like particle (a);

for use in a method for enhancing an immune response in an animal, preferably in a human, comprising introducing said composition into said animal; wherein preferably said composition is further defined as in any of claims 2 to 15.

**20.** The composition for use according to claim 19, wherein said immune response is an enhanced B cell response, an enhanced T cell response or a CTL response, and wherein preferably said T cell response is a Th cell response, and wherein further preferably said Th cell response is a Th1 cell response.

**21.** The composition for use according to claim 19, wherein said composition is introduced into said animal subcutaneously, intramuscularly, intravenously, intranasally or directly into the lymph node.

**22.** Use of a composition comprising:

(a) a virus-like particle; and
(b) an immunostimulatory substance, wherein said immunostimulatory substance is an unmethylated CpG-containing oligonucleotide, and wherein said immunostimulatory substance is packaged within said virus-like particle; and
(c) an antigen, wherein said antigen is an allergen, and wherein said antigen is mixed with said virus- like particle (a);

for the manufacture of a composition for enhancing an immune response in an animal, preferably in a human, wherein said composition is to be introduced into said animal, and wherein preferably said composition is further defined as in any of claims 2 to 15.

**23.** The use of claim 22 wherein said immune response is an enhanced B cell response, an enhanced T cell response or a CTL response, and wherein preferably said T cell response is a Th cell response, and wherein further preferably said Th cell response is a Th1 cell response.

**24.** The use of claim 22, wherein said composition is introduced into said animal subcutaneously, intramuscularly, intravenously, intranasally or directly into the lymph node.

**25.** An immunologically effective amount of the vaccine according to claim 16 for use in a method of immunizing or treating an animal comprising administering said amount to said animal, wherein preferably said animal is a mammal, and further preferably a human.

**Patentansprüche**

**1.** Zusammensetzung zum Verstärken einer Immunantwort in einem Tier, umfassend:

(a) ein virusartiges Partikel;
(b) eine immunstimulatorische Substanz, wobei die immunstimulatorische Substanz ein unmethyliertes CpG enthaltendes Oligonukleotid ist, und wobei die immunstimulatorische Substanz in das virusartige Partikel (a) verpackt ist; und
(c) ein Antigen, wobei das Antigen ein Allergen ist, und wobei das Antigen mit dem virusartigen Partikel (a) vermischt ist.

**2.** Zusammensetzung nach Anspruch 1, wobei das CpG-Motiv des unmethylierten CpG enthaltenden Oligonukleotids Teil einer palindromischen Sequenz ist und wobei die palindromische Sequenz vorzugsweise GACGATCGTC (SEQ ID NO:105) ist, und wobei ferner die palindromische Sequenz vorzugsweise an ihrem 5'-Terminus durch mindestens 4 und höchstens 9 Guanosineinheiten flankiert ist, und wobei die palindromische Sequenz an ihren 3'-Terminus durch mindestens 6 und höchstens 9 Guanosineinheiten flankiert ist.

3. Zusammensetzung nach Anspruch 1, wobei das unmethylierte CpG enthaltende Oligonukleotid eine Nukleinsäuresequenz hat, ausgewählt aus:

(a) GGGGACGATCGTCGGGGGG (SEQ ID NO:106);
(b) GGGGGACGATCGTCGGGGGG (SEQ ID NO:107);
(c) GGGGGGACGATCGTCGGGGGG (SEQ ID NO:108);
(d) GGGGGGGACGATCGTCGGGGGG (SEQ ID NO:109);
(e) GGGGGGGGACGATCGTCGGGGGGG (SEQ ID NO:110);
(f) GGGGGGGGGACGATCGTCGGGGGGGG (SEQ ID NO:111);
(g) GGGGGGGGGGACGATCGTCGGGGGGGGG (SEQ ID NO:112); und
(h) GGGGGGCGACGACGATCGTCGTCGGGGGGG (SEQ ID NO:113).

4. Zusammensetzung nach Anspruch 1, wobei das unmethylierte CpG enthaltende Oligonukleotid umfasst oder alternativ im Wesentlichen besteht aus oder alternativ besteht aus der Sequenz GGGGGGGGGG GACGATCGTC GGGGGGGGGG (SEQ ID NO:122).

5. Zusammensetzung nach Anspruch 1, wobei das unmethylierte CpG enthaltende Oligonukleotid aus der Sequenz GGGGGGGGGG GACGATCGTC GGGGGGGGGG (SEQ ID NO:122) besteht.

6. Zusammensetzung nach einem beliebigen der vorhergehenden Ansprüche, wobei das unmethylierte CpG enthaltende Oligonukleotid nicht durch Phosphorthioatmodifikationen des Phosphodiester-Rückgrats stabilisiert ist.

7. Zusammensetzung nach einem beliebigen der vorhergehenden Ansprüche, wobei die immunstimulatorische Substanz nicht DNAse-Hydrolyse zugänglich ist.

8. Zusammensetzung nach einem beliebigen der vorhergehenden Ansprüche, wobei das virusartige Partikel rekombinante Proteine oder Fragmente davon eines RNA-Phagen umfasst, wobei der RNA-Phage vorzugsweise ausgewählt ist aus der Gruppe bestehend aus:

(a) Bacteriophage Qβ;
(b) Bacteriophage R17;
(c) Bacteriophage fr;
(d) Bacteriophage GA;
(e) Bacteriophage SP;
(f) Bacteriophage MS2;
(g) Bacteriophage M11;
(h) Bacteriophage MX1;
(i) Bacteriophage NL95;
(j) Bacteriophage f2;
(k) Bacteriophage PP7; und
(l) Bacteriophage AP205.

9. Zusammensetzung nach einem beliebigen der vorhergehenden Ansprüche, wobei das virusartige Partikel rekombinante Proteine oder Fragmente davon eines RNA-Phagen umfasst, wobei der RNA-Phage Qβ ist.

10. Zusammensetzung nach Anspruch 9, wobei die rekombinanten Proteine umfassen, im Wesentlichen bestehen aus oder alternativ bestehen aus Hüllproteinen mit einer Aminosäuresequenz von SEQ ID NO:1.

11. Zusammensetzung nach einem beliebigen der Ansprüche 1 bis 7, wobei das virusartige Partikel rekombinante Proteine eines RNA-Phagen umfasst, wobei die rekombinanten Proteine aus Hüllproteinen mit einer Aminosäuresequenz von SEQ ID NO:1 bestehen.

12. Zusammensetzung nach einem beliebigen der vorhergehenden Ansprüche, wobei das Antigen (c) aus einer natürlichen Quelle isoliert ist, wobei die natürliche Quelle vorzugsweise ausgewählt ist aus der Gruppe bestehend aus:

(a) Pollenextrakt;
(b) Staubextrakt;
(c) Staubmilbenextrakt;

(d) Pilzextrakt;
(f) Säugerepidermextrakt;
(g) Federextrakt;
(h) Insektenextrakt;
(i) Lebensmittelextrakt;
(j) Haarextrakt;
(k) Speichelextrakt, und
(l) Serumextrakt.

**13.** Zusammensetzung nach einem beliebigen der Ansprüche 1 bis 11, wobei das Allergen aus Pollenextrakt stammt.

**14.** Zusammensetzung nach einem beliebigen der Ansprüche 1 bis 11, wobei das Allergen aus Staubextrakt oder Staubmilbenextrakt stammt.

**15.** Zusammensetzung nach einem beliebigen der Ansprüche 1 bis 11, wobei das Allergen ausgewählt ist aus der Gruppe bestehend aus:

(a) Bäumen;
(b) Gräsern;
(c) Hausstaub;
(d) Hausstaubmilbe;
(e) Aspergillus;
(f) Tierhaar;
(g) Tierfeder;
(h) Bienengift;
(i) Tierprodukten; und
(j) Pflanzenprodukten.

**16.** Vakzine, umfassend eine immunologisch wirksame Menge der Zusammensetzung nach einem beliebigen der Ansprüche 1 bis 15, zusammen mit einem pharmazeutisch verträglichen Verdünnungsmittel, Träger oder Hilfsstoff, wobei die Vakzine vorzugsweise ferner ein Adjuvans umfasst, und wobei ferner das Adjuvans vorzugsweise Alum oder unvollständiges Freund'sches Adjuvans ist.

**17.** Verwendung einer Zusammensetzung gemäß einem beliebigen der Ansprüche 1 bis 15 oder Verwendung einer Vakzine gemäß Anspruch 16 in der Herstellung eines Pharmazeutikums zur Behandlung von Allergien.

**18.** Zusammensetzung gemäß einem beliebigen der Ansprüche 1 bis 15 oder Vakzine gemäß Anspruch 16 zur Verwendung in der Prävention und/oder Behandlung von Allergien.

**19.** Zusammensetzung, umfassend:

(a) ein virusartiges Partikel; und
(b) eine immunstimulatorische Substanz, wobei die immunstimulatorische Substanz ein unmethyliertes CpG enthaltendes Oligonukleotid ist, und wobei die immunstimulatorische Substanz in das virusartige Partikel verpackt ist; und
(c) ein Antigen, wobei das Antigen ein Allergen ist, und wobei das Antigen mit dem virusartigen Partikel (a) vermischt ist;

zur Verwendung in einem Verfahren zum Verstärken einer Immunantwort in einem Tier, vorzugsweise in einem Menschen, umfassend das Einführen der Zusammensetzung in das Tier; wobei vorzugsweise die Zusammensetzung weiterhin definiert ist wie in einem beliebigen der Ansprüche 2 bis 15.

**20.** Zusammensetzung zur Verwendung gemäß Anspruch 19, wobei die Immunantwort eine verstärkte B-Zell-Antwort, eine verstärkte T-Zell-Antwort oder eine CTL-Antwort ist, und wobei vorzugsweise die T-Zell-Antwort eine Th-Zell-Antwort ist, und wobei ferner die Th-Zell-Antwort vorzugsweise eine Th1-Zell-Antwort ist.

**21.** Zusammensetzung zur Verwendung gemäß Anspruch 19, wobei die Zusammensetzung in das Tier subkutan, intramuskulär, intravenös, intranasal oder direkt in den Lymphknoten eingeführt wird.

**22.** Verwendung einer Zusammensetzung, umfassend:

(a) ein virusartiges Partikel; und
(b) eine immunstimulatorische Substanz, wobei die immunstimulatorische Substanz ein unmethyliertes CpG enthaltendes Oligonukleotid ist, und wobei die immunstimulatorische Substanz in das virusartige Partikel verpackt ist; und
(c) ein Antigen, wobei das Antigen ein Allergen ist, und wobei das Antigen mit dem virusartigen Partikel (a) vermischt ist;

zur Herstellung einer Zusammensetzung zum Verstärken einer Immunantwort in einem Tier, vorzugsweise in einem Menschen, wobei die Zusammensetzung in das Tier einzuführen ist, und wobei die Zusammensetzung ferner vorzugsweise definiert ist wie in einem beliebigen der Ansprüche 2 bis 15.

**23.** Verwendung nach Anspruch 22, wobei die Immunantwort eine verstärkte B-Zell-Antwort, eine verstärkte T-Zell-Antwort oder eine CTL-Antwort ist, und wobei vorzugsweise die T-Zell-Antwort eine Th-Zell-Antwort ist, und wobei ferner vorzugsweise die Th-Zell-Antwort eine Th1-Zell-Antwort ist.

**24.** Verwendung nach Anspruch 22, wobei die Zusammensetzung in das Tier subkutan, intramuskulär, intravenös, intranasal oder direkt in den Lymphknoten eingeführt wird.

**25.** Immunologisch wirksame Menge der Vakzine gemäß Anspruch 16 zur Verwendung in einem Verfahren des Immunisierens oder Behandelns eines Tiers, umfassend das Verabreichen der Menge an das Tier, wobei vorzugsweise das Tier ein Säuger ist und ferner vorzugsweise ein Mensch.

**Revendications**

**1.** Composition destinée à améliorer une réponse immunitaire chez un animal comprenant :

(a) une particule analogue à un virus ;
(b) une substance immunostimulante, où ladite substance immunostimulante est un oligonucléotide contenant CpG non méthylé, et où ladite substance immunostimulante est intégrée dans ladite particule analogue à un virus (a) ; et
(c) un antigène, où ledit antigène est un allergène, et où ledit antigène est mélangé avec ladite particule analogue à un virus (a).

**2.** Composition selon la revendication 1, dans laquelle le motif CpG dudit oligonucléotide contenant CpG non méthylé fait partie d'un palindrome, et dans laquelle de préférence ledit palindrome est GACGATCGTC (SEQ ID NO : 105), et dans laquelle de manière encore préférée ledit palindrome est flanqué par au moins 4 et au plus 9 entités de guanosine à son extrémité 5'-terminale, et dans laquelle ledit palindrome est flanqué par au moins 6 et au plus 9 entités de guanosine à son extrémité 3'-terminale.

**3.** Composition selon la revendication 1, dans laquelle ledit oligonucléotide contenant CpG non méthylé possède une séquence d'acide nucléique choisie parmi :

(a) GGGGACGATCGTCGGGGGG (SEQ ID NO : 106) ;
(b) GGGGGACGATCGTCGGGGGG (SEQ ID NO : 107) ;
(c) GGGGGGACGATCGTCGGGGGG (SEQ ID NO : 108) ;
(d) GGGGGGGACGATCGTCGGGGGG (SEQ ID NO : 109) ;
(e) GGGGGGGGACGATCGTCGGGGGGG (SEQ ID NO : 110) ;
(f) GGGGGGGGGACGATCGTCGGGGGGGG (SEQ ID NO : 111) ;
(g) GGGGGGGGGGACGATCGTCGGGGGGGGG (SEQ ID NO : 112) ; et
(h) GGGGGGCGACGACGATCGTCGTCGGGGGGG (SEQ ID NO : 113).

**4.** Composition selon la revendication 1, dans laquelle ledit oligonucléotide contenant CpG non méthylé comprend, ou en variante est essentiellement constitué de, ou en variante est constitué de la séquence GGGGGGGGGG GACGATCGTC GGGGGGGGGG (SEQ ID NO : 122).

5. Composition selon la revendication 1, dans laquelle ledit oligonucléotide contenant CpG non méthylé est constitué de la séquence GGGGGGGGGG GACGATCGTC GGGGGGGGGG (SEQ ID NO : 122).

6. Composition selon l'une quelconque des revendications précédentes, dans laquelle ledit oligonucléotide contenant CpG non méthylé n'est pas stabilisé par des modifications phosphorothioate du squelette de phosphodiester.

7. Composition selon l'une quelconque des revendications précédentes, dans laquelle ladite substance immunostimulante n'est pas accessible à une hydrolyse par ADNase.

8. Composition selon l'une quelconque des revendications précédentes, dans laquelle ladite particule analogue à un virus comprend des protéines recombinées, ou des fragments de celles-ci, d'un phage à ARN, dans laquelle de préférence ledit phage à ARN est choisi dans le groupe constitué des :

(a) bactériophage Qβ ;
(b) bactériophage R17 ;
(c) bactériophage fr ;
(d) bactériophage GA ;
(e) bactériophage SP ;
(f) bactériophage MS2 ;
(g) bactériophage M11 ;
(h) bactériophage MX1 ;
(i) bactériophage NL95 ;
(j) bactériophage f2 ;
(k) bactériophage PP7 ; et
(l) bactériophage AP205.

9. Composition selon l'une quelconque des revendications précédentes, dans laquelle ladite particule analogue à un virus comprend des protéines recombinées, ou des fragments de celles-ci, d'un phage à ARN, où ledit phage à ARN est Qβ.

10. Composition selon la revendication 9, dans laquelle lesdites protéines recombinées comprennent, sont essentiellement constituées de, ou en variante sont constituées de protéines d'enveloppe possédant une séquence d'acides aminés de SEQ ID NO : 1.

11. Composition selon l'une quelconque des revendications 1 à 7, dans laquelle ladite particule analogue à un virus comprend des particules recombinées d'un phage à ARN, dans laquelle lesdites protéines recombinées sont constituées de protéines d'enveloppe possédant une séquence d'acides aminés de SEQ ID NO : 1.

12. Composition selon l'une quelconque des revendications précédentes, dans laquelle ledit antigène (c) est isolé à partir d'une source naturelle, dans laquelle de préférence ladite source naturelle est choisie dans le groupe constitué des :

(a) extrait de pollen ;
(b) extrait de poussière ;
(c) extrait d'acariens de la poussière ;
(d) extrait fongique ;
(f) extrait épidermique de mammifère ;
(g) extrait de plume ;
(h) extrait d'insecte ;
(i) extrait alimentaire ;
(j) extrait de poil ;
(k) extrait de salive, et
(l) extrait de sérum.

13. Composition selon l'une quelconque des revendications 1 à 11, dans laquelle ledit allergène est dérivé d'extrait de pollen.

14. Composition selon l'une quelconque des revendications 1 à 11, dans laquelle ledit allergène est dérivé d'extrait de poussière ou d'extrait d'acariens de la poussière.

**15.** Composition selon l'une quelconque des revendications 1 à 11, dans laquelle ledit allergène est choisi dans le groupe constitué des :

(a) arbres ;
(b) graminées ;
(c) poussières domestiques ;
(d) acarien de la poussière domestique ;
(e) aspergillus ;
(f) poil d'animal ;
(g) plume d'animal ;
(h) venin d'abeille ;
(i) produits animaux ; et
(j) produits végétaux.

**16.** Vaccin comprenant une quantité efficace sur le plan immunologique de la composition selon l'une quelconque des revendications 1 à 15, conjointement avec un diluant, véhicule ou excipient pharmaceutiquement acceptable, dans lequel de préférence ledit vaccin comprend de plus un adjuvant, et dans lequel de manière encore préférée ledit adjuvant est de l'alun ou un adjuvant incomplet de Freund.

**17.** Utilisation d'une composition selon l'une quelconque des revendications 1 à 15 ou utilisation d'un vaccin selon la revendication 16, dans la fabrication d'un produit pharmaceutique pour le traitement d'allergies.

**18.** Composition selon l'une quelconque des revendications 1 à 15 ou vaccin selon la revendication 16, pour une utilisation dans la prévention et/ou le traitement d'allergies.

**19.** Composition comprenant :

(a) une particule analogue à un virus ; et
(b) une substance immunostimulante, où ladite substance immunostimulante est un oligonucléotide contenant CpG non méthylé, et où ladite substance immunostimulante est intégrée dans ladite particule analogue à un virus ; et
(c) un antigène, où ledit antigène est un allergène, et où ledit antigène est mélangé avec ladite particule analogue à un virus (a) ;

pour une utilisation dans un procédé destiné à améliorer une réponse immunitaire chez un animal, de préférence chez un humain, comprenant l'introduction de ladite composition dans ledit animal ; où de préférence ladite composition est de plus définie comme dans l'une quelconque des revendications 2 à 15.

**20.** Composition pour une utilisation selon la revendication 19, dans laquelle ladite réponse immunitaire est une réponse de cellule B améliorée, une réponse de cellule T améliorée ou une réponse CTL, et dans laquelle de préférence ladite réponse de cellule T est une réponse de cellule Th, et dans laquelle de manière encore préférée ladite réponse de cellule Th est une réponse de cellule Th1.

**21.** Composition pour une utilisation selon la revendication 19, dans laquelle ladite composition est introduite dans ledit animal par voie sous-cutanée, par voie intramusculaire, par voie intraveineuse, par voie intranasale ou directement dans le ganglion lymphatique.

**22.** Utilisation d'une composition comprenant :

(a) une particule analogue à un virus ; et
(b) une substance immunostimulante, où ladite substance immunostimulante est un oligonucléotide contenant CpG non méthylé, et où ladite substance immunostimulante est intégrée dans ladite particule analogue à un virus ; et
(c) un antigène, où ledit antigène est un allergène, et où ledit antigène est mélangé avec ladite particule analogue à un virus (a) ;

pour la fabrication d'une composition destinée à améliorer une réponse immunitaire chez un animal, de préférence chez un humain, où ladite composition doit être introduite dans ledit animal, et où de préférence ladite composition est de plus définie comme dans l'une quelconque des revendications 2 à 15.

**23.** Utilisation selon la revendication 22, dans laquelle ladite réponse immunitaire est une réponse de cellule B améliorée, une réponse de cellule T améliorée ou une réponse CTL, et dans laquelle de préférence ladite réponse de cellule T est une réponse de cellule Th, et dans laquelle de manière encore préférée ladite réponse de cellule Th est une réponse de cellule Th1.

**24.** Utilisation selon la revendication 22, dans laquelle ladite composition est introduite dans ledit animal par voie sous-cutanée, par voie intramusculaire, par voie intraveineuse, par voie intranasale ou directement dans le ganglion lymphatique.

**25.** Quantité efficace sur le plan immunologique du vaccin selon la revendication 16, pour une utilisation dans un procédé d'immunisation ou de traitement d'un animal comprenant l'administration de ladite quantité et audit animal, où de préférence ledit animal est un mammifère, et de manière encore préférée un humain.

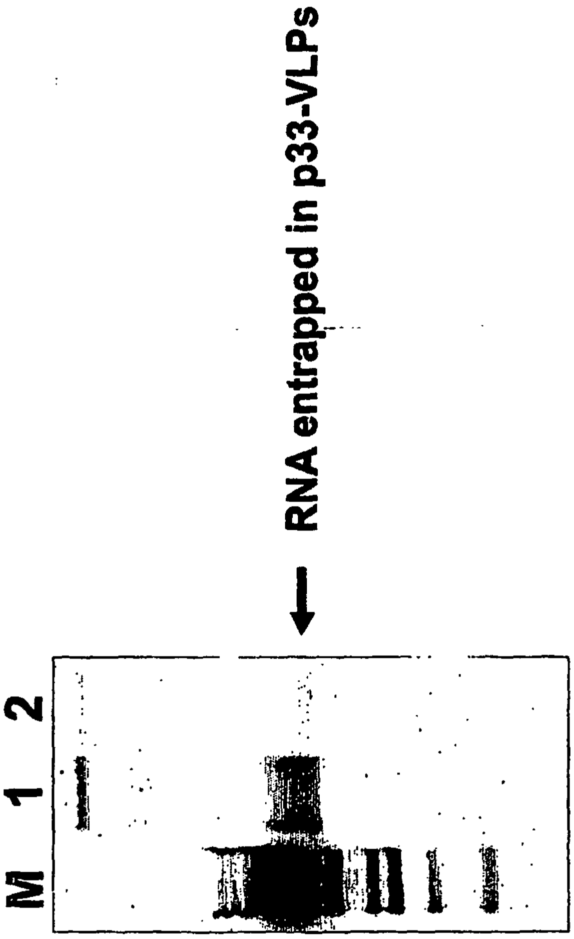


Fig. 1A

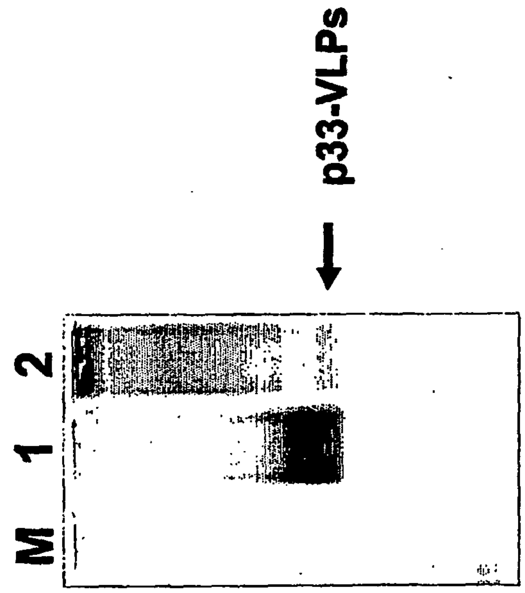


Fig. 1B

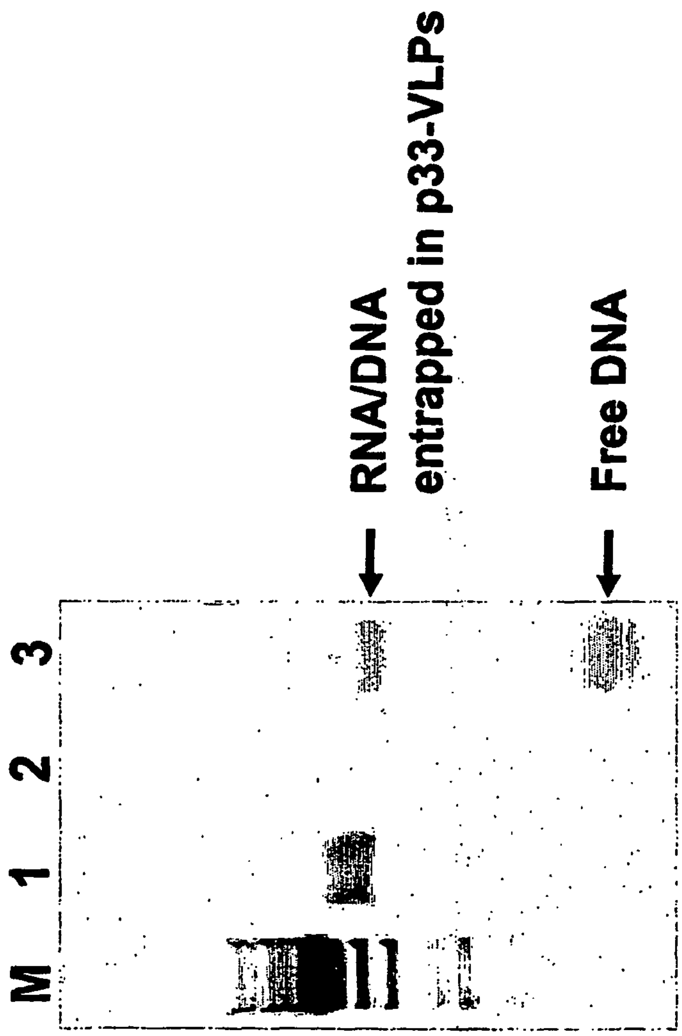


Fig. 2A

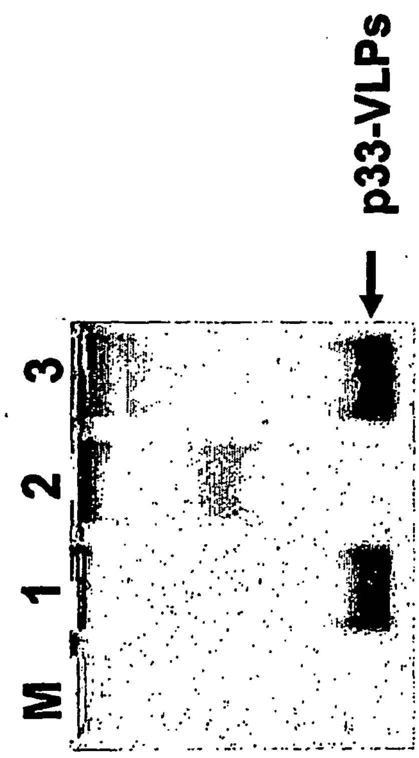


Fig. 2B

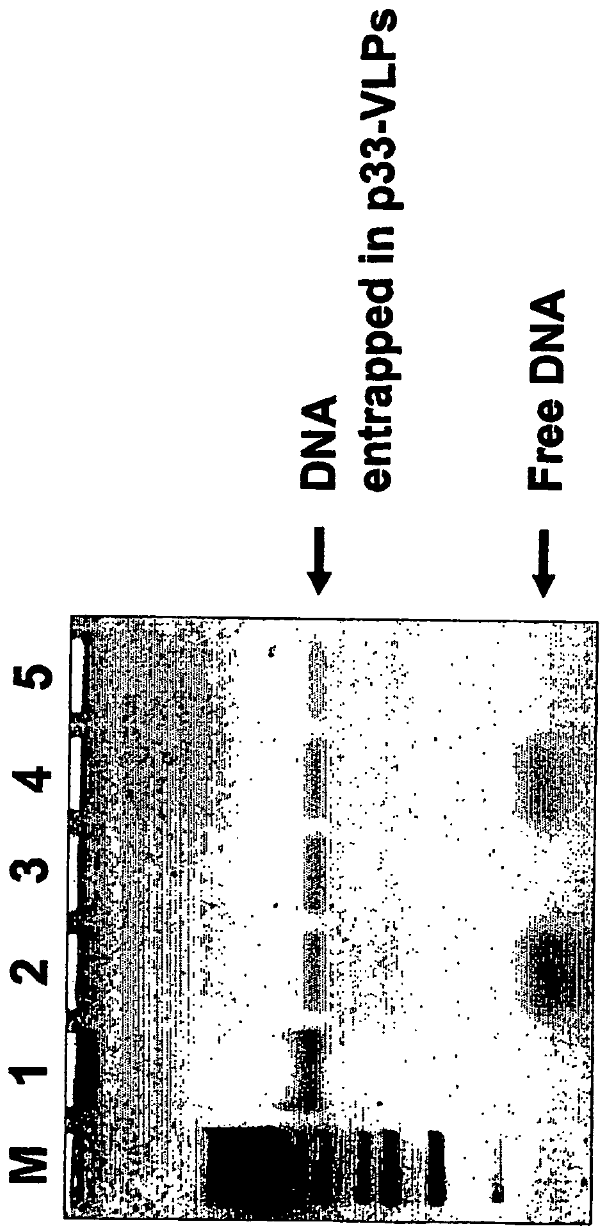


Fig. 3A

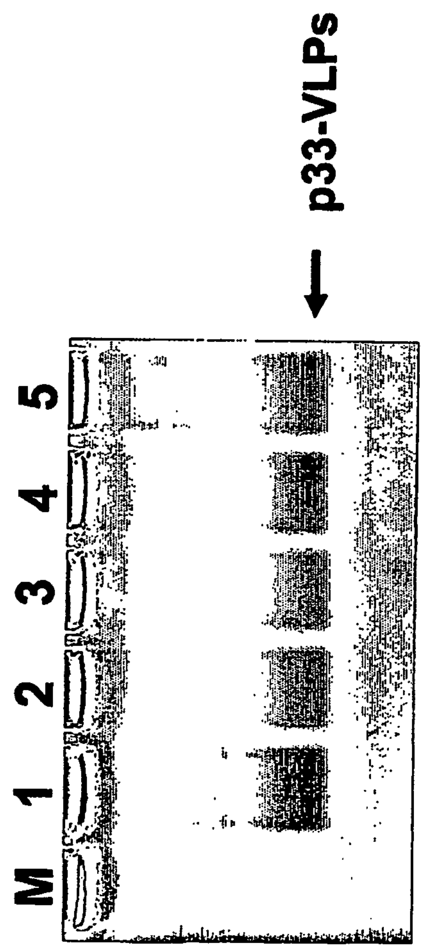


Fig. 3B

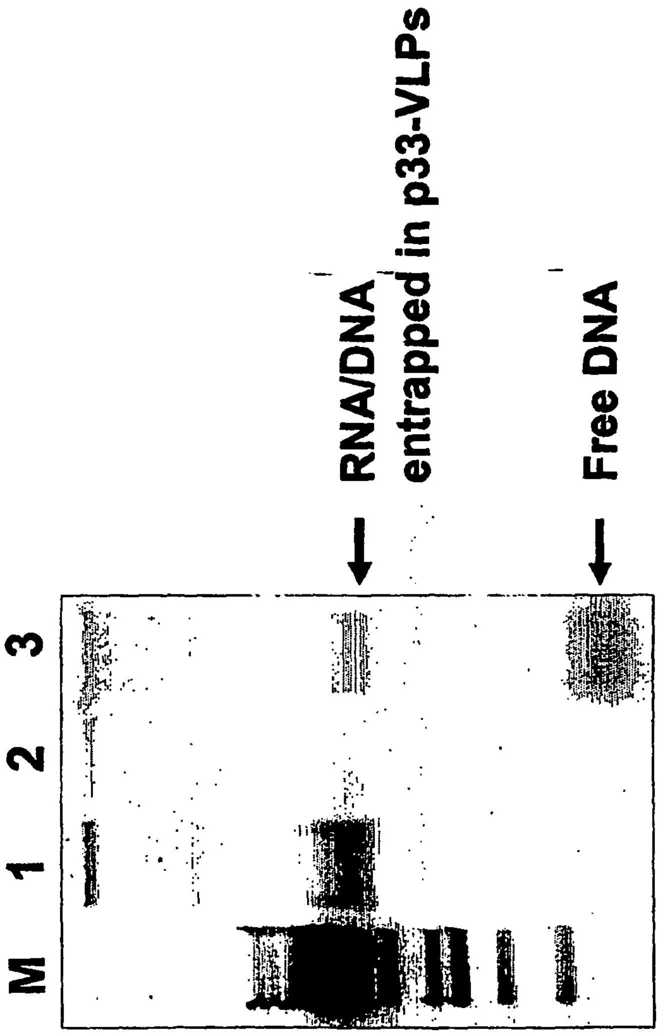


Fig. 4A

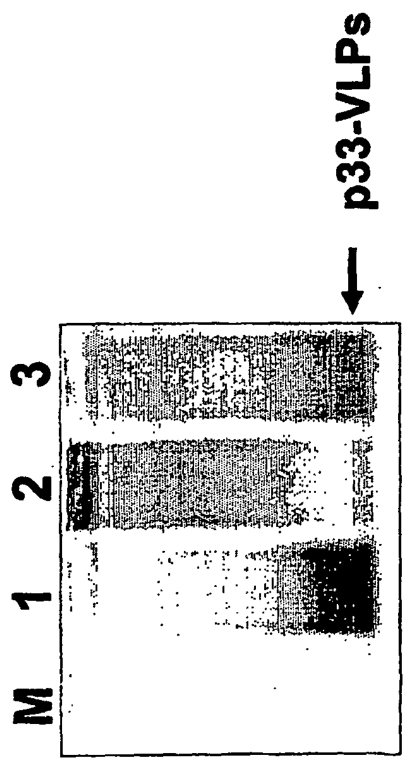


Fig. 4B

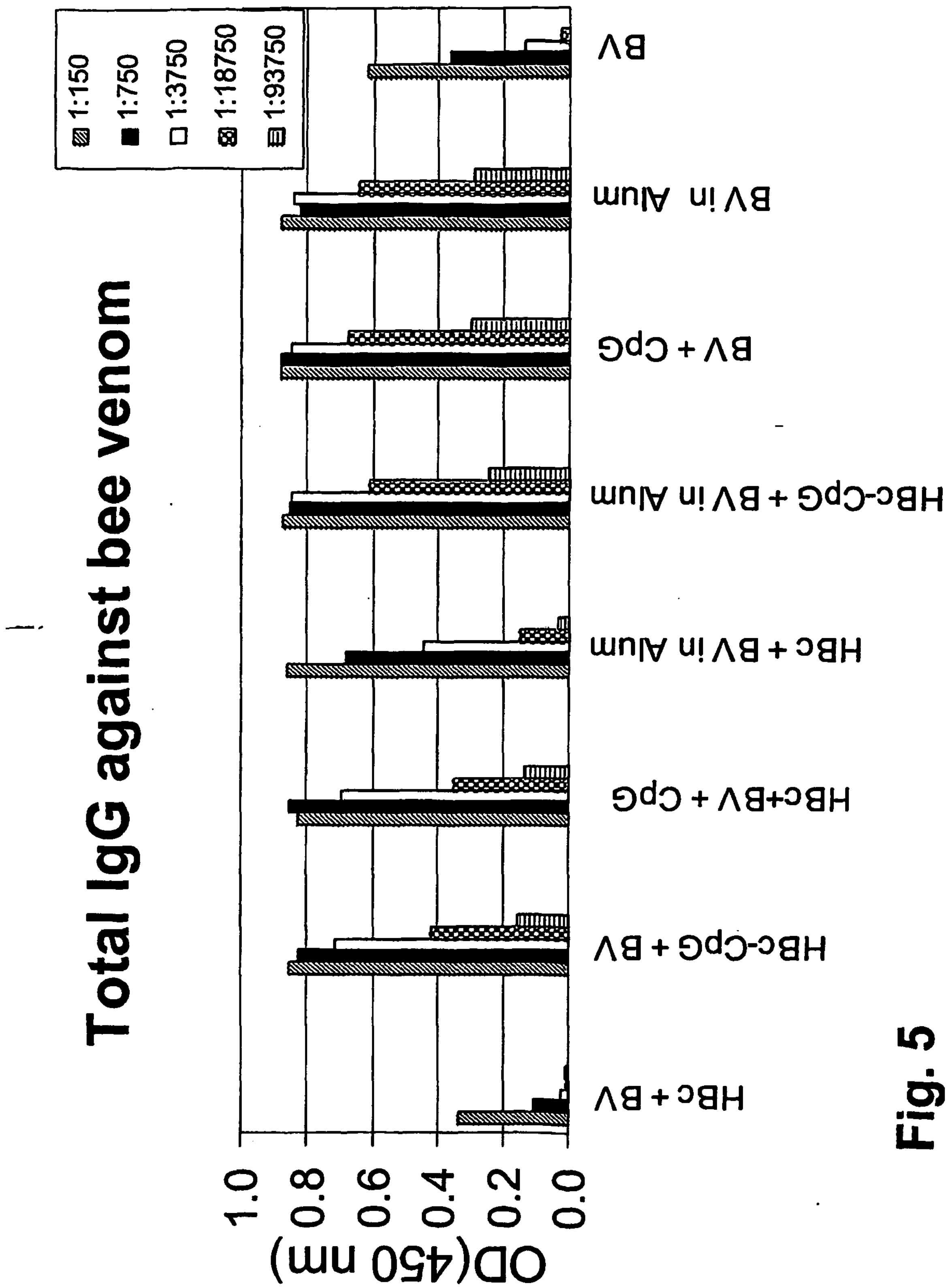
Total IgG against bee venom
1:150
1:750
1:3750
1:18750
1:93750
OD(450 nm)
1.0
0.8
0.6
0.4
0.2
0.0
HBc + BV
HBc-CpG + BV
HBc+BV + CpG
HBc + BV in Alum
HBc-CpG + BV in Alum
BV + CpG
BV in Alum
BV

Fig. 5

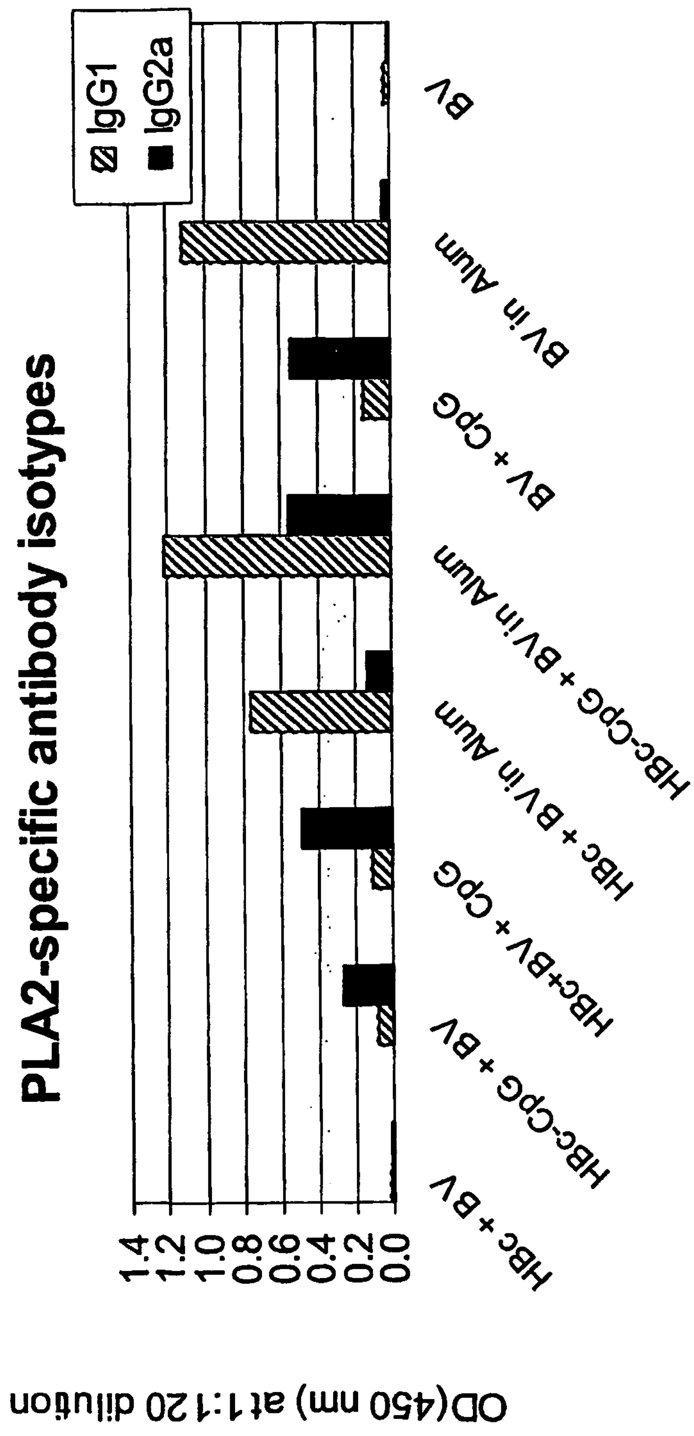
PLA2-specific antibody isotypes
IgG1
IgG2a
OD(450 nm) at 1:120 dilution
1.4
1.2
1.0
0.8
0.6
0.4
0.2
0.0
BV
BV in Alum
BV + CpG
HBC-CpG + BV in Alum
HBc + BV in Alum
HBC+BV + CpG
HBC-CpG + BV
HBc + BV

Fig. 6

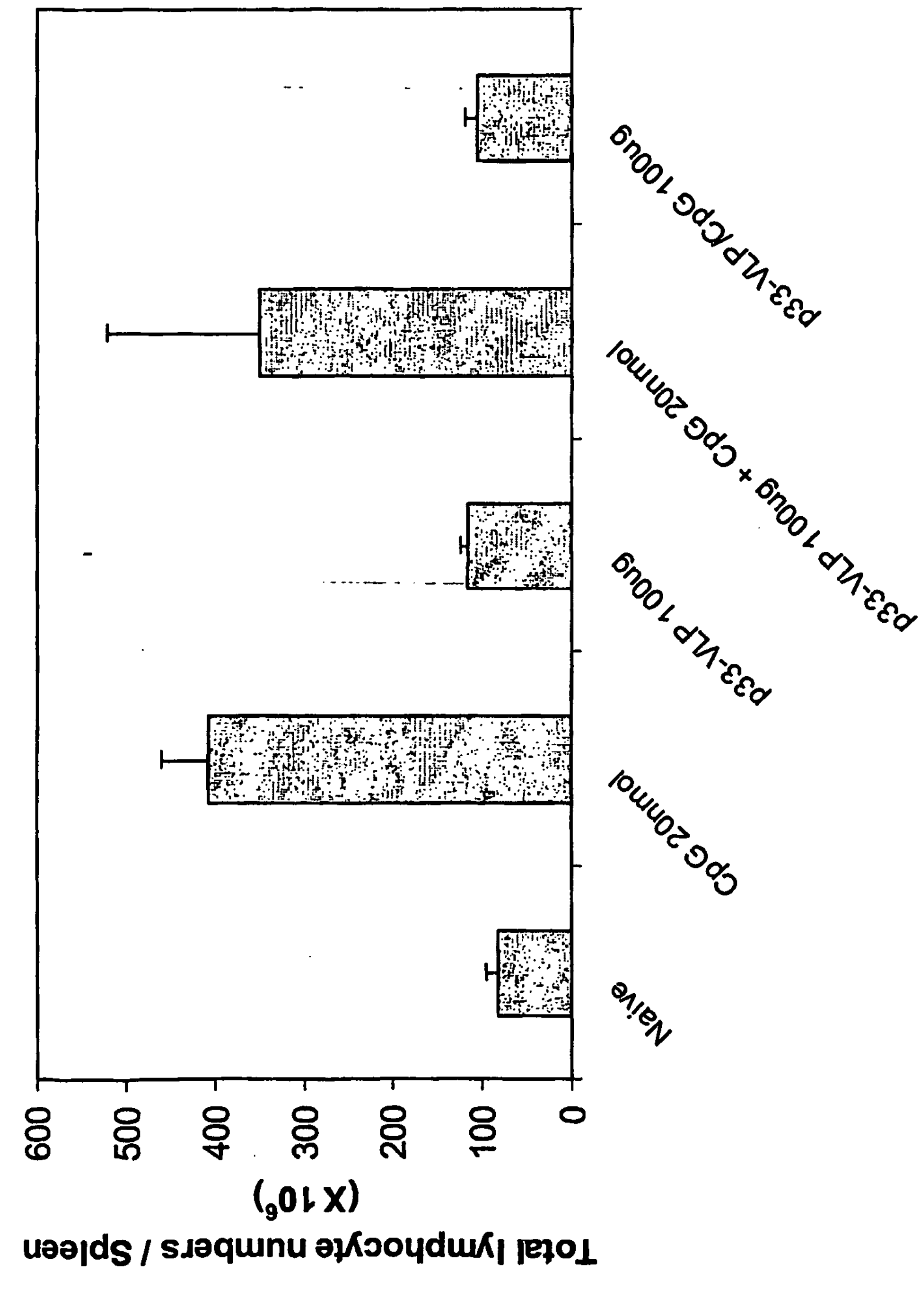
Total lymphocyte numbers / Spleen (X 10^6)
600
500
400
300
200
100
0
Naive
CpG 20nmol
p33-VLP 100ug
p33-VLP 100ug + CpG 20nmol
p33-VLP/CpG 100ug

Fig. 7

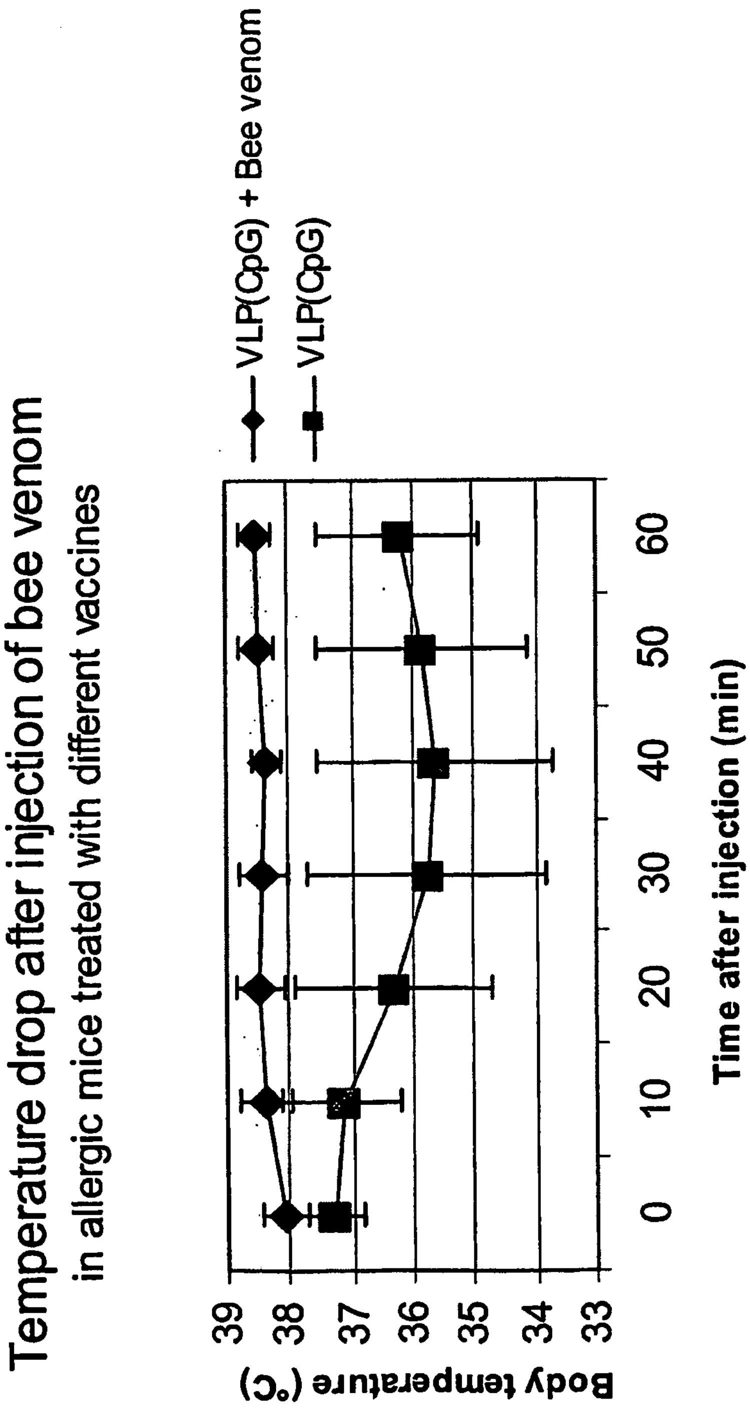
Temperature drop after injection of bee venom
in allergic mice treated with different vaccines
Body temperature (°C)
39
38
37
36
35
34
33
0
10
20
30
40
50
60
Time after injection (min)
VLP(CpG) + Bee venom
VLP(CpG)

Fig. 8

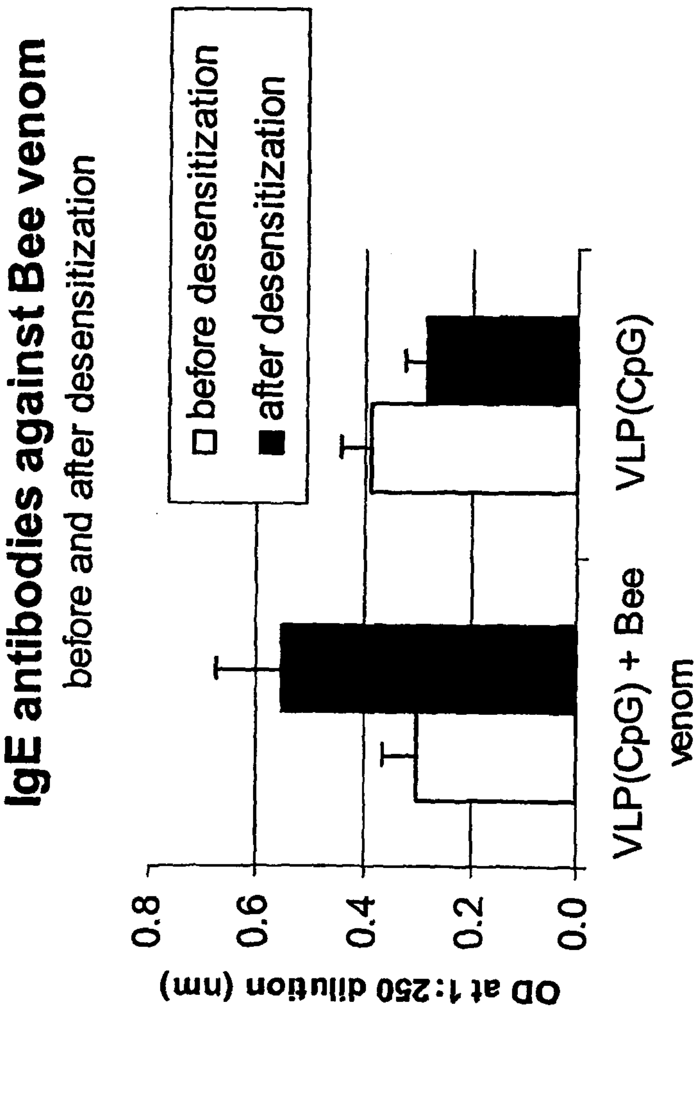
IgE antibodies against Bee venom
before and after desensitization
before desensitization
after desensitization
OD at 1:250 dilution (nm)
0.8
0.6
0.4
0.2
0.0
VLP(CpG) + Bee venom
VLP(CpG)

Fig. 9A

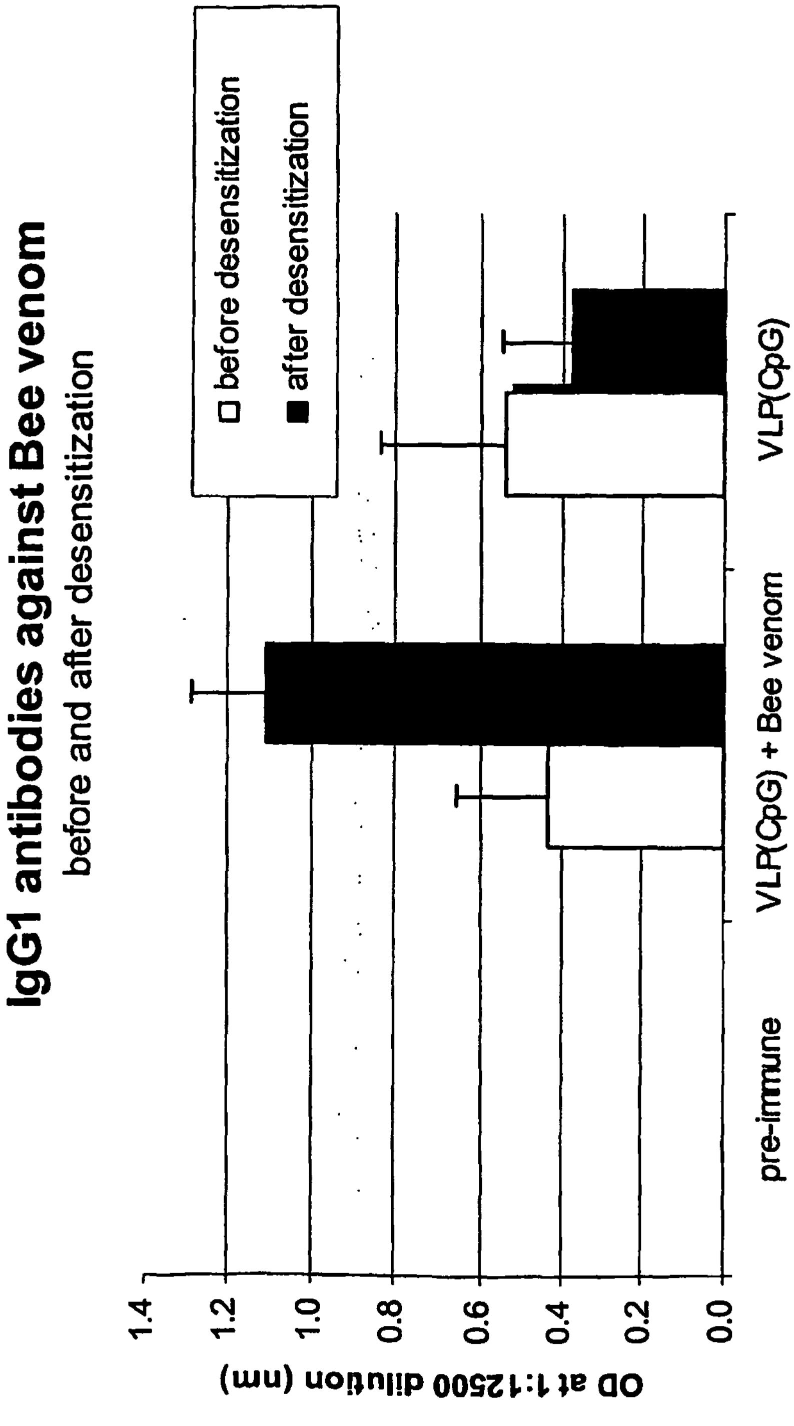
IgG1 antibodies against Bee venom
before and after desensitization
before desensitization
after desensitization
1.4
1.2
1.0
0.8
0.6
0.4
0.2
0.0
OD at 1:12500 dilution (nm)
pre-immune
VLP(CpG) + Bee venom
VLP(CpG)

Fig. 9B

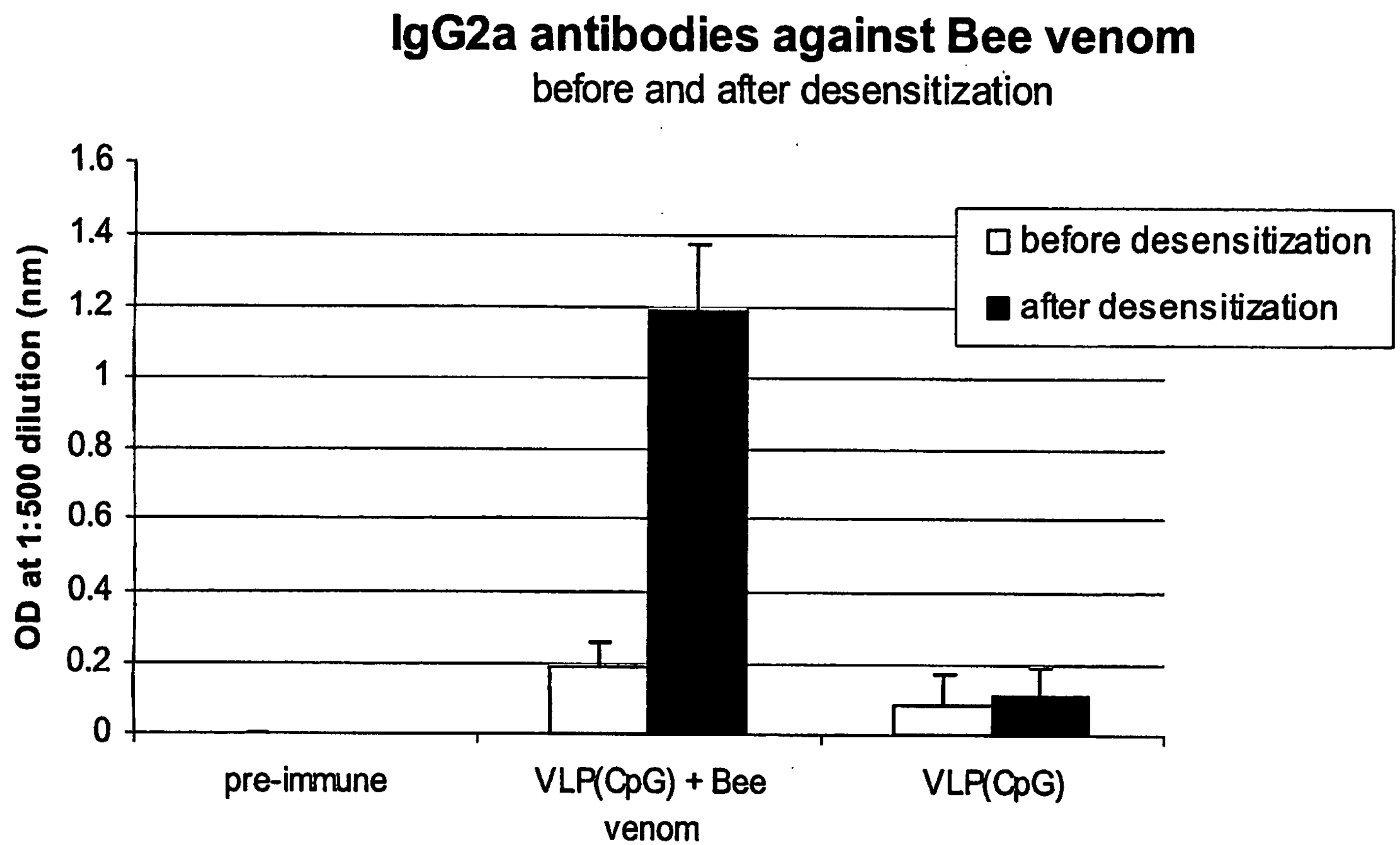
IgG2a antibodies against Bee venom
before and after desensitization
OD at 1:500 dilution (nm)
1.6
1.4
1.2
1
0.8
0.6
0.4
0.2
0
before desensitization
after desensitization
pre-immune
VLP(CpG) + Bee venom
VLP(CpG)

Fig. 9C

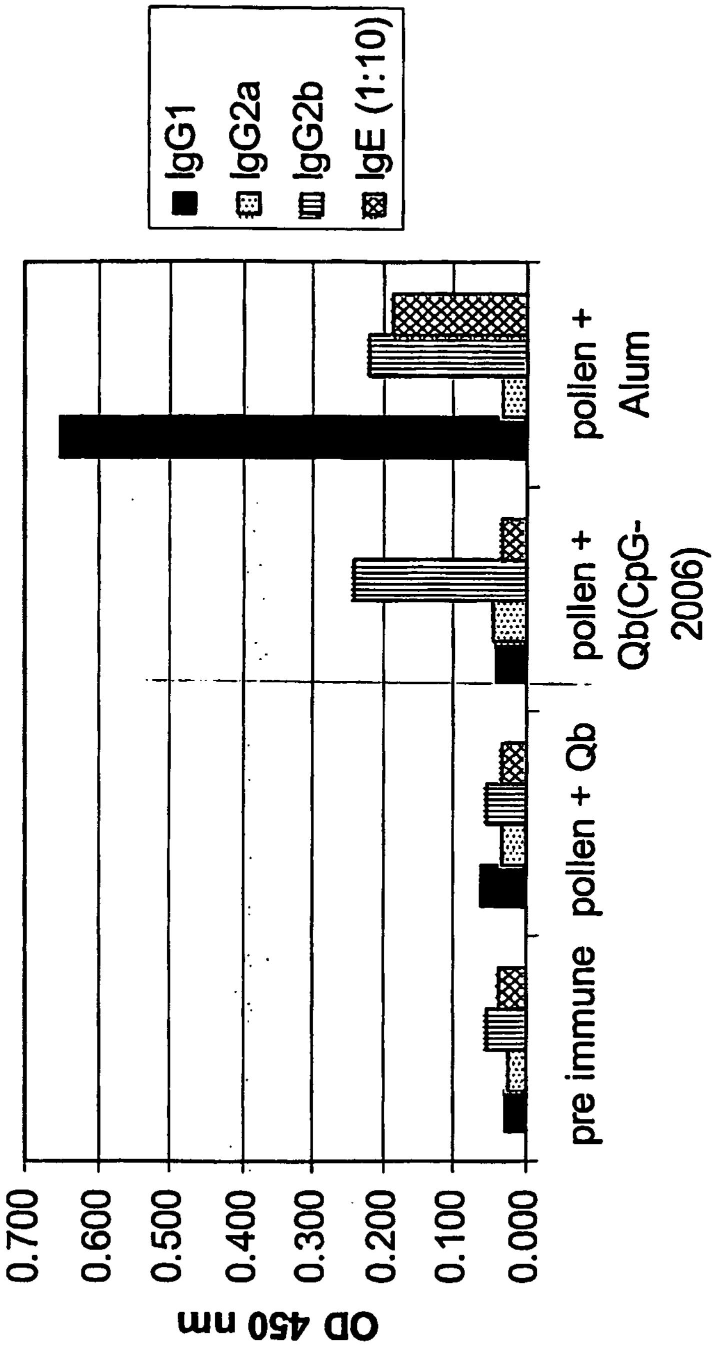
OD 450 nm
0.700
0.600
0.500
0.400
0.300
0.200
0.100
0.000
pre immune
pollen + Qb
pollen + Qb(CpG-2006)
pollen + Alum
IgG1
IgG2a
IgG2b
IgE (1:10)

FIG. 10

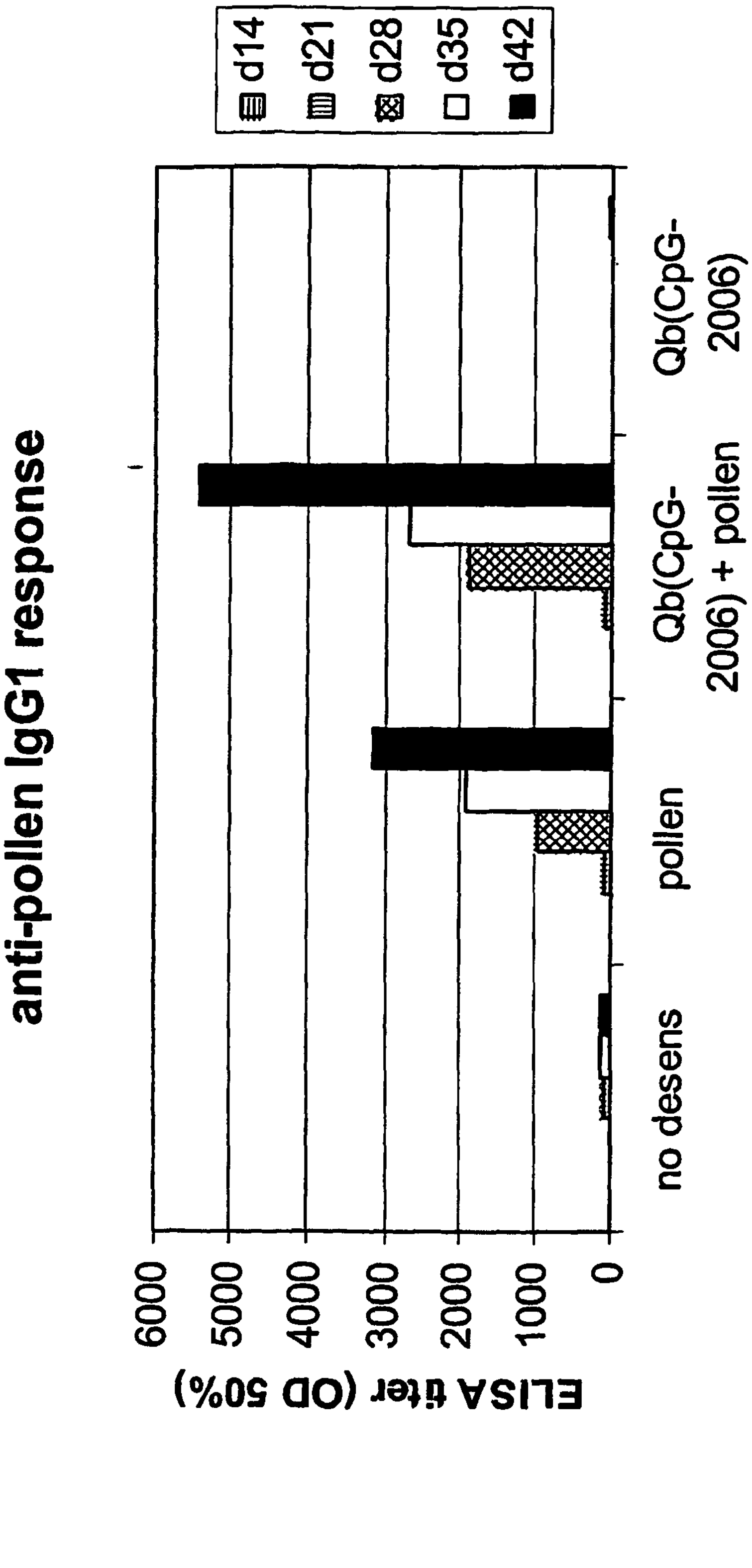
anti-pollen IgG1 response
ELISA titer (OD 50%)
6000
5000
4000
3000
2000
1000
0
no desens
pollen
Qb(CpG-2006) + pollen
Qb(CpG-2006)
d14
d21
d28
d35
d42

FIG. 11 A

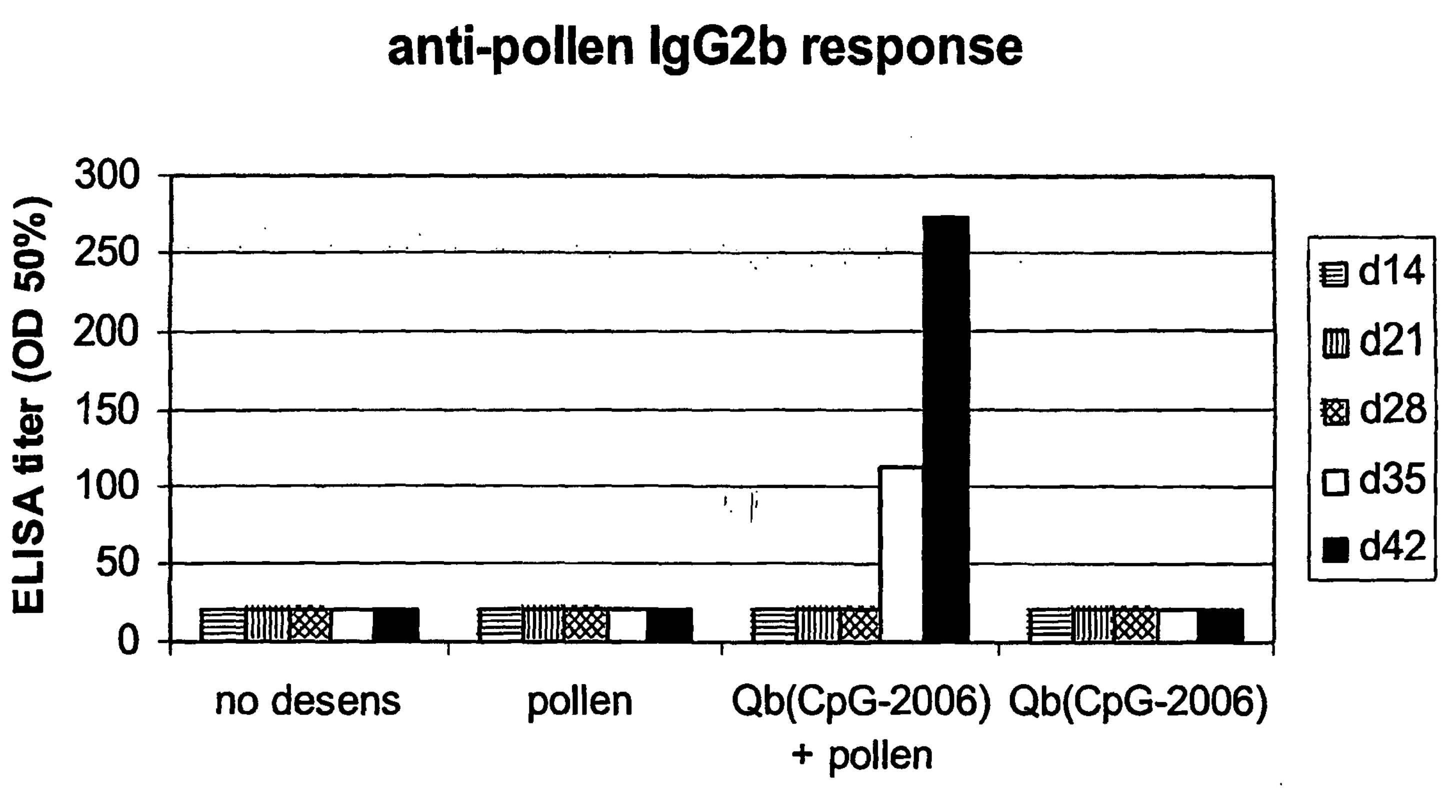
anti-pollen IgG2b response
ELISA titer (OD 50%)
300
250
200
150
100
50
0
no desens
pollen
Qb(CpG-2006) + pollen
Qb(CpG-2006)
d14
d21
d28
d35
d42

FIG. 11 B

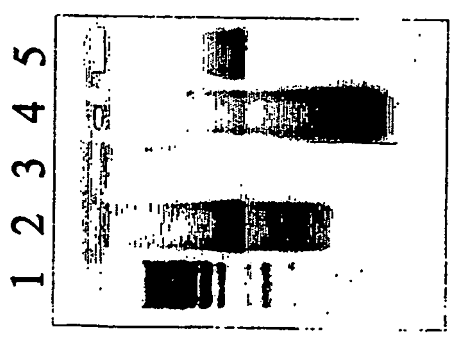


Fig. 12A

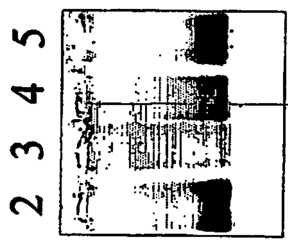


Fig. 12B

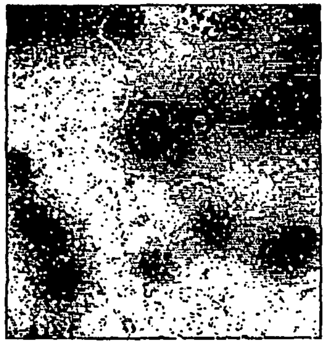
+ CyOpA

Fig. 13A

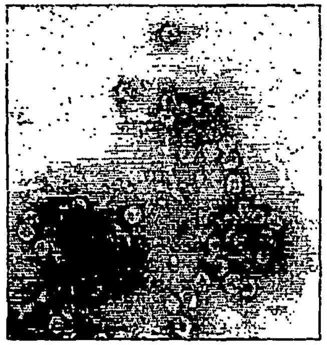
+ $CyCpG_{20}OpA$

Fig. 13B

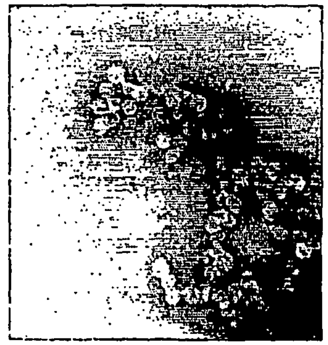
+ $CyCpG_{20}$

Fig. 13C

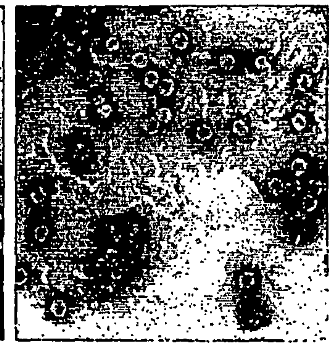
+ CyCyCy

Fig. 13D

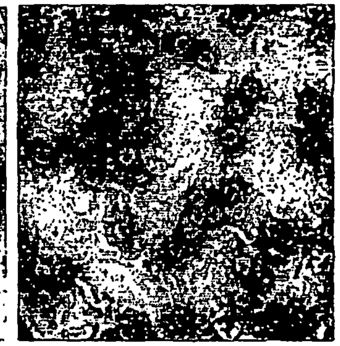
+ $CpG_{20}OpA$

Fig. 13E

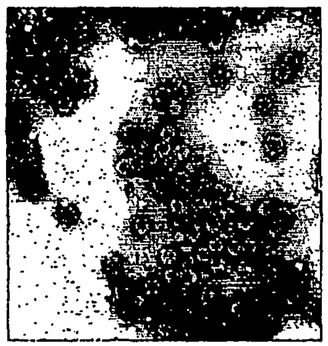
+ tRNA

Fig. 13F

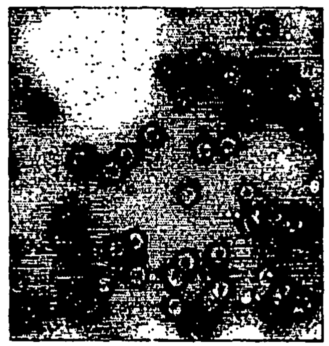
"intact" capsids from *E.coli*

Fig. 13G

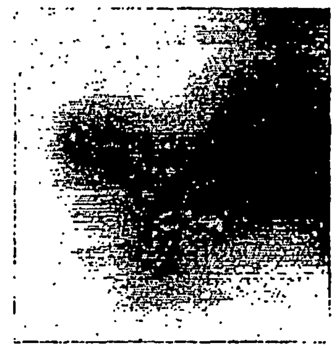
w/o any nucleic acid

Fig. 13H

Fig. 13

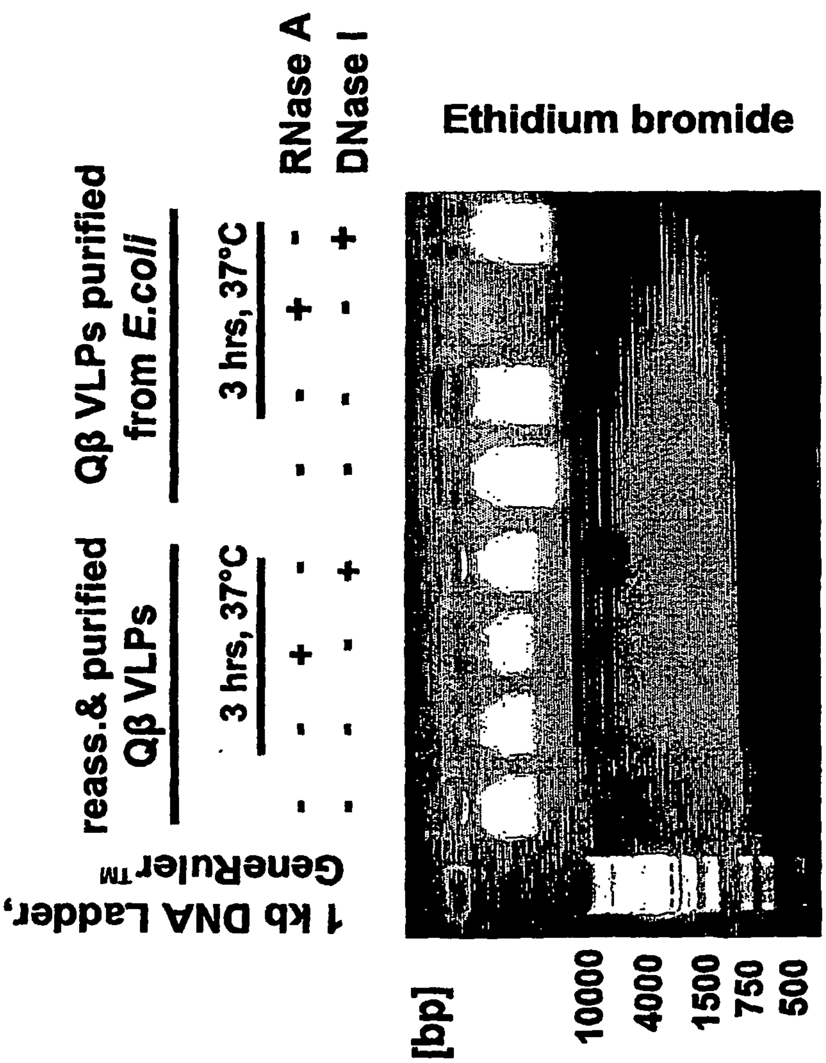
1 kb DNA Ladder, GeneRuler™
reass.& purified Qβ VLPs
Qβ VLPs purified from E.coli
3 hrs, 37°C
3 hrs, 37°C
RNase A
DNase I
Ethidium bromide
[bp]
10000
4000
1500
750
500

Fig. 14A

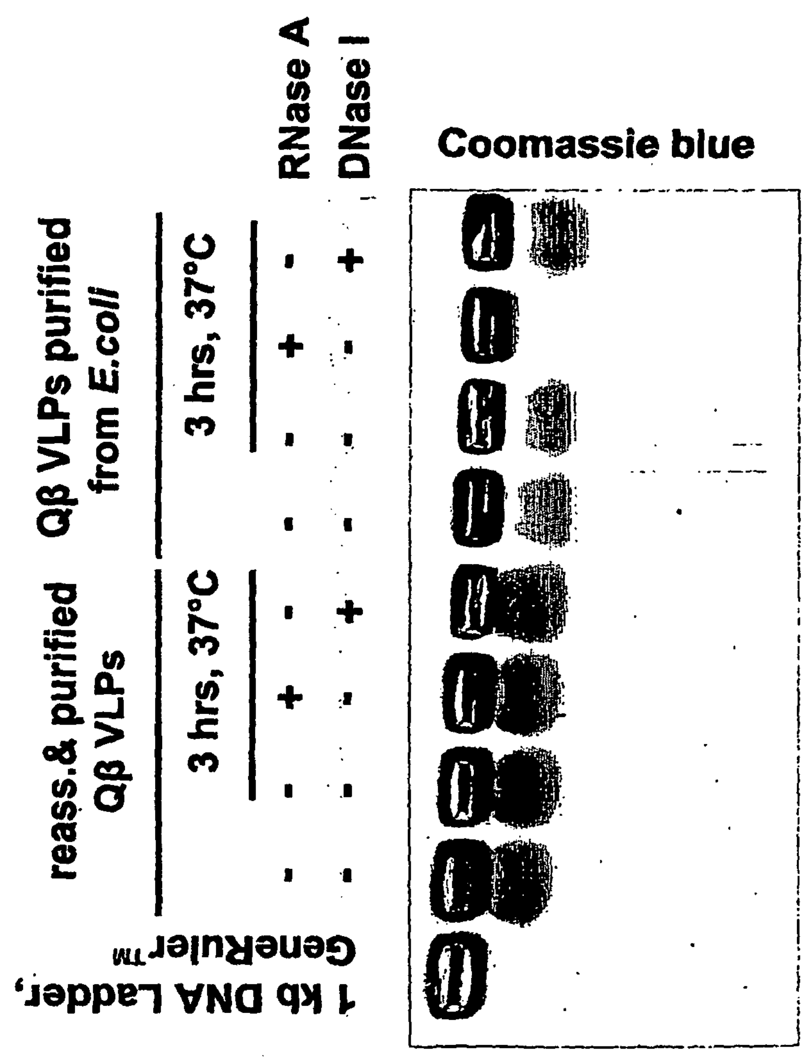
1 kb DNA Ladder, GeneRuler™
reass.& purified Qβ VLPs
Qβ VLPs purified from E.coli
3 hrs, 37°C
3 hrs, 37°C
RNase A
DNase I
- - + - - - + -
- - - + - - - +
Coomassie blue

Fig. 14B

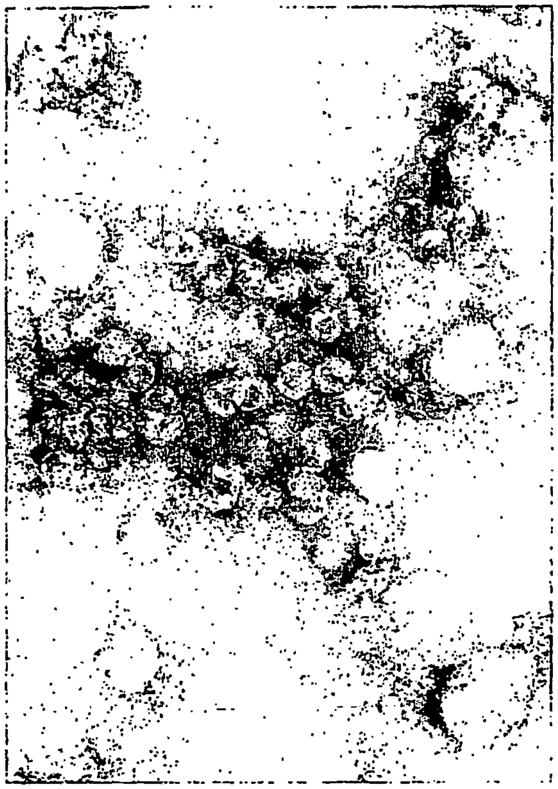

Fig. 15B

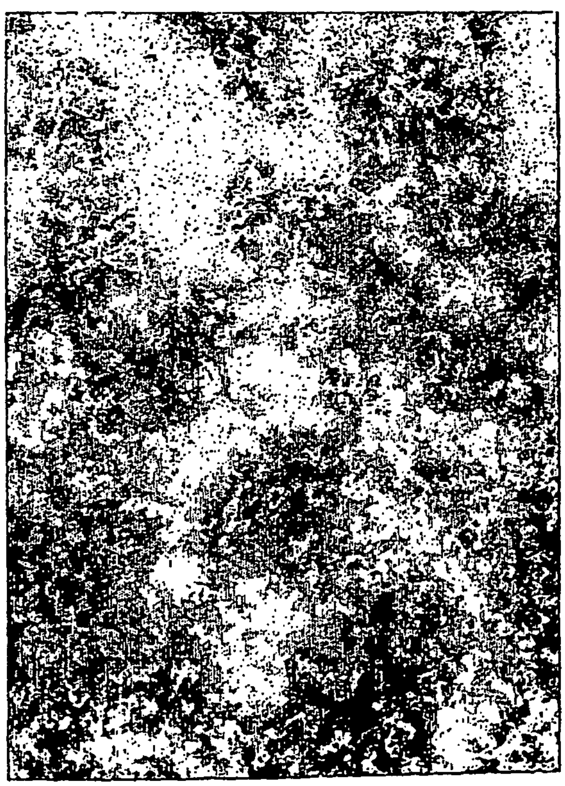

Fig. 15A

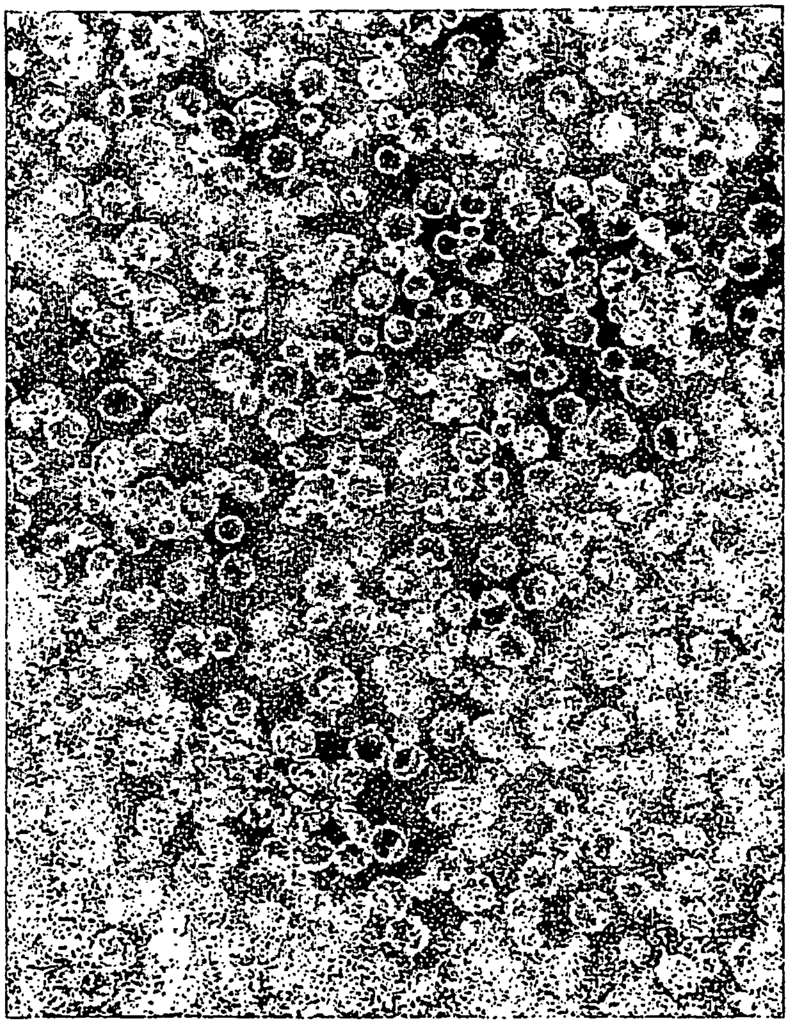

Fig. 15C

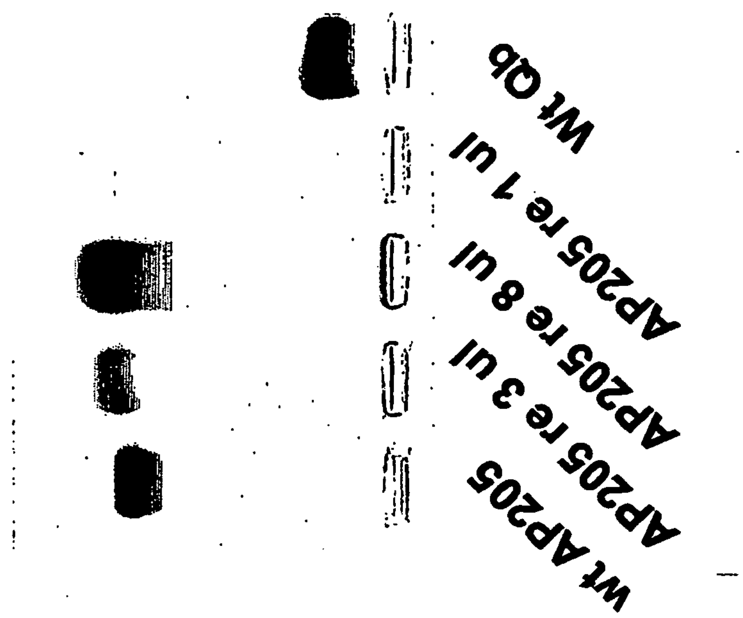


Fig. 16B

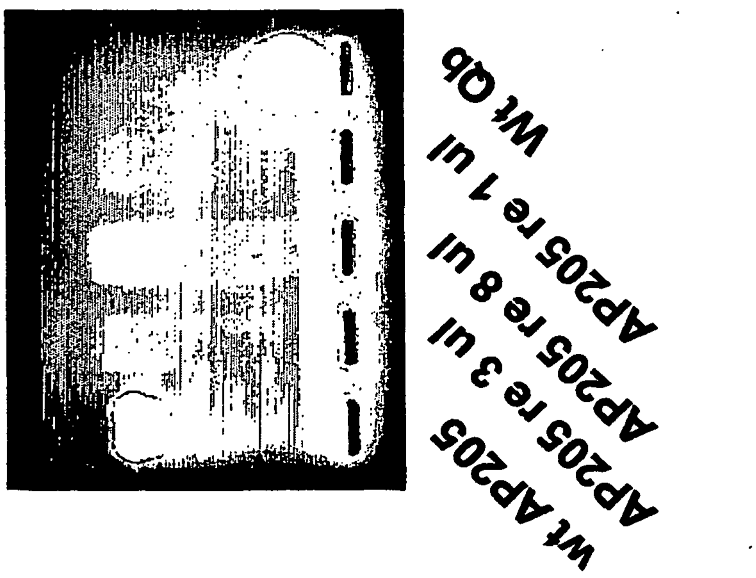


Fig. 16A

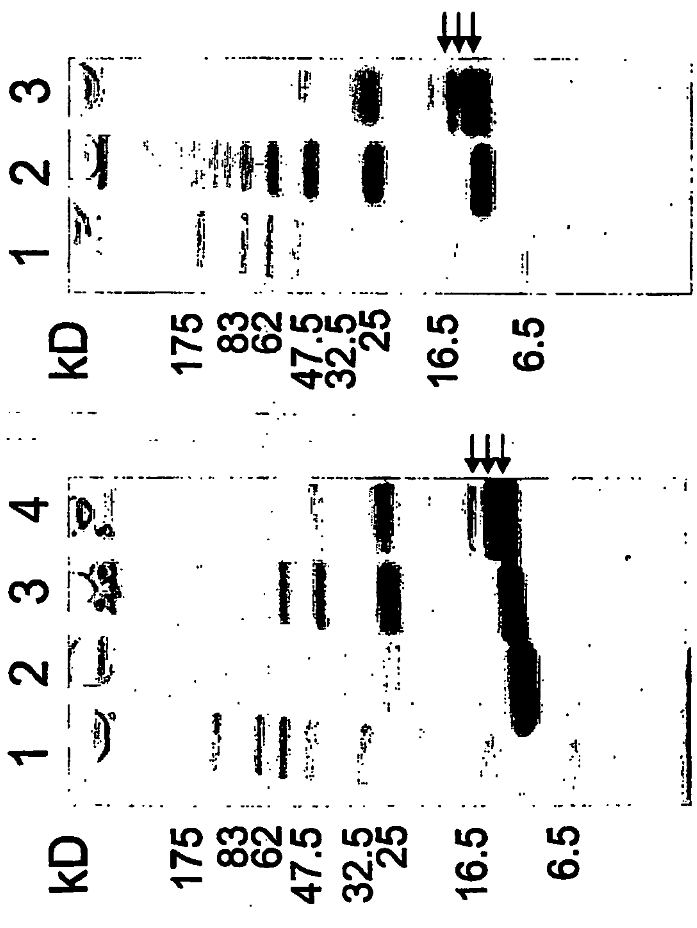
1 2 3
kD
175
83
62
47.5
32.5
25
16.5
6.5
1 2 3 4
kD
175
83
62
47.5
32.5
25
16.5
6.5

FIG. 17

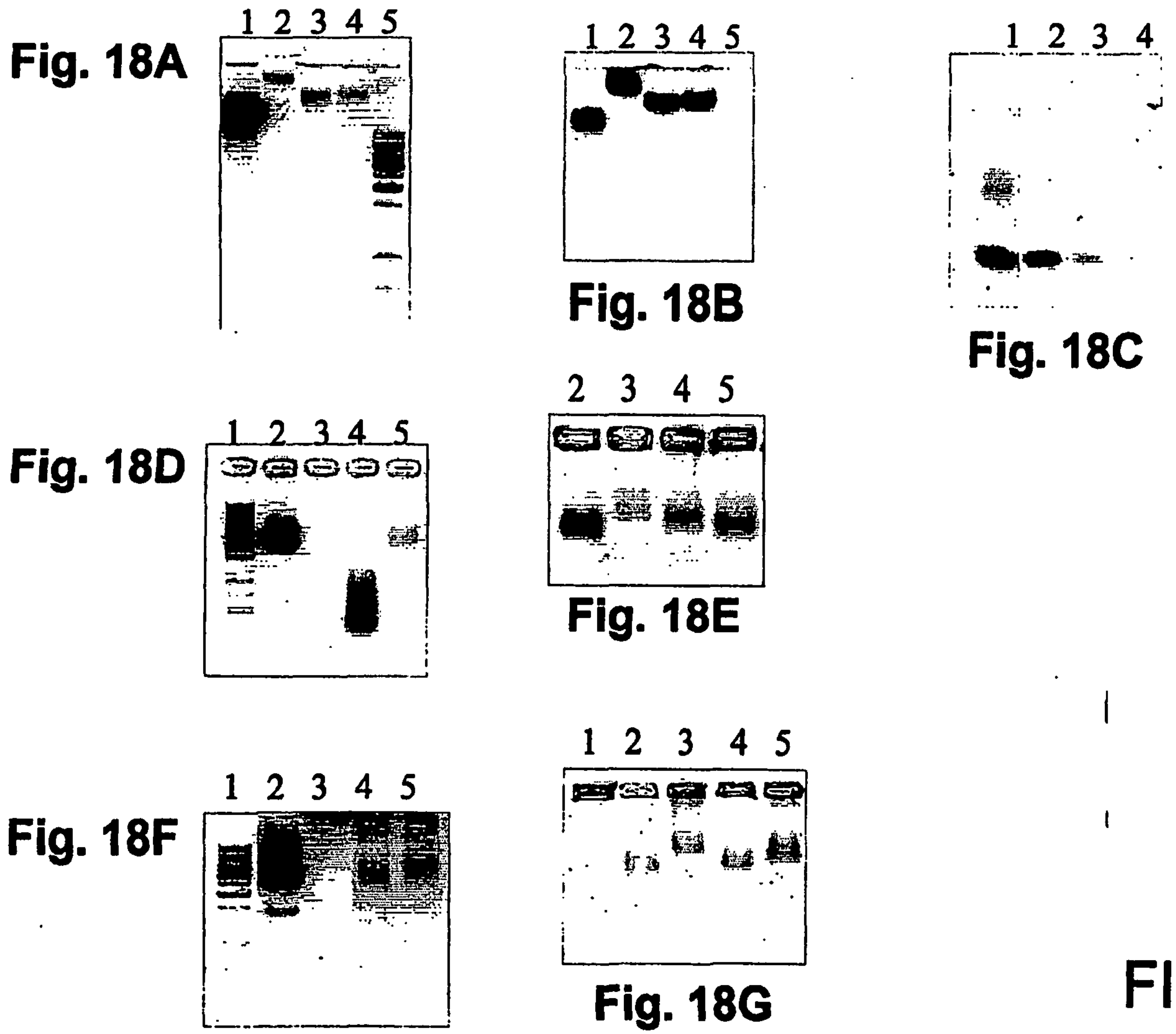
Fig. 18A
1 2 3 4 5
Fig. 18B
1 2 3 4 5
Fig. 18C
1 2 3 4
Fig. 18D
1 2 3 4 5
Fig. 18E
2 3 4 5
Fig. 18F
1 2 3 4 5
Fig. 18G
1 2 3 4 5

FIG. 18

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 5334394 A
- US 5871747 A
- WO 9415585 A
- WO 9850071 A
- WO 03024481 A
- WO 0122972 A
- US 5939598 A, Kucherlapati
- US 5071651 A
- US 5374426 A
- WO 9630523 A
- WO 9211291 A
- WO 9815631 A
- WO 02056905 A
- US 4918166 A
- WO 0032227 A
- WO 0185208 A
- WO 0198333 A
- WO 00177158 A
- WO 00214478 A
- US 5057540 A
- GB 2220211 A
- WO 0000462 A
- US 60389898 B


### Non-patent literature cited in the description


- **Medzhitov, R. ; Janeway, C.A., Jr.** *Cell,* 1997, vol. 91, 295-298
- **Sotomayor, E. M. et al.** *Nat. Med.,* 1999, vol. 5, 780
- **Diehl, L. et al.** *Nat. Med.,* 1999, vol. 5, 774
- **Weigle, W. O.** *Adv. Immunol.,* 1980, vol. 30, 159
- **Nestle et al.** *Nat. Med.,* 1998, vol. 4, 328
- **Weigle, W. O.** *Adv. Immunol.,* 1980, vol. 30, 159
- **Garza, K. M. et al.** *J. Exp. Med.,* 2000, vol. 191, 2021
- **Vella, A. T. et al.** *Immunity,* 1995, vol. 2, 261
- **Ehl, S. et al.** *J. Exp. Med.,* 1998, vol. 187, 763
- **Maxwell, J. R. et al.** *J. Immunol.,* 1999, vol. 162, 2024
- **Husmann, L. A. ; M. J. Bevan.** *Ann. NY. Acad Sci.,* 1988, vol. 532, 158
- **Guerder, S. ; P. Matzinger.** *J. Exp. Med.,* 1992, vol. 176, 553
- **Bennett, S. R. M. et al.** *J. Exp. Med.,* 1997, vol. 186, 65
- **Ossendorp, F. et al.** *J. Exp. Med.,* 1998, vol. 187, 693
- **Foy, T.M. et al.** *Annu. Rev. Immunol.,* 1996, vol. 14, 591
- **Foy, T. M. et al.** *Ann. Rev. Immunol.,* 1996, vol. 14, 591
- **Cella, M. et al.** *Curr. Opin. Immunol.,* 1997, vol. 9, 10
- **Banchereau, J. ; R. M. Steinman.** *Nature,* 1998, vol. 392, 245
- **Ridge, J. P. et al.** *Nature,* vol. 393, 474
- **Bennett, S. R. M. et al.** *Nature,* 1998, vol. 393, 478
- **Schoenenberger, S. P. et al.** *Nature,* 1998, vol. 393, 480
- **Bachmann, M. F. et al.** *J. Immunol.,* 1998, vol. 161, 5791
- **Leist, T. P. et al.** *J. Immunol.,* 1987, vol. 138, 2278
- **Ahmed, R. et al.** *J. Virol.,* 1988, vol. 62, 2102
- **Battegay, M. et al.** *Cell Immunol.,* 1996, vol. 167, 115
- **Borrow, P. et al.** *J Exp. Med.,* 1996, vol. 183, 2129
- **Whitmire, J. K. et al.** *J. Virol.,* 1996, vol. 70, 8375
- **Kündig, T. M. et al.** *Immunity,* vol. 5, 41
- **Tripp, R. A. et al.** *J. Immunol.,* 1995, vol. 155, 2955
- **Leist, T. P. et al.** *Scand. J. Immunol.,* 1989, vol. 30, 679
- **Buller, R. et al.** *Nature,* 1987, vol. 328, 77
- **Albert, M. L. et al.** *J. Exp. Med.,* 1998, vol. 188, 1359
- **Albert, M. L. et al.** *Nature,* 1998, vol. 392, 86
- **Ridge, J. P et al.** *Nature,* 1998, vol. 393, 474
- **Ruedl, C. et al.** *J. Exp. Med.,* 1999, vol. 189, 1875
- **Kündig, T. M. et al.** *Immunity,* 1996, vol. 5, 41
- **Bachmann, M. F. et al.** *J. Exp. Med.,* 1999, vol. 189, 1025
- **Sallusto, F. ; A. Lanzavecchia.** *J Exp Med.,* 1994, vol. 179, 1109

- **Bachmann ; Zinkernagel.** *Ann. Rev. Immunol.,* 1997, vol. 15, 235-270
- **Bachmann ; Zinkernagel.** *Immunol. Today,* 1996, vol. 17, 553-558
- **Fehr, T. et al.** *J. Exp. Med.,* 1997, vol. 185, 1785-1792
- **Townsend ; Bodmer.** *Ann. Rev. Immunol.,* 1989, vol. 7, 601-624
- **Kovacsovics-Bankowski et al.** *Proc. Natl. Acad Sci. USA,* 1993, vol. 90, 4942-4946
- **Bachmann et al.** *Eur. J. Immunol.,* 1996, vol. 26, 2595-2600
- **Harding, C. et al.** *J. Immunology,* 1994, vol. 153, 4925
- **Kovacsovics-Bankowski, M. et al.** *Proc. Natl. Acad Sci. USA,* 1993, vol. 90, 4942-4946
- **Sparwasser et al.** *J. Immunol.,* 1999, vol. 162, 2368-74
- **Romagnani.** *Immunol. Today,* 1997, vol. 18, 263-266
- **Shough, H. et al.** REMINGTON'S PHARMACEUTICAL SCIENCES. Mack Publishing Group, 1995
- **C. G. A. Thomas.** *Medical Microbiology,* 1983
- **Cohen et al.** *Cancer Research,* 1994, vol. 54, 1055
- **Geysen et al.** *Proc. Natl. Acad Sci. USA,* 1983, vol. 81, 3998-4002
- **Kozlovska, T.M. et al.** *Intervirology,* 1996, vol. 39, 9-15
- MOLECULAR CLONING, A LABORATORY MANUAL. Cold Spring Harbor Laboratory Press, 1989
- CURRENT PROTOCOLS IN MOLECULAR BIOLOGY. John H. Wiley & Sons, Inc, 1997
- CELL BIOLOGY. Academic Press, 1998
- **Harlow, E. ; Lane, D.** Antibodies: A Laboratory Manual. Cold Spring Harbor Laboratory, 1988
- Guide to Protein Purification. **Deutscher, M.P.** Meth. Enzymol. Academic Press, 1990, vol. 128
- **Scopes, R.K.** Protein Purification Principles and Practice. Springer-Verlag, 1994
- **Ulrich et al.** *Virus Res.,* 1998, vol. 50, 141-182
- **Warnes et al.** *Gene,* 1995, vol. 160, 173-178
- **Twomey et al.** *Vaccine,* 1995, vol. 13, 1603-1610
- **Jiang, X. et al.** *Science,* 1990, vol. 250, 1580-1583
- **Matsui, S.M. et al.** *J. Clin. Invest.,* 1991, vol. 87, 1456-1461
- **Kozlovska TM. et al.** *GENE,* 1993, vol. 137, 133-137
- **Golmohammadi, R. et al.** *Structure,* 1996, vol. 4, 543-5554
- **Stoll, E. et al.** *J Biol. Chem,* 1977, vol. 252, 990-993
- **Kastelein, RA. et al.** *Gene,* 1983, vol. 23, 245-254
- **Kozlovskaya, TM. et al.** *Dokl. Akad. Nauh SSSR,* 1986, vol. 287, 452-455
- **Adhin, MR. et al.** *Virology,* 1989, vol. 170, 238-242
- **Ni, CZ. et al.** *Protein Sci.,* 1996, vol. 5, 2485-2493
- **Priano, C. et al.** *J. Mol. Biol.,* 1995, vol. 249, 283-297
- **Witherell, GW. ; Uhlenbeck, OC.** *Biochemistry,* vol. 28, 71-76
- **Lim F. et al.** *J. Biol. Chem.,* 1996, vol. 271, 31839-31845
- **Klovins, J. et al.** *J. Gen. Virol.,* 2002, vol. 83, 1523-33
- **Kozlovska, T. M. et al.** *Gene,* 1993, vol. 137, 133-37
- **Golmohammadi, R. et al.** *Structure,* 1996, vol. 4, 543-554
- **Zhou et al.** *J. Virol.,* 1992, vol. 66, 5393-5398
- **Yamamoto et al.** *Springer Semin Immunopathol.,* vol. 22, 11-19
- **Levy, H.B.** *Methods Enzymol.,* 1981, vol. 78, 242-251
- **DeClercq, E.** *Methods Enzymol.,* 1981, vol. 78, 227-236
- **Torrence, P.F.** *Methods Enzymol,* 1981, vol. 78, 326-331
- **Beaucage, S. L. ; Caruthers, M. H.** *Tet. Let.,* 1981, vol. 22, 1859
- **Garegg et al.** *Tet. Let.,* 1986, vol. 27, 4051-4054
- **Froehler et al.** *Nucl. Acid. Res.,* 1986, vol. 14, 5399-5407
- **Garegg et al.** *Tet. Let.,* 1986, vol. 27, 4055-4058
- **Gaffney et al.** *Tet. Let.,* 1988, vol. 29, 2619-2622
- **Sambrook, T. et al.** Molecular Cloning: A Laboratory Manual. Cold Spring Harbor laboratory Press, 1989
- **Engleman, E. G.** *Cytotechnology,* 1997, vol. 25, 1
- **Van Schooten, W. et al.** *Molecular Medicine Today,* June 1997, 255
- **Steinman, R. M.** *Experimental Hematology,* 1996, vol. 24, 849
- **Gluckman, J. C.** *Cytokines, Cellular and Molecular Therapy,* 1997, vol. 3, 187
- **Kuramoto E. et al.** *Japanese Journal Cancer Research,* 1992, vol. 83, 1128-1131
- **Pircher et al.** Tolerance induction in double specific T-cell receptor transgenic mice varies with antigen. *Nature,* 1989, vol. 342, 559

- **Tissot et al.** Characterizing the functionality of recombinant T-cell receptors in vitro: a pMHC tetramer based approach. *J Immunol Methods,* 2000, vol. 236, 147
- **Bachmann et al.** Four types of Ca2+-signals after stimulation of naive T cells with T cell agonists, partial agonists and antagonists. *Eur. J. Immunol.,* 1997, vol. 27, 3414
- **Bachmann et al.** Functional maturation of an anti-viral cytotoxic T cell response. *J. Virol.,* 1997, vol. 71, 5764
- **Bachmann et al.** Peptide induced TCR-down regulation on naive T cell predicts agonist/partial agonist properties and strictly correlates with T cell activation. *Eur. J. Immunol.,* 1997, vol. 27, 2195
- **Bachmann et al.** Distinct roles for LFA-1 and CD28 during activation of naive T cells: adhesion versus costimulation. *Immunity,* 1997, vol. 7, 549
- **Ohashi et al.** Ablation of 'tolerance' and induction of diabetes by virus infection in viral antigen transgenic mice. *Cell,* 1991, vol. 65, 305
- **Kündig et al.** Fibroblasts act as efficient antigen-presenting cells in lymphoid organs. *Science,* 1995, vol. 268, 1343
- **Speiser et al.** CTL tumor therapy specific for an endogenous antigen does not cause autoimmune disease. *J. Exp. Med.,* 1997, vol. 186, 645
- **Marshall, J. et al.** *J. Allergy Clin. Immunol.,* 2001, vol. 108, 191-197
- **Redman, T. et al.** *Exp. Lung Res.,* 2001, vol. 27, 433-451
- **Justice, J. et al.** *Am. J. Physiol. Lung Cell Mol. Physiol.,* 2002, vol. 282, L302-L309
- **Santeliz, J. et al.** *J. Allergy Clin. Immunol.,* 2002, vol. 109, 455-462
- **Arlian, L.** *Current Allergy and Asthma Reports,* 2001, vol. I, 581-586
- **Kircher, M. et al.** *J. Allergy Clin. Immunol.,* 2002, vol. 109, 517-523
- **Clarke, A. et al.** *Int. Arch. Allergy Immunol.,* 1999, vol. 120, 126-134
- **Ericksson, T. et al.** *Clinical and Exp. Allergy,* 2001, vol. 31, 1181-1890
- **Vailes, L. et al.** *J. Allergy Clin. Immunol.,* 2001, vol. 107, 641
- **Shampain, M. et al.** *Am. Rev. Respir. Dis.,* 1982, vol. 126, 493-498
- **Vailes, L. et al.** *Clinical and Exp. Allergy,* 2001, vol. 31, 1891-1895
- **Vailes, L. et al.** *J. Allergy Clin Immunol.,* 2001, vol. 107, 641
- **Sampson, H.** *N. Engl. J. Med.,* 2002, vol. 346 (17), 1294-1299
- **Helm, R et al.** *J. Allergy Clin. Immunol.,* 2002, vol. 109, 136-142
- **Li, X. et al.** *J. Allergy Clin. Immunol.,* 2001, vol. 108, 639-646
- **Sampson, H.** *N. Engl. J. Med.,* vol. 346 (17), 1294-1299
- **Bannon, G. et al.** *Int. Arch. Allergy Immunol.,* 2001, vol. 124, 70-72
- **Li, X. et al.** *J. Allergy Clin. Immunol.,* 2000, vol. 106, 150-158
- **Li, X. et al.** *J Allergy Clin. Immunol.,* 2000, vol. 106, 150-158
- REMINGTON'S PHARMACEUTICAL SCIENCES. Mack Publishing Co, 1990
- **Scott et al.** *Int. Archs. Allergy Appl. Immun.,* 1985, vol. 77, 409
- **Stomi T. et al.** *J Immunol.,* 2002, vol. 168 (6), 2880-6

SEQUENCE LISTING

<110> Cytos Biotechnology AG
BACHMANN, MARTIN F
RENNER, WOLFGANG A

<120> PACKAGING OF DNA INTO VIRUS-LIKE PARTICLES FOR USE AS ADJUVANTS: METHOD OF

<130> PA030WO

<150> US 60/389,898
<151> 2002-06-20

<160> 131

<170> PatentIn version 3.2

<210> 1
<211> 132
<212> PRAT
<213> Bacteriophage Q-beta

<400> 1

Ala Lys Leu Glu Thr Val Thr Leu Gly Asn Ile Gly Lys Asp Gly Lys
1 5 10 15

Gln Thr Leu Val Leu Asn Pro Arg Gly Val Asn Pro Thr Asn Gly Val
20 25 30

Ala Ser Leu Ser Gln Ala Gly Ala Val Pro Ala Leu Glu Lys Arg Val
35 40 45

Thr Val Ser Val Ser Gln Pro Ser Arg Asn Arg Lys Asn Tyr Lys Val
50 55 60

Gln Val Lys Ile Gln Asn Pro Thr Ala Cys Thr Ala Asn Gly Ser Cys
65 70 75 80

Asp Pro Ser Val Thr Arg Gln Ala Tyr Ala Asp Val Thr Phe Ser Phe
85 90 95

Thr Gln Tyr Ser Thr Asp Glu Glu Arg Ala Phe Val Arg Thr Glu Leu
100 105 110

Ala Ala Leu Leu Ala Ser Pro Leu Leu Ile Asp Ala Ile Asp Gln Leu
115 120 125

Asn Pro Ala Tyr
130

<210> 2
<211> 329
<212> PRT
<213> Bacteriophage Q-beta

<400> 2

Met Ala Lys Leu Glu Thr Val Thr Leu Gly Asn Ile Gly Lys Asp Gly

```
1               5                   10                  15
Lys Gln Thr Leu Val Leu Asn Pro Arg Gly Val Asn Pro Thr Asn Gly
            20                  25                  30
Val Ala Ser Leu Ser Gln Ala Gly Ala Val Pro Ala Leu Glu Lys Arg
        35                  40                  45
Val Thr Val Ser Val Ser Gln Pro Ser Arg Asn Arg Lys Asn Tyr Lys
    50                  55                  60
Val Gln Val Lys Ile Gln Asn Pro Thr Ala Cys Thr Ala Asn Gly Ser
65                  70                  75                  80
Cys Asp Pro Ser Val Thr Arg Gln Ala Tyr Ala Asp Val Thr Phe Ser
                85                  90                  95
Phe Thr Gln Tyr Ser Thr Asp Glu Glu Arg Ala Phe Val Arg Thr Glu
            100                 105                 110
Leu Ala Ala Leu Leu Ala Ser Pro Leu Leu Ile Asp Ala Ile Asp Gln
        115                 120                 125
Leu Asn Pro Ala Tyr Trp Thr Leu Leu Ile Ala Gly Gly Gly Ser Gly
    130                 135                 140
Ser Lys Pro Asp Pro Val Ile Pro Asp Pro Pro Ile Asp Pro Pro Pro
145                 150                 155                 160
Gly Thr Gly Lys Tyr Thr Cys Pro Phe Ala Ile Trp Ser Leu Glu Glu
                165                 170                 175
Val Tyr Glu Pro Pro Thr Lys Asn Arg Pro Trp Pro Ile Tyr Asn Ala
            180                 185                 190
Val Glu Leu Gln Pro Arg Glu Phe Asp Val Ala Leu Lys Asp Leu Leu
        195                 200                 205
Gly Asn Thr Lys Trp Arg Asp Trp Asp Ser Arg Leu Ser Tyr Thr Thr
    210                 215                 220
Phe Arg Gly Cys Arg Gly Asn Gly Tyr Ile Asp Leu Asp Ala Thr Tyr
225                 230                 235                 240
Leu Ala Thr Asp Gln Ala Met Arg Asp Gln Lys Tyr Asp Ile Arg Glu
                245                 250                 255
Gly Lys Lys Pro Gly Ala Phe Gly Asn Ile Glu Arg Phe Ile Tyr Leu
            260                 265                 270
Lys Ser Ile Asn Ala Tyr Cys Ser Leu Ser Asp Ile Ala Ala Tyr His
        275                 280                 285
```

Ala Asp Gly Val Ile Val Gly Phe Trp Arg Asp Pro Ser Ser Gly Gly
290 295 300

Ala Ile Pro Phe Asp Phe Thr Lys Phe Asp Lys Thr Lys Cys Pro Ile
305 310 315 320

Gln Ala Val Ile Val Val Pro Arg Ala
325

<210> 3
<211> 129
<212> PRT
<213> Bacteriophage R17

<400> 3

Ala Ser Asn Phe Thr Gln Phe Val Leu Val Asn Asp Gly Gly Thr Gly
1 5 10 15

Asn Val Thr Val Ala Pro Ser Asn Phe Ala Asn Gly Val Ala Glu Trp
20 25 30

Ile Ser Ser Asn Ser Arg Ser Gln Ala Tyr Lys Val Thr Cys Ser Val
35 40 45

Arg Gln Ser Ser Ala Gln Asn Arg Lys Tyr Thr Ile Lys Val Glu Val
50 55 60

Pro Lys Val Ala Thr Gln Thr Val Gly Gly Val Glu Leu Pro Val Ala
65 70 75 80

Ala Trp Arg Ser Tyr Leu Asn Met Glu Leu Thr Ile Pro Ile Phe Ala
85 90 95

Thr Asn Ser Asp Cys Glu Leu Ile Val Lys Ala Met Gln Gly Leu Leu
100 105 110

Lys Asp Gly Asn Pro Ile Pro Ser Ala Ile Ala Ala Asn Ser Gly Ile
115 120 125

Tyr

<210> 4
<211> 130
<212> PRT
<213> Bacteriophage f2

<400> 4

Met Ala Ser Asn Phe Glu Glu Phe Val Leu Val Asp Asn Gly Gly Thr
1 5 10 15

Gly Asp Val Lys Val Ala Pro Ser Asn Phe Ala Asn Gly Val Ala Glu

20 25 30

Trp Ile Ser Ser Asn Ser Arg Ser Gln Ala Tyr Lys Val Thr Cys Ser
35 40 45

Val Arg Gln Ser Ser Ala Asn Asn Arg Lys Tyr Thr Val Lys Val Glu
50 55 60

Val Pro Lys Val Ala Thr Gln Val Gln Gly Gly Val Glu Leu Pro Val
65 70 75 80

Ala Ala Trp Arg Ser Tyr Met Asn Met Glu Leu Thr Ile Pro Val Phe
85 90 95

Ala Thr Asn Asp Asp Cys Ala Leu Ile Val Lys Ala Leu Gln Gly Thr
100 105 110

Phe Lys Thr Gly Asn Pro Ile Ala Thr Ala Ile Ala Ala Asn Ser Gly
115 120 125

Ile Tyr
130

<210> 5
<211> 130
<212> PRT
<213> Bacteriophage GA

<400> 5

Met Ala Thr Leu Arg Ser Phe Val Leu Val Asp Asn Gly Gly Thr Gly
1               5                   10                  15

Asn Val Thr Val Val Pro Val Ser Asn Ala Asn Gly Val Ala Glu Trp
            20                  25                  30

Leu Ser Asn Asn Ser Arg Ser Gln Ala Tyr Arg Val Thr Ala Ser Tyr
        35                  40                  45

Arg Ala Ser Gly Ala Asp Lys Arg Lys Tyr Ala Ile Lys Leu Glu Val
    50                  55                  60

Pro Lys Ile Val Thr Gln Val Val Asn Gly Val Glu Leu Pro Gly Ser
65                  70                  75                  80

Ala Trp Lys Ala Tyr Ala Ser Ile Asp Leu Thr Ile Pro Ile Phe Ala
                85                  90                  95

Ala Thr Asp Asp Val Thr Val Ile Ser Lys Ser Leu Ala Gly Leu Phe
            100                 105                 110

Lys Val Gly Asn Pro Ile Ala Glu Ala Ile Ser Ser Gln Ser Gly Phe
        115                 120                 125

Tyr Ala
    130

<210> 6
<211> 132
<212> PRT
<213> Bacteriophage SP

<400> 6

Met Ala Lys Leu Asn Gln Val Thr Leu Ser Lys Ile Gly Lys Asn Gly
1 5 10 15

Asp Gln Thr Leu Thr Leu Thr Pro Arg Gly Val Asn Pro Thr Asn Gly
20 25 30

Val Ala Ser Leu Ser Glu Ala Gly Ala Val Pro Ala Leu Glu Lys Arg
35 40 45

Val Thr Val Ser Val Ala Gln Pro Ser Arg Asn Arg Lys Asn Phe Lys
50 55 60

Val Gln Ile Lys Leu Gln Asn Pro Thr Ala Cys Thr Arg Asp Ala Cys
65 70 75 80

Asp Pro Ser Val Thr Arg Ser Ala Phe Ala Asp Val Thr Leu Ser Phe
85 90 95

Thr Ser Tyr Ser Thr Asp Glu Glu Arg Ala Leu Ile Arg Thr Glu Leu
100 105 110

Ala Ala Leu Leu Ala Asp Pro Leu Ile Val Asp Ala Ile Asp Asn Leu
115 120 125

Asn Pro Ala Tyr
130

<210> 7
<211> 329
<212> PRT
<213> Bacteriophage SP

<400> 7

Ala Lys Leu Asn Gln Val Thr Leu Ser Lys Ile Gly Lys Asn Gly Asp
1 5 10 15

Gln Thr Leu Thr Leu Thr Pro Arg Gly Val Asn Pro Thr Asn Gly Val
20 25 30

Ala Ser Leu Ser Glu Ala Gly Ala Val Pro Ala Leu Glu Lys Arg Val
35 40 45

Thr Val Ser Val Ala Gln Pro Ser Arg Asn Arg Lys Asn Phe Lys Val
50 55 60

```
Gln Ile Lys Leu Gln Asn Pro Thr Ala Cys Thr Arg Asp Ala Cys Asp
65                  70                  75                  80

Pro Ser Val Thr Arg Ser Ala Phe Ala Asp Val Thr Leu Ser Phe Thr
                85                  90                  95

Ser Tyr Ser Thr Asp Glu Glu Arg Ala Leu Ile Arg Thr Glu Leu Ala
            100                 105                 110

Ala Leu Leu Ala Asp Pro Leu Ile Val Asp Ala Ile Asp Asn Leu Asn
        115                 120                 125

Pro Ala Tyr Trp Ala Ala Leu Leu Val Ala Ser Ser Gly Gly Gly Asp
    130                 135                 140

Asn Pro Ser Asp Pro Asp Val Pro Val Val Pro Asp Val Lys Pro Pro
145                 150                 155                 160

Asp Gly Thr Gly Arg Tyr Lys Cys Pro Phe Ala Cys Tyr Arg Leu Gly
                165                 170                 175

Ser Ile Tyr Glu Val Gly Lys Glu Gly Ser Pro Asp Ile Tyr Glu Arg
            180                 185                 190

Gly Asp Glu Val Ser Val Thr Phe Asp Tyr Ala Leu Glu Asp Phe Leu
        195                 200                 205

Gly Asn Thr Asn Trp Arg Asn Trp Asp Gln Arg Leu Ser Asp Tyr Asp
    210                 215                 220

Ile Ala Asn Arg Arg Arg Cys Arg Gly Asn Gly Tyr Ile Asp Leu Asp
225                 230                 235                 240

Ala Thr Ala Met Gln Ser Asp Asp Phe Val Leu Ser Gly Arg Tyr Gly
                245                 250                 255

Val Arg Lys Val Lys Phe Pro Gly Ala Phe Gly Ser Ile Lys Tyr Leu
            260                 265                 270

Leu Asn Ile Gln Gly Asp Ala Trp Leu Asp Leu Ser Glu Val Thr Ala
        275                 280                 285

Tyr Arg Ser Tyr Gly Met Val Ile Gly Phe Trp Thr Asp Ser Lys Ser
    290                 295                 300

Pro Gln Leu Pro Thr Asp Phe Thr Gln Phe Asn Ser Ala Asn Cys Pro
305                 310                 315                 320

Val Gln Thr Val Ile Ile Ile Pro Ser
                325
```

<210> 8

<211> 130
<212> PRT
<213> Bacteriophage Ms2

<400> 8

Met Ala Ser Asn Phe Thr Gln Phe Val Leu Val Asp Asn Gly Gly Thr
1 5 10 15

Gly Asp Val Thr Val Ala Pro Ser Asn Phe Ala Asn Gly Val Ala Glu
20 25 30

Trp Ile Ser Ser Asn Ser Arg Ser Gln Ala Tyr Lys Val Thr Cys Ser
35 40 45

Val Arg Gln Ser Ser Ala Gln Asn Arg Lys Tyr Thr Ile Lys Val Glu
50 55 60

Val Pro Lys Val Ala Thr Gln Thr Val Gly Gly Val Glu Leu Pro Val
65 70 75 80

Ala Ala Trp Arg Ser Tyr Leu Asn Met Glu Leu Thr Ile Pro Ile Phe
85 90 95

Ala Thr Asn Ser Asp Cys Glu Leu Ile Val Lys Ala Met Gln Gly Leu
100 105 110

Leu Lys Asp Gly Asn Pro Ile Pro Ser Ala Ile Ala Ala Asn Ser Gly
115 120 125

Ile Tyr
130

<210> 9
<211> 133
<212> PRT
<213> Bacteriophage M11

<400> 9

Met Ala Lys Leu Gln Ala Ile Thr Leu Ser Gly Ile Gly Lys Lys Gly
1               5                   10                  15

Asp Val Thr Leu Asp Leu Asn Pro Arg Gly Val Asn Pro Thr Asn Gly
            20                  25                  30

Val Ala Ala Leu Ser Glu Ala Gly Ala Val Pro Ala Leu Glu Lys Arg
        35                  40                  45

Val Thr Ile Ser Val Ser Gln Pro Ser Arg Asn Arg Lys Asn Tyr Lys
    50                  55                  60

Val Gln Val Lys Ile Gln Asn Pro Thr Ser Cys Thr Ala Ser Gly Thr
65                  70                  75                  80

Cys Asp Pro Ser Val Thr Arg Ser Ala Tyr Ser Asp Val Thr Phe Ser
                85                  90                  95

Phe Thr Gln Tyr Ser Thr Val Glu Glu Arg Ala Leu Val Arg Thr Glu
            100                 105                 110

Leu Gln Ala Leu Leu Ala Asp Pro Met Leu Val Asn Ala Ile Asp Asn
        115                 120                 125

Leu Asn Pro Ala Tyr
    130

<210> 10
<211> 133
<212> PRT
<213> Bacteriophage MX1

<400> 10

Met Ala Lys Leu Gln Ala Ile Thr Leu Ser Gly Ile Gly Lys Asn Gly
1               5                   10                  15

Asp Val Thr Leu Asn Leu Asn Pro Arg Gly Val Asn Pro Thr Asn Gly
            20                  25                  30

Val Ala Ala Leu Ser Glu Ala Gly Ala Val Pro Ala Leu Glu Lys Arg
        35                  40                  45

Val Thr Ile Ser Val Ser Gln Pro Ser Arg Asn Arg Lys Asn Tyr Lys
    50                  55                  60

Val Gln Val Lys Ile Gln Asn Pro Thr Ser Cys Thr Ala Ser Gly Thr
65                  70                  75                  80

Cys Asp Pro Ser Val Thr Arg Ser Ala Tyr Ala Asp Val Thr Phe Ser
                85                  90                  95

Phe Thr Gln Tyr Ser Thr Asp Glu Glu Arg Ala Leu Val Arg Thr Glu
            100                 105                 110

Leu Lys Ala Leu Leu Ala Asp Pro Met Leu Ile Asp Ala Ile Asp Asn
        115                 120                 125

Leu Asn Pro Ala Tyr
    130

<210> 11
<211> 330
<212> PRT
<213> Bacteriophage NL95

<400> 11

Met Ala Lys Leu Asn Lys Val Thr Leu Thr Gly Ile Gly Lys Ala Gly

1 5 10 15

Asn Gln Thr Leu Thr Leu Thr Pro Arg Gly Val Asn Pro Thr Asn Gly
20 25 30

Val Ala Ser Leu Ser Glu Ala Gly Ala Val Pro Ala Leu Glu Lys Arg
35 40 45

Val Thr Val Ser Val Ala Gln Pro Ser Arg Asn Arg Lys Asn Tyr Lys
50 55 60

Val Gln Ile Lys Leu Gln Asn Pro Thr Ala Cys Thr Lys Asp Ala Cys
65 70 75 80

Asp Pro Ser Val Thr Arg Ser Gly Ser Arg Asp Val Thr Leu Ser Phe
85 90 95

Thr Ser Tyr Ser Thr Glu Arg Glu Arg Ala Leu Ile Arg Thr Glu Leu
100 105 110

Ala Ala Leu Leu Lys Asp Asp Leu Ile Val Asp Ala Ile Asp Asn Leu
115 120 125

Asn Pro Ala Tyr Trp Ala Ala Leu Leu Ala Ala Ser Pro Gly Gly Gly
130 135 140

Asn Asn Pro Tyr Pro Gly Val Pro Asp Ser Pro Asn Val Lys Pro Pro
145 150 155 160

Gly Gly Thr Gly Thr Tyr Arg Cys Pro Phe Ala Cys Tyr Arg Arg Gly
165 170 175

Glu Leu Ile Thr Glu Ala Lys Asp Gly Ala Cys Ala Leu Tyr Ala Cys
180 185 190

Gly Ser Glu Ala Leu Val Glu Phe Glu Tyr Ala Leu Glu Asp Phe Leu
195 200 205

Gly Asn Glu Phe Trp Arg Asn Trp Asp Gly Arg Leu Ser Lys Tyr Asp
210 215 220

Ile Glu Thr His Arg Arg Cys Arg Gly Asn Gly Tyr Val Asp Leu Asp
225 230 235 240

Ala Ser Val Met Gln Ser Asp Glu Tyr Val Leu Ser Gly Ala Tyr Asp
245 250 255

Val Val Lys Met Gln Pro Pro Gly Thr Phe Asp Ser Pro Arg Tyr Tyr
260 265 270

Leu His Leu Met Asp Gly Ile Tyr Val Asp Leu Ala Glu Val Thr Ala
275 280 285

Tyr Arg Ser Tyr Gly Met Val Ile Gly Phe Trp Thr Asp Ser Lys Ser
290 295 300

Pro Gln Leu Pro Thr Asp Phe Thr Arg Phe Asn Arg His Asn Cys Pro
305 310 315 320

Val Gln Thr Val Ile Val Ile Pro Ser Leu
325 330

<210> 12
<211> 129
<212> PRT
<213> Bacteriophage F2

<400> 12

Ala Ser Asn Phe Thr Gln Phe Val Leu Val Asn Asp Gly Gly Thr Gly
1 5 10 15

Asn Val Thr Val Ala Pro Ser Asn Phe Ala Asn Gly Val Ala Glu Trp
20 25 30

Ile Ser Ser Asn Ser Arg Ser Gln Ala Tyr Lys Val Thr Cys Ser Val
35 40 45

Arg Gln Ser Ser Ala Gln Asn Arg Lys Tyr Thr Ile Lys Val Glu Val
50 55 60

Pro Lys Val Ala Thr Gln Thr Val Gly Gly Val Glu Leu Pro Val Ala
65 70 75 80

Ala Trp Arg Ser Tyr Leu Asn Leu Glu Leu Thr Ile Pro Ile Phe Ala
85 90 95

Thr Asn Ser Asp Cys Glu Leu Ile Val Lys Ala Met Gln Gly Leu Leu
100 105 110

Lys Asp Gly Asn Pro Ile Pro Ser Ala Ile Ala Ala Asn Ser Gly Ile
115 120 125

Tyr

<210> 13
<211> 128
<212> PRT
<213> Bacteriophage PP7

<400> 13

Met Ser Lys Thr Ile Val Leu Ser Val Gly Glu Ala Thr Arg Thr Leu
1               5                   10                  15

Thr Glu Ile Gln Ser Thr Ala Asp Arg Gln Ile Phe Glu Glu Lys Val

            20                  25                  30

Gly Pro Leu Val Gly Arg Leu Arg Leu Thr Ala Ser Leu Arg Gln Asn
        35                  40                  45

Gly Ala Lys Thr Ala Tyr Arg Val Asn Leu Lys Leu Asp Gln Ala Asp
    50                  55                  60

Val Val Asp Cys Ser Thr Ser Val Cys Gly Glu Leu Pro Lys Val Arg
65                  70                  75                  80

Tyr Thr Gln Val Trp Ser His Asp Val Thr Ile Val Ala Asn Ser Thr
                85                  90                  95

Glu Ala Ser Arg Lys Ser Leu Tyr Asp Leu Thr Lys Ser Leu Val Ala
            100                 105                 110

Thr Ser Gln Val Glu Asp Leu Val Val Asn Leu Val Pro Leu Gly Arg
        115                 120                 125

<210> 14
<211> 132
<212> PRT
<213> Artificial sequence

<220>
<223> Bacteriophage Qbeta 240 mutant

<400> 14

```
Ala Lys Leu Glu Thr Val Thr Leu Gly Asn Ile Gly Arg Asp Gly Lys
1               5                   10                  15

Gln Thr Leu Val Leu Asn Pro Arg Gly Val Asn Pro Thr Asn Gly Val
            20                  25                  30

Ala Ser Leu Ser Gln Ala Gly Ala Val Pro Ala Leu Glu Lys Arg Val
        35                  40                  45

Thr Val Ser Val Ser Gln Pro Ser Arg Asn Arg Lys Asn Tyr Lys Val
    50                  55                  60

Gln Val Lys Ile Gln Asn Pro Thr Ala Cys Thr Ala Asn Gly Ser Cys
65                  70                  75                  80

Asp Pro Ser Val Thr Arg Gln Lys Tyr Ala Asp Val Thr Phe Ser Phe
                85                  90                  95

Thr Gln Tyr Ser Thr Asp Glu Glu Arg Ala Phe Val Arg Thr Glu Leu
            100                 105                 110

Ala Ala Leu Leu Ala Ser Pro Leu Leu Ile Asp Ala Ile Asp Gln Leu
        115                 120                 125

Asn Pro Ala Tyr
    130
```

<210> 15
<211> 132
<212> PRT
<213> Artificial Sequence

<220>
<223> Bacteriophage Q-beta 243 mutant

<400> 15

Ala Lys Leu Glu Thr Val Thr Leu Gly Lys Ile Gly Lys Asp Gly Lys
1               5                   10                  15

Gln Thr Leu Val Leu Asn Pro Arg Gly Val Asn Pro Thr Asn Gly Val
            20                  25                  30

Ala Ser Leu Ser Gln Ala Gly Ala Val Pro Ala Leu Glu Lys Arg Val
        35                  40                  45

Thr Val Ser Val Ser Gln Pro Ser Arg Asn Arg Lys Asn Tyr Lys Val
    50                  55                  60

Gln Val Lys Ile Gln Asn Pro Thr Ala Cys Thr Ala Asn Gly Ser Cys
65                  70                  75                  80

Asp Pro Ser Val Thr Arg Gln Lys Tyr Ala Asp Val Thr Phe Ser Phe
                85                  90                  95

Thr Gln Tyr Ser Thr Asp Glu Glu Arg Ala Phe Val Arg Thr Glu Leu
            100                 105                 110

Ala Ala Leu Leu Ala Ser Pro Leu Leu Ile Asp Ala Ile Asp Gln Leu
        115                 120                 125

Asn Pro Ala Tyr
    130

<210> 16
<211> 132
<212> PRT
<213> Artificial Sequence

<220>
<223> Bacteriophage Q-beta 250 mutant

<400> 16

Ala Arg Leu Glu Thr Val Thr Leu Gly Asn Ile Gly Arg Asp Gly Lys
1               5                   10                  15

Gln Thr Leu Val Leu Asn Pro Arg Gly Val Asn Pro Thr Asn Gly Val
            20                  25                  30

Ala Ser Leu Ser Gln Ala Gly Ala Val Pro Ala Leu Glu Lys Arg Val
                                          12

35 40 45

Thr Val Ser Val Ser Gln Pro Ser Arg Asn Arg Lys Asn Tyr Lys Val
50 55 60

Gln Val Lys Ile Gln Asn Pro Thr Ala Cys Thr Ala Asn Gly Ser Cys
65 70 75 80

Asp Pro Ser Val Thr Arg Gln Lys Tyr Ala Asp Val Thr Phe Ser Phe
85 90 95

Thr Gln Tyr Ser Thr Asp Glu Glu Arg Ala Phe Val Arg Thr Glu Leu
100 105 110

Ala Ala Leu Leu Ala Ser Pro Leu Leu Ile Asp Ala Ile Asp Gln Leu
115 120 125

Asn Pro Ala Tyr
130

<210> 17
<211> 132
<212> PRT
<213> Artificial Sequence

<220>
<223> Bacteriophage Q-beta 251 mutant

<400> 17

Ala Lys Leu Glu Thr Val Thr Leu Gly Asn Ile Gly Lys Asp Gly Arg
1               5                   10                  15

Gln Thr Leu Val Leu Asn Pro Arg Gly Val Asn Pro Thr Asn Gly Val
            20                  25                  30

Ala Ser Leu Ser Gln Ala Gly Ala Val Pro Ala Leu Glu Lys Arg Val
        35                  40                  45

Thr Val Ser Val Ser Gln Pro Ser Arg Asn Arg Lys Asn Tyr Lys Val
    50                  55                  60

Gln Val Lys Ile Gln Asn Pro Thr Ala Cys Thr Ala Asn Gly Ser Cys
65                  70                  75                  80

Asp Pro Ser Val Thr Arg Gln Lys Tyr Ala Asp Val Thr Phe Ser Phe
                85                  90                  95

Thr Gln Tyr Ser Thr Asp Glu Glu Arg Ala Phe Val Arg Thr Glu Leu
            100                 105                 110

Ala Ala Leu Leu Ala Ser Pro Leu Leu Ile Asp Ala Ile Asp Gln Leu
        115                 120                 125

Asn Pro Ala Tyr
    130

<210> 18
<211> 132 '
<212> PRT
<213> Artificial sequence

<220>
<223> Bacteriophage Q-beta 259 mutant

<400> 18

```
Ala Arg Leu Glu Thr Val Thr Leu Gly Asn Ile Gly Lys Asp Gly Arg
1               5                   10                  15

Gln Thr Leu Val Leu Asn Pro Arg Gly Val Asn Pro Thr Asn Gly Val
            20                  25                  30

Ala Ser Leu Ser Gln Ala Gly Ala Val Pro Ala Leu Glu Lys Arg Val
        35                  40                  45

Thr Val Ser Val Ser Gln Pro Ser Arg Asn Arg Lys Asn Tyr Lys Val
    50                  55                  60

Gln Val Lys Ile Gln Asn Pro Thr Ala Cys Thr Ala Asn Gly Ser Cys
65                  70                  75                  80

Asp Pro Ser Val Thr Arg Gln Lys Tyr Ala Asp Val Thr Phe Ser Phe
                85                  90                  95

Thr Gln Tyr Ser Thr Asp Glu Glu Arg Ala Phe Val Arg Thr Glu Leu
            100                 105                 110

Ala Ala Leu Leu Ala Ser Pro Leu Leu Ile Asp Ala Ile Asp Gln Leu
        115                 120                 125

Asn Pro Ala Tyr
    130
```

<210> 19
<211> 185
<212> PRT
<213> Hepatitis B virus

<400> 19

```
Met Asp Ile Asp Pro Tyr Lys Glu Phe Gly Ala Thr Val Glu Leu Leu
1               5                   10                  15

Ser Phe Leu Pro Ser Asp Phe Phe Pro Ser Val Arg Asp Leu Leu Asp
            20                  25                  30

Thr Ala Ser Ala Leu Tyr Arg Glu Ala Leu Glu Ser Pro Glu His Cys
        35                  40                  45
```

Ser Pro His His Thr Ala Leu Arg Gln Ala Ile Leu Cys Trp Gly Glu
50 55 60

Leu Met Thr Leu Ala Thr Trp Val Gly Asn Asn Leu Glu Asp Pro Ala
65 70 75 80

Ser Arg Asp Leu Val Val Asn Tyr Val Asn Thr Asn Met Gly Leu Lys
85 90 95

Ile Arg Gln Leu Leu Trp Phe His Ile Ser Cys Leu Thr Phe Gly Arg
100 105 110

Glu Thr Val Leu Glu Tyr Leu Val Ser Phe Gly Val Trp Ile Arg Thr
115 120 125

Pro Pro Ala Tyr Arg Pro Pro Asn Ala Pro Ile Leu Ser Thr Leu Pro
130 135 140

Glu Thr Thr Val Val Arg Arg Arg Asp Arg Gly Arg Ser Pro Arg Arg
145 150 155 160

Arg Thr Pro Ser Pro Arg Arg Arg Arg Ser Gln Ser Pro Arg Arg Arg
165 170 175

Arg Ser Gln Ser Arg Glu Ser Gln Cys
180 185

<210> 20
<211> 183
<212> PRT
<213> Hepatitis B virus

<400> 20

Met Asp Ile Asp Pro Tyr Lys Glu Phe Gly Ala Thr Val Glu Leu Leu
1               5                   10                  15

Ser Phe Leu Pro Ser Asp Phe Phe Pro Ser Val Arg Asp Leu Leu Asp
            20                  25                  30

Thr Ala Ser Ala Leu Tyr Arg Glu Ala Leu Glu Ser Pro Glu His Cys
        35                  40                  45

Ser Pro His His Thr Ala Leu Arg Gln Ala Ile Leu Cys Trp Gly Glu
    50                  55                  60

Leu Met Thr Leu Ala Thr Trp Val Gly Gly Asn Leu Glu Asp Pro Ile
65                  70                  75                  80

Ser Arg Asp Leu Val Val Ser Tyr Val Asn Thr Asn Met Gly Leu Lys
                85                  90                  95

Phe Arg Gln Leu Leu Trp Phe His Ile Ser Cys Leu Thr Phe Gly Arg
            100                 105                 110

Glu Thr Val Ile Glu Tyr Leu Val Ser Phe Gly Val Trp Ile Arg Thr
        115                 120                 125

Pro Pro Ala Tyr Arg Pro Pro Asn Ala Pro Ile Leu Ser Thr Leu Pro
    130                 135                 140

Glu Thr Thr Val Val Arg Arg Arg Gly Arg Ser Pro Arg Arg Arg Thr
145                 150                 155                 160

Pro Ser Pro Arg Arg Arg Arg Ser Gln Ser Pro Arg Arg Arg Arg Ser
                165                 170                 175

Gln Ser Arg Gly Ser Gln Cys
            180

<210> 21
<211> 183
<212> PRT
<213> Hepatitis B virus

<400> 21

Met Asp Ile Asp Pro Tyr Lys Glu Phe Gly Ala Thr Val Glu Leu Leu
1               5                   10                  15

Ser Phe Leu Pro Ser Asp Phe Phe Pro Ser Val Arg Asp Leu Leu Asp
            20                  25                  30

Thr Ala Ser Ala Leu Tyr Arg Glu Ala Leu Glu Ser Pro Glu His Cys
        35                  40                  45

Ser Pro His His Thr Ala Leu Arg Gln Ala Ile Leu Cys Trp Gly Glu
    50                  55                  60

Leu Met Thr Leu Ala Thr Trp Val Gly Gly Asn Leu Glu Asp Pro Thr
65                  70                  75                  80

Ser Arg Asp Leu Val Val Ser Tyr Val Asn Thr Asn Met Gly Leu Lys
                85                  90                  95

Phe Arg Gln Leu Leu Trp Phe His Ile Ser Cys Leu Thr Phe Gly Arg
            100                 105                 110

Glu Thr Val Ile Glu Tyr Leu Val Ser Phe Gly Val Trp Ile Arg Thr
        115                 120                 125

Pro Pro Ala Tyr Arg Pro Thr Asn Ala Pro Ile Leu Ser Thr Leu Pro
    130                 135                 140

Glu Thr Cys Val Ile Arg Arg Arg Gly Arg Ser Pro Arg Arg Arg Thr
145                 150                 155                 160

Pro Ser Pro Arg Arg Arg Arg Ser Gln Ser Pro Arg Arg Arg Arg Ser
                165                 170                 175

Gln Ser Arg Gly Ser Gln Cys
            180

<210> 22
<211> 212
<212> PRT
<213> Hepatitis B virus

<400> 22

Met Gln Leu Phe His Leu Cys Leu Ile Ile Ser Cys Ser Cys Pro Thr
1               5                   10                  15

Val Gln Ala Ser Lys Leu Cys Leu Gly Trp Leu Trp Gly Met Asp Ile
            20                  25                  30

Asp Pro Tyr Lys Glu Phe Gly Ala Thr Val Glu Leu Leu Ser Phe Leu
        35                  40                  45

Pro Ser Asp Phe Phe Pro Ser Val Arg Asp Leu Leu Asp Thr Ala Ser
    50                  55                  60

Ala Leu Tyr Arg Glu Ala Leu Glu Ser Pro Glu His Cys Ser Pro His
65                  70                  75                  80

His Thr Ala Leu Arg Gln Ala Ile Leu Cys Trp Gly Glu Leu Met Thr
                85                  90                  95

Leu Ala Thr Trp Val Gly Gly Asn Leu Glu Asp Pro Ile Ser Arg Asp
            100                 105                 110

Leu Val Val Ser Tyr Val Asn Thr Asn Met Gly Leu Lys Phe Arg Gln
        115                 120                 125

Leu Leu Trp Phe His Ile Ser Cys Leu Thr Phe Gly Arg Glu Thr Val
    130                 135                 140

Ile Glu Tyr Leu Val Ser Phe Gly Val Trp Ile Arg Thr Pro Pro Ala
145                 150                 155                 160

Tyr Arg Pro Pro Asn Ala Pro Ile Leu Ser Thr Leu Pro Glu Thr Thr
                165                 170                 175

Val Val Arg Arg Arg Gly Arg Ser Pro Arg Arg Arg Thr Pro Ser Pro
            180                 185                 190

Arg Arg Arg Arg Ser Gln Ser Pro Arg Arg Arg Arg Ser Gln Ser Arg
        195                 200                 205

Glu Ser Gln Cys
    210

<210> 23
<211> 212
<212> PRT
<213> Hepatitis B virus

<400> 23

Met Gln Leu Phe His Leu Cys Leu Ile Ile Ser Cys Ser Cys Pro Thr
1 5 10 15

Val Gln Ala Ser Lys Leu Cys Leu Gly Trp Leu Trp Gly Met Asp Ile
20 25 30

Asp Pro Tyr Lys Glu Phe Gly Ala Thr Val Glu Leu Leu Ser Phe Leu
35 40 45

Pro Ser Asp Phe Phe Pro Ser Val Arg Asp Leu Leu Asp Asn Ala Ser
50 55 60

Ala Leu Tyr Arg Glu Ala Leu Glu Ser Pro Glu His Cys Ser Pro His
65 70 75 80

His Thr Ala Leu Arg Gln Ala Ile Leu Cys Trp Gly Glu Leu Met Thr
85 90 95

Leu Ala Thr Trp Val Gly Gly Asn Leu Glu Asp Pro Ile Ser Arg Asp
100 105 110

Leu Val Val Ser Tyr Val Asn Thr Asn Met Gly Leu Lys Phe Arg Gln
115 120 125

Leu Leu Trp Phe His Ile Ser Cys Leu Thr Phe Gly Arg Glu Thr Val
130 135 140

Ile Glu Tyr Leu Val Ser Phe Gly Val Trp Ile Arg Thr Pro Pro Ala
145 150 155 160

Tyr Arg Pro Pro Asn Ala Pro Ile Leu Ser Thr Leu Pro Glu Thr Thr
165 170 175

Val Val Arg Arg Arg Gly Arg Ser Pro Arg Arg Arg Thr Pro Ser Pro
180 185 190

Arg Arg Arg Arg Ser Gln Ser Pro Arg Arg Arg Arg Ser Gln Ser Arg
195 200 205

Glu Ser Gln Cys
210

<210> 24
<211> 183
<212> PRT
<213> Hepatitis B virus

<400> 24

Met Asp Ile Asp Pro Tyr Lys Glu Phe Gly Ala Thr Val Glu Leu Leu
1               5                   10                  15

Ser Phe Leu Pro Thr Asp Phe Phe Pro Ser Val Arg Asp Leu Leu Asp
            20                  25                  30

Thr Ala Ser Ala Leu Tyr Arg Glu Ala Leu Glu Ser Pro Glu His Cys
        35                  40                  45

Ser Pro His His Thr Ala Leu Arg Gln Ala Ile Leu Cys Trp Gly Glu
    50                  55                  60

Leu Met Thr Leu Ala Thr Trp Val Gly Val Asn Leu Glu Asp Pro Ala
65                  70                  75                  80

Ser Arg Asp Leu Val Val Ser Tyr Val Asn Thr Asn Met Gly Leu Lys
                85                  90                  95

Phe Arg Gln Leu Leu Trp Phe His Ile Ser Cys Leu Thr Phe Gly Arg
            100                 105                 110

Glu Thr Val Ile Glu Tyr Leu Val Ser Phe Gly Val Trp Ile Arg Thr
        115                 120                 125

Pro Pro Ala Tyr Arg Pro Pro Asn Ala Pro Ile Leu Ser Thr Leu Pro
    130                 135                 140

Glu Thr Cys Val Val Arg Arg Arg Gly Arg Ser Pro Arg Arg Arg Thr
145                 150                 155                 160

Pro Ser Pro Arg Arg Arg Arg Ser Gln Ser Pro Arg Arg Arg Arg Ser
                165                 170                 175

Gln Ser Arg Glu Ser Gln Cys
            180

<210> 25
<211> 212
<212> PRT
<213> Hepatitis B virus

<400> 25

Met Gln Leu Phe His Leu Cys Leu Ile Ile Ser Cys Ser Cys Pro Thr
1               5                   10                  15

Val Gln Ala Ser Lys Leu Cys Leu Gly Trp Leu Trp Gly Met Asp Ile
            20                  25                  30

Asp Pro Tyr Lys Glu Phe Gly Ala Thr Val Glu Leu Leu Ser Phe Leu
        35                  40                  45

Pro Ser Asp Phe Phe Pro Ser Val Arg Asp Leu Leu Asp Thr Ala Ser
50 55 60

Ala Leu Tyr Arg Glu Ala Leu Glu Ser Pro Glu His Cys Ser Pro His
65 70 75 80

His Thr Ala Leu Arg Gln Ala Ile Leu Cys Trp Gly Asp Leu Met Thr
85 90 95

Leu Ala Thr Trp Val Gly Gly Asn Leu Glu Asp Pro Val Ser Arg Asp
100 105 110

Leu Val Val Ser Tyr Val Asn Thr Asn Val Gly Leu Lys Phe Arg Gln
115 120 125

Leu Leu Trp Phe His Ile Ser Cys Leu Thr Phe Gly Arg Glu Thr Val
130 135 140

Ile Glu Tyr Leu Val Ser Phe Gly Val Trp Ile Arg Thr Pro Pro Ala
145 150 155 160

Tyr Arg Pro Pro Asn Ala Pro Ile Leu Ser Thr Leu Pro Glu Thr Thr
165 170 175

Val Val Arg Arg Arg Gly Arg Ser Pro Arg Arg Arg Thr Pro Ser Pro
180 185 190

Arg Arg Arg Arg Ser Gln Ser Pro Arg Arg Arg Arg Ser Gln Ser Arg
195 200 205

Glu Ser Gln Cys
210

<210> 26
<211> 212
<212> PRT
<213> Hepatitis B virus

<400> 26

```
Met Gln Leu Phe His Leu Cys Leu Ile Ile Ser Cys Ser Cys Pro Thr
1               5                   10                  15

Val Gln Ala Ser Lys Leu Cys Leu Gly Trp Leu Trp Asp Met Asp Ile
            20                  25                  30

Asp Pro Tyr Lys Glu Phe Gly Ala Thr Val Glu Leu Leu Ser Phe Leu
        35                  40                  45

Pro Ser Asp Phe Phe Pro Ser Val Arg Asp Leu Leu Asp Thr Ala Ser
    50                  55                  60

Ala Leu Tyr Arg Glu Ala Leu Glu Ser Pro Glu His Cys Ser Pro His


65                  70                  75                  80

His Thr Ala Leu Arg Gln Ala Ile Leu Cys Trp Gly Asp Leu Met Thr
                85                  90                  95

Leu Ala Thr Trp Val Gly Gly Asn Leu Glu Asp Pro Val Ser Arg Asp
            100                 105                 110

Leu Val Val Ser Tyr Val Asn Thr Asn Val Gly Leu Lys Phe Arg Gln
        115                 120                 125

Leu Leu Trp Phe His Ile Ser Cys Leu Thr Phe Gly Arg Glu Thr Val
    130                 135                 140

Ile Glu Tyr Leu Val Ser Phe Gly Val Trp Ile Arg Thr Pro Pro Ala
145                 150                 155                 160

Tyr Arg Pro Pro Asn Ala Pro Ile Leu Ser Thr Leu Pro Glu Thr Thr
                165                 170                 175

Val Val Arg Arg Arg Gly Arg Ser Pro Arg Arg Arg Thr Pro Ser Pro
            180                 185                 190

Arg Arg Arg Arg Ser Gln Ser Pro Arg Arg Arg Arg Ser Gln Ser Arg
        195                 200                 205

Glu Ser Gln Cys
    210
```

<210> 27
<211> 212
<212> PRT
<213> Hepatitis B virus

<400> 27

Met Gln Leu Phe His Leu Cys Leu Ile Ile Ser Cys Ser Cys Pro Thr
1 5 10 15

Val Gln Ala Ser Lys Leu Cys Leu Gly Trp Leu Trp Gly Met Asp Ile
20 25 30

Asp Pro Tyr Lys Glu Phe Gly Ala Thr Val Glu Leu Leu Ser Phe Leu
35 40 45

Pro Ser Asp Phe Phe Pro Ser Val Arg Asp Leu Leu Asp Thr Ala Ser
50 55 60

Ala Leu Tyr Arg Glu Ala Leu Glu Ser Pro Glu His Cys Ser Pro Gln
65 70 75 80

His Thr Ala Leu Arg Gln Ala Ile Leu Cys Trp Gly Glu Leu Met Thr
85 90 95

Leu Ala Thr Trp Val Gly Gly Asn Leu Glu Asp Pro Ile Ser Arg Asp
100 105 110

Leu Val Val Ser Tyr Val Asn Thr Asn Met Gly Leu Lys Phe Arg Gln
115 120 125

Leu Leu Trp Phe His Ile Ser Cys Leu Thr Phe Gly Arg Glu Thr Val
130 135 140

Ile Glu Tyr Leu Val Ser Phe Gly Val Trp Ile Arg Thr Pro Pro Ala
145 150 155 160

Tyr Arg Pro Pro Asn Ala Pro Ile Leu Ser Thr Leu Pro Glu Thr Thr
165 170 175

Val Val Arg Arg Arg Gly Arg Ser Pro Arg Arg Arg Thr Pro Ser Pro
180 185 190

Arg Arg Arg Arg Ser Gln Ser Pro Arg Arg Arg Arg Ser Gln Ser Arg
195 200 205

Glu Ser Gln Cys
210

<210> 28
<211> 212
<212> PRT
<213> Hepatitis B virus

<400> 28

```
Met Gln Leu Phe His Leu Cys Leu Ile Ile Ser Cys Ser Cys Pro Thr
1               5                   10                  15

Val Gln Ala Ser Lys Leu Cys Leu Gly Trp Leu Trp Gly Met Asp Ile
            20                  25                  30

Asp Pro Tyr Lys Glu Phe Gly Ala Thr Val Glu Leu Leu Ser Phe Leu
        35                  40                  45

Pro Ser Asp Phe Phe Pro Ser Val Arg Asp Leu Leu Asp Thr Ala Ser
    50                  55                  60

Ala Leu Tyr Arg Glu Ala Leu Glu Ser Pro Glu His Cys Ser Pro His
65                  70                  75                  80

His Thr Ala Leu Arg Gln Ala Ile Leu Cys Trp Gly Glu Leu Met Thr
                85                  90                  95

Leu Ala Thr Trp Val Gly Val Asn Leu Glu Asp Pro Ala Ser Arg Asp
            100                 105                 110

Leu Val Val Ser Tyr Val Asn Thr Asn Met Gly Leu Lys Phe Arg Gln


        115                 120                 125

Leu Leu Trp Phe His Ile Ser Cys Leu Thr Phe Gly Arg Glu Thr Val
    130                 135                 140

Ile Glu Tyr Leu Val Ser Phe Gly Val Trp Ile Arg Thr Pro Pro Ala
145                 150                 155                 160

Tyr Lys Pro Pro Asn Ala Pro Ile Leu Ser Thr Leu Pro Glu Thr Thr
                165                 170                 175

Val Val Arg Arg Arg Gly Arg Ser Pro Arg Arg Arg Thr Pro Ser Pro
            180                 185                 190

Arg Arg Arg Arg Ser Gln Ser Pro Arg Arg Arg Arg Ser Gln Ser Arg
        195                 200                 205

Gly Ser Gln Cys
    210
```

<210> 29
<211> 183
<212> PRT
<213> Hepatitis B virus

<400> 29

Met Asp Ile Asp Pro Tyr Lys Glu Phe Gly Ala Thr Val Glu Leu Leu
1               5                   10                  15

Ser Phe Leu Pro Ser Asp Phe Phe Pro Ser Val Arg Asp Leu Leu Asp
            20                  25                  30

Thr Ala Ser Ala Leu Phe Arg Asp Ala Leu Glu Ser Pro Glu His Cys
        35                  40                  45

Ser Pro His His Thr Ala Leu Arg Gln Ala Ile Leu Cys Trp Gly Glu
    50                  55                  60

Leu Met Thr Leu Ala Thr Trp Val Gly Gly Asn Leu Glu Asp Pro Ala
65                  70                  75                  80

Ser Arg Asp Leu Val Val Ser Tyr Val Asn Thr Asn Met Gly Leu Lys
                85                  90                  95

Phe Arg Gln Leu Leu Trp Phe His Ile Ser Cys Leu Thr Phe Gly Arg
            100                 105                 110

Asp Thr Val Ile Glu Tyr Leu Val Ser Phe Gly Val Trp Ile Arg Thr
        115                 120                 125

Pro Pro Ala Tyr Arg Pro Ser Asn Ala Pro Ile Leu Ser Thr Leu Pro
    130                 135                 140

Glu Thr Cys Val Val Arg Arg Arg Gly Arg Ser Pro Arg Arg Arg Thr
145                 150                 155                 160

Pro Ser Pro Arg Arg Arg Arg Ser Gln Ser Pro Arg Arg Arg Arg Ser
                165                 170                 175

Gln Ser Arg Glu Ser Gln Cys
            180

<210> 30
<211> 183
<212> PRT
<213> Hepatitis B virus

<400> 30

Met Asp Ile Asp Pro Tyr Lys Glu Phe Gly Ala Thr Val Glu Leu Leu
1 5 10 15

Ser Phe Leu Pro Ser Asp Phe Phe Pro Ser Val Arg Asp Leu Leu Asp
20 25 30

Thr Ala Ser Ala Leu Tyr Arg Glu Ala Leu Glu Ser Pro Glu His Cys
35 40 45

Ser Pro His His Thr Ala Leu Arg Gln Ala Ile Leu Cys Trp Gly Glu
50 55 60

Leu Met Thr Leu Ala Thr Trp Val Gly Val Asn Leu Glu Asp Pro Ala
65 70 75 80

Ser Arg Asp Leu Val Val Ser Tyr Val Asn Thr Asn Met Gly Leu Lys
85 90 95

Phe Arg Gln Leu Leu Trp Phe His Ile Ser Cys Leu Thr Phe Gly Arg
100 105 110

Glu Thr Val Ile Glu Tyr Leu Val Ser Phe Gly Val Trp Ile Arg Thr
115 120 125

Pro Pro Ala Tyr Arg Pro Pro Asn Ala Pro Ile Leu Ser Thr Leu Pro
130 135 140

Glu Thr Thr Val Val Arg Arg Arg Gly Arg Ser Pro Arg Arg Arg Thr
145 150 155 160

Pro Ser Pro Arg Arg Arg Arg Ser Gln Ser Pro Arg Arg Arg Arg Ser
165 170 175

Gln Ser Arg Glu Ser Gln Cys
180

<210> 31
<211> 212
<212> PRT
<213> Hepatitis B virus

<400> 31

Met Gln Leu Phe His Leu Cys Leu Ile Ile Ser Cys Ser Cys Pro Thr
1               5                   10                  15

Val Gln Ala Ser Lys Leu Cys Leu Gly Trp Leu Trp Gly Met Asp Ile
            20                  25                  30

Asp Pro Tyr Lys Glu Phe Gly Ala Thr Val Glu Leu Leu Ser Phe Leu
        35                  40                  45

Pro Ser Asp Phe Phe Pro Ser Val Arg Asp Leu Leu Asp Thr Ala Ser
    50                  55                  60

Ala Leu Tyr Arg Glu Ala Leu Glu Ser Pro Glu His Cys Ser Pro His
65                  70                  75                  80

His Thr Ala Leu Arg His Ala Ile Leu Cys Trp Gly Asp Leu Arg Thr
                85                  90                  95

Leu Ala Thr Trp Val Gly Gly Asn Leu Glu Asp Pro Ile Ser Arg Asp
            100                 105                 110

Leu Val Val Ser Tyr Val Asn Thr Asn Met Gly Leu Lys Phe Arg Gln
        115                 120                 125

Leu Leu Trp Phe His Ile Ser Cys Leu Thr Phe Gly Arg Glu Thr Val
    130                 135                 140

Ile Glu Tyr Leu Val Ser Phe Gly Val Trp Ile Arg Thr Pro Pro Ala
145                 150                 155                 160

Tyr Arg Pro Pro Asn Ala Pro Ile Leu Ser Thr Leu Pro Glu Thr Thr
                165                 170                 175

Val Val Arg Arg Arg Gly Arg Ser Pro Arg Arg Arg Thr Pro Ser Pro
            180                 185                 190

Arg Arg Arg Arg Ser Gln Ser Pro Arg Arg Arg Arg Ser Gln Ser Arg
        195                 200                 205

Glu Ser Gln Cys
    210

<210> 32
<211> 212
<212> PRT
<213> Hepatitis B virus

<400> 32

Met Gln Leu Phe His Leu Cys Leu Ile Ile Ser Cys Ser Cys Pro Thr
1               5                   10                  15

Val Gln Ala Ser Lys Leu Cys Leu Gly Trp Leu Trp Asp Met Asp Ile
            20                  25                  30

Asp Pro Tyr Lys Glu Phe Gly Ala Thr Val Glu Leu Leu Ser Phe Leu
        35                  40                  45

Pro Ser Asp Phe Phe Pro Ser Val Arg Asp Leu Leu Asp Thr Ala Ser
    50                  55                  60

Ala Leu Phe Arg Asp Ala Leu Glu Ser Pro Glu His Cys Ser Pro His
65                  70                  75                  80

His Thr Ala Leu Arg Gln Ala Ile Leu Cys Trp Gly Glu Leu Met Thr
                85                  90                  95

Leu Ala Thr Trp Val Gly Ala Asn Leu Glu Asp Pro Ala Ser Arg Asp
            100                 105                 110

Leu Val Val Ser Tyr Val Asn Thr Asn Met Gly Leu Lys Phe Arg Gln
        115                 120                 125

Leu Leu Trp Phe His Ile Ser Cys Leu Thr Phe Gly Arg Glu Thr Val
    130                 135                 140

Ile Glu Tyr Leu Val Ser Phe Gly Val Trp Ile Arg Thr Pro Gln Ala
145                 150                 155                 160

Tyr Arg Pro Pro Asn Ala Pro Ile Leu Ser Thr Leu Pro Glu Thr Cys
                165                 170                 175

Val Val Arg Arg Arg Gly Arg Ser Pro Arg Arg Arg Thr Pro Ser Pro
            180                 185                 190

Arg Arg Arg Arg Ser Gln Ser Pro Arg Arg Arg Arg Ser Gln Ser Arg
        195                 200                 205

Glu Ser Gln Cys
    210

<210> 33
<211> 183
<212> PRT
<213> Artificial sequence

<220>
<223> Description of Artificial sequence: synthetic human Hepatitus B construct

<400> 33

Met Asp Ile Asp Pro Tyr Lys Glu Phe Gly Ala Thr Val Glu Leu Leu
1 5 10 15

Ser Phe Leu Pro Ser Asp Phe Phe Pro Ser Val Arg Asp Leu Leu Asp
20 25 30

Thr Ala Ser Ala Leu Tyr Arg Glu Ala Leu Glu Ser Pro Glu His Cys
35 40 45

Ser Pro His His Thr Ala Leu Arg Gln Ala Ile Leu Cys Trp Gly Glu
50 55 60

Leu Met Thr Leu Ala Thr Trp Val Gly Val Asn Leu Glu Asp Pro Ala
65 70 75 80

Ser Arg Asp Leu Val Val Ser Tyr Val Asn Thr Asn Met Gly Leu Lys
85 90 95

Phe Arg Gln Leu Leu Trp Phe His Ile Ser Cys Leu Thr Phe Gly Arg
100 105 110

Glu Thr Val Leu Glu Tyr Leu Val Ser Phe Gly Val Trp Ile Arg Thr
115 120 125

Pro Pro Ala Tyr Arg Pro Pro Asn Ala Pro Ile Leu Ser Thr Leu Pro
130 135 140

Glu Thr Thr Val Val Arg Arg Arg Gly Arg Ser Pro Arg Arg Arg Thr
145 150 155 160

Pro Ser Pro Arg Arg Arg Arg Ser Gln Ser Pro Arg Arg Arg Arg Ser
165 170 175

Gln Ser Arg Glu Ser Gln Cys
180

<210> 34
<211> 212
<212> PRT
<213> Hepati ti s B virus

<400> 34

Met Gln Leu Phe His Leu Cys Leu Ile Ile Ser Cys Ser Cys Pro Thr
1 5 10 15

Val Gln Ala Ser Lys Leu Cys Leu Gly Trp Leu Trp Gly Met Asp Ile
20 25 30

Asp Pro Tyr Lys Glu Phe Gly Ala Thr Val Glu Leu Leu Ser Phe Leu
35 40 45

Pro Ser Asp Phe Phe Pro Ser Val Arg Asp Leu Leu Asp Thr Ala Ser
50 55 60

Ala Leu Tyr Arg Glu Ala Leu Glu Ser Pro Glu His Cys Ser Pro His
65 70 75 80

His Thr Ala Leu Arg Gln Ala Ile Leu Cys Trp Gly Asp Leu Met Ser
85 90 95

Leu Ala Thr Trp Val Gly Val Asn Leu Glu Asp Pro Ile Ser Arg Asp
100 105 110

Leu Val Val Ser Tyr Val Asn Thr Asn Met Gly Leu Lys Phe Arg Gln
115 120 125

Leu Leu Trp Phe His Ile Ser Cys Leu Thr Phe Gly Arg Glu Thr Val
130 135 140

Ile Glu Tyr Leu Val Ser Phe Gly Val Trp Ile Arg Thr Pro Pro Ala
145 150 155 160

Tyr Arg Pro Pro Asn Ala Pro Ile Leu Ser Thr Leu Pro Glu Thr Thr
165 170 175

Val Val Arg Arg Arg Gly Arg Ser Pro Arg Arg Arg Thr Pro Ser Pro
180 185 190

Arg Arg Arg Arg Ser Gln Ser Pro Arg Arg Arg Arg Ser Gln Ser Arg
195 200 205

Glu Ser Gln Cys
210

<210> 35
<211> 183
<212> PRT
<213> Hepatitis B virus

<400> 35

Met Asp Ile Asp Pro Tyr Lys Glu Phe Gly Ala Thr Val Glu Leu Leu
1               5                   10                  15

Ser Phe Leu Pro Ser Asp Phe Phe Pro Ser Val Arg Asp Leu Leu Asp
            20                  25                  30

Thr Ala Ser Ala Leu Tyr Arg Asp Ala Leu Glu Ser Pro Glu His Cys
        35                  40                  45

Ser Pro His His Thr Ala Leu Arg Gln Ala Ile Leu Cys Trp Gly Glu
    50                  55                  60

Leu Met Thr Leu Ala Thr Trp Val Gly Val Asn Leu Glu Asp Pro Ala
65                  70                  75                  80

Ser Arg Asp Leu Val Val Ser Tyr Val Asn Thr Asn Met Gly Leu Lys

                85                  90                  95

Phe Arg Gln Leu Leu Trp Phe His Ile Ser Cys Leu Thr Phe Gly Arg
            100                 105                 110

Glu Thr Val Ile Glu Tyr Leu Val Ser Phe Gly Val Trp Ile Arg Thr
        115                 120                 125

Pro Pro Ala Tyr Arg Pro Pro Asn Ala Pro Ile Leu Ser Thr Leu Pro
    130                 135                 140

Glu Thr Thr Val Val Arg Arg Arg Gly Arg Ser Pro Arg Arg Arg Thr
145                 150                 155                 160

Pro Ser Pro Arg Arg Arg Arg Ser Gln Ser Pro Arg Arg Arg Arg Ser
                165                 170                 175

Gln Ser Arg Glu Ser Gln Cys
            180

<210> 36
<211> 183
<212> PRT
<213> Hepatitis B virus

<400> 36

Met Asp Ile Asp Pro Tyr Lys Glu Phe Gly Ala Thr Val Glu Leu Leu
1 5 10 15

Ser Phe Leu Pro Ser Asp Phe Phe Pro Ser Val Arg Asp Leu Leu Asp
20 25 30

Thr Ala Ser Ala Leu Tyr Arg Glu Ala Leu Glu Ser Pro Glu His Cys
35 40 45

Ser Pro His His Thr Ala Leu Arg Gln Ala Ile Leu Cys Trp Gly Asp
50 55 60

Leu Met Thr Leu Ala Thr Trp Val Gly Val Asn Leu Glu Asp Pro Ala
65 70 75 80

Ser Arg Asp Leu Val Val Ser Tyr Val Asn Thr Asn Met Gly Leu Lys
85 90 95

Phe Arg Gln Leu Leu Trp Phe His Ile Ser Cys Leu Thr Phe Gly Arg
100 105 110

Glu Thr Val Ile Glu Tyr Leu Val Ser Phe Gly Val Trp Ile Arg Thr
115 120 125

Pro Pro Ala Tyr Arg Pro Pro Asn Ala Pro Ile Leu Ser Thr Leu Pro
130 135 140

Glu Thr Thr Val Val Arg Arg Arg Gly Arg Ser Pro Arg Arg Arg Thr
145 150 155 160

Pro Ser Pro Arg Arg Arg Arg Ser Gln Ser Pro Arg Arg Arg Arg Ser
165 170 175

Gln Ser Arg Glu Ser Gln Cys
180

<210> 37
<211> 183
<212> PRT
<213> Hepatitis B virus

<400> 37

Met Asp Ile Asp Pro Tyr Lys Glu Phe Gly Ala Thr Val Glu Leu Leu
1 5 10 15

Ser Phe Leu Pro Ser Asp Phe Phe Pro Ser Val Arg Asp Leu Leu Asp
20 25 30

Thr Ala Ser Ala Leu Tyr Arg Asp Ala Leu Glu Ser Pro Glu His Cys
35 40 45

Ser Pro His His Thr Ala Leu Arg Gln Ala Ile Leu Cys Trp Gly Glu
50 55 60

Leu Met Thr Leu Ala Thr Trp Val Gly Ala Asn Leu Glu Asp Pro Ala
65 70 75 80

Ser Arg Asp Leu Val Val Ser Tyr Val Asn Thr Asn Met Gly Leu Lys
85 90 95

Phe Arg Gln Leu Leu Trp Phe His Ile Ser Cys Leu Thr Phe Gly Arg
100 105 110

Glu Thr Val Ile Glu Tyr Leu Val Ser Phe Gly Val Trp Ile Arg Thr
115 120 125

Pro Pro Ala Tyr Arg Pro Pro Asn Ala Pro Ile Leu Ser Thr Leu Pro
130 135 140

Glu Thr Thr Val Val Arg Arg Arg Gly Arg Thr Pro Arg Arg Arg Thr
145 150 155 160

Pro Ser Pro Arg Arg Arg Arg Ser Gln Ser Pro Arg Arg Arg Arg Ser
165 170 175

Gln Ser Arg Glu Ser Gln Cys
180

<210> 38
<211> 212
<212> PRT
<213> Hepatitis B virus

<400> 38

```
Met Gln Leu Phe His Leu Cys Leu Ile Ile Ser Cys Ser Cys Pro Thr
1               5                   10                  15

Val Gln Ala Ser Lys Leu Cys Leu Gly Trp Leu Trp Gly Met Asp Ile
            20                  25                  30

Asp Pro Tyr Lys Glu Phe Gly Ala Thr Val Glu Leu Leu Ser Phe Leu
        35                  40                  45

Pro Ser Asp Phe Phe Pro Ser Val Arg Asp Leu Leu Asp Thr Ala Ser
    50                  55                  60

Ala Leu Tyr Arg Asp Ala Leu Glu Ser Pro Glu His Cys Ser Pro His
65                  70                  75                  80

His Thr Ala Leu Arg Gln Ala Ile Leu Cys Trp Gly Glu Leu Met Thr
                85                  90                  95

Leu Ala Thr Trp Val Gly Val Asn Leu Glu Asp Pro Ala Ser Arg Asp
            100                 105                 110

Leu Val Val Ser Tyr Val Asn Thr Asn Met Gly Leu Lys Phe Arg Gln
        115                 120                 125

Leu Leu Trp Phe His Ile Ser Cys Leu Thr Phe Gly Arg Glu Thr Val
    130                 135                 140

Ile Glu Tyr Leu Val Ser Phe Gly Val Trp Ile Arg Thr Pro Pro Ala
145                 150                 155                 160

Tyr Arg Pro Pro Asn Ala Pro Ile Leu Ser Thr Leu Pro Glu Thr Thr
                165                 170                 175

Val Val Arg Arg Arg Gly Arg Ser Pro Arg Arg Arg Thr Pro Ser Pro
            180                 185                 190

Arg Arg Arg Arg Ser Gln Ser Pro Arg Arg Arg Arg Ser Gln Ser Arg
        195                 200                 205

Glu Ser Gln Cys
    210
```

<210> 39
<211> 212
<212> PRT
<213> Hepatitis B virus

<400> 39

```
Met Gln Leu Phe His Leu Cys Leu Ile Ile Ser Cys Ser Cys Pro Thr
1               5                   10                  15

Val Gln Ala Ser Lys Leu Cys Leu Gly Trp Leu Trp Gly Met Asp Ile
            20                  25                  30

Asp Pro Tyr Lys Glu Phe Gly Ala Thr Val Glu Leu Leu Ser Phe Leu
        35                  40                  45

Pro Ser Asp Phe Phe Pro Ser Val Arg Asp Leu Leu Asp Thr Ala Ser
    50                  55                  60

Ala Leu Tyr Arg Glu Ala Leu Glu Ser Pro Glu His Cys Ser Pro His
65                  70                  75                  80

His Thr Ala Leu Arg Gln Ala Ile Leu Cys Trp Gly Asp Leu Met Thr
                85                  90                  95

Leu Ala Thr Trp Val Gly Val Asn Leu Glu Asp Pro Ala Ser Arg Asp
            100                 105                 110

Leu Val Val Ser Tyr Val Asn Thr Asn Met Gly Leu Lys Phe Arg Gln
        115                 120                 125

Leu Leu Trp Phe His Ile Ser Cys Leu Thr Phe Gly Arg Glu Thr Val
    130                 135                 140

Ile Glu Tyr Leu Val Ser Phe Gly Val Trp Ile Arg Thr Pro Pro Ala
145                 150                 155                 160

Tyr Arg Pro Pro Asn Ala Pro Ile Leu Ser Thr Leu Pro Glu Thr Thr
                165                 170                 175

Val Val Arg Arg Arg Gly Arg Ser Pro Arg Arg Arg Thr Pro Ser Pro
            180                 185                 190

Arg Arg Arg Arg Ser Gln Ser Pro Arg Arg Arg Arg Ser Gln Ser Arg
        195                 200                 205

Glu Ser Gln Cys
    210
```

<210> 40
<211> 212
<212> PRT
<213> Hepatitis B virus

<400> 40

Met Gln Leu Phe His Leu Cys Leu Ile Ile Ser Cys Thr Cys Pro Thr
1 5 10 15

Val Gln Ala Ser Lys Leu Cys Leu Gly Trp Leu Trp Gly Met Asp Ile
20 25 30

Asp Pro Tyr Lys Glu Phe Gly Ala Thr Val Glu Leu Leu Ser Phe Leu
35 40 45

Pro Ser Asp Phe Phe Pro Ser Val Arg Asp Leu Leu Asp Thr Ala Ser
50 55 60

Ala Leu Tyr Arg Glu Ala Leu Glu Ser Pro Glu His Cys Ser Pro His
65 70 75 80

His Thr Ala Leu Arg Gln Ala Ile Leu Cys Trp Gly Glu Leu Met Thr
85 90 95

Leu Ala Thr Trp Val Gly Val Asn Leu Glu Asp Pro Ala Ser Arg Asp
100 105 110

Leu Val Val Ser Tyr Val Asn Thr Asn Met Gly Leu Lys Phe Arg Gln
115 120 125

Leu Leu Trp Phe His Ile Ser Cys Leu Thr Phe Gly Arg Glu Thr Val
130 135 140

Ile Glu Tyr Leu Val Ala Phe Gly Val Trp Ile Arg Thr Pro Pro Ala
145 150 155 160

Tyr Arg Pro Pro Asn Ala Pro Ile Leu Ser Thr Leu Pro Glu Thr Thr
165 170 175

Val Val Arg Arg Arg Gly Arg Ser Pro Arg Arg Arg Thr Pro Ser Pro
180 185 190

Arg Arg Arg Arg Ser Gln Ser Pro Arg Arg Arg Arg Ser Gln Ser Arg
195 200 205

Glu Ser Gln Cys
210

<210> 41
<211> 212
<212> PRAT
<213> Hepatitis B virus

<400> 41

Met Gln Leu Phe His Leu Cys Leu Ile Ile Ser Cys Ser Cys Pro Thr
1               5                   10                  15

Val Gln Ala Ser Lys Leu Cys Leu Gly Trp Leu Trp Gly Met Asp Ile
            20                  25                  30

Asp Pro Tyr Lys Glu Phe Gly Ala Thr Val Glu Leu Leu Ser Phe Leu
        35                  40                  45

Pro Ser Asp Phe Phe Pro Ser Val Arg Asp Leu Leu Asp Thr Ala Ser
    50                  55                  60

Ala Leu Tyr Arg Glu Ala Phe Glu Cys Ser Glu His Cys Ser Pro His
65                  70                  75                  80

His Thr Ala Leu Arg Gln Ala Ile Leu Cys Trp Gly Glu Leu Met Thr
                85                  90                  95

Leu Ala Thr Trp Val Gly Gly Asn Leu Glu Asp Pro Ile Ser Arg Asp
            100                 105                 110

Leu Val Val Ser Tyr Val Asn Thr Asn Met Gly Leu Lys Phe Arg Gln
        115                 120                 125

Leu Leu Trp Phe His Ile Ser Cys Leu Thr Phe Gly Arg Glu Thr Val
    130                 135                 140

Ile Glu Tyr Leu Val Ser Phe Gly Val Trp Ile Arg Thr Pro Pro Ala
145                 150                 155                 160

Tyr Arg Pro Pro Asn Ala Pro Ile Leu Ser Thr Leu Pro Glu Thr Thr
                165                 170                 175

Val Val Arg Arg Arg Gly Arg Ser Pro Arg Arg Arg Thr Pro Ser Pro
            180                 185                 190

Arg Arg Arg Arg Ser Gln Ser Pro Arg Arg Arg Arg Ser Gln Ser Arg
        195                 200                 205

Glu Ser Gln Cys
    210

<210> 42
<211> 212
<212> PRT
<213> Hepatitis B virus

<220>
<221> MIST_FEATURE
<222> (28)..(28)
<223> May be any amino acid

<400> 42

Met Gln Leu Phe His Leu Cys Leu Ile Ile Ser Cys Ser Cys Pro Thr
1               5                   10                  15

Val Gln Ala Ser Lys Leu Cys Leu Gly Trp Leu Xaa Asp Met Asp Ile
            20                  25                  30

Asp Pro Tyr Lys Glu Phe Gly Ala Thr Val Glu Leu Leu Ser Phe Leu
        35                  40                  45

Pro Ser Asp Phe Phe Pro Ser Val Arg Asp Leu Leu Asp Thr Ala Ser
    50                  55                  60

Ala Leu Tyr Arg Glu Ala Leu Glu Ser Pro Glu His Cys Ser Pro His
65                  70                  75                  80

His Thr Ala Leu Arg Gln Ala Ile Leu Cys Trp Gly Asp Leu Ile Thr
                85                  90                  95

Leu Ser Thr Trp Val Gly Gly Asn Leu Glu Asp Pro Thr Ser Arg Asp
            100                 105                 110

Leu Val Val Ser Tyr Val Asn Thr Asn Met Gly Leu Lys Phe Arg Gln
        115                 120                 125

Leu Leu Trp Phe His Ile Ser Cys Leu Thr Phe Gly Arg Glu Thr Val
    130                 135                 140

Ile Glu Tyr Leu Val Ser Phe Gly Val Trp Ile Arg Thr Pro Pro Ala
145                 150                 155                 160

Tyr Arg Pro Pro Asn Ala Pro Ile Leu Ser Thr Leu Pro Glu Thr Thr
                165                 170                 175

Val Val Arg Arg Arg Gly Arg Ser Pro Arg Arg Arg Thr Pro Ser Pro
            180                 185                 190

Arg Arg Arg Arg Ser Gln Ser Pro Arg Arg Arg Arg Thr Gln Ser Arg
        195                 200                 205

Glu Ser Gln Cys
    210

<210> 43
<211> 212
<212> PRT
<213> Hepatitis B virus

<400> 43

```
Met Gln Leu Phe His Leu Cys Leu Ile Ile Ser Cys Ser Cys Pro Thr
1               5                   10                  15

Val Gln Ala Ser Lys Leu Cys Leu Gly Trp Leu Trp Gly Met Asp Ile
            20                  25                  30

Asp Pro Tyr Lys Glu Phe Gly Ala Thr Val Glu Leu Leu Ser Phe Leu
        35                  40                  45

Pro Ser Asp Phe Phe Pro Ser Val Arg Asp Leu Leu Asp Asn Ala Ser
    50                  55                  60

Ala Leu Tyr Arg Glu Ala Leu Glu Ser Pro Glu His Cys Ser Pro His
65                  70                  75                  80

His Thr Ala Leu Arg Gln Ala Ile Leu Cys Trp Gly Glu Leu Met Thr
                85                  90                  95

Leu Ala Thr Trp Val Gly Val Asn Leu Glu Asp Pro Ala Ser Arg Asp
            100                 105                 110

Leu Val Val Ser Tyr Val Asn Thr Asn Met Gly Leu Lys Phe Arg Gln
        115                 120                 125

Leu Leu Trp Phe His Ile Ser Cys Leu Thr Phe Gly Arg Glu Thr Val
    130                 135                 140

Ile Glu Tyr Leu Val Ser Phe Gly Val Trp Ile Arg Thr Pro Pro Ala
145                 150                 155                 160

Tyr Arg Pro Pro Asn Ala Pro Ile Leu Ser Thr Leu Pro Glu Thr Thr
                165                 170                 175

Val Val Arg Arg Arg Gly Arg Ser Pro Arg Arg Arg Thr Pro Ser Pro
            180                 185                 190

Arg Arg Arg Arg Ser Gln Ser Pro Arg Arg Arg Arg Ser Gln Ser Arg
        195                 200                 205

Glu Ser Gln Cys
    210
```

<210> 44
<211> 212
<212> PRT
<213> Hepatitis B virus

<400> 44

Met Gln Leu Phe His Leu Cys Leu Ile Ile Ser Cys Ser Cys Pro Thr
1               5                   10                  15

Val Gln Ala Ser Lys Leu Cys Leu Gly Trp Leu Trp Gly Met Asp Ile
            20                  25                  30

Asp Pro Tyr Lys Glu Phe Gly Ala Thr Val Glu Leu Leu Ser Phe Leu
        35                  40                  45

Pro Ser Asp Phe Phe Pro Ser Val Arg Asp Leu Leu Asp Thr Ala Ser
    50                  55                  60

Ala Leu Tyr Arg Glu Ala Leu Glu Ser Pro Glu His Cys Ser Pro His
65                  70                  75                  80

His Thr Ala Leu Arg Gln Ala Ile Leu Cys Trp Gly Glu Leu Met Thr
                85                  90                  95

Leu Ala Thr Trp Val Gly Val Asn Leu Glu Asp Pro Ala Ser Arg Asp
            100                 105                 110

Leu Val Val Ser Tyr Val Asn Thr Asn Met Gly Leu Lys Phe Arg Gln
        115                 120                 125

Leu Leu Trp Phe His Ile Cys Cys Leu Thr Phe Gly Arg Glu Thr Val
    130                 135                 140

Ile Glu Tyr Leu Val Ser Phe Gly Val Trp Ile Arg Thr Pro Pro Ala
145                 150                 155                 160

Tyr Arg Pro Pro Asn Ala Pro Ile Leu Ser Thr Leu Pro Glu Thr Thr
                165                 170                 175

Val Val Arg Arg Arg Gly Arg Ser Pro Arg Arg Arg Thr Pro Ser Pro
            180                 185                 190

Arg Arg Arg Arg Ser Gln Ser Pro Arg Arg Arg Arg Ser Gln Ser Arg
        195                 200                 205

Glu Ser Gln Cys
    210

<210> 45
<211> 212
<212> PRT
<213> Hepatitis B virus

<400> 45

Met Gln Leu Phe His Leu Cys Leu Ile Ile Ser Cys Ser Cys Pro Thr
1 5 10 15

Val Gln Ala Ser Lys Leu Cys Leu Gly Trp Leu Trp Gly Met Asp Ile
20 25 30

Asp Pro Tyr Lys Glu Phe Gly Ala Thr Val Glu Leu Leu Ser Phe Leu
35 40 45

Pro Ser Asp Phe Phe Pro Ser Val Arg Asp Leu Leu Asp Thr Ala Ser
50 55 60

Ala Leu Tyr Arg Glu Ala Leu Glu Ser Pro Glu His Cys Ser Pro His
65 70 75 80

His Thr Ala Leu Arg Gln Ala Ile Leu Cys Trp Gly Glu Leu Met Thr
85 90 95

Leu Ala Thr Trp Val Gly Val Asn Leu Glu Asp Pro Ala Ser Arg Asp
100 105 110

Leu Val Val Ser Tyr Val Asn Thr Asn Met Gly Leu Lys Phe Arg Gln
115 120 125

Leu Leu Trp Phe His Ile Ser Cys Leu Thr Phe Gly Arg Glu Thr Val
130 135 140

Ile Glu Tyr Leu Val Ser Phe Gly Val Trp Ile Arg Thr Pro Pro Ala
145 150 155 160

Tyr Arg Pro Pro Asn Ala Pro Ile Leu Ser Thr Leu Pro Glu Thr Thr
165 170 175

Val Val Arg Arg Arg Gly Arg Ser Pro Arg Arg Arg Thr Pro Ser Pro
180 185 190

Arg Arg Arg Arg Ser Gln Ser Pro Arg Arg Arg Arg Ser Gln Ser Arg
195 200 205

Glu Pro Gln Cys
210

<210> 46
<211> 212
<212> PRT
<213> Hepatitis B virus

<400> 46

Met Gln Leu Phe His Leu Cys Leu Ile Ile Ser Cys Ser Cys Pro Thr
1               5                   10                  15

Val Gln Ala Ser Lys Leu Cys Leu Gly Trp Leu Trp Gly Met Asp Ile
            20                  25                  30

Asp Pro Tyr Lys Glu Phe Gly Ala Thr Val Glu Leu Leu Ser Phe Leu
        35                  40                  45

Pro Ser Asp Phe Phe Pro Ser Val Arg Asp Leu Leu Ser Thr Ala Ser
    50                  55                  60

Ala Leu Tyr Arg Glu Ala Leu Glu Ser Pro Glu His Cys Ser Pro His
65                  70                  75                  80

His Thr Ala Leu Arg Gln Ala Ile Leu Cys Trp Gly Glu Leu Met Thr
                85                  90                  95

Leu Ala Thr Trp Val Gly Val Asn Leu Glu Asp Pro Ala Ser Arg Asp
            100                 105                 110

Leu Val Val Ser Tyr Val Asn Thr Asn Met Gly Leu Lys Phe Arg Gln
        115                 120                 125

Leu Leu Trp Phe His Ile Ser Cys Leu Thr Phe Gly Arg Glu Thr Val
    130                 135                 140

Ile Glu Tyr Leu Val Ser Phe Gly Val Trp Ile Arg Thr Pro Pro Ala
145                 150                 155                 160

Tyr Arg Pro Pro Asn Ala Pro Ile Leu Ser Thr Leu Pro Glu Thr Thr
                165                 170                 175

Val Val Arg Arg Arg Gly Arg Ser Pro Arg Arg Arg Thr Pro Ser Pro
            180                 185                 190

Arg Arg Arg Arg Ser Gln Ser Pro Arg Arg Arg Arg Ser Gln Ser Arg
        195                 200                 205

Glu Ser Gln Cys
    210

<210> 47
<211> 212
<212> PRT
<213> Hepatitis B virus

<400> 47

Met Gln Leu Phe His Leu Cys Leu Ile Ile Ser Cys Ser Cys Pro Thr
1 5 10 15

Val Gln Ala Ser Lys Leu Cys Leu Gly Trp Leu Trp Gly Met Asp Ile
20 25 30

Asp Pro Tyr Lys Glu Phe Gly Ala Thr Val Glu Leu Leu Ser Phe Leu
35 40 45

Pro Ser Asp Phe Phe Pro Ser Val Arg Asp Leu Leu Asp Thr Ala Ser
50 55 60

Ala Leu Tyr Arg Glu Ala Leu Glu Ser Pro Glu His Cys Ser Pro His
65 70 75 80

His Thr Ala Leu Arg Gln Ala Ile Leu Cys Trp Gly Glu Leu Met Thr
85 90 95

Leu Ala Thr Trp Val Gly Val Asn Leu Glu Asp Pro Ala Ser Arg Asp
100 105 110

Leu Val Val Ser Tyr Val Asn Thr Asn Met Gly Leu Lys Phe Arg Gln
115 120 125

Leu Leu Trp Phe His Ile Ser Cys Leu Thr Phe Gly Arg Glu Thr Val
130 135 140

Ile Glu Tyr Leu Val Ser Phe Gly Val Trp Ile Arg Thr Pro Pro Ala
145 150 155 160

Tyr Arg Pro Pro Asn Ala Pro Ile Leu Leu Thr Leu Pro Glu Thr Thr
165 170 175

Val Val Arg Arg Arg Gly Arg Ser Pro Arg Arg Arg Thr Pro Ser Pro
180 185 190

Arg Arg Arg Arg Ser Gln Ser Pro Arg Arg Arg Arg Ser Gln Ser Arg
195 200 205

Glu Ser Gln Cys
210

<210> 48
<211> 212
<212> PRT
<213> Hepatitis B virus

<400> 48

Met Gln Leu Phe His Leu Cys Leu Ile Ile Ser Cys Ser Cys Pro Thr
1 5 10 15

Val Gln Ala Ser Lys Leu Cys Leu Gly Trp Leu Trp Gly Met Asp Ile
20 25 30

Asp Pro Tyr Lys Glu Phe Gly Ala Thr Val Glu Leu Leu Ser Phe Leu
35 40 45

Pro Ser Asp Phe Phe Pro Ser Val Arg Asp Leu Leu Asp Thr Ala Ser
50 55 60

Ala Leu Tyr Arg Glu Ala Leu Glu Ser Pro Glu His Cys Ser Pro His
65 70 75 80

His Thr Ala Leu Arg Gln Ala Ile Leu Cys Trp Gly Asp Leu Met Thr
85 90 95

Leu Ala Thr Trp Val Gly Val Asn Leu Glu Asp Pro Ala Ser Arg Asp
100 105 110

Leu Val Val Ser Tyr Val Asn Thr Asn Met Gly Leu Lys Phe Lys Gln
115 120 125

Leu Leu Trp Phe His Ile Ser Cys Leu Thr Phe Gly Arg Glu Thr Val
130 135 140

Ile Glu Tyr Leu Val Ser Phe Gly Val Trp Ile Arg Thr Pro Pro Ala
145 150 155 160

Tyr Arg Pro Pro Asn Ala Pro Ile Leu Ser Thr Leu Pro Glu Thr Thr
165 170 175

Val Val Arg Arg Arg Gly Arg Ser Pro Arg Arg Arg Thr Pro Ser Pro
180 185 190

Arg Arg Arg Arg Ser Gln Ser Pro Arg Arg Arg Arg Ser Gln Ser Arg
195 200 205

Glu Ser Gln Cys
210

<210> 49
<211> 212
<212> PRT
<213> Hepatitis B virus

<400> 49

```
Met Gln Leu Phe His Leu Cys Leu Ile Ile Ser Cys Ser Cys Pro Thr
1               5                   10                  15

Val Gln Ala Ser Lys Leu Cys Leu Gly Trp Leu Trp Gly Met Asp Ile
            20                  25                  30

Asp Pro Tyr Lys Glu Phe Gly Ala Thr Val Glu Leu Leu Ser Phe Leu
        35                  40                  45

Pro Ser Asp Phe Phe Pro Ser Val Arg Asp Leu Leu Asp Thr Ala Ala
    50                  55                  60

Ala Leu Tyr Arg Asp Ala Leu Glu Ser Pro Glu His Cys Ser Pro His
65                  70                  75                  80

His Thr Ala Leu Arg Gln Ala Ile Leu Cys Trp Gly Glu Leu Met Thr
                85                  90                  95

Leu Ala Thr Trp Val Gly Thr Asn Leu Glu Asp Pro Ala Ser Arg Asp
            100                 105                 110

Leu Val Val Ser Tyr Val Asn Thr Asn Met Gly Leu Lys Phe Arg Gln
        115                 120                 125

Leu Leu Trp Phe His Ile Ser Cys Leu Thr Phe Gly Arg Glu Thr Val
    130                 135                 140

Leu Glu Tyr Leu Val Ser Phe Gly Val Trp Ile Arg Thr Pro Pro Ala
145                 150                 155                 160

Tyr Arg Pro Pro Asn Ala Pro Ile Leu Ser Thr Leu Pro Glu Thr Thr
                165                 170                 175

Val Val Arg Arg Arg Gly Arg Ser Pro Arg Arg Arg Thr Pro Ser Pro
            180                 185                 190

Arg Arg Arg Arg Ser Gln Ser Pro Arg Arg Arg Arg Ser Gln Ser Arg
        195                 200                 205

Glu Ser Gln Cys
    210
```

<210> 50
<211> 183
<212> PRT
<213> Hepatitis B virus

<400> 50

Met Asp Ile Asp Pro Tyr Lys Glu Phe Gly Ala Ser Met Glu Leu Leu
1 5 10 15

Ser Phe Leu Pro Ser Asp Phe Tyr Pro Ser Val Arg Asp Leu Leu Asp
20 25 30

Thr Ala Ser Ala Leu Tyr Arg Glu Ala Leu Glu Ser Pro Glu His Cys
35 40 45

Thr Pro His His Thr Ala Leu Arg Gln Ala Ile Leu Cys Trp Gly Glu
50 55 60

Leu Met Thr Leu Ala Thr Trp Val Gly Gly Asn Leu Gln Asp Pro Thr
65 70 75 80

Ser Arg Asp Leu Val Val Ser Tyr Val Asn Thr Asn Met Gly Leu Lys
85 90 95

Phe Arg Gln Leu Leu Trp Phe His Val Ser Cys Leu Thr Phe Gly Arg
100 105 110

Glu Thr Val Val Glu Tyr Leu Val Ser Phe Gly Val Trp Ile Arg Thr
115 120 125

Pro Gln Ala Tyr Arg Pro Pro Asn Ala Pro Ile Leu Ser Thr Leu Pro
130 135 140

Glu Thr Cys Val Val Arg Arg Arg Gly Arg Ser Pro Arg Arg Arg Thr
145 150 155 160

Pro Ser Pro Arg Arg Arg Arg Ser Gln Ser Pro Arg Arg Arg Arg Ser
165 170 175

Gln Ser Arg Glu Ser Gln Cys
180

<210> 51
<211> 183
<212> PRT
<213> Hepatitis B virus

<400> 51

Met Asp Ile Asp Pro Tyr Lys Glu Phe Gly Ala Thr Val Glu Leu Leu
1 5 10 15

Ser Phe Leu Pro Ser Asp Phe Phe Pro Ser Val Arg Asp Leu Leu Asp

20 25 30

Thr Ala Ser Ala Leu Tyr Arg Glu Ala Leu Glu Ser Pro Glu His Cys
35 40 45

Ser Pro His His Thr Ala Leu Arg His Val Phe Leu Cys Trp Gly Asp
50 55 60

Leu Met Thr Leu Ala Thr Trp Val Gly Gly Asn Leu Glu Asp Pro Thr
65 70 75 80

Ser Arg Asp Leu Val Val Ser Tyr Val Asn Thr Asn Met Gly Leu Lys
85 90 95

Phe Arg Gln Leu Leu Trp Phe His Ile Ser Cys Leu Thr Phe Gly Arg
100 105 110

Glu Thr Val Ile Glu Tyr Leu Val Ser Phe Gly Val Trp Ile Arg Thr
115 120 125

Pro Pro Ala Tyr Arg Pro Pro Asn Ala Pro Ile Leu Ser Thr Leu Pro
130 135 140

Glu Thr Thr Val Val Arg Arg Arg Gly Arg Ser Pro Arg Arg Arg Thr
145 150 155 160

Pro Ser Pro Arg Arg Arg Arg Ser Gln Ser Pro Arg Arg Arg Arg Ser
165 170 175

Gln Ser Arg Glu Ser Gln Cys
180

<210> 52
<211> 212
<212> PRT
<213> Hepatitis B virus

<400> 52

Met Gln Leu Phe His Leu Cys Leu Ile Ile Ser Cys Ser Cys Pro Thr
1               5                   10                  15

Val Gln Ala Ser Lys Leu Cys Leu Gly Trp Leu Trp Gly Met Asp Ile
            20                  25                  30

Asp Pro Tyr Lys Glu Phe Gly Ala Thr Val Glu Leu Leu Ser Phe Leu
        35                  40                  45

Pro Ser Asp Phe Phe Pro Ser Val Arg Asp Leu Leu Asp Thr Ala Ser
    50                  55                  60

Ala Leu Tyr Arg Glu Ala Leu Glu Ser Pro Glu His Cys Ser Pro His
65                  70                  75                  80

His Thr Ala Leu Arg Gln Ala Ile Leu Cys Trp Gly Asp Leu Thr Thr
                85                  90                  95

Leu Ala Thr Trp Val Gly Val Asn Leu Glu Asp Pro Ala Ser Arg Asp
            100                 105                 110

Leu Val Val Ser Tyr Val Asn Thr Asn Met Gly Leu Lys Phe Arg Gln
        115                 120                 125

Leu Leu Trp Phe His Ile Ser Cys Leu Thr Phe Gly Arg Glu Thr Val
    130                 135                 140

Ile Glu Tyr Leu Val Ser Phe Gly Val Trp Ile Arg Thr Pro Pro Ala
145                 150                 155                 160

Tyr Arg Pro Pro Asn Ala Pro Ile Leu Ser Thr Leu Pro Glu Thr Thr
                165                 170                 175

Val Val Arg Arg Arg Gly Arg Ser Pro Arg Arg Arg Thr Pro Ser Pro
            180                 185                 190

Arg Arg Arg Arg Ser Gln Ser Pro Arg Arg Arg Arg Ser Gln Ser Arg
        195                 200                 205

Glu Ser Gln Cys
    210

<210> 53
<211> 212
<212> PRT
<213> Hepatitis B virus

<400> 53

Met Gln Leu Phe His Leu Cys Leu Ile Ile Ser Cys Ser Cys Pro Thr
1 5 10 15

Val Gln Ala Ser Lys Leu Cys Leu Gly Trp Leu Trp Gly Met Asp Ile
20 25 30

Asp Pro Tyr Lys Glu Phe Gly Ala Thr Val Glu Leu Leu Ser Phe Leu
35 40 45

Pro Ser Asp Phe Phe Pro Ser Val Arg Asp Leu Leu Asp Thr Ala Ser
50 55 60

Ala Leu Tyr Arg Asp Ala Leu Glu Ser Pro Glu His Cys Ser Pro His
65 70 75 80

His Thr Ala Leu Arg Gln Ala Ile Leu Cys Trp Gly Glu Leu Met Thr
85 90 95

Leu Ala Thr Trp Val Gly Val Asn Leu Glu Asp Pro Ala Ser Arg Asp

100 105 110

Leu Val Val Ser Tyr Val Asn Thr Asn Met Gly Leu Lys Phe Arg Gln
115 120 125

Leu Leu Trp Phe His Ile Ser Cys Leu Ile Phe Gly Arg Glu Thr Val
130 135 140

Ile Glu Tyr Leu Val Ser Phe Gly Val Trp Ile Arg Thr Pro Pro Ala
145 150 155 160

Tyr Arg Pro Pro Asn Ala Pro Ile Leu Ser Thr Leu Pro Glu Thr Thr
165 170 175

Val Val Arg Arg Arg Gly Arg Ser Pro Arg Arg Arg Thr Pro Ser Pro
180 185 190

Arg Arg Arg Arg Ser Gln Ser Pro Arg Arg Arg Arg Ser Gln Ser Arg
195 200 205

Glu Ser Gln Cys
210

<210> 54
<211> 183
<212> PRT
<213> Hepatitis B virus

<400> 54

Met Asp Ile Asp Pro Tyr Lys Glu Phe Gly Ala Thr Val Glu Leu Leu
1 5 10 15

Ser Phe Leu Pro Ser Asp Phe Phe Pro Ser Val Arg Asp Leu Leu Asp
20 25 30

Thr Ala Ser Ala Leu Tyr Arg Glu Ala Leu Glu Ser Pro Glu His Cys
35 40 45

Ser Pro His His Thr Ala Leu Arg Gln Ala Ile Leu Cys Trp Gly Asp
50 55 60

Leu Met Thr Leu Ala Thr Trp Val Gly Val Asn Leu Glu Asp Pro Val
65 70 75 80

Ser Arg Asp Leu Val Val Ser Tyr Val Asn Thr Asn Val Gly Leu Lys
85 90 95

Phe Arg Gln Leu Leu Trp Phe His Ile Ser Cys Leu Thr Phe Gly Arg
100 105 110

Glu Thr Val Ile Glu Tyr Leu Val Ser Phe Gly Val Trp Ile Arg Thr
115 120 125

Pro Pro Ala Tyr Arg Pro Pro Asn Ala Pro Ile Leu Ser Thr Leu Pro
130 135 140

Glu Thr Thr Val Val Arg Arg Arg Gly Arg Ser Pro Arg Arg Arg Thr
145 150 155 160

Pro Ser Pro Ala Arg Arg Arg Ser Gln Ser Pro Arg Arg Arg Arg Ser
165 170 175

Gln Ser Arg Glu Ser Gln Cys
180

<210> 55
<211> 212
<212> PRT
<213> Hepatitis B virus

<400> 55

Met Gln Leu Phe His Leu Cys Leu Ile Ile Ser Cys Ser Cys Pro Thr
1               5                   10                  15

Val Gln Ala Ser Lys Leu Cys Leu Gly Trp Leu Trp Gly Met Asp Ile
            20                  25                  30

Asp Pro Tyr Lys Glu Phe Gly Ala Thr Val Glu Leu Leu Ser Phe Leu
        35                  40                  45

Pro Ser Asp Phe Phe Pro Ser Val Arg Asp Leu Leu Asp Thr Ala Ser
    50                  55                  60

Ala Leu Tyr Arg Glu Ala Leu Glu Ser Pro Glu His Cys Ser Pro His
65                  70                  75                  80

His Thr Ala Leu Arg Gln Ala Ile Leu Cys Trp Gly Asp Leu Met Asn
                85                  90                  95

Leu Ala Thr Trp Val Gly Gly Asn Leu Glu Asp Pro Val Ser Arg Asp
            100                 105                 110

Leu Val Val Gly Tyr Val Asn Thr Thr Val Gly Leu Lys Phe Arg Gln
        115                 120                 125

Leu Leu Trp Phe His Ile Ser Cys Leu Thr Phe Gly Arg Glu Thr Val
    130                 135                 140

Ile Glu Tyr Leu Val Ser Phe Gly Val Trp Ile Arg Thr Pro Pro Ala
145                 150                 155                 160

Tyr Arg Pro Pro Asn Ala Pro Ile Leu Ser Thr Leu Pro Glu Thr Thr
                165                 170                 175

Val Val Arg Arg Arg Gly Arg Ser Pro Arg Arg Arg Thr Pro Ser Pro

            180                 185                 190

Arg Arg Arg Arg Ser Gln Ser Pro Arg Arg Arg Arg Ser Gln Ser Arg
        195                 200                 205

Glu Ser Gln Cys
    210

<210> 56
<211> 183
<212> PRT
<213> Hepatitis B virus

<400> 56

```
Met Asp Ile Asp Pro Tyr Lys Glu Phe Gly Ala Thr Val Glu Leu Leu
1               5                   10                  15

Ser Phe Leu Pro Ser Asp Phe Phe Pro Ser Val Arg Asp Leu Leu Asp
            20                  25                  30

Thr Ala Ser Ala Leu Tyr Arg Asp Ala Leu Glu Ser Pro Glu His Cys
        35                  40                  45

Ser Pro His His Thr Ala Leu Arg Gln Ala Ile Leu Cys Trp Gly Asp
    50                  55                  60

Leu Met Thr Leu Ala Thr Trp Val Gly Val Asn Leu Glu Asp Pro Ala
65                  70                  75                  80

Ser Arg Asp Leu Val Val Ser Tyr Val Asn Thr Asn Met Gly Leu Lys
                85                  90                  95

Phe Arg Gln Leu Leu Trp Phe His Ile Ser Cys Leu Thr Phe Gly Arg
            100                 105                 110

Glu Thr Val Ile Glu Tyr Leu Val Ser Phe Gly Val Trp Ile Arg Thr
        115                 120                 125

Pro Pro Ala Tyr Arg Pro Pro Asn Ala Pro Ile Leu Ser Thr Leu Pro
    130                 135                 140

Glu Thr Thr Val Val Arg Arg Arg Gly Arg Thr Pro Arg Arg Arg Thr
145                 150                 155                 160

Pro Ser Pro Arg Arg Arg Arg Ser Gln Ser Pro Arg Arg Arg Arg Ser
                165                 170                 175

Gln Ser Arg Glu Ser Gln Cys
            180
```

<210> 57
<211> 212
<212> PRT
<213> Hepatitis B virus

<400> 57

Met Gln Leu Phe His Leu Cys Leu Ile Ile Ser Cys Ser Cys Pro Thr
1 5 10 15

Val Gln Ala Ser Lys Leu Cys Leu Gly Trp Leu Trp Gly Met Asp Ile
20 25 30

Asp Pro Tyr Lys Glu Phe Gly Ala Thr Val Glu Leu Leu Ser Phe Leu
35 40 45

Pro Ser Asp Phe Phe Pro Ser Val Arg Ala Leu Leu Asp Thr Ala Ser
50 55 60

Ala Leu Tyr Arg Glu Ala Leu Glu Ser Pro Glu His Cys Ser Pro His
65 70 75 80

His Thr Ala Leu Arg Gln Ala Ile Leu Cys Trp Gly Glu Leu Met Thr
85 90 95

Leu Ala Thr Trp Val Gly Val Asn Leu Glu Asp Pro Ala Ser Arg Asp
100 105 110

Leu Val Val Ser Tyr Val Asn Thr Asn Met Gly Leu Lys Phe Arg Gln
115 120 125

Ile Leu Trp Phe His Ile Ser Cys Leu Thr Phe Gly Arg Glu Thr Val
130 135 140

Ile Glu Tyr Leu Val Ser Phe Gly Val Trp Ile Arg Thr Pro Pro Ala
145 150 155 160

Tyr Arg Pro Pro Asn Ala Pro Ile Leu Ser Thr Leu Pro Glu Thr Thr
165 170 175

Val Val Arg Arg Arg Gly Arg Ser Pro Arg Arg Arg Thr Pro Ser Pro
180 185 190

Arg Arg Arg Arg Ser Gln Ser Pro Arg Arg Arg Arg Ser Gln Ser Arg
195 200 205

Glu Ser Gln Cys
210

<210> 58
<211> 212
<212> PRT
<213> Hepatitis B virus

<400> 58

Met Gln Leu Phe His Leu Cys Leu Ile Ile Ser Cys Ser Cys Pro Thr
1 5 10 15

Val Gln Ala Ser Lys Leu Cys Leu Gly Trp Leu Trp Gly Met Asp Ile
20 25 30

Asp Pro Tyr Lys Glu Phe Gly Ala Thr Val Glu Leu Leu Ser Phe Leu
35 40 45

Pro Ser Asp Phe Phe Pro Ser Val Arg Asp Leu Leu Asp Thr Ala Ser
50 55 60

Ala Leu Tyr Arg Glu Ala Leu Glu Ser Pro Glu His Cys Ser Pro His
65 70 75 80

His Thr Ala Leu Arg Gln Ala Ile Leu Cys Trp Gly Asp Leu Met Thr
85 90 95

Leu Ala Thr Trp Val Gly Val Asn Leu Glu Asp Pro Ala Thr Arg Asp
100 105 110

Leu Val Val Ser Tyr Val Asn Thr Asn Val Gly Leu Lys Phe Arg Gln
115 120 125

Leu Leu Trp Phe His Ile Ser Cys Leu Thr Phe Gly Arg Glu Thr Val
130 135 140

Ile Glu Tyr Leu Val Ser Phe Gly Val Trp Ile Arg Thr Pro Pro Ala
145 150 155 160

Tyr Arg Pro Pro Asn Ala Pro Ile Leu Ser Thr Leu Pro Glu Thr Thr
165 170 175

Val Val Arg Arg Arg Gly Arg Ser Pro Arg Arg Arg Thr Pro Ser Pro
180 185 190

Arg Arg Arg Arg Ser Gln Ser Pro Arg Arg Arg Arg Ser Gln Ser Arg
195 200 205

Glu Ser Gln Cys
210

<210> 59
<211> 212
<212> PRT
<213> Hepatitis B virus

<400> 59

Met Gln Leu Phe His Leu Cys Leu Ile Ile Ser Cys Ser Cys Pro Thr
1 5 10 15

Val Gln Ala Ser Lys Leu Cys Leu Gly Trp Leu Trp Gly Met Asp Ile
20 25 30

Asp Pro Tyr Lys Glu Phe Gly Ala Thr Val Glu Leu Leu Ser Phe Leu
35 40 45

Pro Ser Asp Phe Phe Pro Ser Val Arg Asp Leu Leu Asp Thr Ala Ser
50 55 60

Ala Leu Tyr Arg Glu Ala Leu Glu Ser Pro Glu His Cys Ser Pro His
65 70 75 80

His Thr Ala Leu Arg Gln Arg Ile Leu Cys Trp Gly Glu Leu Met Thr
85 90 95

Leu Ala Thr Trp Val Gly Val Asn Leu Glu Asp Pro Ala Ser Arg Asp
100 105 110

Leu Val Val Ser Tyr Val Asn Thr Asn Met Gly Leu Lys Phe Arg Gln
115 120 125

Leu Leu Trp Phe His Ile Ser Cys Leu Thr Phe Gly Arg Glu Thr Val
130 135 140

Ile Glu Tyr Leu Val Ser Phe Gly Val Trp Ile Arg Thr Pro Pro Ala
145 150 155 160

Tyr Arg Pro Pro Asn Ala Pro Ile Leu Ser Thr Leu Pro Glu Thr Thr
165 170 175

Val Val Arg Arg Arg Gly Arg Ser Pro Arg Arg Arg Thr Pro Ser Pro
180 185 190

Arg Arg Arg Arg Ser Gln Ser Pro Arg Arg Thr Arg Ser Gln Ser Arg
195 200 205

Glu Ser Gln Cys
210

<210> 60
<211> 212
<212> PRT
<213> Hepatitis B virus

<400> 60

Met Gln Leu Phe His Leu Cys Leu Val Ile Ser Cys Ser Cys Pro Thr
1 5 10 15

Val Gln Ala Ser Lys Leu Cys Leu Gly Trp Leu Trp Gly Met Asp Ile
20 25 30

Asp Pro Tyr Lys Glu Phe Gly Ala Thr Val Glu Leu Leu Ser Phe Leu
35 40 45

Pro Ser Asp Phe Phe Pro Ser Val Arg Asp Leu Leu Asp Thr Ala Ala
50 55 60

Ala Leu Tyr Arg Glu Ala Leu Glu Ser Pro Glu His Cys Ser Pro His
65 70 75 80

His Thr Ala Leu Arg Gln Ala Ile Leu Cys Trp Gly Glu Leu Met Thr
85 90 95

Leu Ala Thr Trp Val Gly Asn Asn Leu Glu Asp Pro Ala Ser Arg Asp
100 105 110

Leu Val Val Asn Tyr Val Asn Thr Asn Met Gly Leu Lys Ile Arg Gln
115 120 125

Leu Leu Trp Phe His Ile Ser Cys Leu Thr Phe Gly Arg Glu Thr Val
130 135 140

Leu Glu Tyr Leu Val Ser Phe Gly Val Trp Ile Arg Thr Pro Pro Ala
145 150 155 160

Tyr Arg Pro Pro Asn Ala Pro Ile Leu Ser Thr Leu Pro Glu Thr Thr
165 170 175

Val Val Arg Arg Arg Gly Arg Ser Pro Arg Arg Arg Thr Pro Ser Pro
180 185 190

Arg Arg Arg Arg Ser Gln Ser Pro Arg Arg Arg Arg Ser Gln Ser Arg
195 200 205

Glu Ser Gln Cys
210

<210> 61
<211> 212
<212> PRT
<213> Hepatitis B virus

<400> 61

Met Gln Leu Phe His Leu Cys Leu Ile Ile Ser Cys Ser Cys Pro Thr
1 5 10 15

Val Gln Ala Ser Lys Leu Cys Leu Gly Trp Leu Trp Gly Met Asp Ile
20 25 30

Asp Pro Tyr Lys Glu Phe Gly Ala Thr Val Glu Leu Leu Ser Phe Leu
35 40 45

Pro Ser Ala Phe Phe Pro Ser Val Arg Asp Leu Leu Asp Thr Ala Ser
50 55 60

Ala Leu Tyr Arg Glu Ala Leu Glu Ser Pro Glu His Cys Ser Pro His
65 70 75 80

His Thr Ala Leu Arg Gln Ala Ile Leu Cys Trp Gly Asp Leu Met Thr
85 90 95

Leu Ala Thr Trp Val Gly Val Asn Leu Glu Asp Pro Ala Ser Arg Asp
100 105 110

Leu Val Val Ser Tyr Val Asn Thr Asn Met Gly Leu Lys Phe Arg Gln
115 120 125

Leu Leu Trp Phe His Ile Ser Cys Leu Thr Phe Gly Arg Glu Thr Val
130 135 140

Ile Glu Tyr Leu Val Ser Phe Gly Val Trp Ile Arg Thr Pro Pro Ala
145 150 155 160

Tyr Arg Pro Pro Asn Ala Pro Ile Leu Ser Thr Leu Pro Glu Thr Thr
165 170 175

Val Val Arg Arg Arg Gly Arg Ser Pro Arg Arg Arg Thr Pro Ser Pro
180 185 190

Arg Arg Arg Arg Ser Gln Ser Pro Arg Arg Arg Arg Ser Gln Ser Arg
195 200 205

Glu Ser Gln Cys
210

<210> 62
<211> 183
<212> PRT
<213> Hepatitis B virus

<400> 62

Met Asp Ile Asp Pro Tyr Lys Glu Phe Gly Ala Thr Val Glu Leu Leu
1               5                   10                  15

Ser Phe Leu Pro Ser Asp Phe Phe Pro Ser Val Arg Asp Leu Leu Asp
            20                  25                  30

Thr Ala Ala Ala Leu Tyr Arg Glu Ala Leu Glu Ser Pro Glu His Cys
        35                  40                  45

Ser Pro His His Thr Ala Leu Arg Gln Ala Ile Leu Cys Trp Gly Glu
    50                  55                  60

Leu Met Thr Leu Ala Thr Trp Val Gly Asn Asn Leu Glu Asp Pro Ala
65                  70                  75                  80

Ser Arg Asp Leu Val Val Asn Tyr Val Asn Thr Asn Met Gly Leu Lys
                85                  90                  95

Ile Arg Gln Leu Leu Trp Phe His Ile Ser Cys Leu Thr Phe Gly Arg
            100                 105                 110

Glu Thr Val Leu Glu Tyr Leu Val Ser Phe Gly Val Trp Ile Arg Thr
        115                 120                 125

Pro Pro Ala Tyr Arg Pro Pro Asn Ala Pro Ile Leu Ser Thr Leu Pro
    130                 135                 140

Glu Thr Thr Val Val Arg Arg Arg Gly Arg Ser Pro Arg Arg Arg Thr
145                 150                 155                 160

Pro Ser Pro Arg Arg Arg Arg Ser Gln Ser Pro Arg Arg Arg Arg Ser
                165                 170                 175

Gln Ser Arg Glu Ser Gln Cys
            180

<210> 63
<211> 183
<212> PRT
<213> Hepatitis B virus

<400> 63

Met Asp Ile Asp Pro Tyr Lys Glu Phe Gly Ala Thr Val Glu Leu Leu
1 5 10 15

Ser Phe Leu Pro Ser Asp Phe Phe Pro Ser Val Arg Asp Leu Leu Asp
20 25 30

Thr Ala Ser Ala Leu Tyr Arg Glu Ala Leu Glu Ser Pro Glu His Cys
35 40 45

Ser Pro His His Thr Ala Leu Arg Gln Ala Ile Leu Cys Trp Gly Glu
50 55 60

Leu Met Thr Leu Ala Thr Trp Val Gly Gly Asn Leu Glu Asp Pro Ile
65 70 75 80

Ser Arg Asp Leu Val Val Ser Tyr Val Asn Thr Asn Met Gly Leu Lys
85 90 95

Phe Arg Gln Leu Leu Trp Phe His Ile Ser Cys Leu Thr Phe Gly Arg
100 105 110

Glu Thr Val Ile Glu Tyr Leu Val Ser Phe Gly Val Trp Ile Arg Thr
115 120 125

Pro Pro Ala Tyr Arg Pro Pro Asn Ala Pro Ile Leu Ser Thr Leu Pro
130 135 140

Glu Thr Cys Val Val Arg Arg Arg Gly Arg Ser Pro Arg Arg Arg Thr
145 150 155 160

Pro Ser Pro Arg Arg Arg Arg Ser Gln Ser Pro Arg Arg Arg Arg Ser
165 170 175

Gln Ser Arg Gly Ser Gln Cys
180

<210> 64
<211> 188
<212> PRT
<213> Hepatitis B virus

<400> 64

Met Asp Ile Asp Pro Tyr Lys Glu Phe Gly Ser Ser Tyr Gln Leu Leu
1 5 10 15

Asn Phe Leu Pro Leu Asp Phe Phe Pro Asp Leu Asn Ala Leu Val Asp
20 25 30

Thr Ala Thr Ala Leu Tyr Glu Glu Glu Leu Thr Gly Arg Glu His Cys
35 40 45

Ser Pro His His Thr Ala Ile Arg Gln Ala Leu Val Cys Trp Asp Glu
50 55 60

Leu Thr Lys Leu Ile Ala Trp Met Ser Ser Asn Ile Thr Ser Glu Gln
65 70 75 80

Val Arg Thr Ile Ile Val Asn His Val Asn Asp Thr Trp Gly Leu Lys
85 90 95

Val Arg Gln Ser Leu Trp Phe His Leu Ser Cys Leu Thr Phe Gly Gln
100 105 110

His Thr Val Gln Glu Phe Leu Val Ser Phe Gly Val Trp Ile Arg Thr
115 120 125

Pro Ala Pro Tyr Arg Pro Pro Asn Ala Pro Ile Leu Ser Thr Leu Pro
130 135 140

Glu His Thr Val Ile Arg Arg Arg Gly Gly Ala Arg Ala Ser Arg Ser
145 150 155 160

Pro Arg Arg Arg Thr Pro Ser Pro Arg Arg Arg Arg Ser Gln Ser Pro
165 170 175

Arg Arg Arg Arg Ser Gln Ser Pro Ser Thr Asn Cys
180 185

<210> 65
<211> 217
<212> PRT
<213> Hepatitis B virus

<400> 65

Met Tyr Leu Phe His Leu Cys Leu Val Phe Ala Cys Val Pro Cys Pro
1 5 10 15

Thr Val Gln Ala Ser Lys Leu Cys Leu Gly Trp Leu Trp Asp Met Asp
20 25 30

Ile Asp Pro Tyr Lys Glu Phe Gly Ser Ser Tyr Gln Leu Leu Asn Phe
35 40 45

Leu Pro Leu Asp Phe Phe Pro Asp Leu Asn Ala Leu Val Asp Thr Ala
50 55 60

Ala Ala Leu Tyr Glu Glu Glu Leu Thr Gly Arg Glu His Cys Ser Pro
65 70 75 80

His His Thr Ala Ile Arg Gln Ala Leu Val Cys Trp Glu Glu Leu Thr
85 90 95

Arg Leu Ile Thr Trp Met Ser Glu Asn Thr Thr Glu Glu Val Arg Arg
100 105 110

Ile Ile Val Asp His Val Asn Asn Thr Trp Gly Leu Lys Val Arg Gln
115 120 125

Thr Leu Trp Phe His Leu Ser Cys Leu Thr Phe Gly Gln His Thr Val
130 135 140

Gln Glu Phe Leu Val Ser Phe Gly Val Trp Ile Arg Thr Pro Ala Pro
145 150 155 160

Tyr Arg Pro Pro Asn Ala Pro Ile Leu Ser Thr Leu Pro Glu His Thr
165 170 175

Val Ile Arg Arg Arg Gly Gly Ser Arg Ala Ala Arg Ser Pro Arg Arg
180 185 190

Arg Thr Pro Ser Pro Arg Arg Arg Arg Ser Gln Ser Pro Arg Arg Arg
195 200 205

Arg Ser Gln Ser Pro Ala Ser Asn Cys
210 215

<210> 66
<211> 262
<212> PRT
<213> Hepatitis B virus

<400> 66

Met Asp Val Asn Ala Ser Arg Ala Leu Ala Asn Val Tyr Asp Leu Pro
1               5                   10                  15

Asp Asp Phe Phe Pro Lys Ile Glu Asp Leu Val Arg Asp Ala Lys Asp

20 25 30

Ala Leu Glu Pro Tyr Trp Lys Ser Asp Ser Ile Lys Lys His Val Leu
35 40 45

Ile Ala Thr His Phe Val Asp Leu Ile Glu Asp Phe Trp Gln Thr Thr
50 55 60

Gln Gly Met His Glu Ile Ala Glu Ala Ile Arg Ala Val Ile Pro Pro
65 70 75 80

Thr Thr Ala Pro Val Pro Ser Gly Tyr Leu Ile Gln His Asp Glu Ala
85 90 95

Glu Glu Ile Pro Leu Gly Asp Leu Phe Lys Glu Gln Glu Glu Arg Ile
100 105 110

Val Ser Phe Gln Pro Asp Tyr Pro Ile Thr Ala Arg Ile His Ala His
115 120 125

Leu Lys Ala Tyr Ala Lys Ile Asn Glu Glu Ser Leu Asp Arg Ala Arg
130 135 140

Arg Leu Leu Trp Trp His Tyr Asn Cys Leu Leu Trp Gly Glu Ala Thr
145 150 155 160

Val Thr Asn Tyr Ile Ser Arg Leu Arg Thr Trp Leu Ser Thr Pro Glu
165 170 175

Lys Tyr Arg Gly Arg Asp Ala Pro Thr Ile Glu Ala Ile Thr Arg Pro
180 185 190

Ile Gln Val Ala Gln Gly Gly Arg Lys Thr Ser Thr Ala Thr Arg Lys
195 200 205

Pro Arg Gly Leu Glu Pro Arg Arg Arg Lys Val Lys Thr Thr Val Val
210 215 220

Tyr Gly Arg Arg Arg Ser Lys Ser Arg Glu Arg Arg Ala Ser Ser Pro
225 230 235 240

Gln Arg Ala Gly Ser Pro Leu Pro Arg Ser Ser Ser Ser His His Arg
245 250 255

Ser Pro Ser Pro Arg Lys
260

<210> 67
<211> 305
<212> PRT
<213> Hepatitis B virus

<400> 67

Met Trp Asp Leu Arg Leu His Pro Ser Pro Phe Gly Ala Ala Cys Gln
1               5                   10                  15

Gly Ile Phe Thr Ser Ser Leu Leu Leu Phe Leu Val Thr Val Pro Leu
            20                  25                  30

Val Cys Thr Ile Val Tyr Asp Ser Cys Leu Cys Met Asp Ile Asn Ala
        35                  40                  45

Ser Arg Ala Leu Ala Asn Val Tyr Asp Leu Pro Asp Asp Phe Phe Pro
    50                  55                  60

Lys Ile Asp Asp Leu Val Arg Asp Ala Lys Asp Ala Leu Glu Pro Tyr
65                  70                  75                  80

Trp Arg Asn Asp Ser Ile Lys Lys His Val Leu Ile Ala Thr His Phe
                85                  90                  95

Val Asp Leu Ile Glu Asp Phe Trp Gln Thr Thr Gln Gly Met His Glu
            100                 105                 110

Ile Ala Glu Ala Leu Arg Ala Ile Ile Pro Ala Thr Thr Ala Pro Val
        115                 120                 125

Pro Gln Gly Phe Leu Val Gln His Glu Glu Ala Glu Glu Ile Pro Leu
    130                 135                 140

Gly Glu Leu Phe Arg Tyr Gln Glu Glu Arg Leu Thr Asn Phe Gln Pro
145                 150                 155                 160

Asp Tyr Pro Val Thr Ala Arg Ile His Ala His Leu Lys Ala Tyr Ala
                165                 170                 175

Lys Ile Asn Glu Glu Ser Leu Asp Arg Ala Arg Arg Leu Leu Trp Trp
            180                 185                 190

His Tyr Asn Cys Leu Leu Trp Gly Glu Pro Asn Val Thr Asn Tyr Ile
        195                 200                 205

Ser Arg Leu Arg Thr Trp Leu Ser Thr Pro Glu Lys Tyr Arg Gly Lys
    210                 215                 220

Asp Ala Pro Thr Ile Glu Ala Ile Thr Arg Pro Ile Gln Val Ala Gln
225                 230                 235                 240

Gly Gly Arg Asn Lys Thr Gln Gly Val Arg Lys Ser Arg Gly Leu Glu
                245                 250                 255

Pro Arg Arg Arg Arg Val Lys Thr Thr Ile Val Tyr Gly Arg Arg Arg
            260                 265                 270

Ser Lys Ser Arg Glu Arg Arg Ala Pro Thr Pro Gln Arg Ala Gly Ser
275 280 285

Pro Leu Pro Arg Thr Ser Arg Asp His His Arg Ser Pro Ser Pro Arg
290 295 300

Glu
305

<210> 68
<211> 185
<212> PRT
<213> Hepatitis B virus

<400> 68

Met Asp Ile Asp Pro Tyr Lys Glu Phe Gly Ala Thr Val Glu Leu Leu
1 5 10 15

Ser Phe Leu Pro Ser Asp Phe Phe Pro Ser Val Arg Asp Leu Leu Asp
20 25 30

Thr Ala Ser Ala Leu Tyr Arg Glu Ala Leu Glu Ser Pro Glu His Cys
35 40 45

Ser Pro His His Thr Ala Leu Arg Gln Ala Ile Leu Cys Trp Gly Glu
50 55 60

Leu Met Thr Leu Ala Thr Trp Val Gly Asn Asn Leu Glu Asp Pro Ala
65 70 75 80

Ser Arg Asp Leu Val Val Asn Tyr Val Asn Thr Asn Met Gly Leu Lys
85 90 95

Ile Arg Gln Leu Leu Trp Phe His Ile Ser Cys Leu Thr Phe Gly Arg
100 105 110

Glu Thr Val Leu Glu Tyr Leu Val Ser Phe Gly Val Trp Ile Arg Thr
115 120 125

Pro Pro Ala Tyr Arg Pro Pro Asn Ala Pro Ile Leu Ser Thr Leu Pro
130 135 140

Glu Thr Thr Val Val Arg Arg Arg Asp Arg Gly Arg Ser Pro Arg Arg
145 150 155 160

Arg Thr Pro Ser Pro Arg Arg Arg Arg Ser Gln Ser Pro Arg Arg Arg
165 170 175

Arg Ser Gln Ser Arg Glu Ser Gln Cys
180 185

<210> 69
<211> 21
<212> DNA
<213> Artificial sequence

<220>
<223> CyCpG

<400> 69
tccatgacgt tcctgaataa t 21

<210> 70
<211> 594
<212> DNA
<213> Hepatitis B virus

<220>
<221> CDS
<222> (1)..(594)

<400> 70

```
atg gac att gac cct tat aaa gaa ttt gga gct act gtg gag tta ctc      48
Met Asp Ile Asp Pro Tyr Lys Glu Phe Gly Ala Thr Val Glu Leu Leu
1               5                   10                  15

tcg ttt ttg cct tct gac ttc ttt cct tcc gtc aga gat ctc cta gac      96
Ser Phe Leu Pro Ser Asp Phe Phe Pro Ser Val Arg Asp Leu Leu Asp
            20                  25                  30

acc gcc tca gct ctg tat cga gaa gcc tta gag tct cct gag cat tgc     144
Thr Ala Ser Ala Leu Tyr Arg Glu Ala Leu Glu Ser Pro Glu His Cys
        35                  40                  45

tca cct cac cat act gca ctc agg caa gcc att ctc tgc tgg ggg gaa     192
Ser Pro His His Thr Ala Leu Arg Gln Ala Ile Leu Cys Trp Gly Glu
    50                  55                  60

ttg atg act cta gct acc tgg gtg ggt aat aat ttg gaa gat cca gca     240
Leu Met Thr Leu Ala Thr Trp Val Gly Asn Asn Leu Glu Asp Pro Ala
65                  70                  75                  80

tcc agg gat cta gta gtc aat tat gtt aat act aac atg ggt tta aag     288
Ser Arg Asp Leu Val Val Asn Tyr Val Asn Thr Asn Met Gly Leu Lys
                85                  90                  95

atc agg caa cta ttg tgg ttt cat ata tct tgc ctt act ttt gga aga     336
Ile Arg Gln Leu Leu Trp Phe His Ile Ser Cys Leu Thr Phe Gly Arg
            100                 105                 110

gag act gta ctt gaa tat ttg gtc tct ttc gga gtg tgg att cgc act     384
Glu Thr Val Leu Glu Tyr Leu Val Ser Phe Gly Val Trp Ile Arg Thr
        115                 120                 125

cct cca gcc tat aga cca cca aat gcc cct atc tta tca aca ctt ccg     432
Pro Pro Ala Tyr Arg Pro Pro Asn Ala Pro Ile Leu Ser Thr Leu Pro
    130                 135                 140

gaa act act gtt gtt aga cga cgg gac cga ggc agg tcc cct aga aga     480
Glu Thr Thr Val Val Arg Arg Arg Asp Arg Gly Arg Ser Pro Arg Arg
145                 150                 155                 160

aga act ccc tcg cct cgc aga cgc aga tct caa tcg ccg cgt cgc aga     528
Arg Thr Pro Ser Pro Arg Arg Arg Arg Ser Gln Ser Pro Arg Arg Arg
                165                 170                 175

aga tct caa tct cgg gaa tct caa tgt ctt ctc ctt aaa gct gtt tac     576
Arg Ser Gln Ser Arg Glu Ser Gln Cys Leu Leu Leu Lys Ala Val Tyr
            180                 185                 190

aac ttc gct acc atg taa                                             594
Asn Phe Ala Thr Met
        195
```

<210> 71
<211> 197
<212> PRT
<213> Hepatitis B virus

<400> 71

```
Met Asp Ile Asp Pro Tyr Lys Glu Phe Gly Ala Thr Val Glu Leu Leu
1               5                   10                  15

Ser Phe Leu Pro Ser Asp Phe Phe Pro Ser Val Arg Asp Leu Leu Asp
            20                  25                  30

Thr Ala Ser Ala Leu Tyr Arg Glu Ala Leu Glu Ser Pro Glu His Cys
        35                  40                  45

Ser Pro His His Thr Ala Leu Arg Gln Ala Ile Leu Cys Trp Gly Glu
    50                  55                  60

Leu Met Thr Leu Ala Thr Trp Val Gly Asn Asn Leu Glu Asp Pro Ala
65                  70                  75                  80

Ser Arg Asp Leu Val Val Asn Tyr Val Asn Thr Asn Met Gly Leu Lys
                85                  90                  95

Ile Arg Gln Leu Leu Trp Phe His Ile Ser Cys Leu Thr Phe Gly Arg
            100                 105                 110

Glu Thr Val Leu Glu Tyr Leu Val Ser Phe Gly Val Trp Ile Arg Thr
        115                 120                 125

Pro Pro Ala Tyr Arg Pro Pro Asn Ala Pro Ile Leu Ser Thr Leu Pro
    130                 135                 140

Glu Thr Thr Val Val Arg Arg Arg Asp Arg Gly Arg Ser Pro Arg Arg
145                 150                 155                 160

Arg Thr Pro Ser Pro Arg Arg Arg Arg Ser Gln Ser Pro Arg Arg Arg
                165                 170                 175

Arg Ser Gln Ser Arg Glu Ser Gln Cys Leu Leu Leu Lys Ala Val Tyr
            180                 185                 190

Asn Phe Ala Thr Met
        195
```

<210> 72
<211> 9
<212> PRT
<213> Homo sapiens

<400> 72

```
Lys Thr Trp Gly Gln Tyr Trp Gln Val
1               5
```

<210> 73
<211> 9
<212> PRT

<213> Homo sapiens

<400> 73

Ile Thr Asp Gln Val Pro Phe Ser Val
1 5

<210> 74
<211> 9
<212> PRT
<213> Homo sapiens

<400> 74

Tyr Leu Glu Pro Gly Pro Val Thr Ala
1 5

<210> 75
<211> 10
<212> PRT
<213> Homo sapiens

<400> 75

Leu Leu Asp Gly Thr Ala Thr Leu Arg Leu
1 5 10

<210> 76
<211> 10
<212> PRT
<213> Homo sapiens

<400> 76

Val Leu Tyr Arg Tyr Gly Ser Phe Ser Val
1 5 10

<210> 77
<211> 9
<212> PRT
<213> Homo sapiens

<400> 77

Ala Ala Gly Ile Gly Ile Leu Thr Val
1 5

<210> 78
<211> 9

<212> PRT
<213> Homo sapiens

<400> 78

Ile Leu Thr Val Ile Leu Gly Val Leu
1 5

<210> 79
<211> 9
<212> PRT
<213> Homo sapiens

<400> 79

Met Leu Leu Ala Val Leu Tyr Cys Leu
1 5

<210> 80
<211> 9
<212> PRT
<213> Homo sapiens

<400> 80

Tyr Met Asp Gly Thr Met Ser Gln Val
1 5

<210> 81
<211> 9
<212> PRT
<213> Homo sapiens

<400> 81

Val Leu Pro Asp Val Phe Ile Arg Cys
1 5

<210> 82
<211> 9
<212> PRT
<213> Homo sapiens

<400> 82

Phe Leu Trp Gly Pro Arg Ala Leu Val
1 5

<210> 83
<211> 9
<212> PRT
<213> Homo sapiens

<400> 83

Tyr Leu Ser Gly Ala Asn Leu Asn Leu
1 5

<210> 84
<211> 9
<212> PRT
<213> Homo sapiens

<400> 84

Arg Met Pro Glu Ala Ala Pro Pro Val
1 5

<210> 85
<211> 9
<212> PRT
<213> Homo sapiens

<400> 85

Ser Thr Pro Pro Pro Gly Thr Arg Val
1 5

<21Q> 86
<211> 9
<212> PRT
<213> Homo sapiens

<400> 86

Leu Leu Gly Arg Asn Ser Phe Glu Val
1 5

<210> 87
<211> 9
<212> PRT
<213> Homo sapiens

<400> 87

Lys Ile Phe Gly Ser Leu Ala Phe Leu
1 5

<210> 88
<211> 9
<212> PRT
<213> Homo sapiens

<400> 88

Ile Ile Ser Ala Val Val Gly Ile Leu
1 5

<210> 89
<211> 8
<212> PRT
<213> Homo sapiens

<400> 89

Thr Leu Gly Ile Val Cys Pro Ile
1 5

<210> 90
<211> 131
<212> PRT
<213> Bacteriophage AP205

<400> 90

Met Ala Asn Lys Pro Met Gln Pro Ile Thr Ser Thr Ala Asn Lys Ile
1 5 10 15

Val Trp Ser Asp Pro Thr Arg Leu Ser Thr Thr Phe Ser Ala Ser Leu
20 25 30

Leu Arg Gln Arg Val Lys Val Gly Ile Ala Glu Leu Asn Asn Val Ser
35 40 45

Gly Gln Tyr Val Ser Val Tyr Lys Arg Pro Ala Pro Lys Pro Glu Gly
50 55 60

Cys Ala Asp Ala Cys Val Ile Met Pro Asn Glu Asn Gln Ser Ile Arg
65 70 75 80

Thr Val Ile Ser Gly Ser Ala Glu Asn Leu Ala Thr Leu Lys Ala Glu
85 90 95

Trp Glu Thr His Lys Arg Asn Val Asp Thr Leu Phe Ala Ser Gly Asn
100 105 110

Ala Gly Leu Gly Phe Leu Asp Pro Thr Ala Ala Ile Val Ser Ser Asp
115 120 125

Thr Thr Ala
130

<210> 91
<211> 3635
<212> DNA
<213> Artificial Sequence

<220>
<223> Plasmid, pAP283-58, encoding RNA phage AP205 coat protein

<400> 91

```
cgagctcgcc cctggcttat cgaaattaat acgactcact atagggagac cggaattcga      60
gctcgcccgg ggatcctcta gaattttctg cgcacccatc ccgggtggcg cccaaagtga     120
ggaaaatcac atggcaaata agccaatgca accgatcaca tctacagcaa ataaaattgt     180
gtggtcggat ccaactcgtt tatcaactac attttcagca agtctgttac gccaacgtgt     240
taaagttggt atagccgaac tgaataatgt ttcaggtcaa tatgtatctg tttataagcg     300
tcctgcacct aaaccggaag gttgtgcaga tgcctgtgtc attatgccga atgaaaacca     360
atccattcgc acagtgattt cagggtcagc cgaaaacttg gctaccttaa aagcagaatg     420
ggaaactcac aaacgtaacg ttgacacact cttcgcgagc ggcaacgccg gtttgggttt     480
ccttgaccct actgcggcta tcgtatcgtc tgatactact gcttaagctt gtattctata     540
gtgtcaccta aatcgtatgt gtatgataca taaggttatg tattaattgt agccgcgttc     600
taacgacaat atgtacaagc ctaattgtgt agcatctggc ttactgaagc agaccctatc     660
atctctctcg taaactgccg tcagagtcgg tttggttgga cgaaccttct gagtttctgg     720
taacgccgtt ccgcaccccg gaaatggtca ccgaaccaat cagcagggtc atcgctagcc     780
agatcctcta cgccggacgc atcgtggccg gcatcaccgg cgccacaggt gcggttgctg     840
gcgcctatat cgccgacatc accgatgggg aagatcgggc tcgccacttc gggctcatga     900
gcgcttgttt cggcgtgggt atggtggcag gccccgtggc cgggggactg ttgggcgcca     960
```

tctccttgca tgcaccattc cttgcggcgg cggtgctcaa cggcctcaac ctactactgg 1020
gctgcttcct aatgcaggag tcgcataagg gagagcgtcg atatggtgca ctctcagtac 1080
aatctgctct gatgccgcat agttaagcca actccgctat cgctacgtga ctgggtcatg 1140
gctgcgcccc gacacccgcc aacacccgct gacgcgccct gacgggcttg tctgctcccg 1200
gcatccgctt acagacaagc tgtgaccgtc tccgggagct gcatgtgtca gaggttttca 1260
ccgtcatcac cgaaacgcgc gaggcagctt gaagacgaaa gggcctcgtg atacgcctat 1320
ttttataggt taatgtcatg ataataatgg tttcttagac gtcaggtggc acttttcggg 1380
gaaatgtgcg cggaacccct atttgtttat ttttctaaat acattcaaat atgtatccgc 1440
tcatgagaca ataaccctga taaatgcttc aataatattg aaaaaggaag agtatgagta 1500
ttcaacattt ccgtgtcgcc cttattccct tttttgcggc attttgcctt cctgtttttg 1560
ctcacccaga aacgctggtg aaagtaaaag atgctgaaga tcagttgggt gcacgagtgg 1620
gttacatcga actggatctc aacagcggta agatccttga gagttttcgc cccgaagaac 1680
gttttccaat gatgagcact tttaaagttc tgctatgtgg cgcggtatta tcccgtattg 1740
acgccgggca agagcaactc ggtcgccgca tacactattc tcagaatgac ttggttgagt 1800
actcaccagt cacagaaaag catcttacgg atggcatgac agtaagagaa ttatgcagtg 1860
ctgccataac catgagtgat aacactgcgg ccaacttact tctgacaacg atcggaggac 1920
cgaaggagct aaccgctttt ttgcacaaca tgggggatca tgtaactcgc cttgatcgtt 1980
gggaaccgga gctgaatgaa gccataccaa acgacgagcg tgacaccacg atgcctgtag 2040
caatggcaac aacgttgcgc aaactattaa ctggcgaact acttactcta gcttcccggc 2100
aacaattaat agactggatg gaggcggata aagttgcagg accacttctg cgctcggccc 2160
ttccggctgg ctggtttatt gctgataaat ctggagccgg tgagcgtggg tctcgcggta 2220
tcattgcagc actggggcca gatggtaagc cctcccgtat cgtagttatc tacacgacgg 2280
ggagtcaggc aactatggat gaacgaaata gacagatcgc tgagataggt gcctcactga 2340
ttaagcattg gtaactgtca gaccaagttt actcatatat actttagatt gatttaaaac 2400
ttcattttta atttaaaagg atctaggtga agatcctttt tgataatctc atgaccaaaa 2460
tcccttaacg tgagttttcg ttccactgag cgtcagaccc cgtagaaaag atcaaaggat 2520
cttcttgaga tccttttttt ctgcgcgtaa tctgctgctt gcaaacaaaa aaaccaccgc 2580
taccagcggt ggtttgtttg ccggatcaag agctaccaac tctttttccg aaggtaactg 2640
gcttcagcag agcgcagata ccaaatactg tccttctagt gtagccgtag ttaggccacc 2700
acttcaagaa ctctgtagca ccgcctacat acctcgctct gctaatcctg ttaccagtgg 2760
ctgctgccag tggcgataag tcgtgtctta ccgggttgga ctcaagacga tagttaccgg 2820
ataaggcgca gcggtcgggc tgaacggggg gttcgtgcac acagcccagc ttggagcgaa 2880
cgacctacac cgaactgaga tacctacagc gcgagcattg agaaagcgcc acgcttcccg 2940
aagggagaaa ggcggacagg tatccggtaa gcggcagggt cggaacagga gagcgcacga 3000

gggagcttcc agggggaaac gcctggtatc tttatagtcc tgtcgggttt cgccacctct 3060
gacttgagcg tcgatttttg tgatgctcgt caggggggcg gagcctatgg aaaaacgcca 3120
gcaacgcggc ctttttacgg ttcctggcct tttgctggcc ttttgctcac atgttctttc 3180
ctgcgttatc ccctgattct gtggataacc gtattaccgc ctttgagtga gctgataccg 3240
ctcgccgcag ccgaacgacc gagcgcagcg agtcagtgag cgaggaagcg gaagagcgcc 3300
caatacgcaa accgcctctc cccgcgcgtt ggccgattca ttaatgcagc tgtggtgtca 3360
tggtcggtga tcgccagggt gccgacgcgc atctcgactg catggtgcac caatgcttct 3420
ggcgtcaggc agccatcgga agctgtggta tggccgtgca ggtcgtaaat cactgcataa 3480
ttcgtgtcgc tcaaggcgca ctcccgttct ggataatgtt ttttgcgccg acatcataac 3540
ggttctggca aatattctga aatgagctgt tgacaattaa tcatcgaact agttaactag 3600
tacgcaagtt cacgtaaaaa gggtatcgcg gaatt 3635

<210> 92
<211> 35
<212> DNA
<213> Artificial sequence

<220>
<223> vector pQb185

<400> 92
tctagattaa cccaacgcgt aggagtcagg ccatg 35

<210> 93
<211> 131
<212> PRT
<213> Artificial sequence

<220>
<223> Bacteriophage AP205 mutant

<400> 93

Met Ala Asn Lys Thr Met Gln Pro Ile Thr Ser Thr Ala Asn Lys Ile
1               5                   10                  15

Val Trp Ser Asp Pro Thr Arg Leu Ser Thr Thr Phe Ser Ala Ser Leu
            20                  25                  30

Leu Arg Gln Arg Val Lys Val Gly Ile Ala Glu Leu Asn Asn Val Ser
        35                  40                  45

Gly Gln Tyr Val Ser Val Tyr Lys Arg Pro Ala Pro Lys Pro Glu Gly
    50                  55                  60

Cys Ala Asp Ala Cys Val Ile Met Pro Asn Glu Asn Gln Ser Ile Arg
65                  70                  75                  80

Thr Val Ile Ser Gly Ser Ala Glu Asn Leu Ala Thr Leu Lys Ala Glu
                85                  90                  95

Trp Glu Thr His Lys Arg Asn Val Asp Thr Leu Phe Ala Ser Gly Asn
            100                 105                 110

Ala Gly Leu Gly Phe Leu Asp Pro Thr Ala Ala Ile Val Ser Ser Asp
        115                 120                 125

Thr Thr Ala
    130

<210> 94
<211> 3613
<212> DNA
<213> Artificial sequence

<220>
<223> Plasmid, pAP281-32, encoding RNA phage AP205 coat protein

<400> 94

cgagctcgcc cctggcttat cgaaattaat acgactcact atagggagac cggaattcga 60
gctcgcccgg ggatcctcta gattaaccca acgcgtagga gtcaggccat ggcaaataag 120
acaatgcaac cgatcacatc tacagcaaat aaaattgtgt ggtcggatcc aactcgttta 180
tcaactacat tttcagcaag tctgttacgc caacgtgtta aagttggtat agccgaactg 240
aataatgttt caggtcaata tgtatctgtt tataagcgtc ctgcacctaa accggaaggt 300
tgtgcagatg cctgtgtcat tatgccgaat gaaaaccaat ccattcgcac agtgatttca 360
gggtcagccg aaaacttggc taccttaaaa gcagaatggg aaactcacaa acgtaacgtt 420
gacacactct tcgcgagcgg caacgccggt ttgggtttcc ttgaccctac tgcggctatc 480
gtatcgtctg atactactgc ttaagcttgt attctatagt gtcacctaaa tcgtatgtgt 540
atgatacata aggttatgta ttaattgtag ccgcgttcta acgacaatat gtacaagcct 600
aattgtgtag catctggctt actgaagcag accctatcat ctctctcgta aactgccgtc 660
agagtcggtt tggttggacg aaccttctga gtttctggta acgccgttcc gcaccccgga 720
aatggtcacc gaaccaatca gcagggtcat cgctagccag atcctctacg ccggacgcat 780
cgtggccggc atcaccggcg ccacaggtgc ggttgctggc gcctatatcg ccgacatcac 840
cgatggggaa gatcgggctc gccacttcgg gctcatgagc gcttgtttcg gcgtgggtat 900
ggtggcaggc cccgtggccg ggggactgtt gggcgccatc tccttgcatg caccattcct 960
tgcggcggcg gtgctcaacg gcctcaacct actactgggc tgcttcctaa tgcaggagtc 1020
gcataaggga gagcgtcgat atggtgcact ctcagtacaa tctgctctga tgccgcatag 1080
ttaagccaac tccgctatcg ctacgtgact gggtcatggc tgcgccccga cacccgccaa 1140
cacccgctga cgcgccctga cgggcttgtc tgctcccggc atccgcttac agacaagctg 1200
tgaccgtctc cgggagctgc atgtgtcaga ggttttcacc gtcatcaccg aaacgcgcga 1260
ggcagcttga agacgaaagg gcctcgtgat acgcctattt ttataggtta atgtcatgat 1320
aataatggtt tcttagacgt caggtggcac ttttcgggga aatgtgcgcg gaacccctat 1380
ttgtttattt ttctaaatac attcaaatat gtatccgctc atgagacaat aaccctgata 1440

aatgcttcaa taatattgaa aaaggaagag tatgagtatt caacatttcc gtgtcgccct 1500
tattcccttt tttgcggcat tttgccttcc tgtttttgct cacccagaaa cgctggtgaa 1560
agtaaaagat gctgaagatc agttgggtgc acgagtgggt tacatcgaac tggatctcaa 1620
cagcggtaag atccttgaga gttttcgccc cgaagaacgt tttccaatga tgagcacttt 1680
taaagttctg ctatgtggcg cggtattatc ccgtattgac gccgggcaag agcaactcgg 1740
tcgccgcata cactattctc agaatgactt ggttgagtac tcaccagtca cagaaaagca 1800
tcttacggat ggcatgacag taagagaatt atgcagtgct gccataacca tgagtgataa 1860
cactgcggcc aacttacttc tgacaacgat cggaggaccg aaggagctaa ccgctttttt 1920
gcacaacatg ggggatcatg taactcgcct tgatcgttgg gaaccggagc tgaatgaagc 1980
cataccaaac gacgagcgtg acaccacgat gcctgtagca atggcaacaa cgttgcgcaa 2040
actattaact ggcgaactac ttactctagc ttcccggcaa caattaatag actggatgga 2100
ggcggataaa gttgcaggac cacttctgcg ctcggccctt ccggctggct ggtttattgc 2160
tgataaatct ggagccggtg agcgtgggtc tcgcggtatc attgcagcac tggggccaga 2220
tggtaagccc tcccgtatcg tagttatcta cacgacgggg agtcaggcaa ctatggatga 2280
acgaaataga cagatcgctg agataggtgc ctcactgatt aagcattggt aactgtcaga 2340
ccaagtttac tcatatatac tttagattga tttaaaactt catttttaat ttaaaaggat 2400
ctaggtgaag atcctttttg ataatctcat gaccaaaatc ccttaacgtg agttttcgtt 2460
ccactgagcg tcagaccccg tagaaaagat caaaggatct tcttgagatc ctttttttct 2520
gcgcgtaatc tgctgcttgc aaacaaaaaa accaccgcta ccagcggtgg tttgtttgcc 2580
ggatcaagag ctaccaactc tttttccgaa ggtaactggc ttcagcagag cgcagatacc 2640
aaatactgtc cttctagtgt agccgtagtt aggccaccac ttcaagaact ctgtagcacc 2700
gcctacatac ctcgctctgc taatcctgtt accagtggct gctgccagtg gcgataagtc 2760
gtgtcttacc gggttggact caagacgata gttaccggat aaggcgcagc ggtcgggctg 2820
aacggggggt tcgtgcacac agcccagctt ggagcgaacg acctacaccg aactgagata 2880
cctacagcgc gagcattgag aaagcgccac gcttcccgaa gggagaaagg cggacaggta 2940
tccggtaagc ggcagggtcg gaacaggaga gcgcacgagg gagcttccag ggggaaacgc 3000
ctggtatctt tatagtcctg tcgggtttcg ccacctctga cttgagcgtc gatttttgtg 3060
atgctcgtca ggggggcgga gcctatggaa aaacgccagc aacgcggcct ttttacggtt 3120
cctggccttt tgctggcctt ttgctcacat gttctttcct gcgttatccc ctgattctgt 3180
ggataaccgt attaccgcct ttgagtgagc tgataccgct cgccgcagcc gaacgaccga 3240
gcgcagcgag tcagtgagcg aggaagcgga agagcgccca atacgcaaac cgcctctccc 3300
cgcgcgttgg ccgattcatt aatgcagctg tggtgtcatg gtcggtgatc gccagggtgc 3360
cgacgcgcat ctcgactgca tggtgcacca atgcttctgg cgtcaggcag ccatcggaag 3420
ctgtggtatg gccgtgcagg tcgtaaatca ctgcataatt cgtgtcgctc aaggcgcact 3480
cccgttctgg ataatgtttt ttgcgccgac atcataacgg ttctggcaaa tattctgaaa 3540

tgagctgttg acaattaatc atcgaactag ttaactagta cgcaagttca cgtaaaaagg 3600

gtatcgcgga att 3613

<210> 95
<211> 5
<212> PRT
<213> Artificial sequence

<220>
<223> HBcAg peptide

<400> 95

Gly Gly Lys Gly Gly
1 5

<210> 96
<211> 15Z
<212> PRT
<213> Artificial sequence

<220>
<223> HBcAg variant

<400> 96

Met Asp Ile Asp Pro Tyr Lys Glu Phe Gly Ala Thr Val Glu Leu Leu
1 5 10 15

Ser Phe Leu Pro Ser Asp Phe Phe Pro Ser Val Arg Asp Leu Leu Asp
20 25 30

Thr Ala Ala Ala Leu Tyr Arg Asp Ala Leu Glu Ser Pro Glu His Cys
35 40 45

Ser Pro His His Thr Ala Leu Arg Gln Ala Ile Leu Cys Trp Gly Asp
50 55 60

Leu Met Thr Leu Ala Thr Trp Val Gly Thr Asn Leu Glu Asp Gly Gly
65 70 75 80

Lys Gly Gly Ser Arg Asp Leu Val Val Ser Tyr Val Asn Thr Asn Val
85 90 95

Gly Leu Lys Phe Arg Gln Leu Leu Trp Phe His Ile Ser Cys Leu Thr
100 105 110

Phe Gly Arg Gly Thr Val Leu Glu Tyr Leu Val Ser Phe Gly Val Trp
115 120 125

Ile Arg Thr Pro Pro Ala Tyr Arg Pro Pro Asn Ala Pro Ile Leu Ser
130 135 140

Thr Leu Pro Glu Thr Thr Val Val
145 150

<210> 97
<211> 185
<212> PRT
<213> Artificial sequence

<220>
<223> HBcAg variant

<400> 97

Met Asp Ile Asp Pro Tyr Lys Glu Phe Gly Ala Thr Val Glu Leu Leu
1 5 10 15

Ser Phe Leu Pro Ser Asp Phe Phe Pro Ser Val Arg Asp Leu Leu Asp
20 25 30

Thr Ala Ser Ala Leu Tyr Arg Glu Ala Leu Glu Ser Pro Glu His Ser
35 40 45

Ser Pro His His Thr Ala Leu Arg Gln Ala Ile Leu Cys Trp Gly Glu
50 55 60

Leu Met Thr Leu Ala Thr Trp Val Gly Asn Asn Leu Glu Asp Pro Ala
65 70 75 80

Ser Arg Asp Leu Val Val Asn Tyr Val Asn Thr Asn Met Gly Leu Lys
85 90 95

Ile Arg Gln Leu Leu Trp Phe His Ile Ser Ser Leu Thr Phe Gly Arg
100 105 110

Glu Thr Val Leu Glu Tyr Leu Val Ser Phe Gly Val Trp Ile Arg Thr
115 120 125

Pro Pro Ala Tyr Arg Pro Pro Asn Ala Pro Ile Leu Ser Thr Leu Pro
130 135 140

Glu Thr Thr Val Val Arg Arg Arg Asp Arg Gly Arg Ser Pro Arg Arg
145 150 155 160

Arg Thr Pro Ser Pro Arg Arg Arg Arg Ser Gln Ser Pro Arg Arg Arg
165 170 175

Arg Ser Gln Ser Arg Glu Ser Gln Cys
180 185

<210> 98
<211> 10
<212> PRAT
<213> Homo sapiens

<400> 98

Glu Ala Ala Gly Ile Gly Ile Leu Thr Val
1 5 10

<210> 99
<211> 10
<212> PRT
<213> Homo sapiens

<400> 99

Glu Leu Ala Gly Ile Gly Ile Cys Thr Val
1 5 10

<210> 100
<211> 28
<212> DNA
<213> Artificial sequence

<220>
<223> primer p1.44

<220>
<221> misc_feature
<222> (1)..(2)
<223> n can be any nucleotide, preferably a

<400> 100
nnccatggca aataagccaa tgcaaccg 28

<210> 101
<211> 30
<212> DNA
<213> Artificial sequence

<220>
<223> primer p1.45

<220>
<221> misc_feature
<222> (1)..(2)
<223> n can be any nucleotide, preferably a

<400> 101
nntctagaat tttctgcgca cccatcccgg 30

<210> 102
<211> 31
<212> DNA
<213> Artificial sequence

<220>
<223> primer p1.46

<220>
<221> misc_feature
<222> (1)..(2)
<223> n can be any nucleotide, preferably a

<400> 102
nnaagcttaa gcagtagtat cagacgatac g 31

<210> 103
<211> 43
<212> DNA
<213> Artificial sequence

<220>
<223> primer p1.47

<400> 103
gagtgatcca actcgtttat caactacatt ttcagcaagt ctg 43

<210> 104
<211> 43
<212> DNA
<213> Artificial Sequence

<220>
<223> p1.48

<400> 104
cagacttgct gaaaatgtag ttgataaacg agttggatca ctc 43

<210> 105
<211> 10
<212> DNA
<213> Artificial sequence

<220>
<223> oligonucleotide ISS

<400> 105
gacgatcgtc 10

<210> 106
<211> 19
<212> DNA
<213> Artificial sequence

<220>
<223> oligonucleotide G3-6

<400> 106
ggggacgatc gtcgggggg 19

<210> 107
<211> 20
<212> DNA
<213> Artificial sequence

<220>
<223> oligonucleotide G4-6

<400> 107
gggggacgat cgtcgggggg 20

<210> 108
<211> 21
<212> DNA
<213> Artificial sequence

<220>
<223> oligonucleotide G5-6

<400> 108
ggggggacga tcgtcggggg g 21

<210> 109
<211> 22
<212> DNA
<213> Artificial sequence

<220>
<223> oligonucleotide G6-6

<400> 109
gggggggacg atcgtcgggg gg 22

<210> 110
<211> 24
<212> DNA
<213> Artificial sequence

<220>
<223> oligonucleotide G7-7

<400> 110
ggggggggac gatcgtcggg gggg 24

<210> 111
<211> 26
<212> DNA
<213> Artificial sequence

<220>
<223> oligonucleotide G8-8

<400> 111
ggggggggga cgatcgtcgg gggggg 26

<210> 112
<211> 28
<212> DNA
<213> Artificial sequence

<220>
<223> oligonucleotide G9-9

<400> 112
gggggggggg acgatcgtcg gggggggg 28

<210> 113
<211> 30
<212> DNA
<213> Artificial sequence

<220>
<223> oligonucleotide G6

<400> 113
ggggggcgac gacgatcgtc gtcggggggg 30

<210> 114
<211> 23
<212> DNA
<213> Artificial sequence

<220>
<223> CpG-2006, deoxynucleotides connected via phosphorothioate bonds

<400> 114
tcgtcgtttt gtcgttttgt cgt 23

<210> 115
<211> 12
<212> PRT
<213> Artificial sequence

<220>
<223> p33 peptide containing CGG n-terminal

<400> 115

```
Cys Gly Gly Lys Ala Val Tyr Asn Phe Ala Thr Met
1               5                   10
```

<210> 116
<211> 21
<212> DNA
<213> Artificial sequence

<220>
<223> CyCpGpt, deoxynucleotides connected via phosphorothioate bonds

<400> 116
tccatgacgt tcctgaataa t 21

<210> 117
<211> 20
<212> DNA
<213> Artificial sequence

<220>
<223> B-CpGpt, deoxynucleotides connected via phosphorothioate bonds

<400> 117
tccatgacgt tcctgacgtt 20

<210> 118
<211> 20
<212> DNA
<213> Artificial sequence

<220>
<223> B-CpG

<400> 118
tccatgacgt tcctgacgtt 20

<210> 119
<211> 19
<212> DNA
<213> Artificial sequence

<220>
<223> NKCpGpt, deoxynucleotides connected via phosphorothioate bonds

<400> 119
ggggtcaacg ttgaggggg 19

<210> 120
<211> 19
<212> DNA
<213> Artificial Sequence

<220>
<223> NKCpG

<400> 120
ggggtcaacg ttgaggggg 19

<210> 121
<211> 21
<212> DNA
<213> Artificial Sequence

<220>
<223> CyCpG-rev-pt, deoxynucleotides connected via phosphorothioate bonds

<400> 121
attattcagg aacgtcatgg a 21

<210> 122
<211> 30
<212> DNA
<213> Artificial sequence

<220>
<223> g10gacga-PO (G10-PO)

<400> 122
gggggggggg gacgatcgtc gggggggggg 30

<210> 123
<211> 30
<212> DNA
<213> Artificial sequence

<220>
<223> g10gacga-PS (G10-PS), deoxynucleotides connected via phosphorothioate bonds

<400> 123
gggggggggg gacgatcgtc gggggggggg 30

<210> 124
<211> 62
<212> DNA

<213> Artificial sequence

<220>
<223> (CpG)200pA

<400> 124

cgcgcgcgcg cgcgcgcgcg cgcgcgcgcg cgcgcgcgcg aaatgcatgt caaagacagc 60
at 62

<210> 125
<211> 61
<212> DNA
<213> Artificial sequence

<220>
<223> Cy(CpG)20

<400> 125

tccatgacgt tcctgaataa tcgcgcgcgc gcgcgcgcgc gcgcgcgcgc gcgcgcgcgc 60
g 61

<210> 126
<211> 83
<212> DNA
<213> Artificial Sequence

<220>
<223> Cy(CpG)20-OpA

<400> 126

tccatgacgt tcctgaataa tcgcgcgcgc gcgcgcgcgc gcgcgcgcgc gcgcgcgcgc 60
gaaatgcatg tcaaagacag cat 83

<210> 127
<211> 43
<212> DNA
<213> Artificial Sequence

<220>
<223> CyopA

<400> 127
tccatgacgt tcctgaataa taaatgcatg tcaaagacag cat 43

<210> 128
<211> 63
<212> DNA
<213> Artificial Sequence

<220>
<223> CyCyCy

<400> 128

tccatgacgt tcctgaataa ttccatgacg ttcctgaata attccatgac gttcctgaat 60
aat 63

<210> 129
<211> 150
<212> DNA
<213> Artificial Sequence

<220>
<223> Cy150-1

<400> 129

tccatgacgt tcctgaataa ttccatgacg ttcctgaata attccatgac gttcctgaat 60
aattggatga cgttggtgaa taattccatg acgttcctga ataattccat gacgttcctg 120
aataattcca tgacgttcct gaataattcc 150

<210> 130
<211> 253
<212> DNA
<213> Artificial sequence

<220>
<223> dscycpG-253

<400> 130

ctagaactag tggatccccc gggctgcagg aattcgattc atgacttcct gaataattcc 60
atgacgttgg tgaataattc catgacgttc ctgaataatt ccatgacgtt cctgaataat 120
tccatgacgt tcctgaataa ttccatgacg ttcctgaata attccatgac gttcctgaat 180
aattccatga cgttcctgaa taattccatg acgttcctga aaattccaat caagcttatc 240
gataccgtcg acc 253

<210> 131
<211> 12
<212> PRT
<213> Artificial sequence

<220>
<223> p33 peptides containing a c-terminal GGC

<400> 131

```
Lys Ala Val Tyr Asn Phe Ala Thr Met Gly Gly Cys
1               5                   10
```